# EUROPÄISCHE PATENTSCHRIFT

(11) **EP 1 899 330 B1**
(45) Veröffentlichungstag und Bekanntmachung des Hinweises auf die Patenterteilung: **24.11.2010**
(21) Anmeldenummer: 06763910.4
(22) Anmeldetag: 28.06.2006
(51) Int. Cl.: C07D 409/14, C07D 409/12, C07D 487/04, C07D 413/04, C07D 417/14, C07D 471/04, A61K 31/55, A61P 29/00, A61P 35/00, A61P 9/00

(54) **SUBSTITUIERTE GLYCINAMIDE MIT ANTITHROMBOTISCHER UND FAKTOR XA-INHIBIERENDER WIRKUNG**
SUBSTITUTED GLYCINAMIDES HAVING AN ANTITHROMBOTIC AND FACTOR XA-INHIBITING EFFECT
GLYCINAMIDES SUBSTITUES A EFFET ANTITHROMBOTIQUE ET INHIBANT LE FACTEUR XA

(30) Priorität: 30.06.2005 EP 05014270
(43) Veröffentlichungstag der Anmeldung: 19.03.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: WIENEN, Wolfgang, 88400 Biberach (DE); PFAU, Roland, 88400 Biberach (DE); GERLACH, Kai, 88400 Biberach (DE); DAHMANN, Georg, 88448 Attenweiler (DE); PRIEPKE, Henning, 88447 Warthausen (DE); NAR, Herbert, 88416 Ochsenhausen (DE); HANDSCHUH, Sandra, 88400 Biberach (DE); SCHULER-METZ, Annette, 89081 Ulm (DE)
(74) Vertreter: Hammann, Heinz
(86) Internationale Anmeldenummer: PCT/EP2006/063611
(87) Internationale Veröffentlichungsnummer: WO 2007/003536

(56) Entgegenhaltungen:
- WO-A-01/47919
- WO-A-2004/046138

## Beschreibung

WO 2004/046138 offenbart Glycinamid-Derivate mit anti-thrombotischer und Faktor Xa-inhibierender Wirkung. Gegenstand der vorliegenden Erfindung sind neue substituierte Glycinamide der allgemeinen Formel (I) 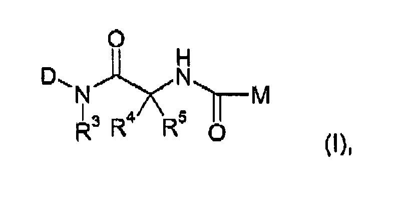 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel (I) sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

Gegenstand der vorliegenden Anmeldung sind neue Verbindungen der obigen allgemeinen Formel (I), deren Herstellung, die die pharmakologisch wirksamen Verbindungen enthaltenden Arzneimittel, deren Herstellung und Verwendung.

Eine 1. Ausführungsform der vorliegenden Erfindung umfasst diejenigen Verbindungen der allgemeinen Formel (I), in denen
- D: ein substituiertes bicyclisches Ringsystem der Formel (II) darstellt, 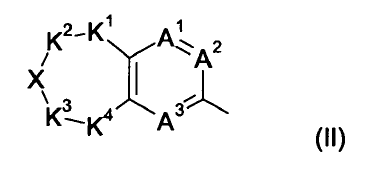 in dem
K¹ und K⁴ jeweils unabhängig voneinander eine -CH₂-, -CHR^{7a}-, -CR^{7b}R^{7c}- oder eine -C(O)-Gruppe bedeuten, und wobei
R^{7a}/R^{7b}/R^{7c} jeweils unabhängig voneinander ein Fluoratom, eine Hydroxy-, C_{1- 5}-Alkyloxy-, Tetrahydrofuranyl-, Oxetanyl-, Amino-, C₁₋₅-Alkylamino-, Di-(C₁₋₅-alkyl)-amino, C₃₋₅-Cycloalkylenimino- oder C₁₋₅-Alkylcarbonylaminogruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁- ₅-alkyl-, C₁₋₅-Alkylamino-C₁₋₅-alkyl-, Di-(C₁₋₅-alkyl)-amino-C₁₋₅-alkyl- , C₄₋₇-Cycloalkylenimino-C₁₋₅-alkyl-, Carboxy-C₀₋₅-alkyl-, C₁₋₅- Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl- C₀₋₅-alkyl-, C₄₋₇-Cycloalkyleniminocarbonyl-C₀₋₅-alkyl-gruppe, eine Phenylgruppe oder eine 5- oder 6-gliedrige Heteroarylgruppe, die durch 1-2 Substituenten ausgewählt aus einer Nitro-, Amino-, Hydroxy- Methoxy-, Cyano-, C₁₋₅-alkyl-gruppe oder einem Fluor-, Chlor- oder Bromatom substituiert sein kann, bedeutet, wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe,
oder zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2- Imidazolidinon-, 2-Oxazolidinon-, Tetrahydro-2(1H)-pyrimidinon-, [1,3]Oxazinan-2-on-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine-CH₂-Gruppe neben einem N-Atom durch eine -CO-Gruppe ersetzt sein kann, und/oder dessen Iminogruppen jeweils durch eine C₁₋₃-Alkyl- oder C₁- ₃-Alkylcarbonyl-gruppe substituiert sein können, und/oder bei dem das Schwefelatom zu einer Sulfoxid oder Sulfongruppe oxidiert sein kann,
K² und K³ jeweils unabhängig voneinander eine -CH₂-, -CHR^{8a}-, -CR^{8b}R^{8c}- oder eine -C(O)- Gruppe bedeuten, und wobei
R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁- ₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyk-C₀₋₅-alkyl-, C₁₋₅-Alkylaminocarbonyl-C₀₋₅-alkyl-, Di- (C₁₋₅-alkyl)-aminocarbonyl-C₀₋₅-alkyl-, C₄₋₇- Cycloalkyleniminocarbonyl- C₀₋₅-alkyl-gruppe bedeutet, oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)- pyrimidinon-, [1,3]Oxazinan-2-on-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem Stickstoffatom durch eine -CO-Gruppe ersetzt sein kann, und/oder dessen Iminogruppen jeweils durch eine C₁₋₃-Alkyl- oder C₁₋ ₃-Alkylcarbonyl-gruppe substituiert sein können, und/oder bei dem das Schwefelatom zu einer Sulfoxid oder Sulfongruppe oxidiert sein kann, mit der Massgabe, dass ein durch R^{8b} oder R^{8c} eingebrachtes Heteroatom nicht durch nur ein Kohlenstoffatom von X in Formel (I) entfernt sein darf, und insgesamt in Formel (II) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Sulfen-, Sulfon-, oder eine NR¹-Gruppe bedeutet, in der
R¹ ein Wasserstoffatom oder eine Hydoxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆- Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl- sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅- Alkyloxycarbonyl-, C₄-₇-Cycloalkyleniminocarbonyl-gruppe bedeutet, wobei die in den voranstehend genannten Gruppen befindlichen Methylen- und Methylgruppen zusätzlich durch eine C₁₋₃-Alkyl-, Carboxy-, C₁₋₅-Alkoxycarbonyl- gruppe substituiert sein können oder durch eine Hydroxy-, C₁₋₅-Alkyloxy-, Amino-, C₁₋₅-Alkylamino-, C₁- ₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind, und in dem
- A¹: entweder N oder CR¹⁰ bedeutet,
- A²: entweder N oder CR¹¹ bedeutet,
- A³: entweder N oder CR¹² bedeutet,
wobei R¹⁰, R¹¹ und R¹² jeweils unabhängig voneinander
ein Wasserstoff- , Fluor-, Chlor-, Brom- oder lodatom, oder eine C₁- ₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄- ₇-Cycloalkylenimino-gruppe bedeuten, oder
- D: eine der vier Gruppen (II-1), (II-2), (II-3) oder (II-4) darstellt 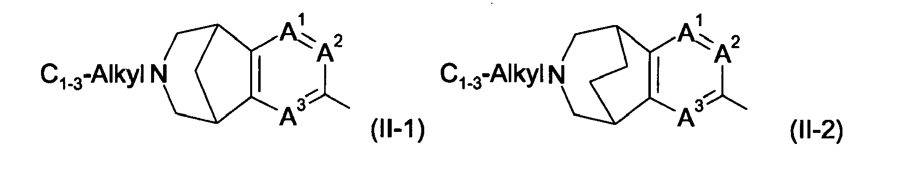 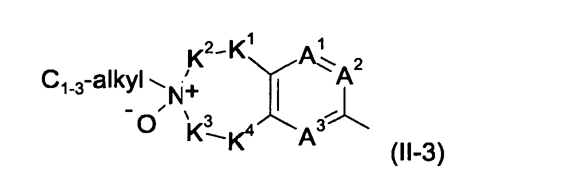 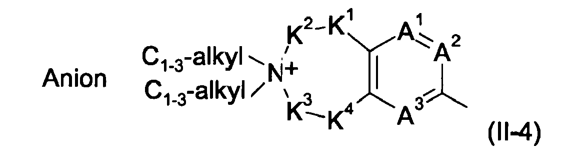 in der die Reste A1, A2, A3, K1, K2, K3, K4 wie oben definiert sind, und Anion in (II-4) ein Fluorid, Chlorid, Bromid, Iodid, Sulfat, Hydrogensulfat, Phosphat, Hydrogenphosphat, Benzoat, Salicylat, Succinat, Citrat oder Tartrat bedeutet,
- R³: ein Wasserstoffatom oder eine C₁₋₃-Alkylgruppe bedeutet, und
- R⁴ und R⁵: jeweils unabhängig voneinander ein Wasserstoffatom, eine geradkettige oder verzweigte C₁₋₆-Alkyl-, C₂₋₆-Alkenyl- oder wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder wobei ein oder zwei Wasserstoffatome der geradkettigen oder verzweigten C₁₋₆-Alkyl-, C₂₋₆-Alkenyl- oder C₂₋₆-Alkinylgruppe in ihren Methylen- und/oder Methylfragmenten gegebenenfalls jeweils unabhängig voneinander durch eine C₃₋₇- Cycloalkylgruppe, eine Nitril-, Hydroxy-oder C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Phenylmethyloxy-, Phenethyloxy-, C₁₋₅-Alkylcarbonyloxy-, C₁₋₅-Alkyloxycarbonyloxy-, Carboxy- C₁₋₅-alkyloxy-, C₁₋₅-Alkyloxycarbonyl-C₁₋₅-alkyloxy-, C₁₋₅-Alkyloxy- C₂₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₁₋₅-Alkyl-aminocarbonyloxy-, Di-(C₁₋₅-alkyl)- aminocarbonyloxy-, C₄₋₇-Cycloalkyleniminocarbonyl-, Aminosulfonyl-, C₁₋₅-Alkylaminosulfonyl-, Di-(C₁₋₅-alkyl)- aminosulfonyl-, C₄₋₇-Cycloalkyleniminosulfonyl-, Di-( C₁₋₅-alkyl)- phosphoryl-, Amino-, C₁₋₅-Alkylamino-, Di-(C₁₋₅-alkyl)-amino-, C₁₋₅-Alkylcarbonylamino-, Trifluoracetylamino-, C₁₋₅-Alkyloxy- C₁₋₅-alkylcarbonylamino-, Phenylcarbonylamino-, C₁₋₅-Alkylaminocarbonylamino-, Di-(C₁₋₅-alkyl)- aminocarbonylamino-, C₁₋₅-Alkyloxy-carbonylamino-, Phenylmethyloxy-carbonylamino-, C₁₋₅-Alkyloxy-C₂₋₅-alkyloxy-C₁₋₂- alkylcarbonylamino-, C₁₋₅-Alkylsulfonylamino-, *N-*(C₁₋₅-Alkylsulfonyl)-C₁₋₅-alkylamino-, C₃₋₆-Cycloalkylcarbonyl- aminogruppe, 4-Morpholinocarbonylamino-, oder eine Morpholinyl- , Thiomorpholinyl-, Pyrrolidinyl-, Piperidinyl-, Piperazinyl-, Tetrahydrofuranyl-, Tetrahydropyranylgruppe ersetzt sein können, wobei die vorgenannten Carbo- und Heterocyclen im Ring jeweils durch 1 bis 4 C₁₋₃-Alkyl- oder C₁₋₃- Alkylcarbonylgruppen oder jeweils durch 1 oder 2 Oxogruppen substituiert sein können, und/oder wobei die vorgenannten Phenyl und Heteroarylreste durch 1 oder 2 Substituenten ausgewahlt aus Fluor-, Chlor-, Brom-, Methyl-, Methoxy-, Amino- oder Trifluormethyl ersetzt sein können oder zwei benachbarte Kohlenstoffatome eines Phenylrings durch eine -CH₂-O- CH₂-Gruppe substituiert sein können, und/oder wobei die vorgenannten Alkylgruppen durch eine Cyano- C₁₋₅-Alkyloxycarbonyl- oder Carboxy-gruppe substituiert sein können, wobei die vorgenannten Carbonsäure- oder Sulfonsäure- amide gegebenenfalls am Stickstoff zusätzlich durch eine C₁₋₅-Alkylgruppe substituiert sein können, und/oder wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, eine Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl-, C₄₋₇-Cycloalkyleniminocarbonylgruppe, eine Phenyl-, mono- oder bicyclische Heteroaryl-, Phenyl-C₁₋₅-alkyl- oder mono- oder bicyclische Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom- und lodatome, und C₁₋₅-Alkyl-, Trifluormethyl-, Benzyl-, Amino-, Nitro-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann, oder zwei benachbarte Kohlenstoffatome eines Phenylrings durch eine -CH₂-O-CH₂-Gruppe substituiert sein können, bedeuten, oder eine C₃₋₇Cycloalkyl-, Morpholinyl-, Thiomorpholinyl-, Pyrrolidinyl-, Piperidinyl-, Piperazinyl-, Tetrahydrofuranyl-, Tetrahydropyranylgruppe bedeuten, die gegebenenfalls durch ein bis zwei unabhängig voneinander ausgewählte Reste aus C₁₋₃-Alkyl-, Acetyl-, C₁₋₅Alkyloxycarbonyl-, und Hydroxycarbonyl- substituiert sein kann, oder
- R⁴ und R⁵: zusammen mit dem Kohlenstoffatom, an dem sie gebunden sind, eine C₃₋₇-Cycloalkyl- oder C₅₋₇-Cycloalkenylgruppe bilden, wobei eine der Methylengruppen einer C₄₋₇-Cycloalkylgruppe durch ein Sauerstoff- oder Schwefelatom oder eine -NH-, -N(C₁₋₅- Alkyl), -N(C₁₋₄-Alkylcarbonyl)-, -N(C₁₋₄-Alkyloxycarbonyl)- oder eine Carbonyl-, Sulfinyl- oder Sulfonylgruppe ersetzt sein kann, und/oder wobei zwei direkt benachbarte Methylengruppen einer C₄₋₇-Cycloalkylgruppe zusammen durch eine -C(O)NH-, - C(O)N(C₁₋₅-Alkyl)-, -S(O)₂NH-, oder -S(O)₂N(C₁₋₅-Alkyl)-Gruppe ersetzt sein können, und/oder wobei 1 bis 3 Kohlenstoffatome einer C₃₋₇-Cycloalkylgruppe gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, Formyloxy-, C₁₋₅-Alkylcarbonyloxy-, C₁₋₅-Alkylsulfanyl-, C₁₋₅-Alkylsulfonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino-, C₃₋₆-Cycloalkylcarbonylamino-, Nitril-, Carboxy-C₁₋₅-alkyl, C₁₋₅- Alkyloxycarbonyl-C₁₋₅-alkyl, Carboxy-, C₁₋₅-Alkyloxycarbonyl-, Aminocarbonyl-, C₁₋₅-Alkylaminocarbonyl-, Di-(C₁₋₅-alkyl)- aminocarbonyl- oder C₄₋₇-Cycloalkyleniminocarbonylgruppe substituiert sein können, und/oder wobei 1 bis 2 Kohlenstoffatome einer C₅₋₇-Cycloalkenylgruppe gegebenenfalls unabhängig voneinander durch jeweils eine C₁₋₅- Alkyl-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonylgruppen oder 1-2 Fluoratome substituiert sein können, und/oder 1 bis 2 Kohlenstoffatome einer C₄₋₇-Cycloalkenylgruppe, die nicht durch eine Doppelbindung an ein anderes Kohlenstoffatom gebunden sind, gegebenenfalls unabhängig voneinander durch eine Hydroxy-, C₁₋₅-Alkyloxy-, C₁₋₅-Alkylcarbonyloxy-, C₁₋₅-Alkylsulfanyl-, C₁₋₅-Alkylsulfonyl-, Amino-, C₁₋₅-Alkylamino-, Di-(C₁₋₅-alkyl)-amino-, C₁₋₅-Alkylcarbonylamino-, C₁₋₅-Alkyl- sulfonylamino-, *N-*(C₁₋₅-Alkylsulfonyl)-C₁₋₅-alkylamino- oder C₃₋₆-Cycloalkylcarbonylaminogruppen substituiert sein können, mit der Maßgabe, dass eine solche, zusammen aus R⁴ und R⁵ gebildete C₃₋₇-Cycloalkyl- oder C₅₋₇-Cycloalkenylgruppe, in der zwei Heteroatome im Cyclus aus der Gruppe Sauerstoff und Stickstoff durch genau eine gegebenenfalls substituierte -CH₂-Gruppe voneinander getrennt sind, und/oder in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder in der ein an die cyclische Gruppe gebundener Substituent, der sich dadurch auszeichnet, daß ein Heteroatom aus der Gruppe Sauerstoff, Stickstoff, Schwefel und Fluor direkt an die cyclische Gruppe gebunden ist, von einem anderen Heteroatom aus der Gruppe Sauerstoff, Stickstoff und Schwefel, mit Ausnahme der Sulfongruppe, durch genau eine, gegebenenfalls substituierte, Methylengruppe getrennt ist, und/oder in der zwei Atome im Ring eine -O-O- oder-S-O-Bindung bilden, ausgeschlossen ist,
- M: einen Thiophenring gemäß Formel (III) darstellt, 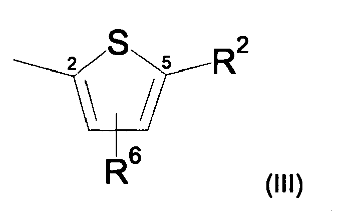 der über die 2-Position an die Carbonylgruppe in Formel (I) gebunden ist und der in 5-Position durch R² und gegebenenfalls zusätzlich durch R⁶ substituiert ist, wobei R² ein Wasserstoff- , Fluor-, Chlor-, Brom- oder lodatom oder eine Methoxy-, C₁₋₂-Alkyl-, Formyl-, NH₂CO- oder Ethinyl-Gruppe bedeutet, R⁶ ein Wasserstoff-, Fluor-, Chlor-, Brom- oder lodatom oder eine C₁₋₂- Alkyl- oder Amino-Gruppe bedeutet,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält, und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)- amino- oder C₃₋₆-Cycloalkyleniminogruppe substituierter Phenylring ankondensiert sein kann, und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und lod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt wurde, ganz oder teilweise durch Fluoratome ersetzt sein können,
deren Tautomere, deren Enantiomere, deren Diastereomere, deren Gemische und deren Salze.

Beispiele für monocyclische Heteroarylgruppen sind die Pyridyl-, *N-*Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thiophenyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[*c*]furanyl-, Benzothiophenyl-, Benzo[*c*]thiophenyl-, Benzothiazolyl-, Benzo[*c*]isothiazolyl-, Benzo[*d*]isothiazolyl-, Benzooxazolyl-, Benzo[*c*]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]triazinyl-, Benzo[1,2,4]triazinyl-, Benzotriazolyl-, Cinnolinyl-, Chinolinyl-, *N-*Oxy-chinolinyl-, Isochinolinyl-, Chinazolinyl-, *N-*Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylgruppen sind die Methyl-, Ethyl-, 1-Propyl-, 2-Propyl-, *n-*Butyl-, *sec-*Butyl-, *tert*-Butyl-, 1-Pentyl-, 2-Pentyl-, 3-Pentyl-, *neo*-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxy-,*n-*Butyloxy-, *sec-*Butyloxy-, *tert*-Butyloxy-, 1-Pentyloxy-, 2-Pentyloxy-, 3-Pentyloxy- oder *neo*-Pentyloxy-Gruppe.

Beispiele für die voranstehend in den Definitionen erwähnten C₂₋₅-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl- oder 2-Ethyl-prop-2-en-1-yl -Gruppe,

Beispiele für die voranstehend in den Definitionen erwähnten C₂₋₅-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl- oder 3-Methyl-1-butin-3-yl-Gruppe.

Eine 2. Ausführungsform der vorliegenden Erfindung umfasst diejenigen Verbindungen der allgemeinen Formel (I), in denen
- D: ein substituiertes bicyclisches Ringsystem der Formel (II) darstellt, 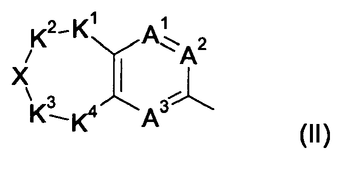 in dem
K¹und K⁴ jeweils unabhängig voneinander eine -CH₂-, -CHR^{7a}-, -CR^{7b}R^{7c}- oder eine -C(O)-Gruppe bedeuten, wobei
R^{7a}/R^{7b}/R^{7c} jeweils unabhängig voneinander ein Fluoratom, eine Hydroxy-, C₁₋ ₅-Alkyloxygruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅- alkyl-gruppe bedeutet, oder eine Phenylgruppe, die durch 1-2 Substituenten ausgewählt aus einer Nitro-, Amino-, Hydroxy- Methoxy-, Cyano-, C₁₋₅-alkyl-gruppe oder einem Fluor-, Chlor-
oder Bromatom substituiert sein kann, oder eine 5- oder 6- gliedriger Heteroarylgruppe, bedeutet wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Tetrahydrofuran- oder Tetrahydropyran-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und
K² und K³ jeweils unabhängig voneinander eine -CH₂-, -CHR^{8a}-, -CR^{8b}R^{8c}- oder eine -C(O)- Gruppe bedeuten, und wobei R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁₋ ₅-Alkyloxy-C₁₋₅-alkyl-gruppe bedeutet, oder zwei Reste R^{8b}/R^{8c} zusammen mit dem Ringkohlenstoffatom einen 3-, 4-, 5-, 6- oder 7-gliedrigen Carbocyclus oder einen Cyclopenten-, Cyclohexen-, Oxetan-, Tetrahydrofuran-, Tetrahydropyran-ring bilden können, wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, mit der Massgabe, dass ein durch R^{8b} oder R^{8c} eingebrachtes Heteroatom nicht durch nur ein Kohlenstoffatom von X in Formel (I) entfernt sein darf, und insgesamt in Formel (II) maximal vier Reste ausgewählt aus R^{7a}, R^{7b}, R^{7c}, R^{8a} , R^{8b} und R^{8c} vorhanden sein dürfen, und
X ein Sauerstoff- oder Schwefelatom, eine Sulfen-, Sulfon- oder eine NR¹-Gruppe bedeutet, in der
R¹ ein Wasserstoffatom oder eine C₁₋₅-Alkyl-, C₂₋₅-Alkenyl-CH₂-, C₂₋₅-Alkinyl-CH₂, C₃₋₆- Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl- sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅- Alkyloxycarbonyl-, C₄₋₇-Cycloalkyleniminocarbonyl-gruppe bedeutet, wobei die in den voranstehend genannten Gruppen befindlichen Methylen- und Methylgruppen zusätzlich durch eine C₁₋₃Alkyl-, Carboxy-, C₁₋₅-Alkoxycarbonyl- gruppe substituiert sein können oder durch eine Hydroxy-, C₁₋₅-Alkyloxy-, Amino-, C₁₋₅-Alkylamino-, C₁- ₅-Dialkylamino- oder C₄₋₇Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹, A², und A³ jeweils wie in der 1. Ausführungsform beschrieben definiert sind, oder
- D: eine der vier Gruppen (II-1a), (II-2a), (II-3) oder (II-4) darstellt 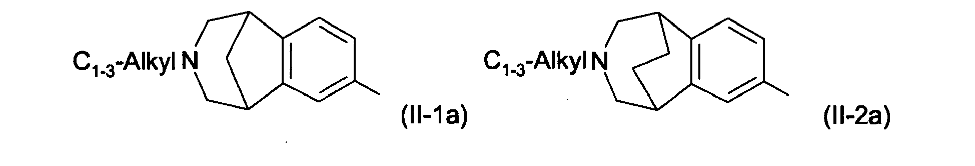 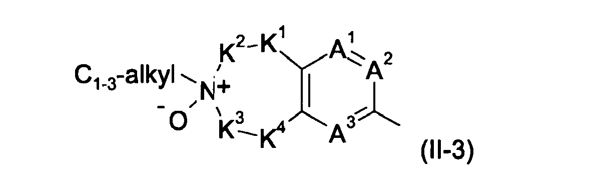 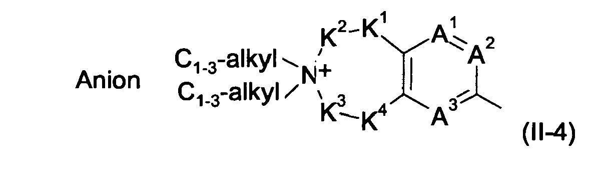 in der die Reste A1, A2, A3, K1, K2, K3, K4 wie oben definiert sind, und Anion in (II-4) ein Fluorid, Chlorid, Bromid, Iodid, Sulfat, Hydrogensulfat, Phosphat, Hydrogenphosphat, Benzoat, Salicylat, Succinat, Citrat oder Tartrat bedeutet, und
- R³: ein Wasserstoffatom bedeutet, und
- R⁴, R⁵: und M jeweils wie in der 1. Ausführungsform beschrieben definiert sind, deren Tautomere, deren Enantiomere, deren Diastereomere, deren Gemische und deren Salze. Eine 3. Ausführungsform der vorliegenden Erfindung umfasst diejenigen Verbindungen der allgemeinen Formel (I), in denen
- D: ein substituiertes bicyclisches Ringsystem der Formel (II) darstellt, 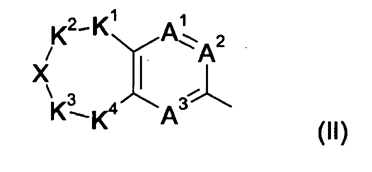 in dem K1, K2, K3 und K4 wie in der 1. oder 2. Ausführungsform beschrieben definiert sind, und wobei
X eine NR¹-Gruppe bedeutet, in der R¹ ein Wasserstoffatom oder eine C₁₋₅-Alkyl-, C₂₋₅-Alkenyl-CH₂-, C₂₋₅-Alkinyl-CH₂, C₃₋₆- Cycloalkyl-, C₄₋₆-Cycloalkenylgruppe bedeutet, wobei die in den voranstehend genannten Gruppen befindlichen Methylen- und Methylgruppen zusätzlich durch eine C₁₋₃-Alkyl-, Carboxy-, C₁₋₅- Alkoxycarbonyl-gruppe substituiert sein können oder durch eine Hydroxy-, C₁₋₅-Alkyloxy-, Amino-, C₁₋₅- Alkylamino-, C₁₋₅-Dialkylamino- oder C₄₋₇- Cycloalkyleniminogruppe substituiert sein können, sofern die Methylen- oder Methylgruppen nicht direkt an ein Heteroatom aus der Gruppe O, N oder S gebunden sind, und/oder
ein bis drei Wasserstoffatome durch Fluoratome ersetzt sein können, sofern die Methylen- oder Methylgruppen nicht direkt an das Stickstoff atom gebunden sind, und in dem
- A¹: entweder N oder CR¹⁰ bedeutet,
- A²: entweder N oder CR¹¹ bedeutet,
- A³: entweder N oder CR¹² bedeutet, wobei R¹⁰, R¹¹ und R¹² jeweils unabhängig voneinander ein Wasserstoff- , Fluor-, Chlor-, Bromatom oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O- CHF₂O- CH₂FO-gruppe bedeuten, oder
- D: eine der vier Gruppen (II-1a), (II-2a), (II-3) oder (II-4) darstellt 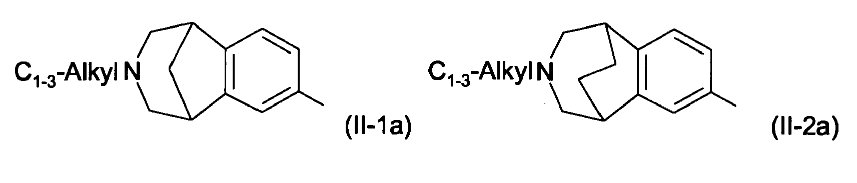 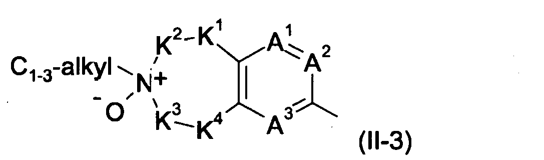 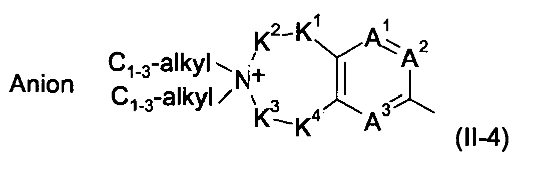 in der die Reste A1, A2, A3, K1, K2, K3, K4 wie oben definiert sind, und Anion in (II-4) aus der Gruppe Fluorid, Chlorid, Bromid, Iodid, Sulfat, Phosphat, Benzoat, Salicylat, Succinat, Citrat und Tartrat ausgewählt werden kann,und R³, R⁴, R⁵ und M jeweils wie in der 1. oder 2. Ausführungsform definiert sind, wobei R⁶ ein Wasserstoffatom bedeutet, deren Tautomere, deren Enantiomere, deren Diastereomere, deren Gemische und deren Salze. Eine 4. Ausführungsform der vorliegenden Erfindung umfasst diejenigen Verbindungen der allgemeinen Formel (I), in denen
- D, R³ und M: jeweils wie in der 1., 2. oder 3. Ausführungsform beschrieben definiert sind, und
- R⁴: eine geradkettige oder verzweigte C₃₋₆-Alkenyl- oder C₃₋₆-Alkinylgruppe, eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und bei der gegebenenfalls ein bis zwei Wasserstoffatome unabhängig voneinander durch eine C₃₋₇-Cycloalkyl-, Hydroxy-, C₁₋₅-Alkyloxy-, Phenylmethyloxy-, Phenethyloxy-, Carboxy- C₁₋₅-alkyloxy-, C₁₋₅-Alkyloxycarbonyl-C₁₋₅-alkyloxy-, C₁₋₅-Alkyloxy- C₂₋₅-alkyloxy-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₁₋₅-Alkyl-aminocarbonyloxy-, Di-(C₁₋₅-alkyl)- aminocarbonyloxy-, C₄₋₇-Cycloalkyleniminocarbonyl-, Amino-, C₁₋₅- Alkylamino- oder Di-(C₁₋₅-alkyl)-aminogruppe C₁₋₅-Alkylcarbonylamino-, Trifluoracetylamino-, C₁₋₅-Alkyloxy- C₁₋₅-alkylcarbonylamino-, Phenylcarbonylamino-, C₁₋₅-Alkylaminocarbonylamino-, Di-(C₁₋₅-alkyl)- aminocarbonylamino-, C₁₋₅-Alkyloxy-carbonylamino-, Phenylmethyloxy-carbonylamino-, C₁₋₅-Alkyloxy-C₂₋₅-alkyloxy-C₁₋₂- alkylcarbonylamino-, C₁₋₅-Alkylsulfonylamino-, C₃₋₆-Cycloalkylcarbonylaminogruppe, 4-Morpholinocarbonylamino- gruppe ersetzt sein können, wobei die vorgenannten Carbo- und Heterocyclen im Ring jeweils durch 1 bis 4 C₁₋₃-Alkyl- oder C₁₋₃- Alkylcarbonylgruppen oder jeweils durch 1 oder 2 Oxogruppen substituiert sein können, und/oder wobei die vorgenannten Phenyl und Heteroarylreste durch 1 bis 2 Substituenten ausgewahlt aus Fluor-, Chlor-, Brom-, Methyl-, Methoxy-, oder Trifluormethyl ersetzt sein können, oder zwei benachbarte Kohlenstoffatome eines Phenylrings durch eine -CH₂-O-CH₂-Gruppe substituiert sein können, und/oder wobei die vorgenannten Alkylgruppen durch eine Cyano- C₁₋₅-Alkyloxycarbonyl- oder Carboxy-gruppe substituiert sein können, wobei die vorgenannten Carbonsäure- oder Sulfonsäure- amide gegebenenfalls am Stickstoff zusätzlich durch eine C₁₋₅-Alkylgruppe substituiert sein können, eine Phenyl-, Phenyl-C₁₋₂-alkyl-, Heteroaryl-C₁₋₂-alkyl- oder C-verknüpfte Heteroarylgruppe, wobei die Heteroarylgruppe ausgewählt ist aus der Gruppe bestehend aus Imidazolyl-, Furanyl-, Thiophenyl-, Thiazolyl-, Pyrazolyl-, Tetrazolyl-, Benzimidazolyl-, Indolyl-, Pyrimidinyl-, Pyrazinyl- Oxazolyl-, 1,2,4-Triazolyl- und Pyridinyl-, und die im Phenyl- oder Heteroarylteil gegebenenfalls ein- bis zweifach durch gleiche oder unterschiedliche Substituenten ausgewählt aus Chlor- oder Fluoratomen oder C₁₋₃-Alkyl-, Benzyl-, Hydroxy-, Amino-, CF₃-, CH₃O- oder CHF₂O- gruppen, substituiert sein kann, bedeutet,
- R⁵: ein Wasserstoffatom, eine geradkettige oder verzweigte C₁₋₄-Alkylgruppe, wobei die Wasserstoffatome der geradkettigen oder verzweigten C₁₋₄-Alkylgruppen gegebenenfalls ganz oder teilweise durch Fluoratome ersetzt sein können, oder eine Propargyl- oder C₁₋₃Alkyloxy- C₁₋₃-alkylgruppe, bedeutet, oder
- R⁴ und R⁵: zusammen mit dem Kohlenstoffatom, an dem sie gebunden sind, eine C₅₋₆-Cycloalkenyl- oder C₃₋₇-Cycloalkylgruppe bilden, wobei eine der Methylengruppen einer C₄₋₇-Cycloalkylgruppe durch ein Sauerstoff oder Schwefelatom oder eine -NH-, -N(C₁₋₅- Alkyl), -N(C₁₋₄-Alkylcarbonyl)-, Carbonyl-, Sulfinyl- oder eine Sulfonyl-Gruppe ersetzt sein kann, oder zwei direkt benachbarte Methylengruppen einer C₄₋₇-Cycloalkylgruppe zusammen durch eine -C(O)NH-, -C(O)N(C₁₋₅-Alkyl)-, -S(O)₂NH-, oder -S(O)₂N(C₁₋ ₅-Alkyl)-Gruppe ersetzt sein können, und/oder wobei 1 bis 2 Kohlenstoffatome einer C₃₋₇-Cycloalkylgruppe gegebenenfalls unabhängig voneinander durch jeweils ein oder zwei Fluoratome oder eine oder zwei C₁₋₅- Alkyl-gruppen oder eine Hydroxy-, C₁₋₅-Alkyloxy-, Formyloxy-, Amino-, C₁₋₅-Alkylamino-, Di-(C₁₋₅-alkyl)-amino-, C₁₋₅-Alkylcarbonylamino-, C₃₋₆-Cycloalkylcarbonylamino-, Nitril-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können, mit der Maßgabe, dass eine solche, zusammen aus R⁴ und R⁵ gebildete C₃₋₇-Cycloalkylgruppe, in der zwei Heteroatome im Cyclus aus der Gruppe Sauerstoff und Stickstoff durch genau eine gegebenenfalls substituierte -CH₂-Gruppe voneinander getrennt sind, und/oder in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder in der ein an die cyclische Gruppe gebundener Substituent, der sich dadurch auszeichnet, daß ein Heteroatom aus der Gruppe Sauerstoff, Stickstoff, Schwefel und Fluoratom direkt an die cyclische Gruppe gebunden ist, von einem anderen Heteroatom aus der Gruppe Sauerstoff, Stickstoff und Schwefel durch genau eine, gegebenenfalls substituierte, Methylengruppe getrennt ist, und/oder in der zwei Atome im Ring eine -O-O- oder -S-O-Bindung bilden, ausgeschlossen ist,
deren Tautomere, deren Enantiomere, deren Diastereomere, deren Gemische und deren Salze.

Eine 5. Ausführungsform der vorliegenden Erfindung umfasst diejenigen Verbindungen der allgemeinen Formel (I), in denen
- D: ein substituiertes bicyclisches Ringsystem der Formel (II) darstellt, 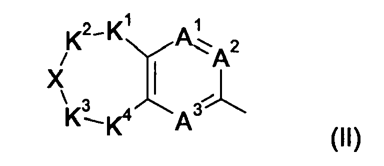 in dem
K¹ und K⁴ jeweils unabhängig voneinander eine -CH₂-, -CHR^{7a}- oder eine - CR^{7b}R^{7c}- Gruppe bedeuten, wobei
R^{7a}/R^{7b}/R^{7c} jeweils unabhängig voneinander eine C₁₋₂-Alkylgruppe oder eine Phenylgruppe, die durch 1 oder 2 Substituenten ausgewählt aus einer Nitro-, Amino-, Hydroxy- Methoxy-, Cyano-, C₁₋₅-alkyl-gruppe oder einem Fluor-, Chlor- oder Bromatom substituiert sein kann, bedeutet,
K² und K³ jeweils eine -CH₂-Gruppe bedeutet
X eine NR¹-Gruppe bedeutet, in der
R¹ ein Wasserstoffatom oder eine C₁₋₅-Alkyl-, C₂₋₄-Alkenyl-CH₂-, C₂₋₄-Alkinyl-CH₂- oder C₃₋₆-Cycloalkylgruppe bedeutet, wobei die in den voranstehend genannten C₂₋₅-Alkyl-Gruppen befindlichen Methylen- und Methylgruppen durch ein bis drei Fluoratome substituiert sein können, sofern die Methylen- oder Methylgruppen nicht direkt an das Stickstoffatom gebunden sind, und in dem
A¹ entweder N oder CR¹⁰ bedeutet,
A² entweder N oder CR¹¹ bedeutet,
A³ entweder N oder CR¹² bedeutet, wobei R¹⁰, R¹¹ und R¹² jeweils unabhängig voneinander ein Wasserstoff- , Fluor- oder Chloratom, oder eine C₁₋₃-Alkyl-, CF₃-, Hydroxy oder CH₃O-gruppe bedeuten, oder
- D: eine der Gruppen (II-3) oder (II-4) darstellt 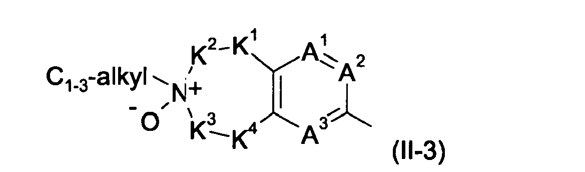 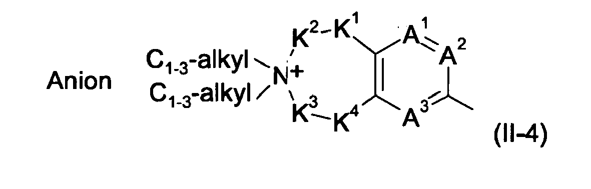 in der die Reste A1, A2, A3, K1, K2, K3, K4 wie oben definiert sind, und das Anion in (II-4) aus der Gruppe Fluorid, Chlorid, Bromid, Iodid, Sulfat, Phosphat, Benzoat, Salicylat, Succinat, Citrat oder Tartrat ausgewählt werden kann,und
- R³: ein Wasserstoffatom bedeutet,
- R⁴: eine geradkettige oder verzweigte C₃₋₆-Alkenyl- oder C₃₋₆-Alkinylgruppe, eine geradkettige oder verzweigte C₁₋₄-Alkylgruppe, wobei die Wasserstoffatome der geradkettigen oder verzweigten C₁₋₄-Alkylgruppe gegebenenfalls teilweise durch bis zu vier Fluoratome ersetzt sein können, und bei der gegebenenfalls ein bis zwei Wasserstoffatome unabhängig voneinander durch eine C₃₋₇-Cycloalkyl-, Hydroxy-, C₁₋₅-Alkyloxy-, Phenylmethyloxy-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino- oder Di-(C₁₋₅-alkyl)-amino-, C₁₋₅-Alkylcarbonylamino-, Carboxy-C₁₋₅-alkylcarbonylamino- oder eine C₁₋₅-Alkyloxycarbonyl-C₁₋₅-alkylcarbonylamino-gruppe ersetzt sein können, wobei die vorgenannten Phenylreste durch 1 oder 2 Substituenten ausgewählt aus Fluor-, Chlor-, Brom-, Methyl-, Methoxy-, oder Trifluormethyl- ersetzt sein können, oder wobei die vorgenannten Carbonsäureamide gegebenenfalls am Stickstoff zusätzlich durch eine C₁₋₅- Alkylgruppe substituiert sein können, eine Phenyl-, Phenyl-C₁₋₂-alkyl-, Heteroaryl-C₁₋₂-alkyl- oder C-verknüpfte Heteroarylgruppe, wobei die Heteroarylgruppe ausgewählt ist aus der Gruppe bestehend aus Imidazolyl-, Furanyl-, Thiophenyl-, Thiazolyl-, Pyrazolyl-, Tetrazolyl-, Benzimidazolyl-, Indolyl-, Pyrimidinyl-, Pyrazinyl-, Oxazolyl-, und Pyridinyl-, und die im Phenyl- oder Heteroarylteil gegebenenfalls ein- bis zweifach durch gleiche oder unterschiedliche Substituenten ausgewählt aus Chlor- oder Fluoratomen oder C₁₋₃-Alkyl-, CF₃-, HO-, CH₃O- oder CHF₂O-gruppen, substituiert sein kann, bedeutet,
- R⁵: ein Wasserstoffatom, eine geradkettige oder verzweigte C₁₋₄-Alkylgruppe, eine Propargyl- oder C₁₋₃Alkyloxy- C₁₋₃-alkylgruppe, bedeutet, oder
- R⁴ und R⁵: zusammen mit dem Kohlenstoffatom, an dem sie gebunden sind, eine C₅₋₆-Cycloalkenyl- oder C₃₋₇-Cycloalkylgruppe bilden, wobei eine der Methylengruppen einer C₄₋₇-Cycloalkylgruppe durch ein Sauerstoff- oder Schwefelatom oder eine Sulfonyl- Gruppe ersetzt sein kann, oder wobei 1 bis 2 Kohlenstoffatome einer C₃₋₇-Cycloalkylgruppe gegebenenfalls unabhängig voneinander durch jeweils ein oder zwei Fluoratome, oder eine oder zwei C₁₋₅-Alkyl-gruppen, oder eine Hydroxy-, C₁₋₅-Alkyloxy-, Formyloxy-, Nitril-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe, substituiert sein können, mit der Maßgabe, dass eine solche, zusammen aus R⁴ und R⁵ gebildete C₃₋₇-Cycloalkylgruppe, in der eine der Methylengruppen des Cyclus, die direkt mit dem Kohlenstoffatom verbunden ist, an dem die Reste R⁴ und R⁵ angebunden sind, durch ein Sauerstoff- oder Schwefelatom ersetzt ist, ausgeschlossen ist, und
- M: einen Thiophenring gemäß Formel (III) darstellt, 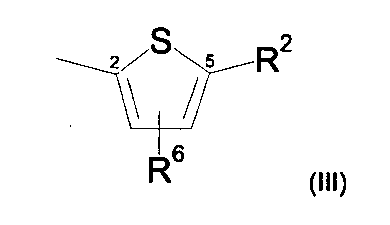 der über die 2-Position an die Carbonylgruppe in Formel (I) gebunden ist und der in 5-Position durch R² substituiert ist, wobei
R² ein Chlor- oder Bromatom oder eine Ethinyl-Gruppe bedeutet, und
R⁶ ein Wasserstoffatom bedeutet, wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können, deren Tautomere, deren Enantiomere, deren Diastereomere, deren Gemische und deren Salze. Eine 6. Ausführungsform der vorliegenden Erfindung umfasst diejenigen Verbindungen der allgemeinen Formel (I), entsprechend den Ausführungsformen 1, 2, 3, 4 oder 5, in denen der Rest
- D: ein substituiertes bicyclisches Ringsystem der Formel (II) darstellt, 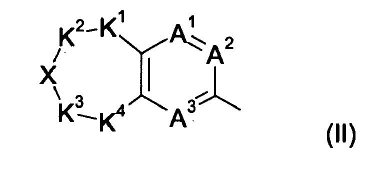 in dem
K¹ und K⁴ jeweils unabhängig voneinander eine -CH₂-, -CHR^{7a}- oder eine - CR^{7b}R^{7c}- Gruppe bedeuten, wobei
R^{7a}/R^{7b}/R^{7c} jeweils unabhängig voneinander eine C₁₋₂-Alkylgruppe bedeutet,
- K² und K³: jeweils eine -CH₂-Gruppe bedeutet,
- X: eine NR¹-Gruppe bedeutet, in der
R¹ ein Wasserstoffatom oder eine C₁₋₅-Alkyl- oder C₃₋₆-Cycloalkylgruppe bedeutet, wobei die in den voranstehend genannten Gruppen befindlichen Methylen- und Methylgruppen ein bis drei Wasserstoffatome durch Fluoratome ersetzt sein können, sofern die Methylen- oder Methylgruppen nicht direkt an das Stickstoffatom gebunden sind, und in dem
A¹ CR¹⁰ bedeutet,
A² CR ¹¹ bedeutet,
A³ CR¹² bedeutet, wobei R¹⁰, R¹¹ und R¹² jeweils unabhängig voneinander ein Wasserstoff- , Fluor- oder Chloratom, oder eine C₁₋₃-Alkyl-, CF₃-, HO-, CH₃O -gruppe, bedeuten, oder
- D: die Gruppe (II-4) darstellt 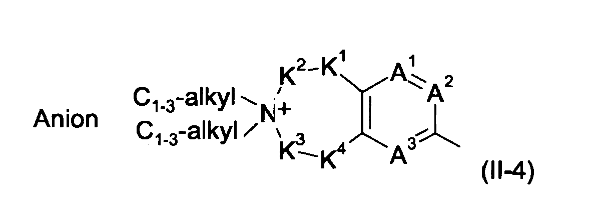 in der die Reste A1, A2, A3, K1, K2, K3, K4 wie oben definiert sind, und das Anion in (II-4) aus der Gruppe Fluorid, Chlorid, Bromid, Iodid, Sulfat, Phosphat, Benzoat, Salicylat, Succinat, Citrat oder Tartrat ausgewählt werden kann,
deren Tautomere, deren Enantiomere, deren Diastereomere, deren Gemische und deren Salze.

Eine 7. Ausführungsform der vorliegenden Erfindung umfasst diejenigen Verbindungen der allgemeinen Formel (I), entsprechend den Ausführungsformen 1, 2, 3, 4, 5 oder 6, in denen weder

R⁴ noch R⁵ ein Wasserstoffatom bedeuten dürfen,
deren Tautomere, deren Enantiomere, deren Diastereomere, deren Gemische und deren Salze.

Eine 8. Ausführungsform der vorliegenden Erfindung umfasst diejenigen Verbindungen der allgemeinen Formel (I), entsprechend den Ausführungsformen 1, 2, 3, 4, 5 oder 6, in denen
- R⁴ und R⁵: zusammen mit dem Kohlenstoffatom, an dem sie gebunden sind, eine C₅₋₆-Cycloalkenyl- oder C₃₋₇-Cycloalkylgruppe bilden, wobei eine der Methylengruppen einer C₄₋₇-Cycloalkylgruppe durch ein Sauerstoff- oder Schwefelatom ersetzt sein kann, mit der Maßgabe, dass eine solche, zusammen aus R⁴ und R⁵ gebildete C₃₋₇-Cycloalkylgruppe, in der eine der Methylengruppen des Cyclus, die direkt mit dem Kohlenstoffatom verbunden ist, an dem die Reste R⁴ und R⁵ angebunden sind, durch ein Sauerstoff- oder Schwefelatom ersetzt ist, ausgeschlossen ist,
deren Tautomere, deren Enantiomere, deren Diastereomere, deren Gemische und deren Salze.

Als besonders bevorzugte Beispiele für die cyclischen Gruppen, die aus R4/R5 und dem Kohlenstoffatom an das sie gebunden sind, gebildet werden können, seien die folgenden Ringsysteme genannt:

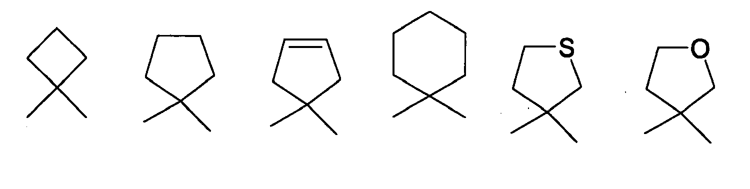

Beispielsweise seien folgende bevorzugte Verbindungen der allgemeinen Formel (I) erwähnt, sowohl als deren Tautomere, deren Enantiomere, deren Diastereomere, deren Gemische und deren Salze:
3-[(5-Brom-thiophen-2-yl)-carbonylamino]-*N-*(3-methyl-2,3,4,5-tetrahydro-1*H-*benzo[*d*]azepin-7-yl)-tetrahydrofuran-3-carbonsäureamid,
3-[(5-Chlor-thiophen-2-yl)-carbonylamino]-*N-*(3-methyl-2,3,4,5-tetrahydro-1*H-*benzo[*d*]azepin-7-yl)-tetrahydrofuran-3-carbonsäureamid,
5-Chlor-thiophen-2-carbonsäure-*N-*[1-(3-ethyl-2,3,4,5-tetrahydro-1*H-*benzo[*d*]azepin-7-ylcarbamoyl)-1-methyl-ethyl]-amid,
5-Ethinyl-N-[1-methyl-1-(3-methyl-2,3,4,5-tetrahydro-1*H-*benzo[*d*]azepin-7-ylcarbamoyl)-ethyl]-thiophen-2-carbonsäureamid,
5-Chlor-thiophen-2-carbonsäure-*N-*[1-methyl-1-(3-methyl-2,3,4,5-tetrahydro-1*H-*benzo[d]azepin-7-ylcarbamoyl]-ethyl}-amid,
5-Brom-thiophen-2-carbonsäure-*N-*[1-methyl-1-(3-methyl-2,3,4,5-tetrahydro-1*H-*benzo[d]azepin-7-ylcarbamoyl]-ethyl}-amid,
5-Chlor-thiophen-2-carbonsäure-*N-*[2-methoxy-1-(3-methyl-2,3,4,5-tetrahydro-1*H-*benzo[*d*]azepin-7-ylcarbamoyl)-ethyl]-amid,
1-[(5-Brom-thiophen-2-yl)-carbonylamino]-*N-*(3-methyl-2,3,4,5-tetrahydro-1*H-*benzo[*d*]azepin-7-yl)-cyclopentan-1-carbonsäureamid,
1-[(5-Chlor-thiophen-2-yl)-carbonylamino]-*N-*(3-methyl-2,3,4,5-tetrahydro-1*H-*benzo[*d*]azepin-7-yl)-cyclopentan-1-carbonsäureamid,
3-[(5-Chlor-thiophen-2-yl)-carbonylamino]-*N-*(3-methyl-2,3,4,5-tetrahydro-1*H-*benzo[d]azepin-7-yl)-tetrahydrothiophen-3-carbonsäureamid,
1-[(5-Chlor-thiophen-2-yl)carbonylamino]-N-(3-methyl-2,3,4,5-tetrahydro-1H-benzo[d]azepin-7-yl)-cyclobutan-1-carbonsäureamid,
1-[(5-Brom-thiophen-2-yl)carbonylamino]-N-(3-methyl-2,3,4,5-tetrahydro-1H-benzo[d]azepin-7-yl)-cyclopent-3-en-1-carbonsäureamid,
1-[(5-Chlor-thiophen-2-yl)carbonylamino]-N-(3-methyl-2,3,4,5-tetrahydro-1H-benzo[d]azepin-7-yl)-cyclohexan-1-carbonsäureamid,
(R)-5-Chlor-thiophen-2-carbonsäure-*N-*[2-benzyloxy-1-(3-methyl-2,3,4,5-tetrahydro-1*H-*benzo[*d*]azepin-7-ylcarbamoyl)-ethyl]-amid,
5-Chlor-thiophen-2-carbonsäure-*N-*[2-benzyloxy-1-methyl-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-hydroxy-1-methyl-1-(3-methyl-2,3,4,5-tetrahydro-1*H-*benzo[*d*]azepin-7-ylcarbamoyl)-ethyl]-amid,
5-Brom-thiophen-2-carbonsäure-N- [3-hydroxy-1-methyl-1-(3-methyl-2,3,4,5-tetrahydro-1H-benzo[d]azepin-7-ylcarbamoyl)-propyl]-amid,
5-Chlor-thiophen-2-carbonsäure-N-[1-methyl-3-dimethylaminocarbonyl-1-(3-methyl-2,3,4,5-tetrahydro-1*H-*benzo[*d*]azepin-7-ylcarbamoyl)-propyl]-amid,
5-Chlor-thiophen-2-carbonsäure-*N-*[2-(4-hydroxy-phenyl)-1-methyl-1-(3-methyl-2,3,4,5-tetrahydro-1*H-*benzo[*d*]azepin-7-ylcarbamoyl)-ethyl]-amid,
5-Chlor-thiophen-2-carbonsäure-*N-*[1-methyl-1-(3,5-dimethyl-2,3,4,5-tetrahydro-1*H-*benzo[*d*]azepin-7-ylcarbamoyl)-ethyl]-amid,
5-Chlor-thiophen-2-carbonsäure-*N-*{1-methyl-1-[3-methyl-5-(4-aminophenyl)-2,3,4,5-tetrahydro-1*H-*benzo[*d*]azepin-7-ylcarbamoyl]-ethyl}-amid,
5-Chlor-thiophen-2-carbonsäure-*N-*[2-ethoxy-1-(3-methyl-2,3,4,5-tetrahydro-1*H-*benzo[*d*]azepin-7-ylcarbamoyl)-ethyl]-amid,
5-Chlor-thiophen-2-carbonsäure-*N-*[3-methoxy-1-(3-methyl-2,3,4,5-tetrahydro-1*H-*benzo[*d*]azepin-7-ylcarbamoyl)-propyl]-amid,
5-Chlor-thiophen-2-carbonsäure-*N-*[2-isopropyloxy-1-(3-methyl-2,3,4,5-tetrahydro-1*H-*benzo[*d*]azepin-7-ylcarbamoyl)ethyl]-amid,
(R)-5-Chlor-thiophen-2-carbonsäure-*N-*[3-benzyloxy-1-(3-methyl-2,3,4,5-tetrahydro-1*H-*benzo[*d*]azepin-7-ylcarbamoyl)-propyl]-amid,
1-[(5-Chlor-thiophen-2-yl)-carbonylamino]-3,4-dimethoxy-*N-*(3-methyl-2,3,4,5-tetrahydro-1*H-*benzo[*d*]azepin-7-yl)-cyclopentan-1-carbonsäureamid,
5-Chlor-thiophen-2-carbonsäure-*N-*[C-(1-methyl-pyrazol-3-yl)-C-(3-methyl-2,3,4,5-tetrahydro-1*H-*benzo[*d*]azepin-7-ylcarbamoyl)-methyl]-amid,
(R)-5-Chlor-thiophen-2-carbonsäure-*N-*[2-phenyl-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-(furan-2-yl)-1-(3-methyl-2,3,4,5-tetrahydro-1*H-*benzo[*d*]azepin-7-ylcarbamoyl)-ethyl]-amid,
5-Chlor-thiophen-2-carbonsäure-*N-*[2-(4-methoxyphenyl)-1-(3-methyl-2,3,4,5-tetrahydro-1*H-*benzo[*d*]azepin-7-ylcarbamoyl)-ethyl]-amid,
5-Chlor-thiophen-2-carbonsäure-*N-*[2-(4-hydroxy-3-nitro-phenyl)-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-(4-hydroxy-phenyl)-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-cyclohexyl-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[3-aminocarbonyl-1-(3-methyl-2,3,4,5-tetrahydro-1*H-*benzo[*d*]azepin-7-ylcarbamoyl)-propyl]-amid,
(R)-5-Chlor-thiophen-2-carbonsäure-*N-*[2-acetylamino-1-(3-methyl-2,3,4,5-tetrahydro-1*H-*benzo[*d*]azepin-7-ylcarbamoyl)-ethyl]-amid,
(R)-5-Brom-thiophen-2-carbonsäure-*N-*[2-benzoylamino-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-(2-hydroxycarbonyl-ethyl)carbonylamino-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-(2-hydroxycarbonyl-ethyl)carbonylamino-1-(3-methyl-2,3,4,5-tetrahydro-1*H-*benzo[*d*]azepin-7-ylcarbamoyl)-ethyl]-amid,
(R)-5-Chlor-thiophen-2-carbonsäure-*N-*[2-(4-methoxycarbonyl-butyl)carbonylamino-1-(3-methyl-2,3,4,5-tetrahydro-1*H-*benzo[*d*]azepin-7-ylcarbamoyl)-ethyl]-amid,
5-Chlor-thiophen-2-carbonsäure-*N-*[1-methyl-1-(3,3-dimethyl-2,3,4,5-tetrahydro-1*H-*benzo[d]azepinium-7-ylcarbamoyl]-ethyl}-amid iodid,
3-[(5-Chlor-thiophen-2-yl)-carbonylamino]-*N-*(3,5-dimethyl-2,3,4,5-tetrahydro-1*H-*benzo[*d*]azepin-7-yl)-tetrahydrofuran-3-carbonsäureamid.

Gegenstand der Erfindung sind auch physiologisch verträgliche Salze der Verbindungen gemäß den vorhin definierten Ausführungsformen und Beispiele. Gegenstand der Erfindung sind auch Arzneimittel, enthaltend eine Verbindung oder ein physiologisch verträgliches Salz einer Verbindung gemäß den vorhin definierten Ausführungsformen und Beispiele, neben gegebenenfalls einem oder mehreren inerten Trägerstoffen und/oder Verdünnungsmitteln.

Gegenstand der Erfindung ist auch die Verwendung einer Verbindung oder ein physiologisch verträgliches Salz einer Verbindung gemäß den vorhin definierten Ausführungsformen und Beispiele, zur Herstellung eines Arzneimittels mit einem inhibitorischen Effekt auf Faktor Xa und/oder einem inhibitorischen Effekt auf verwandte Serinproteasen.

Gegenstand der Erfindung ist auch ein Verfahren zur Herstellung eines Arzneimittels, dadurch gekennzeichnet, dass auf nichtchemischem Wege eine Verbindung oder ein physiologisch verträgliches Salz einer Verbindung gemäß den vorhin definierten Ausführungsformen und Beispiele in einen oder mehrere inerte Trägerstoffe und/oder Verdünnungsmittel eingearbeitet wird.

Erfindungsgemäß erhält man die Verbindungen der allgemeinen Formel (I) nach an sich bekannten Verfahren, beispielsweise nach folgenden Verfahren:

### (a) Die Herstellung einer Verbindung der allgemeinen Formel (la)

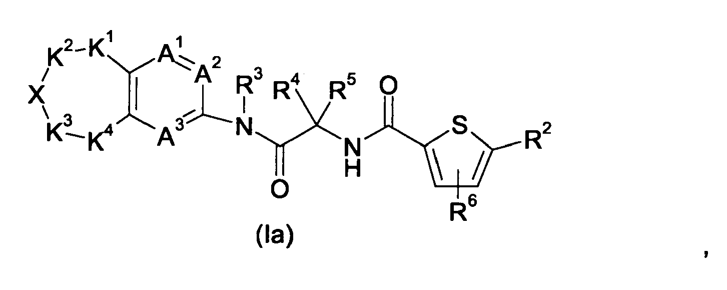

in der A¹bis A³, K¹bis K⁴, X und R¹ bis R⁶ wie in Ausführungsform 1 erwähnt definiert sind,
und die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis" beschriebenen geschützt sein kann und deren Schutzgruppen in literaturbekannterweise abgespalten werden können.
wird in den Ausführungsbeispielen beschrieben oder kann beispielsweise nach einem der folgenden Formelschemata 1 und 2 durchgeführt werden: 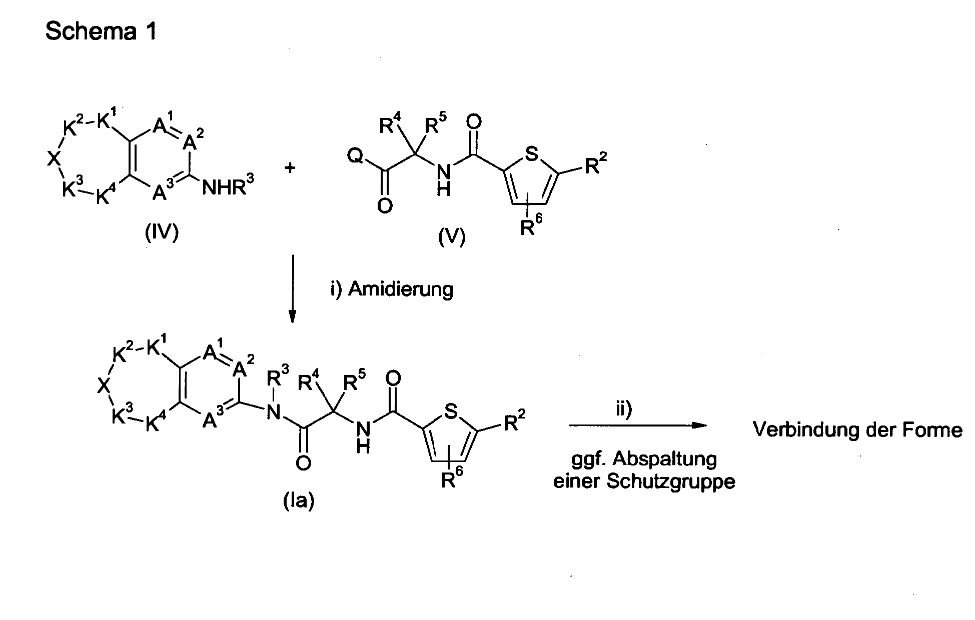 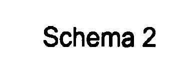 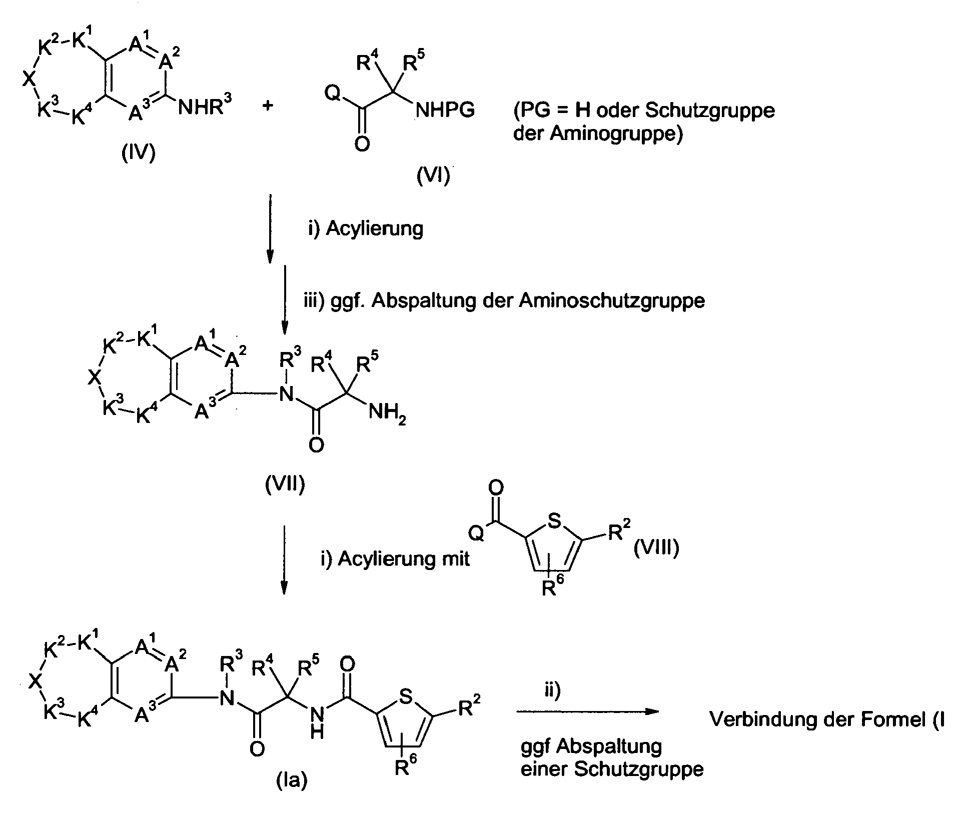 wobei
Q eine Hydroxy- oder C₁₋₄-Alkyloxygruppe, ein Halogenatom oder eine Alkyloxycarbonyloxy- oder Acyloxygruppe darstellt und
PG ein Wasserstoffatom oder eine literaturbekannte Schutzgruppe der Aminofunktion wie beispielsweise eine tert.-Butoxycarbonyl-, Benzyloxycarbonyl- oder eine Trifluoracetylgruppe darstellt.

Die in Schema 1 und 2 beschriebenen Reaktionsstufen i) -iii) können auf die in den Beispielen beschriebene Weise oder nach literaturbekannten Bedingungen beispielsweise wie folgt durchgeführt werden:
i) Acylierung eines Amins (IV) oder (VII) mit einer gegebenenfalls aktivierten Carbonsäure (V) beziehungsweise (VI) oder (VIII)

Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 160°C, durchgeführt.

Die Acylierung kann jedoch auch mit der freien Säure gegebenenfalls in Gegenwart eines die Säure aktivierenden Mittels oder eines wasserentziehenden Mittels, beispielsweise in Gegenwart von Chlorameisensäureisobutylester, Thionylchlorid, Trimethylchlorsilan, Chlorwasserstoff, Schwefelsäure, Methansulfonsäure, *p*-Toluolsulfonsäure, Phosphortrichlorid, Phosphorpentoxid, 2-Ethoxy-1-ethoxycarbonyl-1,2-dihydrochinolin (EEDQ), *N,N'*-Dicyclohexylcarbodümid, *N,N'*-Dicyclohexylcarbodiimid/Camphersulfonsäure, *N,N'*-Dicyclohexylcarbodiimid/*N-*Hydroxysuccinimid oder 1-Hydroxy-benztriazol, *N,N'*-Carbonyldiimidazol, *O*-(Benzotriazol-1-yl)-*N,N,N',N'*-tetramethyluroniumtetrafluorborat/*N-*Methylmorpholin, *O-*(Benzotriazol-1-yl)-*N,N,N',N'*-tetramethyl-uroniumtetrafluorborat/N-Ethyldiisopropylamin, *O-*Pentafluorophenyl-*N,N,N',N'-*tetramethyluronium-hexafluorophosphat/Triethylamin, *N,N'*-Thionyldümidazol oder Triphenylphosphin/Tetrachlorkohlenstoff, bei Temperaturen zwischen -20 und 200°C, vorzugsweise jedoch bei Temperaturen zwischen -10 und 160°C, durchgeführt werden.

Die Acylierung kann auch mit einem Carbonsäureester (V) oder (VI) und dem Amin (IV) durch Aktivierung mit Trimethylaluminium durchgeführt werden.

Die Acylierung einer Verbindung der allgemeinen Formel (IV) kann aber auch mit einem reaktiven Carbonsäurederivat der allgemeinen Formel (IX) 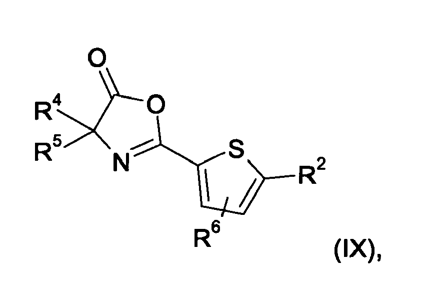 in der R⁴ bis R⁶ und R² wie in Ausführungsform 1 erwähnt definiert sind durchgeführt werden. Die Acylierung wird dann zweckmäßigerweise in einem Lösungsmittel wie beispielsweise Toluol, Tetrahydofuran oder Dimethylformamid unter Zusatz einer Säure wie Essigsäure oder Camphersulfonsäure oder gegebenenfalls in Gegenwart einer Lewis-Säure wie Zinkchloridoder Kupfer(II)chlorid und gegebenenfalls durch Zusatz von Aminbasen wie beispielsweise Diisopropylethylamin, Triethylamin oder N-Methylmorpholin bei Temperaturen zwischen -10 und 100°C, beispielsweise unter Verwendung eines Mikrowellenofens oder wie in P.Wipf et al., Helvetica Chimica Acta, 69, 1986, 1153 beschrieben, durchgeführt.

Verbindungen der allgemeinen Formel (IX) können aus Verbindungen der allgemeinen Formel (V) zweckmäßigerweise in einem Lösungsmittel oder Lösungsmittelgemisch wie Dichlormethan, Trichlormethan, Tetrachlorkohlenstoff, Benzol, Chlorbenzol, Toluol, Xylol, Hexamethyldisiloxan, Ether, Tetrahydrofuran, Dioxan, Acetonitril, Pyridin, gegebenenfalls in Gegenwart von *N*,*N*'*-*Dicyclohexylcarbodiimid, *N*,*N'*-Dicyclohexylcarbodiimid/*N-*Hydroxysuccinimid oder 1-Hydroxy-benzotriazol, *N,N'*-Carbonyldiimidazol, *O-*(Benzotriazol-1-yl)-*N,N,N',N'*-tetramethyl-uroniumtetrafluorborat/*N-*Methylmorpholin, *O-*(Benzotriazol-1-yl)-*N,N,N',N'*-tetramethyl-uroniumtetrafluorborat/*N-*Ethyldiisopropylamin, oder in Acetanhydrid bei Temperaturen zwischen -20 und 200°C, vorzugsweise jedoch bei Temperaturen zwischen -10 und 100°C, hergestellt werden.

Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995 oder auch im Houben-Weyl Ergänzungsband 22, Thieme Verlag, 2003 und der dort zitierten Literatur beschrieben.

### ii) bzw. iii) Abspaltung einer Schutzgruppe

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung ener Schutzgruppe kann aber auch nach den in T.W. Greene, P.G.M. Wuts in "Protective Groups in Organic Synthesis" beschriebenen Verfahren durchgeführt werden.

### (b) Die Bausteine der allgemeinen Formel

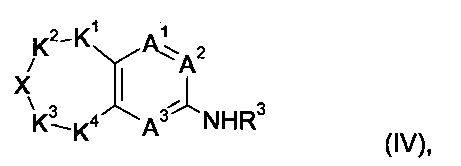

in denen A¹, A², A³, K¹, K², K³, K⁴, X und R³ wie in Ausführungsform 1 erwähnt definiert sind, und
die gegebenenfalls an vorhandenen Amino-, Hydroxy-, Carboxy-oder Thiolgruppen durch gängige Schutzgruppen, wie beispielsweise denen in T.W. Greene, P.G.M. Wuts in "Protective Groups in Organic Synthesis" beschriebenen geschützt sein können, und deren Schutzgruppen in literaturbekannterweise im Verlauf der Synthesesequenz zu Verbindungen der Formel (I) abgespalten werden können,
sind literaturbekannt, oder deren Synthese wird in den Ausführungsbeispielen beschrieben, oder sie können beispielsweise nach literaturbekannten Syntheseverfahren oder in Analogie zu literaturbekannten Syntheseverfahren wie beispielsweise in DE4429079, US4490369, DE3515864, US5175157, DE1921861, WO85/00808 bzw. in G. Bobowski et al., J.Heterocyclic Chem. 16, 1525, 1979 oder in P.D. Johnson et al., Bioorg. Med. Chem. Lett 2003, 4197, beschrieben hergestellt werden. Im Azepinteil verbrückte Fragmente wie in Formel II-1 oder II-2 dargestellt können beispielsweise analog zu J.W. Coe et al. J. Med. Chem., 2005, 48, 3474 oder J.W. Coe et al. US Patent application US2005/0020616 hergestellt werden.

Die in Formel II-3 bzw. II-4 dargestellten Benzazepinmodifikationen können beispielsweise durch Oxidation mittels meta-Chlorperbenzoesäure bzw. Alkylierung mit einem Alkylhalogenid aus geeigneten Benzepinvorstufen wie im experimentellen Teil ausgeführt hergestellt werden

Beispielsweise kann eine Verbindung der allgemeinen Formel (IV), in der R³ ein Wasserstoffatom bedeutet und A1, A2, A3, K1, K2, K3, K4 und X wie in Ausführungsform 1 erwähnt definiert sind durch Reduktion der Nitrogruppe einer Verbindungen der allgemeinen Formel (III) 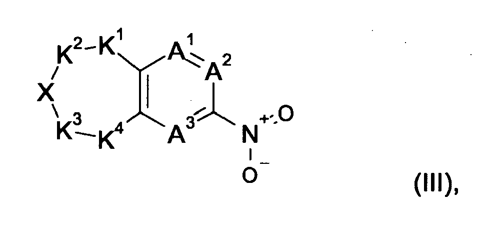 in der A1, A2, A3, K1, K2, K3, K4 und X wie in Ausführungsform 1 erwähnt definiert sind, wie folgt hergestellt werden.

Die Reduktion der Nitrogruppe wird beispielsweise zweckmäßigerweise in einem Lösungsmittel oder Lösungsmittelgemisch wie Wasser, wäßriger Ammoniumchlorid-Lösung, Salzsäure, Schwefelsäure, Phosphorsäure, Ameisensäure, Essigsäure, Acetanhydrid mit unedlen Metallen wie Eisen, Zink, Zinn oder Schwefelverbindungen wie Ammoniumsulfid, Natriumsulfid oder Natriumdithionit oder durch katalytische Hydrierung mit Wasserstoff, beispielsweise unter einem Druck zwischen 0.5 und 100 bar, vorzugsweise jedoch zwischen 1 und 50 bar, oder mit Hydrazin als Reduktionsmittel, zweckmäßigerweise in Gegenwart eines Katalysators wie beispielsweise Raney-Nickel, Palladiumkohle, Platinoxid, Platin auf Mineralfaser oder Rhodium, oder mit komplexen Hydriden wie Lithiumaluminiumhydrid, Natriumborhydrid, Natriumcyanborhydrid, Diisobutylaluminiumhydrid, zweckmäßigerweise in einem Lösungsmittel oder Lösungsmittelgemisch wie Wasser, Methanol, Ethanol, Isopropanol, Pentan, Hexan, Cyclohexan, Heptan, Benzol, Toluol, Xylol, Ethylacetat, Methylpropionat, Glykol, Glykoldimethylether, Diethylenglykoldimethylether, Dioxan, Tetrahydrofuran, *N-*Methylpyrrolidinon, oder aber *N-*Ethyl-diisopropylamin, *N-*C₁₋₅-Alkylmorpholin, *N-*C₁₋₅-Alkylpiperidin, *N-*C₁₋₅-Alkylpyrrolidin, Triethylamin, Pyridin, beispielsweise bei Temperaturen zwischen -30 und 250°C, vorzugsweise jedoch zwischen 0 und 150°C, durchgeführt.

### (c) Die Bausteine der allgemeinen Formel

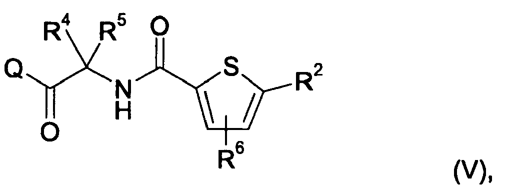

in denen R⁴, R5, R⁶ und R² wie in Ausführungsform 1 erwähnt definiert sind, und wobei Q eine Hydroxy- oder C₁₋₄-Alkyloxygruppe, ein Halogenatom oder eine Alkyloxycarbonyloxy- oder Acyloxygruppe darstellt die gegebenenfalls an vorhandenen Amino-, Hydroxy-, Carboxy-oder Thiolgruppen durch gängige Schutzgruppen wie beispielsweise denen in T.W. Greene, P.G.M. Wuts in "Protective Groups in Organic Synthesis" beschriebenen geschützt sein können und deren Schutzgruppen in literaturbekannterweise im Verlauf der Synthesesequenz zu Verbindungen der Formel (I) abgespalten werden können, sind literaturbekannt, oder deren Synthese wird in den Ausführungsbeispielen beschrieben, oder sie können nach literaturbekannten Syntheseverfahren oder in Analogie zu literaturbekannten Syntheseverfahren wie beispielsweise in WO04/4613 beschrieben, hergestellt werden.

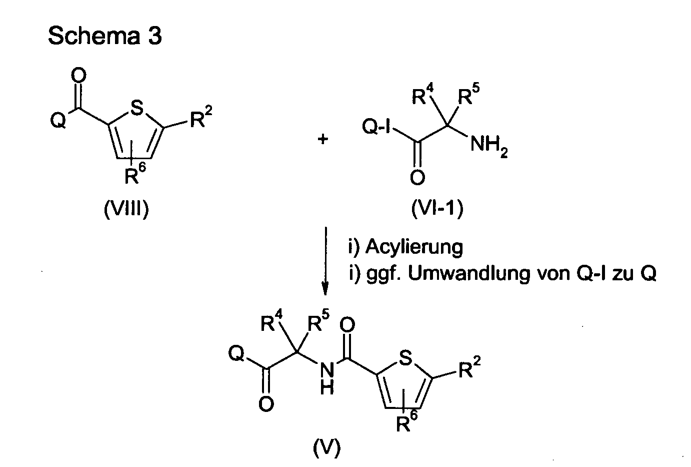

Beispielsweise können sie auch nach Schema 3 durch Umsetzung einer Verbindung (VIII) mit einem Amin (VI-1) hergestellt werden, wobei Q eine Hydroxy- oder C₁₋₄-Alkyloxygruppe, ein Halogenatom oder eine Alkyloxycarbonyloxy- oder Acyloxygruppe darstellt und Q-I eine Hydroxy oder C₁₋₄-Alkyloxygruppe darstellt, die nach dem Acylierungsschritt gegebenenfalls durch Verseifung und Aktivierung wie oben beschrieben in Q überführt werden kann. Die Acylierung kann nach den oben beschriebenen Acylierungsbedingungen durchgeführt werden.

Die Aminosäurederivate (VI-1) sind literaturbekannt oder können in Analogie zu literaturbekannten Verfahren wie sie beispielsweise in den Beispielen ausgeführt sind aus kommerziell erhältlichen Aminosäurederivaten hergestellt werden.

Bei den vorstehend beschriebenen Umsetzungen können gegebenenfalls vorhandene reaktive Gruppen wie Hydroxy-, Carboxy-, Amino-, Alkylamino-oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, *tert*.-Butyl-, Trityl-, Benzyl- oder Tetrahydropyranylgruppe in Betracht.

Beispielsweise kommt als Schutzrest für eine Carboxylgruppe die Trimethylsilyl-, Methyl-, Ethyl-, *tert*.-Butyl-, Benzyl- oder Tetrahydropyranylgruppe in Betracht.

Beispielsweise kommt als Schutzrest für eine Amino-, Alkylamino- oder Iminogruppe die Acetyl-, Trifluoracetyl-, Benzoyl-, Ethoxycarbonyl-, *tert*.-Butoxycarbonyl-, Benzyloxycarbonyl-, Benzyl-, Methoxybenzyl- oder 2,4-Dimethoxybenzylgruppe, und für die Aminogruppe zusätzlich die Phthalylgruppe in Betracht.

Beispielsweise kommt als Schutzrest für eine Ethinylgruppe die Trimethylsilyl-, Diphenylmethylsilyl-, tert.Butyldimethylsilyl- oder eine 1-Hydroxy-1-methyl-ethylgruppe in Betracht.

Weitere Schutzgruppen die eingesetzt werden können und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder *n-*Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel (I) in ihre Enantiomeren und/oder Diastereomeren aufgetrennt werden.

So lassen sich beispielsweise die erhaltenen Verbindungen der allgemeinen Formel (I), welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel (I) mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können. Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-*o*-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel (I), falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel (I) sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor VIIa, Faktor IX, Faktor XI und Faktor XII.

Die im Experimentellen Teil angeführten Verbindungen wurden auf ihre Wirkung auf die Hemmung des Faktors Xa wie folgt untersucht:

### Methodik:

Enzymkinetische Messung mit chromogenem Substrat. Die durch humanen Faktor Xa aus dem farblosen chromogenen Substrat freigesetzte Menge an *p-*Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material:

Tris(hydroxymethyl)-aminomethan-Puffer (100 mmol) und Natriumchlorid (150 mmol), pH 8.0 plus 1 mg/ml Human Albumin Fraction V, Proteasefrei
Faktor Xa (Calbiochem), Spez. Aktivität: 217 IU/mg, Endkonzentration: 7 IU/ml pro Reaktionsansatz
Substrat S 2765 (Chromogenix), Endkonzentration: 0.3 mM/l (1 KM) pro Reaktionsansatz
Testsubstanz: Endkonzentration 100, 30, 10, 3, 1, 0.3, 0.1, 0.03, 0.01, 0.003, 0.001 µMol/l

Durchführung: 10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Ausweitung:

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigten IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulcerativen Colitis, der Prophylaxe und Behandlung der Koronarthrombose, der Prophylaxe des Schlaganfalls und der Verhinderung der Occlusion von Shunts.

Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von Fibrin-abhängigen Gewebsadhäsionen und/oder Narbengewebebildung sowie zur Förderung von Wundheilungsprozessen geeignet.

Aufgrund ihrer pharmakologischen Eigenschaften eignen sich außerdem die neuen Verbindungen und deren physiologisch verträgliche Salze zur Behandlung der Alzheimer- und Parkinson'schen Krankheit. Eine Rationale dafür ergibt sich zum Beispiel aus folgende Befunden, aus denen man schließen kann, dass Thrombinhemmer bzw. Faktor Xa Hemmer, durch Hemmung der Thrombinbildung bzw. -aktivität, wertvolle Medikamente in der Behandlung der Alzheimer- und Parkinson'schen Krankheit darstellen könnten. Klinische und experimentelle Studien legen nahe, dass neurotoxische Mechanismen, beispielsweise die mit der Aktivierung von Proteasen der Gerinnungskaskade einhergehende Entzündung, beteiligt ist am Absterben von Neuronen infolge von Hirntraumata. Verschiedene Studien deuten auf eine Beteiligung von Thrombin bei neurodegenerativen Prozessen hin, beispielsweise infolge eines Schlaganfalls, wiederholter Bypassoperation oder traumatischen Hirnverletzungen. Eine erhöhte Thrombinaktivität konnte beispielsweise noch Tage nach peripherer Nervenverletzung nachgewiesen werden. Es konnte weiterhin gezeigt werden, dass Thrombin eine Neuritenretraktion, sowie Glia-Proliferation, und Apoptose in Primärkulturen von Neuronen und Neuroblastomzellen hervorruft (zur Übersicht siehe: Neurobiol. Aging 2004, 25(6), 783-793). Darüberhinaus deuten verschiedene in vitro Studien an Gehirnen von Patienten mit Alzheimer-Krankheit daruf hin, dass Thrombin in der Pathogenese dieser Krankheit eine Rolle spielt (Neurosci. Lett. 1992, 146, 152-54). Eine Anreicherung immunreaktiven Thrombins konnte in Neuriten-Plaques in Gehirnen von Alzheimer-Patienten nachgewiesen werden. In vitro wurde gezeigt, dass Thrombin ebenfalls eine Rolle bei der Regulation und Stimulation der Produktion des "Amyloid Precursor Proteins" (APP) spielt sowie bei der Spaltung des APP in Fragmente, welche in den Amyloid-Plaques im Gehirn von Alzheimer-Patienten nachgewiesen werden können. Weiterhin konnte gezeigt werden, dass die thrombin-induzierte mikrogliale Aktivierung in vivo zur Degeneration von nigralen dopaminergen Neuronen führt. Diese Befunde lassen den Schluss zu, dass mikrogliale Aktivierung -ausgelöst durch endogene Substanz(en) wie beispielsweise Thrombin- beteiligt sind am neuropathologischen Prozess des Zelltodes dopaminerger Neurone, wie er bei Patienten mit Parkinson'scher Krankheit vorkommt (J. Neurosci. 2003, 23, 5877-86).

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel (I), gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

### Experimenteller Teil

Die nachfolgenden Beispiele sollen die Erfindung näher erläutern, ohne diese jedoch in ihrem Umfang zu beschränken.

Für die hergestellten Verbindungen liegen in der Regel Schmelzpunkte und/oder IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX*^{™}*, 35-70 µm) und RP-Material von der Firma Varian (Microsorb, 60-8µm;C18 Dynamax) verwendet. Falls nähere Angaben zur Konfiguration fehlen, bleibt offen, ob es sich um reine Stereoisomere oder um Enantiomeren-/Diastereomerengemische handelt.

In den Versuchsbeschreibungen werden die folgenden Abkürzungen verwendet:

| | |
|---|---|
| BOC | tert.-Butoxycarbonyl |
| DIPEA | *N-*Ethyl-diisopropylamin |
| DMF | *N*,*N-*Dimethylformamid |
| ges. | gesättigt |
| h | Stunde(n) |
| HATU | *O-*(7-Azabenzotriazol-1-yl)-*N,N,N',N'*-tetramethyluronium-hexafluorphosphat |
| NaHMDS | Natriumhexamethyldisilazid |
| i. Vak. | im Vakuum |
| konz. | konzentriert |
| min | Minute(n) |
| NMM | *N-*Methyl-morpholin |
| R_{f} | Retentionsfaktor |
| Rt | Retentionszeit |
| TBTU | *O-*(Benzotriazol-1-yl)-*N,N,N',N'*-tetramethyluroniumtetrafluorborat |
| TEA | Triethylamin |
| TFA | Trifluoressigsäure |
| THF | Tetrahydrofuran. |

Unter dem Begriff "thiophen-2-yl" oder "thien-2-yl" ist der im Kasten gezeichnete Rest zu verstehen:

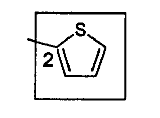

Die HPLC-MS Daten wurden unter den folgenden Bedingungen erzeugt:
Methode 1:
   Waters ZQ2000 Massen Spektrometer, Gilson 215 Autosampler, HP1100 HPLC und Diodenarraydetektor

Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.00 |
| 0.40 | 95 | 5 | 1.00 |
| 4.00 | 2 | 98 | 1.00 |
| 4.35 | 2 | 98 | 1.00 |
| 4.50 | 95 | 5 | 1.00 |

Als stationäre Phase diente eine Säule X-Terra MS C18, 3.5 µm, 4.6 mm x 50 mm.

Die Diodenarraydetektion erfolgte im Wellenlängenbereich 210-500 nm.

### Methode 2:

Waters Alliance 2695, PDA Detektor 2996, ZQ 2002

Als mobile Phase wurde eingesetzt:
A: Wasser mit 0.10% TFA
B: Acetonitril mit 0.10% TFA

| Zeit in min | %A | %B | Flussrate in ml/min |
|---|---|---|---|
| 0.00 | 95 | 5 | 1.00 |
| 0.10 | 95 | 5 | 1.00 |
| 3.10 | 2 | 98 | 1.00 |
| 4.50 | 2 | 98 | 1.00 |
| 5.00 | 95 | 5 | 1.00 |

Als stationäre Phase diente eine Säule Waters X-Terra MS C18, 2.5 µm, 4.6 mm x 30 mm

### Methode 3:

Waters Alliance 2695, PDA Detektor 2996

Als mobile Phase wurde eingesetzt:
A: Wasser mit 0.13% TFA
B: Acetonitril mit 0.10% TFA

| Zeit in min | %A | %B | Flussrate in ml/min |
|---|---|---|---|
| 0.00 | 95 | 5 | 1.00 |
| 0.75 | 95 | 5 | 1.00 |
| 5.25 | 2 | 98 | 1.00 |
| 5.75 | 2 | 98 | 1.00 |
| 6.05 | 95 | 5 | 1.00 |
| 6.55 | 95 | 5 | 1.00 |

Als stationäre Phase diente eine Säule Varian Microsorb 100 C18; 3,5µm; 4,6 mm x 50 mm

### Methode 3a

Waters Alliance 2695, PDA Detektor 2996

Als mobile Phase wurde eingesetzt:
A: Wasser mit 0. 1 % TFA
B: Acetonitril mit 0.1% TFA

| Zeit in min | %A | %B | Flussrate in ml/min |
|---|---|---|---|
| 0.00 | 95 | 5 | 1.00 |
| 0.1 | 95 | 5 | 1.00 |
| 3.1 | 2 | 98 | 1.00 |
| 4.5 | 2 | 98 | 1.00 |
| 5.0 | 95 | 5 | 1.00 |

Als stationäre Phase diente eine Säule Varian Microsorb 100 C18; 2,5µm; 4,6 mm x 30 mm

### Methode 4:

### Agilent 1100

Als mobile Phase wurde eingesetzt:
A: Wasser mit 0.10% Ameisensäure
B: Acetonitril mit 0.10% Ameisensäure

| Zeit in min | %A | %B | Flussrate in ml/min |
|---|---|---|---|
| 0.00 | 95 | 5 | 1.60 |
| 4.50 | 10 | 90 | 1.60 |
| 5.00 | 10 | 90 | 1.60 |
| 5.50 | 90 | 10 | 1.60 |

Als stationäre Phase diente eine Säule Zorbax StableBond C18; 3,5µm ; 4,6 mm x 75 mm

### Methode 5:

Waters Alliance 2695, PDA Detektor 2996

Als mobile Phase wurde eingesetzt:
A: Wasser mit 0.1% TFA
B: Acetonitril mit 0. 1 % TFA

| Zeit in min | %A | %B | Flussrate in ml/min |
|---|---|---|---|
| 0.00 | 95 | 5 | 5.00 |
| 0.20 | 95 | 5 | 5.00 |
| 1.35 | 2 | 98 | 5.00 |
| 1.55 | 2 | 98 | 5.00 |
| 1.65 | 95 | 5 | 5.00 |
| 1.85 | 95 | 5 | 5.00 |

Als stationäre Phase diente eine Säule Interchim HS Strategy 5 C18-2; 5µm; 4,6 mm x 50 mm

### Methode 6:

Waters Alliance 26905, PDA Detektor 996

Als mobile Phase wurde eingesetzt:
A: Wasser mit 0. 1 % TFA
B: Acetonitril mit 0.1 % TFA

| Zeit in min | %A | %B | Flussrate in ml/min |
|---|---|---|---|
| 0.00 | 95 | 5 | 2.00 |
| 0.10 | 95 | 5 | 2.00 |
| 2.10 | 2 | 98 | 2.00 |
| 3.00 | 2 | 98 | 2.00 |
| 3.25 | 95 | 5 | 2.00 |

Als stationäre Phase diente eine Säule MerckChromolith SpeedRod RP-18e; 4,6 mm x 50 mm

### Beispiel 1

### 3-[(5-Brom-thiophen-2-yl)-carbonylamino]-N-(3-methyl-2,3,4,5-tetrahydro-1H-benzo[d]azepin-7-yl)-tetrahydrofuran-3-carbonsäureamid

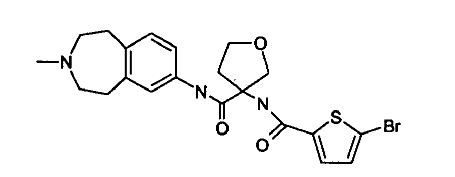

### (a) 7-Nitro-2,3,4,5-tetrahydro-1H-benzo[d][azepin

8.4 g (29.0 mmol) 3-Trifluoracetyl-7-nitro-2,3,4,5-tetrahydro-1*H*-benzo[*d*]azepin werden unter Stickstoffatmosphäre in 80 ml Methanol suspendiert und mit 5 ml NaOH-Lösung (50%) versetzt und 2 h bei 70°C gerührt.

Das Methanol wird am Rotationsverdampfer abdestilliert, der Rückstand mit Wasser versetzt und mit *tert*.-Butylethylether extrahiert. Die organische Phase wird mit NaOH-Lösung (50%) und ges. Natriumchlorid-Lösung gewaschen, über Natriumsulfat getrocknet und i. Vak. bis zur Trockene eingeengt.
Ausbeute: 5.1 g (91%)
R_{f}-Wert: 0.28 (Aluminiumoxid; Dichlormethan/Ethanol = 95:5)
C₁₀H₁₂N₂O₂ (192.22)
Massenspektrum: (M+H)⁺ = 193

### (b) 3-Methyl-7-nitro-2,3,4,5-tetrahydro-1H-benzo[d]azepin

5.0 g (26.0 mmol) 7-Nitro-2,3,4,5-tetrahydro-1*H*-benzo[*d*][azepin werden in 9.8 ml (260.1 mol) Ameisensäure unter Rühren bei Raumtemperatur mit 15.5 ml (208.1 mmol) Formalin-Lösung in Wasser (37%) versetzt und bei 70°C über Nacht gerührt. Die Reaktionsmischung wird unter Eisbadkühlung mit NaOH-Lösung (50%) alkalisch gestellt und mit *tert*.-Butylmethylether extrahiert. Die organische Phase wird über Natriumsulfat getrocknet und i. Vak bis zur Trockene eingeengt.
Ausbeute: 4.8 g (90%)
R_{f}-Wert: 0.65 (Aluminiumoxid; Dichlormethan/Ethanol = 95:5)
C₁₁H₁₄N₂O₂ (206.24)
Massenspektrum: (M+H)⁺ = 207

### (c) 3-Methyl-2,3,4,5-tetrahydro-1H-benzo[d]azepin-7-ylamin

4.8 g (23.2 mmol) 3-Methyl-7-nitro-2,3,4,5-tetrahydro-1*H*-benzo[*d*]azepin werden in 45 ml Methanol gelöst und mit 400 mg Pd/C 10% versetzt. Man hydriert in einer Parr-Apparatur bei Raumtemperatur bei 3 bar Wasserstoff-Druck für 5 Stunden. Anschließend wird der Katalysator abfiltriert und das Filtrat i. Vak. eingeengt.
Ausbeute: 3.9 g (96 %)
R_{f}-Wert: 0.36 (Aluminiumoxid; Dichlormethan/Ethanol = 98:2)
C₁₁H₁₆N₂ (176.26)
Massenspektrum: (M+H)⁺ = 177

### (d) 3-Amino-tetrahydro-furan-3-carbonsäure-hydrochlorid

3.5 g (15.1 mmol) 3-*tert*.-Butoxycarbonylamino-tetrahydro-furan-3-carbonsäure werden in 150 ml 1-molarer Salzsäure gelöst und 1h bei Raumtemperatur gerührt. Anschließend lyophilisiert man das Reaktionsgemisch.
Ausbeute: 2.5 g (100 %)
C₅H₉NO₃*HCl (167.59)
Massenspektrum: (M+H)⁺ = 132

### (e) 3-[(5-Brom-thiophhen-2-yl)-carbonylamino]-tetrahydro-furan-3-carbonsäure

3.1 g (14.9 mmol) 5-Brom-thiophen-2-carbonsäure werden in 50 ml Dichlormethan unter Rühren bei Raumtemperatur mit 5.4 ml (74.6 mmol) Thionylchlorid versetzt und 3.5 h unter Rückfluß gerührt. Anschließend engt man das Reaktionsgemisch bis zur Trockene ein.

2.5 g (14.9 mmol) 3-Amino-tetrahydro-furan-3-carbonsäure-hydrochlorid werden in 2.0 ml (14.9 mmol) TEA und 150 ml Acetonitril gelöst und unter Rühren mit 5.9 ml (22.4 mmol) *N*,*O-*bis-(trimethylsilyl)-trifluor-acetamid versetzt und 4 h unter Rückfluß gerührt. Die Reaktionsmischung wird mit 4.1 ml (29.8 mmol) TEA und der Lösung des hergestellten Säurechlorides in 50 ml Acetonitril versetzt, 15 min unter Rückfluß gerührt und dann langsam auf Raumtemperatur abgekühlt. Anschließend wird i.Vak. bis zur Trockene eingeengt, der Rückstand mit Wasser und 2-molarer Natriumcarbonat-Lösung versetzt und mit Diethylether gewaschen. Die wässrige Phase wird mit 20 ml konz. Salzsäure auf pH 1 gestellt, der Niederschlag abgesaugt und bei 50°C im Vakuumtrockenschrank getrocknet.
Ausbeute: 3.6 g (75 %)
C₁₀H₁₀BrNO₄S (320.16)
Massenspektrum: (M-H)⁻ = 318/320 (Bromisotope)

### (f) 3-[(5-Brom-thiophen-2-yl)-carbonylamino]-N-(3-methyl-2,3,4,5-tetrahydro-1H-benzo[d]azepin-7-yl)-tetrahydrofuran-3-carbonsäureamid

700.0 mg (2.19 mmol) 3-[(5-Brom-thiophen-2-yl)-carbonylamino]-tetrahydrofuran-3-carbonsäure werden in 10 ml DMF unter Rühren bei Raumtemperatur mit 890.0 mg (2.34 mmol) HATU und 601.0 µl (5.47 mmol) NMM versetzt und 10 min gerührt. Anschließend werden 385.0 mg (2.19 mmol) 3-Methyl-2,3,4,5-tetrahydro-1*H*-benzo[*d*]azepin-7-ylamin zugegeben und die Mischung über Nacht bei 65°C gerührt. Die Reaktionsmischung wird mit Wasser und ges. Natriumhydrogencarbonat-Lösung versetzt, der Niederschlag abfiltriert und chromatographisch an Aluminiumoxid (Laufmittel: Dichlormethan/Ethanol 100:0 bis 98:2) gereinigt.
Ausbeute: 850.0 mg (81 %)
R_{f}-Wert: 0.62 (Aluminiumoxid; Dichlormethan/Ethanol = 95:5)
C₂₁H₂₄BrN₃O₃S (478.40)
Massenspektrum: (M+H)⁺ = 478/480 (Bromisotope)

### Beispiel 2

### 3-[(5-Chlor-thiophen-2-yl)-carbonylamino]-N-(3-methyl-2,3,4,5-tetrahydro-1H-benzo[d]azepin-7-yl)-tetrahydrofuran-3-carbonsäureamid

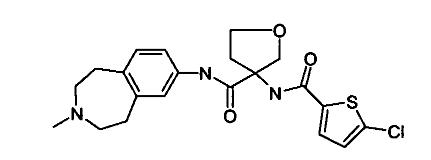

### (a) 3[(5-Chlor-thiophen-2-yl)-carbonylamino]-tetrahydro-furan-3-carbonsäure-benzylester

1.59 g (9.8 mmol) 5-Chlor-thiophen-2-carbonsäure wird in 30 ml DMF gelöst und mit 3.61 g (10.7 mmol) 3-Amino-tetrahydro-furan-3-carbonsäure-benzylester und 3.46 g (10.8 mmol) TBTU und 4.3 ml (39 mmol) NMM bei Raumtemperatur 20 h gerührt. Anschließend wird eingeengt und durch Chromatographie an Kieselgel (Laufmittel: Dichlormethan/Ethanol 100:0 bis 94:6) gereinigt.
Ausbeute: quantitativ
R_{f}-Wert: 0.59 (Kieselgel; Dichlormethan/Ethanol = 9:1)
C₁₇H₁₆ClNO₄S (365.83)
Massenspektrum: (M+H)⁺ = 366/368 (Chlorisotope)

### (b) 3-[(5-Chlor-thiophen-2-yl)-carbonylamino]-tetrahydro-furan-3-carbonsäure

3.6 g (9.8 mmol) 3-[(5-Chlor-thiophen-2-yl)-carbonylamino]-tetrahydro-furan-3-carbonsäure-benzylester werden in 60 ml Ethanol gelöst und mit 39.1 ml (39.1 mmol) 1-molarer wässriger Natronlauge versetzt und bei Raumtemperatur für 6 h gerührt. Nach Einengen i. Vak. wird der Rückstand unter Eisbadkühlung mit 1-molarer wässriger Salzsäure versetzt, der Niederschlag abgesaugt und bei 60°C im Vakuumtrockenschrank getrocknet.
Ausbeute: 2.5 g (91 %)
R_{f}-Wert: 0.13 (Kieselgel; Dichlormethan/Ethanol 9:1)
C₁₀H₁₀ClNO₄S (275.71)
Massenspektrum: (M-H)⁻ = 274/276 (Chlorisotope)

### (c) 3-[(5-Chlor-thiophen-2-yl)-carbonylamino]-N-(3-methyl-2,3,4,5-tetrahydro-1H-benzo[d]azepin-7-yl)-tetrahydrofuran-3-carbonsäureamid

Hergestellt analog Beispiel 2(a) aus 3-[(5-Chlor-thiophen-2-yl)-carbonylamino]-tetrahydro-furan-3-carbonsäure und 3-Methyl-2,3,4,5-tetrahydro-1*H-*benzo[*d*]azepin-7-ylamin mit TBTU und TEA in THF bei Raumtemperatur mit anschließender Reinigung durch Chromatographie mit Aluminiumoxid (Laufmittel: Dichlormethan/Ethanol 100:0 bis 97:3).
Ausbeute: 67 %
R_{f}-Wert: 0.63 (Aluminiumoxid; Dichlormethan/Ethanol = 95:5)
C₂₁H₂₄ClN₃O₃S (433.95)
Massenspektrum: (M+H)⁺ = 434/436 (Chlorisotope)

### Beispiel 3

### 5-Chlor-thiophen-2-carbonsäure-N-[1-(3-cyclopropyl-2,3,4,5-tetrahydro-1H-benzo[d]azepin-7-ylcarbamoyl)-1-methyl-ethyl]-amid

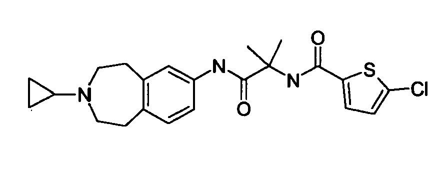

### (a) 2-(5-Chlor-thiophen-2-yl)-4,4-dimethyl-4H-oxazol-5-on

1.0 g (4.0 mmol) 2-[(5-Chlor-thiophen-2-yl)-carbonylamino]-2-methyl-propionsäure in 30 ml Essigsäureanhydrid werden 1h bei 85°C gerührt. Anschließend engt man das Reaktionsgemisch bis zur Trockene ein.
Ausbeute: 927.3 mg (100 %)
C₉H₈ClNO₂S (229.68)
Massenspektrum: (M+H)⁺ = 230

### (b) 5-Chlor-thiophen-2-carbonsäure-N-[1-(3-cyclopropyl-2,3,4,5-tetrahydro-1H-benzo[d]azepin-7-ylcarbamoyl)-1-methyl-ethyl]-amid

200.0 µl (10.0 µmol) einer 0.05 molaren 2-(5-Chlor-thiophen-2-yl)-4,4-dimethyl-4*H-*oxazol-5-on-Lösung in Toluol/Essigsäure 9:1 werden mit 200.0 µl (10.0 µmol) einer 0.05-molaren 3-Cyclopropyl-2,3,4,5-tetrahydro-1*H*-benzo[*d*]azepin-7-ylamin-Lösung in DMF und 1.7 µl (10.0 µmol) DIPEA versetzt, über Nacht bei 80°C erwärmt und 2 Tage bei Raumtemperatur stehen gelassen. Die Reaktionslösung wird über basisches Aluminiumoxid filtriert und das Filtrat i. Vak. eingeengt.
Ausbeute: quantitativ
Rₜ-Wert: 3.31 min (HPLC-MS, Methode 1)
C₂₂H₂₆ClN₃O₂S (431.99)
Massenspektrum: (M+H)⁺ = 432/434 (Chlorisotope)

### Beispiel 4

### 5-Chlor-thiophen-2-carbonsäure-N-{1-methyl-1-[3-(2,2,2-trifluor-acetyl)-2,3,4,5-tetrahydro-1H-benzo[d]azepin-7-ylcarbamoyl]-ethyl}-amid

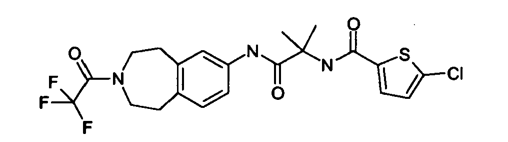

### (a) 2-[(5-Chlor-thiophen-2-yl)-carbonylaminol]2-methyl-propionsäure

4.5 g (27.7 mmol) 5-Chlor-thiophen-2-carbonsäure werden in 250 ml Dichlormethan unter Rühren bei Raumtemperatur mit 8.0 ml (110.7 mmol) Thionylchlorid versetzt und 3 h unter Rückfluß gerührt. Anschließend engt man das Reaktionsgemisch bis zur Trockene ein.

2.9 g (27.7 mmol) 2-Amino-isobuttersäure werden in 300 ml Acetonitril unter Rühren mit 8.0 ml (30.4 mmol) *N*,*O-*bis-(trimethylsilyl)-trifluor-acetamid versetzt und 3.5 h unter Rückfluß gerührt. Die Reaktionsmischung wird mit 8.5 ml (60.9 mmol) TEA und der Lösung des hergestellten Säurechlorides in 75 ml Acetonitril versetzt, 15 min unter Rückfluß gerührt und dann langsam auf Raumtemperatur abgekühlt. Die Reaktionsmischung wird i. Vak. bis zur Trockene eingeengt, der Rückstand mit Wasser und 2-molarer Natriumcarbonat-Lösung versetzt und mit Diethylether gewaschen. Die wässrige Phase wird mit 20 ml konz. Salzsäure auf pH 1 gestellt, der Niederschlag abgesaugt und bei 50°C im Vakuumtrockenschrank getrocknet.
Ausbeute: 5.9 g (86 %)
C₉H₁₀ClNO₃S (247.70)
Massenspektrum: (M+H)⁺ = 248/250 (Chlorisotope)

### (b) 5-Chlor-thiophen-2-carbonsäure-N-{1-methyl-1-[3-(2,2,2-trifluor-acetyl)-2,3,4,5-tetrahydro-1H-benzo[d]azepin-7-ylcarbamoyl]-ethyl}-amid

Hergestellt analog Beispiel 2-c aus 2-[(5-Chlor-thiophen-2-yl)-carbonylamino]-2-methyl-propionsäure und 3-(2,2,2-Trifluoracetyl)-2,3,4,5-tetrahydro-1*H-*benzo[*d*]azepin-7-yl)-amin mit TBTU und NMM in DMF bei Raumtemperatur mit anschließender Filtration über Aluminiumoxid.
Ausbeute: (95 %)
R_{f}-Wert: 0.65 (Kieselgel; Dichlormethan/Ethanol = 9:1)
C₂₁H₂₁ClF₃N₃O₃S (487.92)
Massenspektrum: (M-H)⁻ = 486/488 (Chlorisotope)

### Beispiel 5

### 5-Chlor-thiophen-2-carbonsäure-N-[1-methyl-1-(2,3,4,5-tetrahydro-1H-benzo[d]azepin-7-ylcarbamoyl)-ethyl]-amid

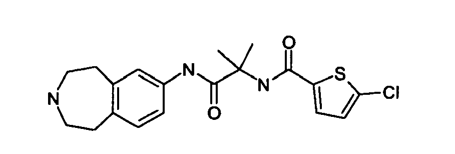

500.0 mg (1.03 mmol) 5-Chlor-thiophen-2-carbonsäure-*N-*{1-methyl-1-[3-(2,2,2-trifluor-acetyl)-2,3,4,5-tetrahydro-1*H-*benzo[*d*]azepin-7-ylcarbamoyl]-ethyl}-amid werden in 15 ml Methanol und 10 ml Wasser unter Rühren bei Raumtemperatur mit 637.0 mg (4.61 mmol) Kaliumcarbonat versetzt und 3 h unter Rückfluß gerührt. Die Reaktionsmischung wird i. Vak. eingeengt, der Rückstand mit Wasser versetzt, der Niederschlag abfiltriert und im Vakuumtrockenschrank bei 50°C getrocknet.
Ausbeute: 340.0 mg (85 %)
R_{f}-Wert: 0.20 (Kieselgel; Dichlormethan/Methanol/konz. Ammoniak-Lösung = 80:20:2)
C₁₉H₂₂ClN₃O₂S (391.92)
Massenspektrum: (M+H)⁻ = 390/392 (Chlorisotope)

### Beispiel 6

### 5-Chlor-thiophen-2-carbonsäure-N-[1-(3-acetyl-2,3,4,5-tetrahydro-1H-benzo[d]azepin-7-ylcarbamoyl)-1-methyl-ethyl]-amid

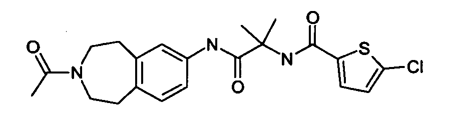

100.0 mg (0.26 mmol) 5-Chlor-thiophen-2-carbonsäure-*N-*[1-methyl-1-(2,3,4,5-tetrahydro-1*H-*benzo[*d*]azepin-7-ylcarbamoyl)-ethyl]-amid werden in 5 ml THF unter Rühren bei Raumtemperatur mit 43.0 µl (0.31 mmol) TEA und 27.0 µl (0.29 mmol) Essigsäureanhydrid versetzt und 2 h bei Raumtemperatur gerührt. Die Reaktionsmischung wird mit Wasser versetzt, der Niederschlag abfiltriert und im Vakuumtrockenschrank bei 50°C getrocknet.
Ausbeute: 80.0 mg (72 %)
R_{f}-Wert: 0.90 (Kieselgel; Dichlormethan/Methanol/konz. Ammoniak-Lösung = 80:20:2)
C₂₁H₂₄ClN₃O₃S (433.95)
Massenspektrum: (M+H)⁺ = 434/436 (Chlorisotope)

### Beispiel 7

### 5-Chlor-thiophen-2-carbonsäure-N-[1-(3-ethyl-2,3,4,5-tetrahydro-1H-benzo[d]azepin-7-ylcarbamoyl)-1-methyl-ethyl]-amid

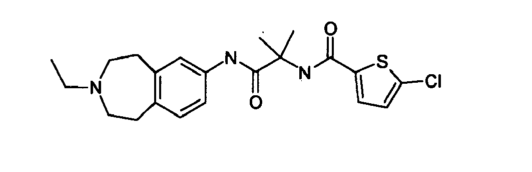

100.0 mg (0.26 mmol) 5-Chlor-thiophen-2-carbonsäure-*N-*[1-methyl-1-(2,3,4,5-tetrahydro-1*H-*benzo[*d*]azepin-7-ylcarbamoyl)-ethyl]-amid in 5 ml Aceton werden unter Rühren bei Raumtemperatur mit 74.0 mg (0.54 mmol) Kaliumcarbonat und 21.0 µl (0.26 mmol) lodethan versetzt und 3 h unter Rückfluß gerührt. Die Reaktionsmischung wird bei Raumtemperatur mit Wasser versetzt, der Niederschlag abfiltriert und im Vakuumtrockenschrank bei 50°C getrocknet.
Ausbeute: 60.0 mg (56 %)
R_{f}-Wert: 0.30 (Kieselgel; Dichlormethan/Methanol/konz. Ammoniak-Lösung = 80:20:2)
C₂₁H₂₆ClN₃O₂S (419.97)
Massenspektrum: (M+H)⁺ = 420/422 (Chlorisotope)

### Beispiel 8

### 5-Ethinyl-N-[1-methyl-1-(3-methyl-2,3,4,5-tetrahydro-1H-benzo[d]azepin-7-ylcarbamoyl)-ethyl]-thiophen-2-carbonsäureamid

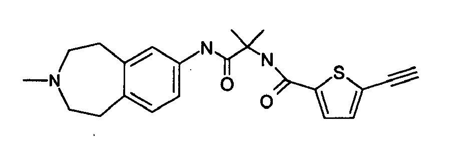

### (a) 5-Trimethylsilylethinyl-thiophen-2-carbonsäure-ethylester

Unter Argonatmosphäre wird eine Lösung aus 32.5 g (138.0 mmol) 5-Brom-thiophen-2-carbonsäure-ethylester in 320 ml Acetonitril und 640 ml THF unter Rühren mit 1.3 g (7.0 mmol) Kupfer-(I)-iodid und 39.0 g (276.0 mmol) Trimethylsilylacetylen versetzt, 5 min gerührt und anschließend werden 5.6 g (7.0 mmol) 1,1-Bis-(diphenylphosphino)-ferrocen-dichlorpalladium(II))-PdCl₂ im Komplex mit CH₂Cl₂ 1/1 und 57.4 ml (414.0 mmol) TEA zugegeben. Die Reaktionsmischung wird bei Raumtemperatur über Nacht gerührt, i. Vak. eingeengt, der Rückstand mit Essigsäureethylester versetzt und mit Ammoniak-Lösung (5%) und Wasser gewaschen. Die organische Phase wird über Natriumsulfat getrocknet und i. Vak. bis zur Trockene eingeengt. Der Rückstand wird mit Diethylether versetzt, der Niederschlag abgesaugt und getrocknet.
Ausbeute: quantitativ
R_{f}-Wert: 0.71 (Kieselgel; Petrolether/Essigsäureethylester = 8:2)
C₁₂H₁₆O₂SSi (252.41)
Massenspektrum: (M+H)⁺ = 253

### (b) 5-Ethinyl-thiophen-2-carbonsäure

Die Lösung aus 36.2 g (114.0 mmol) 5-Trimethylsilanylethinyl-thiophen-2-carbonsäure-ethylester in 185 ml Ethanol wird mit 730.0 ml (1.46 mol) 2-molarer wässriger Natronlauge versetzt und über Nacht bei 50°C gerührt. Das Reaktionsgemisch wird i. Vak. bis zur Trockene eingeengt, mit Wasser versetzt und mit Dichlormethan gewaschen. Die Wasser-Phase wird unter Eisbadkühlung mit 6-molarer wässriger Salzsäure sauer gestellt, der Niederschlag abfiltriert und bei 50°C im Vakuumtrockenschrank getrocknet.
Ausbeute: 8.9 g (41 %)
R_{f}-Wert: 0.64 (RP-8; Methanol/NaCl-Lösung (5%) = 6:4)
C₇H₄O₂S (152.17)
Massenspektrum: (M-H)⁻ = 151

### (c) 2-[(5-Ethinyl-thiophen-2-yl)-carbonylamino]-2-methyl-propionsäure-methylester

Eine Lösung aus 6.0 g (39.4 mmol) 5-Ethinyl-thiophen-2-carbonsäure in 180 ml THF wird unter Rühren mit 20.6 ml (118.3 mmol) DIPEA und 13.9 g (43.3 mmol) TBTU versetzt und 10 min gerührt. Anschließend wird 6.1 g (39.4 mmol) 2-Amino-isobuttersäure-methylester-hydrochlorid zugegeben und über Nacht bei Raumtemperatur gerührt. Das Reaktionsgemisch wird i. Vak. bis zur Trockene eingeengt, mit Essigsäureethylester versetzt und mit Wasser und Natriumhydrogencarbonat-Lösung (5%) gewaschen. Die organische Phase wird mit Natriumsulfat getrocknet und i.Vak. eingeengt.
Ausbeute: 9.3 g (94 %)
R_{f}-Wert: 0.71 (Kieselgel; Dichlormethan/Ethanol = 9:1)
C₁₂H₁₃NO₃S (251.30)
Massenspektrum: (M+H)⁺ = 252

### (d) 2-[(5-Ethinyl-thiophen-2-yl)-carbonylamino]-2-methyl-propionsäure

9.3 g (37.1 mmol) 2-[(5-Ethinyl-thiophen-2-yl)-carbonylamino]-2-methyl-propionsäure-methylester werden in 550 ml Wasser und 370 ml THF gelöst und mit 74.2 ml (74.2 mmol) 1-molarer wässriger Lithiumhydroxid-Lösung versetzt und bei Raumtemperatur für 2 h gerührt. Man destilliert i. Vak. THF ab und extrahiert mit Dichlormethan. Die Wasser-Phase wird mit 3-molarer wässriger Salzsäure sauer gestellt, der Niederschlag abfiltriert und bei 50°C im Vakuumtrockenschrank getrocknet.
Ausbeute: 8.4 g (95 %)
R_{f}-Wert: 0.20 (Kieselgel; Dichlormethan/Ethanol = 9:1)
C₁₁H₁₁NO₃S (237.28)
Massenspektrum: (M+H)⁺ = 238

### e) 2-(5-Ethinyl-thiophen-2-yl)-4,4,-dimethyl-4H-oxazol-5-on

Hergestellt analog Beispiel 3-a aus 2-[(5-Ethinyl-thiophen-2-yl)-carbonylamino]-2-methyl-propionsäure in Essigsäureanhydrid bei 65°C mit anschließender Reinigung durch Chromatographie an Kieselgel mit dem Laufmittel (Petrolether/Essigsäureethylester = 2:1).
Ausbeute: 3.1 g (84%)
R_{f}-Wert: 0.67 (Kieselgel; Petrolether/Essigsäureethylester = 7:3)
C₁₁H₉NO₂S (219.26)
Massenspektrum: (M+H)⁺ = 220

### f) 5-Ethinyl-N-[1-methyl-1-(3-methyl-2,3,4,5-tetrahydro-1H-benzo[d]azepin-7-ylcarbamoyl)-ethyl]-thiophen-2-carbonsäureamid

Eine Lösung aus 150.0 mg (0.7 mmol) 2-(5-Ethinyl-thiophen-2-yl)-4,4,-dimethyl-4*H-*oxazol-5-on in 7.5 ml Toluol und 850.0 µl Essigsäure wird unter Rühren mit der Lösung aus 134.2 mg (0.8 mmol) 3-Methyl-2,3,4,5-tetrahydro-1*H-*benzo[*d*]azepin-7-ylamin in 8.5 ml DMF versetzt und über Nacht bei 65°C gerührt. Das Reaktionsgemisch wird i. Vak. bis zur Trockene eingeengt, in Dichlormethan und Ethanol gelöst und über Aluminiumoxid filtriert. Das Filtrat wird mit Wasser versetzt, der Niederschlag abgesaugt und im Vakuumtrockenschrank bei 55°C getrocknet.
Ausbeute: 260.0 mg (96 %)
R_{f}-Wert: 0.43 (RP-8; Methanol/NaCL-Lösung (5%) = 6:4)
C₂₂H₂₅N₃O₂S (395.52)
Massenspektrum: (M+H)⁺ = 396

### Beispiel 9

### 5-Chlor-thiophen-2-carbonsäure-N-[1-methyl-1-(4-oxo-2,3,4,5-tetrahydro-1H-benzo[d]azepin-7-ylcarbamoyl)-1-methyl-ethyl]-amid/ 5-Chlor-thiophen-2-carbonsäure-N-[1-methyl-1-(2-oxo-2,3,4,5-tetrahydro-1H-benzo[d]azepin-7-ylcarbamoyl)-1-methyl-ethyl]-amid

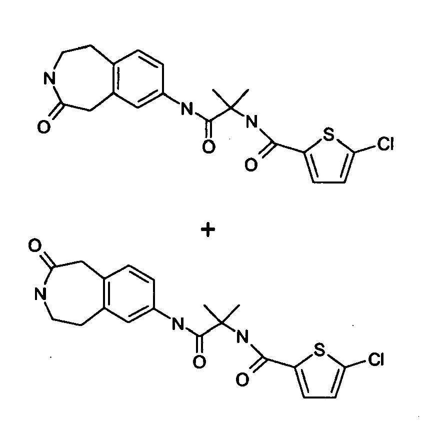

### (a) 3-Trifluoracetyl-7-nitro-2,3,4,5-tetrahydro-1H-benzo[d]azepin

12.5 g (51.39 mmol) 3-Trifluoracetyl-2,3,4,5-tetrahydro-1*H-*benzo[*d*]azepin in 30 ml Essigsäureanhydrid werden in 18.9 ml (352.0 mmol) Schwefelsäure (konz.) gelöst und bei - 5°C bis 0°C langsam mit 3.6 ml (51.39 mmol) Salpetersäure (65%) versetzt und 1 h bei 0°C gerührt. Man gibt die Reaktionsmischung auf Wasser, extrahiert mit Essigsäureethylester, trocknet die organische Phase über Natriumsulfat und engt bis zur Trockene ein. Den Rückstand kristallisiert man aus Ethanol um.
Ausbeute: 10.4 g (70 %)
R_{f}-Wert: 0.78 (Kieselgel; Dichlormethan/Ethanol = 95:5)
C₁₂H₁₁F₃N₂O₃ (288.22)
Massenspektrum: (M+H)⁺ = 289

### (b) 7-Nitro-2,3,4,5-tetrahydro-benzo[d]azepin-3-carbonsäure-tert.-butylester

5.0 g (17.34 mmol) 3-Trifluoracetyl-7-nitro-2,3,4,5-tetrahydro-1*H-*benzo[*d*]azepin in 50 ml THF werden mit 10.4 ml (20.81 mmol) 2-molarer Natronlauge versetzt und 30 min bei Raumtemperatur gerührt. Man versetzt die Reaktionslösung mit 1.88 g (17.69 mmol) Natriumcarbonat und 5.0 ml Wasser und dosiert unter Eisbadkühlung die Lösung aus 3.98 g (18.21 mmol) Di-*tert*.-butyl-dicarbonat in 15 ml THF zu und rührt 1 h bei Raumtemperatur. Anschließend filtriert man vom
Unlöslichen ab, versetzt das Filtrat mit Essigsäureethylester und wäscht mit ges. Natriumchlorid-Lösung. Die organische Phase wird über Natriumsulfat getrocknet und das Filtrat i. Vak. bis zur Trockene eingeengt.
Ausbeute: quantitativ
R_{f}-Wert: 0.81 (Kieselgel; Dichlormethan/Ethanol = 95:5)
C₁₅H₂₀N₂O₄ (292.33)
Massenspektrum: (M-Isobuten+H)⁺ = 237

### (c) 8-Nitro-1,3,4,5-tetrahydro-benzo[d]azepin-2-on/ 7-Nitro-1,3,4,5-tetrahydro-benzo[d]azepin-2-on

5.1 g (17.3 mmol) 7-Nitro-2,3,4,5-tetrahydro-benzo[*d*]azepin-3-carbonsäure-*tert*.-butylester in 50 ml Essigsäureethylester und 70 ml Wasser werden unter starkem Rühren mit 8.9 g (41.63 mmol) Natriummetaperiodat, 0.54 g (2.60 mmol) Rutheniumchlorid*H₂O versetzt und 3.5 h bei Raumtemperatur gerührt. Anschließend filtriert man vom Unlöslichen ab, versetzt das Filtrat mit Essigsäureethylester und wäscht mit Natriumdisulfit-Lösung (10%) und ges. Natriumchlorid-Lösung. Die organische Phase wird über Natriumsulfat getrocknet und das Filtrat i. Vak. bis zur Trockene eingeengt. Den Rückstand löst man in 60 ml Dichlormethan, gibt 6.0 ml TFA zu und rührt über Nacht. Die Reaktionslösung wird i. Vak. bis zur Trockene eingeengt, mit Dichlormethan versetzt, mit Wasser und ges Natriumhydrogencarbonat-Lösung gewaschen, über Natriumsulfat getrocknet und i. Vak. bis zur Trockene eingeengt.
Ausbeute: 2.3 g (65%)
R_{f}-Wert: 0.52 (Kieselgel; Dichlormethan/Ethanol = 9:1)
C₁₀H₁₀N₂O₃ (206.20)
Massenspektrum: (M+H)⁺ = 207

### (d) 8-Amino-1,3,4,5-tetrahydro-benzo[d]azepin-2-on/ 7-Amino-1,3,4,5-tetrahydro-benzo[d]azepin-2-on

2.3 g (11.2 mmol) 8-Nitro-1,3,4,5-tetrahydro-benzo[*d*]azepin-2-on / 7-Nitro-1,3,4,5-tetrahydro-benzo[*d*]azepin-2-on werden in 40 ml Methanol gelöst und mit 300 mg Pd/C 10% versetzt. Man hydriert in einer Parr-Apparatur bei Raumtemperatur bei 3 bar Wasserstoff-Druck für 17 Stunden. Anschließend wird der Katalysator abfiltriert und das Filtrat i. Vak. eingeengt.
Ausbeute: 1.51 g (77 %)
R_{f}-Wert: 0.10 (Kieselgel; Dichlormethan/Ethanol = 95:5)
C₁₀H₁₂N₂O (176.22)
Massenspektrum: (M+H)⁺ = 177

### (e) 5-Chlor-thiophen-2-carbonsäure-N-[1-methyl-1-(4-oxo-2,3,4,5-tetrahydro-1H-benzo[d]azepin-7-ylcarbamoyl)-1-methyl-ethyl]-amid/ 5-Chlor-thiophen-2-carbonsäure-N-[1-methyl-1-(2-oxo-2,3,4,5-tetrahydro-1H-benzo[d]azepin-7-ylcarbamoyl)-1-methyl-ethyl]-amid

Hergestellt analog Beispiel 1 aus 2-[(5-Chlor-thiophen-2-yl)-carbonylamino-2-methyl-propionsäure mit HATU, NMM und 8-Amino-1,3,4,5-tetrahydro-benzo[*d*]azepin-2-on/7-Amino-1,3,4,5-tetrahydro-benzo[*d*]azepin-2-on in DMF mit anschließender Reinigung durch Chromatographie an Kieselgel mit dem Laufmittel (Dichlormethan/Ethanol 100:0 bis 92:8).
Ausbeute: quantitativ
R_{f}-Wert: 0.40 (Kieselgel; Dichlormethan/Ethanol = 9:1)
C₁₉H₂₀ClN₃O₃S (405.90)
Massenspektrum: (M+H)⁺ = 406/408 (Chlorisotope)

Analog Beispiel 1 können folgende Verbindungen hergestellt werden:

| **Nr.** | **Strukturformel** | **Ausbeute Letzte Stufe** | **Massenpeak(s)** | **R_{f}-Wert bzw. Rₜ** |
|---|---|---|---|---|
| | **Name** | | | |
| **10** | 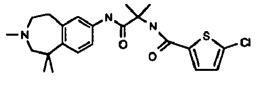 | 68% | (M+H)⁺ = 434/436 (Chlorisotope) | 0.33 (RP-8; Methanol: 5% NaCl-Lösung = 6:4) |
| | 5-Chlor-thiophen-2-carbonsäure-*N-*[1-methyl-1-(1,1,3-trimethyl-2,3,4,5-tetrahydro-1*H-*benzo[*d*]azepin-7-ylcarbamoyl]-ethyl}-amid | | | |
| **11** | 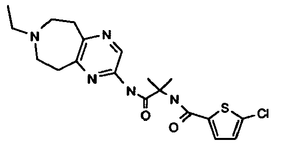 | 1 % | (M+H)⁺ = 422/424 (Chlorisotope) | 0.17 (RP-8; Methanol: 5% NaCl-Lösung = 6:4) |
| | 5-Chlor-thiophen-2-carbonsäure-*N-*[1-(7-ethyl-6,7,8,9-tetrahydro-5*H-*pyrazino[2,3-*d*]azepin-2-ylcarbamoyl)-1-methyl-ethyl]-amid | | | |
| **12** | 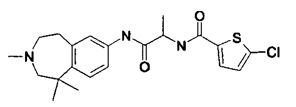 | 100% | (M+H)⁺ = 420/422 (Chlorisotope) | 3.29 min (HPLC-MS) Methode 1 |
| | 5-Chlor-thiophen-2-carbonsäure-*N-*[1-[1,1,3-trimethyl-2,3,4,5-tetrahydro-1*H-*benzo[*d*]azepin-7-ylcarbamoyl]-ethyl}-amid | | | |
| **13** | 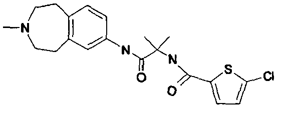 | 64% | (M+H)⁺ = 406/408 (Chlorisotope) | 0.40 (RP-8; Methanol: 5% NaCl-Lösung = 6:4) |
| | 5-Chlor-thiophen-2-carbonsäure-*N-*[1-methyl-1-(3-methyl-2,3,4,5-tetrahydro-1*H-*benzo[*d*]azepin-7-ylcarbamoyl]-ethyl}-amid | | | |
| **14** | 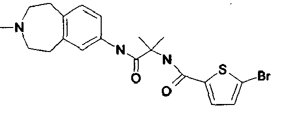 | 99% | (M+H)⁺ = 450/452 (Bromisotope) | 0.40 (RP-8; Methanol: 5% NaCl-Lösung = 6:4) |
| | 5-Brom-thiophen-2-carbonsäure-*N-*[1-methyl-1-(3-methyl-2,3,4,5-tetrahydro-1*H-*benzo[*d*]azepin-7-ylcarbamoyl]-ethyl}-amid | | | |
| **18** | 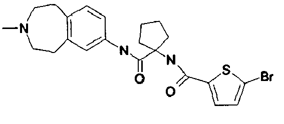 | 42 % | (M+H)+= 476/78 (Bromisotope) | 0.61 (Aluminiu moxid;Dic hlormetha n/Ethanol = 95:5) |
| | 1-[(5-Brom-thiophen-2-yl)-carbonylamino]-*N-*(3-methyl-2,3,4,5-tetrahydro-1*H-*benzo[*d*]azepin-7-yl)-cyclopentan-1-carbonsäureamid | | | |
| **19** | 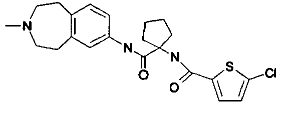 | 75% | (M+H)+= 432/34 (Chlorisotope) | 0.32 (RP-8;Methan ol/ 5%NaCl-Lösung = 6 :4) |
| | 1-[(5-Chlor-thiophen-2-yl)-carbonylamino]-*N-*(3-methyl-2,3,4,5-tetrahydro-1*H*-benzo[*d*]azepin-7-yl)-cyclopentan-1-carbonsäureamid | | | |
| **20** | 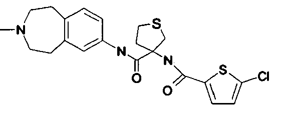 | 62% | (M+H)+= 450/52 (Chlorisotope) | |
| | 3-[(5-Chlor-thiophen-2-yl)-carbonylamino]-*N-*(3-methyl-2,3,4,5-tetrahydro-1*H-*benzo[*d*]azepin-7-yl)-tetrahydrothiophen-3-carbonsäureamid | | | |
| **21** | 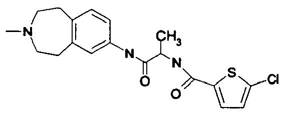 | 48% | (M+H)+= 392/94 (Chlorisotope) | |
| | 5-Chlor-thiophen-2-carbonsäure-*N-*[1-(3-methyl-2,3,4,5-tetrahydro-1*H-*benzo[*d*]azepin-7-ylcarbamoyl]-ethyl}-amid | | | |
| **22** | 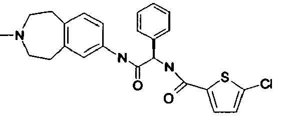 | 26% | (M-H)-= 452/4 (Chlorisotope) | 0.45 (RP-8;Methan ol/ 5%NaCl-Lösung = 6:4) |
| | (R)-5-Chlor-thiophen-2-carbonsäure-*N-*[1-(3-methyl-2,3,4,5-tetrahydro-1*H-*benzo[*d*]azepin-7-ylcarbamoyl]-1-phenyl-methyl}-amid | | | |
| **23** | 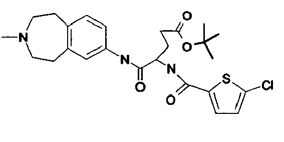 | 57% | (M+H)+= 506/08 (Chlorisotope) | |
| | 5-Chlor-thiophen-2-carbonsäure-*N-*[3-tert.-butoxycarbonyl-1-(3-methyl-2,3,4,5-tetrahydro-1*H-*benzo[*d*]azepin-7-ylcarbamoyl]-propyl}-amid | | | |
| **24** | 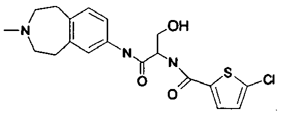 | 2% | (M+H)+= 408/10 (Chlorisotope) | 0.48 (RP-8;Methan ol/ 5%NaCl-Lösung = 6 :4) |
| | 5-Chlor-thiophen-2-carbonsäure-*N-*[2-hydroxy-1-(3-methyl-2,3,4,5-tetrahydro-1*H-*benzo[*d*]azepin-7-ylcarbamoyl]-ethyl}-amid | | | |
| **25** | 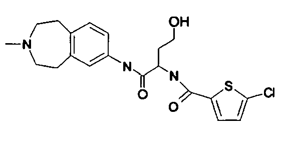 | 20% | (M+H)+= 422/24 (Chlorisotope) | 0.51 (RP-8;Methan ol/ 5%NaCl-Lösung = 6 :4) |
| | 5-Chlor-thiophen-2-carbonsäure-*N-*[3-hydroxy-1-(3-methyl-2,3,4,5-tetrahydro-1*H-*benzo[*d*]azepin-7-ylcarbamoyl]-propyl}-amid | | | |
| **26** | 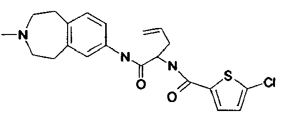 | 71 % | (M+H)+= 418/20 (Chlorisotope) | |
| | 5-Chlor-thiophen-2-carbonsäure-*N-*[1-(3-methyl-2,3,4,5-tetrahydro-1*H-*benzo[*d*]azepin-7-ylcarbamoyl]-but-3-enyl}-amid | | | |
| **27** | 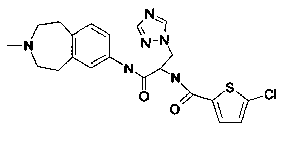 | 55% | (M+H)+= 459/61 (Chlorisotope) (M-H)-= 457/9 (Chlorisotope) | 0.46 (RP-8;Methan ol/ 5%NaCl-Lösung = 6 :4) |
| | 5-Chlor-thiophen-2-carbonsäure-*N-*[2-(1,2,4-triazol-1-yl)-1-(3-methyl-2,3,4,5-tetrahydro-1*H-*benzo[*d*]azepin-7-ylcarbamoyl]-ethyl}-amid | | | |
| **28** | 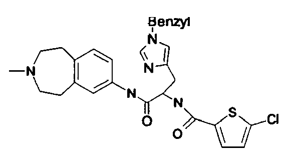 | | | |
| | 5-Chlor-thiophen-2-carbonsäure-*N-*[2-(1-benzyl-imidazol-4-yl)-1-(3-methyl-2,3,4,5-tetrahydro-1*H-*benzo[*d*]azepin-7-ylcarbamoyl]-ethyl}-amid | | | |
| **29** | 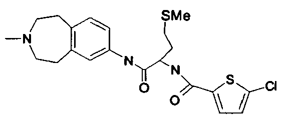 | 54% | (M+H)+= 452/54 (Chlorisotope) | |
| | 5-Chlor-thiophen-2-carbonsäure-*N-*[3-methylsulfanyl-1-(3-methyl-2,3,4,5-tetrahydro-1*H-*benzo[*d*]azepin-7-ylcarbamoyl]-propyl}-amid | | | |
| **34** | 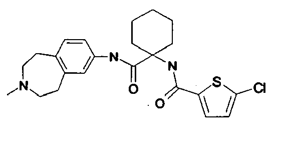 | 8% | (M+H)+= 446/8 (Chlorisotope) | 0.79 (Aluminiu moxid; Dichlorme than/Etha nol = 95:5) |
| | 1-[(5-Chlor-thiophen-2-yl)carbonylamino]-N-(3-methyl-2,3,4,5-tetrahydro-1*H-*benzo[d]azepin-7-yl)-cyclohexan-1-carbonsäureamid | | | |

Analog Beispiel 2 können folgende Verbindungen hergestellt werden:

| **Nr.** | **Strukturformel** | **Ausbeute Letzte Stufe** | **Massenpeak(s)** | **R_{f}-Wert** bzw. **Rt** |
|---|---|---|---|---|
| | **Name** | | | |
| **15** | 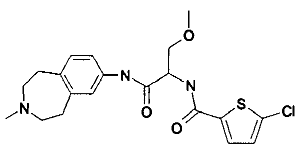 | 86% | (M+H)⁺ = 422/424 Chlorisotope | 0.52 (Aluminiu moxid; Dichlorme than/Etha nol = 95:5) |
| | 5-Chlor-thiophen-2-carbonsäure-*N-*[2-methoxy-1-(3-methyl-2,3,4,5-tetrahydro-1*H-*benzo[*d*]azepin-7-ylcarbamoyl)-ethyl]-amid | | | |
| **30** | 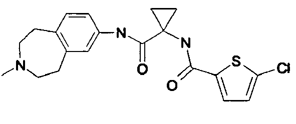 | 40% | (M+H)⁺ = 404/406 Chlorisotope | 0.41 (RP-8;Methan ol/ 5%NaCl-Lösung = 6 :4) |
| | 1-[(5-Chlor-thiophen-2-yl)carbonylamino]-N-(3-methyl-2,3,4,5-tetrahydro-1H-benzo[d]azepin-7-yl)-cyclopropan-1-carbonsäureamid | | | |
| **31** | 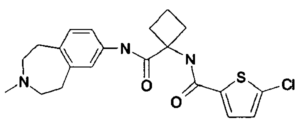 | 60% | (M+H)⁺ = 418/420 Chlorisotope | 0.62 (Kieselgel Dichlorme than/ Ethanol/ Ammonia k = 80:20:2) |
| | 1-[(5-Chlor-thiophen-2-yl)carbonylamino]-N-(3-methyl-2,3,4,5-tetrahydro-1H-benzo[d]azepin-7-yl)-cyclobutan-1-carbonsäureamid | | | |
| **32** | 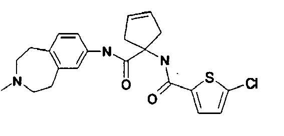 | 70% | (M+H)+= 430/32 (Chlorisotope) (M-H)-= 428/30 (Chlorisotope | 0.34 (RP-8;Methan ol/ 5%NaCl-Lösung = 6 :4) |
| | 1-[(5-Chlor-thiophen-2-yl)carbonylamino]-N-(3-methyl-2,3,4,5-tetrahydro-1H-benzo[d]azepin-7-yl)-cyclopent-3-en-1-carbonsäureamid | | | |
| **33** | 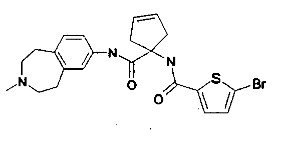 | 85% | (M+H)+= 474/6 (Bromisotope) | 0.32 (RP-8;Methan ol/ 5%NaCl-Lösung = 6 :4) |
| | 1-[(5-Brom-thiophen-2-yl)carbonylamino]-N-(3-methyl-2,3,4,5-tetrahydro-1H-benzo[d]azepin-7-yl)-cyclopent-3-en-1-carbonsäureamid | | | |

Analog Beispiel 8 können folgende Verbindungen hergestellt werden:

| **Nr.** | **Strukturformel** | **Ausbeute** Letzte Stufe | **Massenpeak(s)** | **R_{f}-wert** bzw. Rt |
|---|---|---|---|---|
| | **Name** | | | |
| **16** | 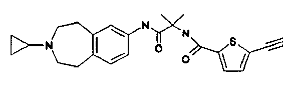 | quantitativ | (M+H)⁺ = 422 | 3.27 min (HPLC-MS; Methode 2) |
| | 5-Ethinyl-N-[1-(3-cyclopropyl-2,3,4,5-tetrahydro-4H-benzo[d]azepin-7-ylcarbamoyl)-1-methyl-ethyl]-thiophen-2-carbonsäureamid | | | |
| **17** | 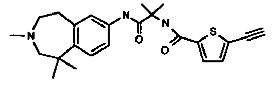 | quantitativ | (M+H)⁺ = 424 | 3.29 min (HPLC-MS; Methode 2) |
| | 5-Ethinyl-*N-*[1-methyl-1-(1,1,3-trimethyl-2,3,4,5-tetrahydro-4*H-*benzo[*d*]azepin-7-ylcarbamoyl)-ethyl]-thiophen-2-carbonsäureamid | | | |

### Beispiel 35

### 5-Chlor-thiophen-2-carbonsäure-N-[3-hydroxycarbonyl-1-(3-methyl-2,3,4,5-tetrahydro-1H-benzo[d]azepin-7-ylcarbamoyl]propyl-amid

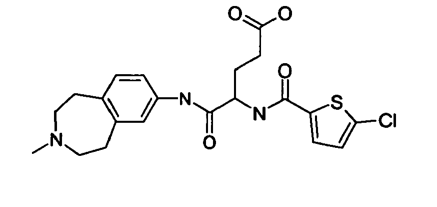

93 mg (0,15 mmol) 5-Chlor-thiophen-2-carbonsäure-*N-*[3-tert.-butoxycarbonyl-1-(3-methyl-2,3,4,5-tetrahydro-1*H-*benzo[*d*]azepin-7-ylcarbamoyl]-propyl}-amid werden in 1 ml Dichlormethan unter Rühren bei RT mit 1 ml Trifluoressigsäure
versetzt und 1,5 h bei Raumtemperatur gerührt und eingeengt.
Ausbeute: 78.0 mg (92 %)
C₂₁H₂₄ClN₃O₄S (449.959)
Massenspektrum: (M+H)⁺ = 450/52 (Chlorisotope)

### Beispiel 36

### (R)-5-Chlor-thiophen-2-carbonsäure-N-[3-(1H-tetrazol-5-yl)-1-(3-methyl-2,3,4,5-tetrahydro-1H-benzo[d]azepin-7-ylcarbamoyl)-propyl]-amid

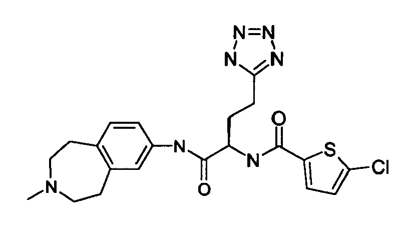

### (a) (R)-2-tert.-Butoxycarbonylamino-4-cyano-buttersäure

5.0 g (20,3 mmol) (R)-N-α-(tert.-Butoxycarbonyl)-D-glutamin werden in 55 ml Pyridin unter Rühren bei Raumtemperatur mit 2,30 ml (24,3 mmol) Essigsäureanhydrid versetzt und 20 h gerührt. Das Reaktionsgemisch wird i. Vak. eingeengt, der Rückstand mit Essigester versetzt und 3 mal mit 5%iger Zitronensäure und 3 mal mit ges. NaCL-Lösung gewaschen. Die organische Phase wird über Natriumsulfat getrocknet und i.Vak. eingeengt. Anschließend wird durch Chromatographie an Kieselgel (Laufmittel: Dichlormethan/Methanol 90:10) gereinigt.
Ausbeute: 3.16 g (68 %)
R_{f}-Wert: 0.3 (Kieselgel; Dichlormethan/Methanol = 9:1)
C₁₀H₁₆N₂O₄ (228.25)
Massenspektrum: (M-H)⁻ = 227

### (b) (R)-[3-Cyano-1-(3-methyl-2,3,4,5-tetrahydro-1H-benzo[d]azepin-7-ylcarbamoyl)-propyl]-carbaminsäure tert.-butyl ester

1.0 g (4,0 mmol) (R)-2-tert.-Butoxycarbonylamino-4-cyano-buttersäure wird in 10 ml DMF gelöst und mit 1,29 g( 4,03 mmol) TBTU und 2,79 ml (20,1 mmol) TEA bei Raumtemperatur 30 min gerührt. Anschließend wird 1.0 g (4,01 mmol) 3-Methyl-2,3,4,5-tetrahydro-1*H-*benzo[d]azepin-7-ylamin zugegeben und über Nacht bei 35 °C gerührt. Das Reaktionsgemisch wird i. Vak. bis zur Trockene eingeengt und mit Essigester extrahiert. Die Organische Phase wird mit Natriumsulfat getrocknet und i. Vak. eingeengt. Anschließend wird durch Chromatographie an Kieselgel (Laufmittel: Dichlormethan/Methanol 95:5) gereinigt.
Ausbeute: 450 mg (29 %)
R_{f}-Wert: 0.15 (Kieselgel; Dichlormethan/Methanol = 95:5)
C₂₁H₃₀N₄O₃ (386.49)
Massenspektrum: (M+H)⁺ = 387

### (c) (R)-[1-(3-Methyl-2,3,4,5-tetrahydro-1H-benzo[d]azepin-7-ylcarbamoyl)-3-(1H-tetrazol-5-yl)-propyl]-carbaminsäure tert.-butyl ester

200 mg (0,52mmol) (R)-[3-Cyano-1-(3-methyl-2,3,4,5-tetrahydro-1*H-*benzo[*d*]azepin-7-ylcarbamoyl)-propyl]carbaminsäure tert.-butyl ester werden in 1 ml DMF vorgelegt und mit 80 mg (1,23 mmol) Natriumazid und 66 mg (1,23 mmol) Ammoniumchlorid versetzt. Das Reaktionsgemisch wird über Nacht bei 100 °C gerührt, anschließend mit TFA sauer gestellt und über RP-Material (Laufmittel: Wasser/Acetonitril 95 :5=> 5:95) getrennt.
Ausbeute: 49.8 mg (18%)
C₂₁H₃₁N₇O₃ (429.53)
Massenspektrum: (M+H)⁺ = 430

### (d) (R)-2-Amino-N-(3-methyl-2,3,4,5-tetrahydro-1H-benzo[d]azepin-7-yl)-4-(1H-tetrazol-5-yl)-butyramide

49,8 mg (90 µmol) (R)-[1-(3-Methyl-2,3,4,5-tetrahydro-1*H-*benzo[*d*]azepin-7-ylcarbamoyl)-3-(1H-tetrazol-5-yl)-propyl]-carbaminsäure tert.-butyl ester werden in 1 ml 2 molarer wässriger Salzsäure bei 50°C 2,5 h gerührt und anschließend zur Trockene eingeengt.
Ausbeute: 32.7 mg (89 %)
C₁₆H₂₃N₇O (329.41)
Massenspektrum: (M+H)⁺ = 330

### (e) (R)-5-Chlor-thiophen-2-carbonsäure-N-[3-(1H-tetrazol-5-yl)-1-(3-methyl-2,3,4,5-tetrahydro-1H-benzo[d]azepin-7-ylcarbamoyl)-propyl]-amide

13 mg (0,08 mmol) 5-Chlor-thiophen-2-carbonsäure werden in 0,5 ml DMF gelöst, bei Raumtemperatur mit 26 mg (0,08 mmol) TBTU und 50 µl (0,45 mmol) NMM versetzt und 15 min gerührt. Zum Reaktionsgemisch wird eine Lösung von 32 mg (0,08 mmol) (R)-2-Amino-N-(3-methyl-2,3,4,5-tetrahydro-1*H-*benzo[*d*]azepin-7-yl)-4-(1H-tetrazol-5-yl)-butyramide in 1 ml DMF zugegeben und über Nacht bei Raumtemperatur gerührt. Das Reaktionsgemisch wird mit TFA sauer gestellt, chromatographisch über RP-Material (Laufmittel: Wasser/Acetonitril 95:5=>5:95) getrennt und anschließend gefriergetrocknet.
Ausbeute: 18,8 mg (40 %)
Rₜ-Wert: 2,29 min (HPLC-MS; Methode 2)
C₂₁H₂₄ClN₇O₂S (473.99)
Massenspektrum: (M+H)⁺ = 474/6 (Chlorisotope)

### Beispiel 37

### (R)-5-Chlor-thiophen-2-carbonsäure-N-[2-benzyloxy-1-(3-methyl-2,3,4,5-tetrahydro-1H-benzo[d]azepin-7-ylcarbamoyl)-ethyl]-amid

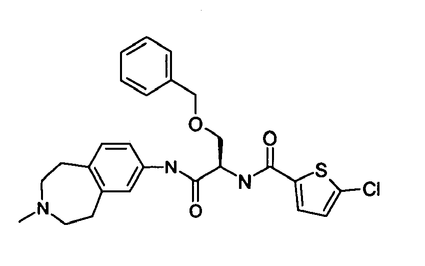

### (a) (R) 2-Amino-3-benzyloxy-N-(3-methyl-2,3,4,5-tetrahydro-1H-benzo[d]azepin-7-yl)-propionamid

0,117 g (0,60 mmol) (R)-3-Benzyloxy-2-tert.-butoxycarbonylamino-propionsäure werden analog Beispiel 2 (a) mit 0,116 g (0,66 mmol) 3-Methyl-2,3,4,5-tetrahydro-1*H-*benzo[*d*]azepin-7-ylamin, TBTU, DIPEA in THF umgesetzt. Anschließend wird analog Beispiel 1 (d) die BOC-Schutzgruppe abgespalten, mit Natriumhydroxid basisch gestellt und mit Essigester extrahiert.
Ausbeute: quantitativ
R_{f}-Wert: 0,48 (RP-8;Methanol/ 5%NaCl-Lösung = 6 :4)
C₂₁ H₂₇N₃O₂ (353.46)
Massenspektrum: (M+H)⁺ = 354

### (b) (R)-5-Chlor-thiophen-2-carbonsäure-N-[2-benzyloxy-1-(3-methyl-2,3,4,5-tetrahydro-1H-benzo[d]azepin-7-ylcarbamoyl)-ethyl]-amid

0,117 g (0,72 mmol) 5-Chlor-thiophen-2-carbonsäure werden in 15 ml Dichlormethan unter Rühren bei Raumtemperatur mit 0,26 ml (3,60 mmol) Thionylchlorid und 0,01 ml DMF versetzt und 2 h am Rückfluß gerührt. Anschließend engt man das Reaktionsgemisch i.Vak. ein.

0,230 g (0,65 mmol) (R) 2-Amino-3-benzyloxy-*N-*(3-methyl-2,3,4,5-tetrahydro-1*H-*benzo[*d*]azepin-7-yl)-propionamid werden in 10 ml THF unter Rühren mit 0,27 ml (1,95 mmol) TEA und der Lösung des hergestellten Säurechlorides in 10 ml THF bei Raumtemperatur versetzt und 15 h gerührt. Anschließend wird mit Essigester verdünnt, mit Wasser gewaschen, über Natriumsulfat getrocknet und i.Vak. eingeengt. Die Reinigung erfolgt chromatographisch über Aluminiumoxid (Laufmittel: Dichlormethan/Ethanol 100 :0=>98,5:1,5) Ausbeute: 0,14 g (43 %)
R_{f}-Wert: 0.81 (Aluminiumoxid; Dichlormethan/Ethanol = 95:5) C₂₆H₂₈ClN₃O₂S (498.04)
Massenspektrum: (M+H)⁺ = 498/00 (Chlorisotope)

### Beispiel 38

### 5-Chlor-thiophen-2-carbonsäure-N-[1-(3-isopropyl-2,3,4,5-tetrahydro-1H-benzo[d]azepin-7-ylcarbamoyl)-1-methyl-ethyl]-amid

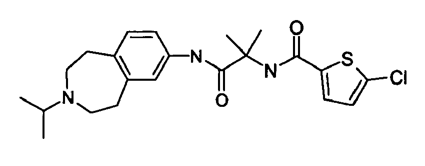

### (a) 3-Isopropyl-7-nitro-2,3,4,5-tetrahydro-1H-benzo[d]azepin

0,96g (5,00 mmol) 7-nitro-2,3,4,5-tetrahydro-1*H-*benzo[*d*]azepin und 0,404 ml (5,50 mmol) Aceton werden in 20 ml THF gelöst, anschließend wird 0,414 ml (7,50 mmol) Essigsäure und 0,10 g p-Toluolsulfonsäure zugegeben und bei Raumtemperatur 30 min gerührt. Bei Raumtemperatur werden 1,378 g (6,50 mmol) Natriumtriacetoxyborhydrid zugegeben und 23 h gerührt. Danach wird mit ges. Natriumhydrogencarbonat- Lösung alkalisch gestellt und mit Essigester extrahiert, anschließend wird die organische Phase mit Natriumsulfat getrocknet und i.Vak. eingeengt.
Ausbeute: quantitativ
C₁₃H₁₈N₂O₂ (234.29)
Massenspektrum: (M+H)⁺ = 235

### (b) 3-Isopropyl-2,3,4,5-tetrahydro-1H-benzo[d]azepin-7-ylamin

Hergestellt analog Beispiel 1 (c) aus 3-Isopropyl-7-nitro-2,3,4,5-tetrahydro-1*H-*benzo[*d*]azepin in Methanol mit Pd/C 10 % bei Raumtemperatur. Anschließend wird das Produkt eingeengt.
Ausbeute: 0,920 g (89 %)
R_{f}-Wert: 0.14 (Kieselgel; Dichlormethan/Ethanol = 9:1)
C₁₃H₂₀N₂ (204.31)
Massenspektrum: (M+H)⁺ = 205

### (c) 5-Chlor-thiophen-2-carbonsäure-N-[1-(3-isopropyl-2,3,4,5-tetrahydro-1H-benzo[d]azepin-7-ylcarbamoyl)-1-methyl-ethyl]-amid

Hergestellt analog Beispiel 1(f) aus 3-Isopropyl-2,3,4,5-tetrahydro-1*H-*benzo[*d*]azepin-7-ylamin und 2-[(5-Chlor-thiophen-2-yl)-carbonylamino]-2-methyl-propionsäure mit HATU und NMM in DMF bei Raumtemperatur mit anschließender Chromatographie über Aluminiumoxid (Laufmittel: Dichlormethan/ Ethanol 98:2) und über RP-Material ( Zorbax StableBond C18; 7µm 220g; Laufmittel: Wasser/Acetonitril/Ameisensäure = 95:5:0,1 => 10:90:0,1 ) und und Lyophilisation.
Ausbeute: 0,386 g (40 %)
R_{f}-Wert: 0.70 (Aluminiumoxid; Dichlormethan/Ethanol = 95:5)
C₂₂H₂₈ClN₃O₂S (434.0)
Massenspektrum: (M+H)+ = 434/6 (Chlorisotope)

### Beispiel 39

### 5-Chlor-thiophen-2-carbonsäure-N-[1-(8-methoxy-3-methyl-2,3,4,5-tetrahydro-1H-benzo[d]azepin-7-ylcarbamoyl)-1-methyl-ethyl]-amid

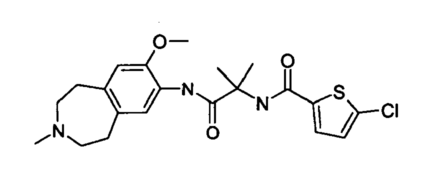

### (a) 2,2,2-Trifluor-1-(7-methoxy-8-nitro-1,2,4,5-tetrahydro-benzo[d]azepin-3-yl)-ethanon

1,30 g (4,27 mmol) 2,2,2-Trifluor-1-(7-hydroxy-8-nitro-1,2,4,5-tetrahydro-benzo[*d*]azepin-3-yl)-ethanon werden in 20 ml DMF gelöst. Bei Raumtemperatur werden 0,59 g (4,27 mmol) Kaliumcarbonat und 0,26 ml (4,27 mmol) Methyliodid zugegeben und über Nacht gerührt. Anschließend wird filtriert und das Filtrat i.Vak. eingeengt, der Rückstand wird mit Wasser verrieben und abgesaugt und im Umlufttrockenschrank bei 45°C getrocknet.
Ausbeute: 1,30 g (96 %)
R_{f}-Wert: 0.59 (Kieselgel; Petrolether/Ethylacetat = 1:1)
C₁₃H₁₃F₃N₂O₄ (318.25)
Massenspektrum: (M+H)+ = 319

### (b) 7-Methoxy-8-nitro-2,3,4,5-tetrahydro-1H-benzo[d]azepin

1,30 g (4,08 mmol) 2,2,2-Trifluor-1-(7-methoxy-8-nitro-1,2,4,5-tetrahydro-benzo[*d*]azepin-3-yl)-ethanon und 3,06 ml (6,13 mmol) 2 molare Natronlauge werden bei Raumtemperatur in 20 ml THF gelöst und 3 h gerührt. Anschließend wird i.Vak. eingeengt, mit Wasser verdünnt und mit tert.-Butylmethylether extrahiert. Die organische Phase wird mit 50 % iger Natronlauge und mit ges. NaCL-Lösung gewaschen, über Natriumsulfat getrocknet und i.Vak. eingeengt.
Ausbeute: 0,87 g (96 %)
R_{f}-Wert: 0.15 (Kieselgel; Petrolether/Ethylacetat = 1:1)
C₁₁H₁₄N₂O₃ (222.24)
Massenspektrum: (M+H)+ = 223

### (c) 7-Methoxy-3-methyl-8-nitro-2,3,4,5-tetrahydro-1H-benzo[d]azepin

Hergestellt analog Beispiel 1 (b) aus 7-Methoxy-8-nitro-2,3,4,5-tetrahydro-1*H-*benzo[*d*]azepin und wässriger Formalin-Lösung in Ameisensäure.
Ausbeute: 0,80 g (86 %)
R_{f}-Wert: 0.59 (Aluminiumoxid; Dichlormethan/Ethanol = 95:5)
C₁₂H₁₆N₂O₃ (236.27)
Massenspektrum: (M+H)+ = 237

### (d) 8-Methoxy-3-methyl-2,3,4,5-tetrahydro-1H-benzo[d]azepin-7-ylamin

Hergestellt analog Beispiel 1 (c) aus 7-Methoxy-3-methyl-8-nitro-2,3,4,5-tetrahydro-1*H*-benzo[*d*]azepin mit Pd/C 10 % in Methanol.
Ausbeute: 0,67 g (96 %)
R_{f}-Wert: 0.63 (Aluminiumoxid; Dichlormethan/Ethanol = 95:5)
C₁₂H₁₈N₂O (236.27)
Massenspektrum: (M+H)+ = 207

### (e) 5-Chlor-thiophen-2-carbonsäure-N-[1-(8-methoxy-3-methyl-2,3,4,5-tetrahydro-1H-benzo[d]azepin-7-ylcarbamoyl)-1-methyl-ethyl]-amid

Hergestellt analog Beispiel 1 (f) aus 8-Methoxy-3-methyl-2,3,4,5-tetrahydro-1*H-*benzo[*d*]azepin-7-ylamin und 2-[(5-Chlor-thiophen-2-yl)-carbonylamino]-2-methyl-propionsäure mit HATU und NMM in DMF bei Raumtemperatur. Anschließend wird i.Vak eingeengt und chromatographisch über Kieselgel (Laufmittel: Dichlormethan/ Ethanol 100:0=>98:2) gereinigt.
Ausbeute: 0,55 g (63 %)
R_{f}-Wert: 0.61 (Aluminiumoxid; Dichlormethan/Ethanol = 95:5)
C₂₁H₂₆ClN₃O₃S (435.97)
Massenspektrum: (M+H)+ = 436/8 (Chlorisotope)

### Beispiel 40

### 5-Chlor-thiophen-2-carbonsäure-N-[1-(8-hydroxy-3-methyl-2,3,4,5-tetrahydro-1H-benzo[d]azepin-7-ylcarbamoyl)-1-methyl-ethyl]-amid

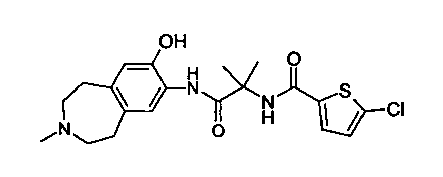

### (a) 1-(7-Benzyloxy-8-nitro-1,2,4,5-tetrahydro-benzo[d]azepin-3-yl)-2,2,2-trifluor-ethanon

1,30 g (4,27 mmol) 2,2,2-Trifluor-1-(7-hydroxy-8-nitro-1,2,4,5-tetrahydro-benzo[*d*]azepin-3-yl)-ethanon werden in 20 ml DMF vorgelegt und bei Raumtemperatur werden 0,65 g (4,70 mmol) Kaliumcarbonat und 0,508 ml (4,27 mmol) Benzylbromid zugegeben und 3 h gerührt. Anschließend wird filtriert und das Filtrat i.Vak. eingeengt, der Rückstand wird mit Wasser verrieben, abgesaugt und im Umlufttrockenschrank bei 45 °C getrocknet.
Ausbeute: 1,67 g (99 %)
R_{f}-Wert: 0.64 (Kieselgel; Petrolether/Ethylacetat = 7:3)
C₁₉H₁₇F₃N₂O₄ (394.34)
Massenspektrum: (M+NH₄)+ = 412

### (b) 7-Benzyloxy-8-nitro-2,3,4,5-tetrahydro-1H-benzo[d]azepin

Hergestellt analog Beispiel 39 (b) aus 1-(7-Benzyloxy-8-nitro-1,2,4,5-tetrahydro-benzo[*d*]azepin-3-yl)-2,2,2-trifluor-ethanon.
Ausbeute: 1,04 g (83 %)
R_{f}-Wert: 0.46 (Kieselgel; Petrolether/Ethylacetat = 3:7)
C₁₇H₁₈N₂O₃ (298.34)
Massenspektrum: (M+H)+ = 299

### (c) 7-Benzyloxy-3-methyl-8-nitro-2,3,4,5-tetrahydro-1H-benzo[d]azepin

Hergestellt analog Beispiel 1 (b) aus 7-Benzyloxy-8-nitro-2,3,4,5-tetrahydro-1*H-*benzo[*d*]azepin und wässriger Formalin-Lösung in Ameisensäure.
Ausbeute: 1,08 g (99 %)
R_{f}-Wert: 0.8 (Aluminiumoxid; Dichlormethan/Ethanol = 95:5)
C₁₈H₂₀N₂O₃ (312.36)
Massenspektrum: (M+H)+ = 313

### (d) 8-Amino-3-methyl-2,3,4,5-tetrahydro-1H-benzo[d]azepin-7-ol

0,50 g (1,60 mmol) 7-Benzyloxy-3-methyl-8-nitro-2,3,4,5-tetrahydro-1*H-*benzo[*d*]azepin werden in 20 ml Methanol gelöst und mit 100 mg Pd/C 10 % versetzt. Man hydriert in einer Parr- Apparatur bei Raumtemperatur bei 3 bar Wasserstoff- Druck für 2 h. Anschließend wird der Katalysator abfiltriert und das Filtrat i.Vak. eingeengt.
Ausbeute: 0,26 g (84 %)
R_{f}-Wert: 0.1 (Aluminiumoxid; Dichlormethan/Ethanol = 95:5)
C₁₁H₁₆N₂O (192.26)
Massenspektrum: (M+H)+ = 193

### (e) 5-Chlor-thiophen-2-carbonsäure-N-[1-(8-hydroxy-3-methyl-2,3,4,5-tetrahydro-1H-benzo[d]azepin-7-ylcarbamoyl)-1-methyl-ethyl]-amid

Hergestellt analog Beispiel 1 (f) aus 8-Amino-3-methyl-2,3,4,5-tetrahydro-1*H-*benzo[*d*]azepin-7-ol und 2-[(5-Chlor-thiophen-2-yl)-carbonylamino]-2-methyl-propionsäure mit HATU und NMM in DMF bei Raumtemperatur. Anschließend wird i.Vak eingeengt und chromatographisch über RP-Material ( Zorbax StableBond C18; 8µm; Laufmittel: Wasser mit 1,5% Ameisensäure/ Acetonitril = 95:5 => 5:95) gereinigt.
Ausbeute: 0,30 g (49 %)
R_{f}-Wert: 0.40 (Aluminiumoxid; Dichlormethan/Ethanol = 95:5)
C₂₀H₂₄ClN₃O₃S (421.95)
Massenspektrum: (M+H)+ = 422/4 (Chlorisotope)

In Analogie zu den, in den obigen Beispielen beschriebenen Synthesewegen können die folgenden Verbindungen aus gegebenenfalls geschützten Aminosäurederivaten, Benzazepinderivaten und Thiophencarbonsäurederivaten hergestellt werden:

| **Nr.** | **Strukturformel** | **Ausbeute (letzte Stufe)** | **Massenpeak(s)** | **R_{f}-Wert bzw. Rₜ** |
|---|---|---|---|---|
| | **Name** | | | |
| **41** | 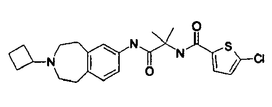 | 32% | (M+H)⁺= 446/448 (Chlorisotope) | Rt = 2.50 min HPLC-Methode 3a |
| | 5-Chlor-thiophen-2-carbonsäure-*N*-[1-(3-cyclobutyl-2,3,4,5-tetrahydro-1*H*-benzo[*d*]azepin-7-ylcarbamoyl)-1-methyl-ethyl]-amid | | | |
| **42** | 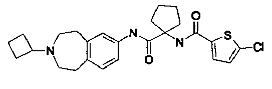 | 46% | (M+H)⁺ = 472/474 (Chlorisotope) | Rt = 2.66 min HPLC-Methode 3a |
| | 1-[(5-Chlor-thiophen-2-yl)-carbonylamino]-*N*-(3-cyclobutyl-2,3,4,5-tetrahydro-1*H*-benzo[*d*]azepin-7-yl)-cyclopentan-1-carbonsäureamid | | | |
| **43** | 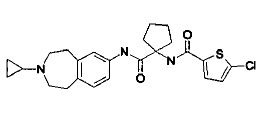 | 49% | (M-H)+ = 458/460 (Chlorisotope) | Rt = 4.41 min HPLC-Methode 3 |
| | 1-[(5-Chlor-thiophen-2-yl)-carbonylamino]-*N*-(3-cyclopropyl-2,3,4,5-tetrahydro-1*H*-benzo[*d*]azepin-7-yl)-cyclopentan-1-carbonsäureamid | | | |
| **44** | 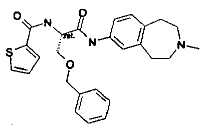 | 70% | (M+H)⁺ = 464 | 0,31 (RP-8;Methanol/ 5% NaCl-Lösung = 6:4) |
| | (S)-Thiophen-2-carbonsäure-*N*-[2-benzyloxy-1-(3-methyl-2,3,4,5-tetrahydro-1*H*-benzo[*d*]azepin-7-ylcarbamoyl)-ethyl]-amid | | | |
| **45** | 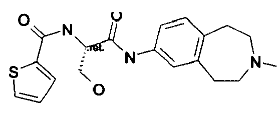 | 11 % | (M+H)⁺ = 374 | 0,60 (RP-8;Methanol/ 5% NaCl-Lösung = 6:4) |
| | (S)-Thiophen-2-carbonsäure-*N*-[2-hydroxy-1-(3-methyl-2,3,4,5-tetrahydro-1*H*-benzo[*d*]azepin-7-ylcarbamoyl)-ethyl]-amid | | | |
| **46** | 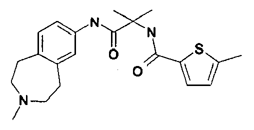 | 54 % | (M-H)- = 386 | 0,63 (Aluminium oxid;Dichlor methan/ Ethanol= 95:5) |
| | 5-Methyl-thiophen-2-carbonsäure-*N*-[1-methyl-1-(3-methyl-2,3,4,5-tetrahydro-1*H*-benzo[*d*]azepin-7-ylcarbamoyl)-ethyl]-amid | | | |
| **47** | 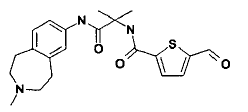 | 42% | (M+H)⁺ = 400 | 0,66 (Aluminium oxid;Dichlor methan/ Ethanol= 95:5) |
| | 5-Formyl-thiophen-2-carbonsäure-*N*-[1-methyl-1-(3-methyl-2,3,4,5-tetrahydro-1*H*-benzo[*d*]azepin-7-ylcarbamoyl)-ethyl]-amid | | | |
| **48** | 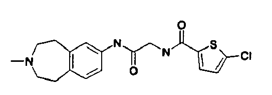 | 38% | (M+H)⁺ = 378/380 | 0.86 min (HPLC-MS; Methode 5) |
| | 5-Chlor-thiophen-2-carbonsäure-*N*-[(3-methyl-2,3,4,5-tetrahydro-1*H*-benzo[*d*]azepin-7-ylcarbamoyl)-methyl]-amid | | | |
| **49** | 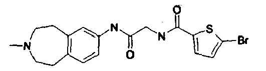 | 29 % 5-Brom-thiophen-2-carbonsäure-*N*-[(3-methyl-2,3,4,5-tetrahydro-1*H*- | (M+H)⁺ = 422/424 (Bromisotope) | 0.88 min (HPLC-MS; Methode 5) |
| | benzo[*d*]azepin-7-ylcarbamoyl)-methyl]-amid | | | |
| **50** | 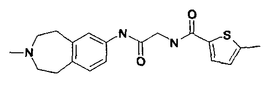 | 34 % | (M-H)- = 358 | 0.82 min (HPLC-MS; Methode 5) |
| | 5-Methyl-thiophen-2-carbonsäure-*N*-[(3-methyl-2,3,4,5-tetrahydro-1*H-*benzo[*d*]azepin-7-ylcarbamoyl)-methyl]-amid | | | |
| **51** | 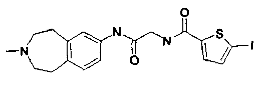 | 29 % | (M+H)⁺ = 470 | 0,89 min (HPLC-MS; Methode 5) |
| | 5-Iod-thiophen-2-carbonsäure-*N*-[(3-methyl-2,3,4,5-tetrahydro-1*H*-benzo[*d*]azepin-7-ylcarbamoyl)-methyl]-amid | | | |

### Beispiel 52

### 3-[(5-Chlor-thiophen-2-yl)carbonylamino]-N-(3-methyl-2,3,4,5-tetrahydro-1H-benzo[d]azepin-7-yl)-1-oxo-tetrahydro-thiophen-3-carbonsäureamid

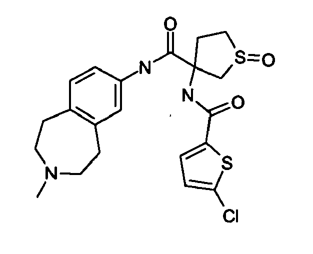

90 mg (0,16 mmol) 3-[(5-Chlor-thiophen-2-yl)-carbonylamino]-*N*-(3-methyl-2,3,4,5-tetrahydro-1*H*-benzo[*d*]azepin-7-yl)-tetrahydrothiophen-3-carbonsäureamid (Bsp. 20) werden in 3,4 ml Dichlormethan und 0,34 ml Eisessig gelöst und bei -5 °C werden 39,3 mg (0,16 mmol) 3-Chlorperoxybenzoesäure zugegeben. Anschließend wird 1 h bei 0 °C gerührt dann auf Raumtemperatur erwärmt und 3 h nachgerührt. Die Reaktion wird mit 5%iger Natriumhydrogencarbonat-Lsg. gewaschen und die organische Phase mit Natriumsulfat getrocknet und eingeengt. Das Rohprodukt wird mit RP-Material (Zorbax StableBond C18; 3,5µm, 4,6x75 mm Laufmittel: Wasser/Acetonitril/Ameisensäure = 95:5:0,1 =>10:90:0,1) gereinigt.
Ausbeute: 14,6 mg (18 %)
R_{f}-Wert: 0.25 (Kieselgel; Dichlormethan/Ethanol/Ammoniak = 8:2:0.2) C₂₁H₂₄ClN₃O₃S₂ (466.02)
Massenspektrum: (M+H)⁺ = 466/468 (Chlorisotope)

### Beispiel 53

### 3-[(5-Chlor-thiophen-2-yl)carbonylamino]-N-(3-methyl-2,3,4,5-tetrahydro-1H-benzo[d]azepin-7-yl)-1,1-dioxo-tetrahydro-thiophen-3-carbonsäureamid

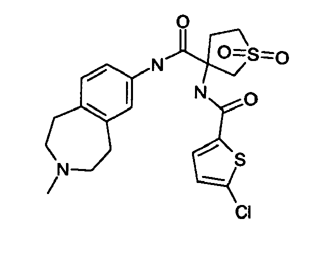

wurde als Nebenprodukt bei der Herstellung von Beispiel 52 isoliert.
Ausbeute: 1,8 mg (2 %)
R_{f}-Wert: 0.11 (Kieselgel; Dichlormethan/Ethanol/Ammoniak = 8:2:0.2) C₂₁H₂₄ClN₃O₄S₂ (482.02)
Massenspektrum: (M+H)⁺ = 482/484 (Chlorisotope)

### Beispiel 54

### 3-[(5-Chlor-thiophen-2-yl)-carbonylamino]-N-(3-methyl-2,3,4,5-tetrahydro-1H-benzo[d]azepin-7-yl)-pyrrolidin-1-tert.butoxycarbonyl-3-carbonsäureamid

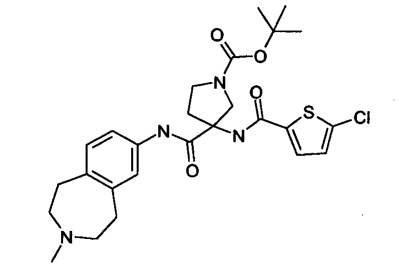

Die Titelverbindung wurde analog der Synthesesequenz Beispiel 1e/1f aus 3-Amino-pyrrolidin-1-tert.butoxycarbonyl-3-carbonsäure, 5-Chlorthiophen-2-carbonsäurechlorid und 3-Methyl-2,3,4,5-tetrahydro-1*H*-benzo[*d*]azepin-7-ylamin hergestellt.
Ausbeute: 15,5 mg
R_{f}-Wert: 0.34 (RP-8; Methanol/5%ige NaCL-Lsg. = 6 : 4)
C₂₆H₃₃ClN₄O₄S (533.09)
Massenspektrum: (M+H)⁺ = 533/535 (Chlorisotope)

### Beispiel 55

### 3-[(5-Chlor-thiophen-2-yl)-carbonylamino]-N-(3-methyl-2,3,4,5-tetrahydro-1H-benzo[d]azepin-7-yl)- pyrrolidin-3-carbonsäureamid

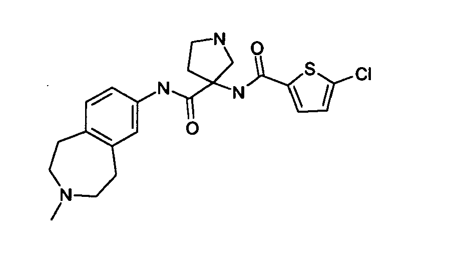

10 mg (0,02 mmol) 3-[(5-Chlor-thiophen-2-yl)-carbonylamino]-*N*-(3-methyl-2,3,4,5-tetrahydro-1*H*-benzo[*d*]azepin-7-yl)-pyrrolidin-1-tert.butoxycarbonyl-3-carbonsäureamid werden in 0,25 ml THF gelöst und bei Raumtemperatur werden 0,3 ml 6 molare Salzsäure zugegeben und es wird 1 h nachgerührt. Anschließend wird i.Vak. eingeengt.
Ausbeute: 10 mg (80 %)
R_{f}-Wert: 0.44 (RP-8; Methanol/5%ige NaCL-Lsg. = 6 : 4)
C₂₁H₂₅ClN₄O₂S (432.98)
Massenspektrum: (M+H)⁺ = 433/435 (Chlorisotope)

In Analogie zu den, in den obigen Beispielen beschriebenen Synthesewegen oder Literatursynthesewegen können die folgenden Verbindungen aus Aminosäurederivaten, Benzazepinderivaten und Thiophencarbonsäurederivaten hergestellt werden:

| **Nr.** | **Strukturformel** | **Ausbeute Letzte Stufe** | **Massenpeak( s)** | **R_{f}-Wert bzw. Rₜ** |
|---|---|---|---|---|
| | **Name** | | | |
| **56** | 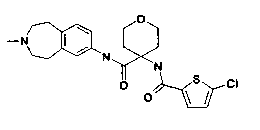 | 68 % | (M+H)⁺ = 448/450 (Chlorisotope) | 2.7 min (HPLC-MS; Methode 4) |
| | 4-[(5-Chlor-thiophen-2-yl)-carbonylamino]-*N*-(3-methyl-2,3,4,5-tetrahydro-1*H*-benzo[*d*]azepin-7-yl)-tetrahydro-pyran-4-carbonsäureamid | | | |
| **57** | 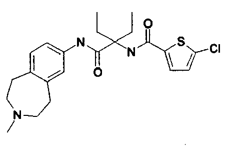 | 25 % | (M+H)⁺ = 434/436 (Chlorisotope) | 0,31 (RP-8;Methan ol/ 5% NaCl-Lösung = 6:4) |
| | 5-Chlor-thiophen-2-carbonsäure-*N*-[1-ethyl-1-(3-methyl-2,3,4,5-tetrahydro-1*H*-benzo[*d*]azepin-7-ylcarbamoyl)-propyl]-amid | | | |
| **59** | 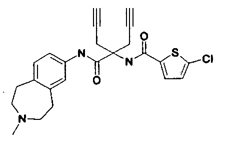 | 45 % | (M+H)⁺ = 454/456 (Chlorisotope) | 0,3 (Kieselgel Dichlorme than/ Ethanol/Ei sessig = 90:10:1) |
| | 5-Chlor-thiophen-2-carbonsäure-*N*-[1-(3-methyl-2,3,4,5-tetrahydro-1*H*-benzo[*d*]azepin-7-ylcarbamoyl)-1-(prop-2-ynyl)-but-3-ynyl]-amid | | | |

### Beispiel 58

### 5-Chlor-thiophen-2-carbonsäure-N-[2-methoxy-1-methoxymethyl-1-(3-methyl-2,3,4,5-tetrahydro-1H-benzo[d]azepin-7-ylcarbamoyl)-ethyl]-amid

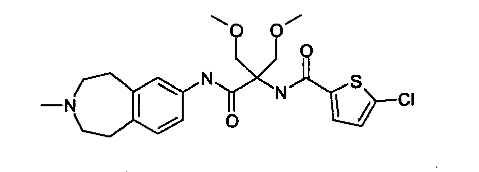

### (a) 5-Chlor-thiophen-2-carbonsäure(1-hydroxymethyl-2-methoxy-1-methoxymethyl-ethyl)-amid

1,14 g (6,16 mmol) 2-Amino-3-methoxy-2-methoxymethyl-propan-1-ol werden in 9 ml THF suspendiert, im Eisbad abgekühlt und mit 2,56 ml (18,4 mmol) TEA versetzt. 1,12 g (6,19 mmol) 5-Chlorthiophen-2-carbonsäurechlorid werden in 6 ml THF gelöst und zugetropft. Nach 1,5 h wird filtriert und das Filtrat i.Vak. eingeengt. Anschließend wird chromatographisch über RP-Material (Microsorb C18 Varian; Laufmittel: Wasser/Acetonitril/Trifluoressigsäure = 90:10:0,1 =>0:100:0,1) gereinigt.
Ausbeute: 0,20 g (8 %)
Rₜ-Wert: 4.25 min (HPLC-MS Methode 3)
C₁₁H₁₆ClNO₄S (293.77)
Massenspektrum: (M+H)⁺ = 294/296 (Chlorisotope)

### (b) 2-[(5-Chlor-thiophen-2-carbonyl)-amino]3-methoxy-2-methoxymethyl-propionsäure

0,20 g (0,49 mmol) 5-Chlor-thiophen-2-carbonsäure(1-hydroxymethyl-2-methoxy-1-methoxymethyl-ethyl)-amid werden bei Raumtemperatur in 3 ml Wasser / 3 ml Acetonitril gelöst und mit 0,08 g (0,51 mmol) 2,2,6,6-Tetramethyl-1-piperidinyloxy-Radikal (TEMPO) und 0,13 g (1,55 mmol) Natriumhydrogencarbonat versetzt. Anschließend wird 1,5 ml (3,14 mmol) 13%iges Natriumhypochlorit zugetropft und 2 h gerührt. Das Reaktionsgemisch wird i.Vak. eingeengt und chromatographisch über RP-Material (Microsorb C18 Varian; Laufmittel: Wasser/ Acetonitril/ Trifluoressigsäure = 90:10:0,1 =>0:100:0,1) gereinigt.
Ausbeute: 0,10 g (66 %)
Rₜ-Wert: 4,16 min (HPLC-MS Methode 3)
C₁₁H₁₄ClNO₅S (307.75)
Massenspektrum: (M+H)⁺ = 306/308 (Chlorisotope)

### (c) 5-Chlor-thiophen-2-carbonsäure-N-[2-methoxy-1-methoxymethyl-1-(3-methyl-2,3,4,5-tetrahydro-1H-benzo[d]azepin-7-ylcarbamoyl)-ethyl]-amid

0,05 g (0,16 mmol) 2-[(5-Chlor-thiophen-2-carbonyl)-amino]-3-methoxy-2-methoxymethyl-propionsäure werden in 1,5 ml THF gelöst und bei Raumtemperatur werden 0,04 g (0,16 mmol) 2-Ethoxy-1-ethoxycarbonyl-1,2-dihydrochinolin (EEDQ) zugegeben. Nach 30 min wird 0,029 g (0,16 mmol) 3-Methyl-2,3,4,5-tetrahydro-1*H*-benzo[*d*]azepin-7-ylamin zugegeben und 12 h bei 40 °C nachgerührt. Anschließend wird i.Vak. eingeengt und chromatographisch über RP-Material (Microsorb C18 Varian; Laufmittel: Wasser/ Acetonitril/ Trifluoressigsäure = 90:10:0,1 =>0:100:0,1) gereinigt.
Ausbeute: 13,9 mg (15%)
Rₜ-Wert: 4,09 min (HPLC-MS Methode 3)
C₂₂H₂₈ClN₃O₄S (466.00)
Massenspektrum: (M+H)⁺ = 466/468 (Chlorisotope)

### Beispiel 60

### 5-Chlor-thiophen-2-carbonsäure-N-[3-methoxy-1-methyl-1-(3-methyl-2,3,4,5-tetrahydro-1H-benzo[d]azepin-7-ylcarbamoyl)-propyl]-amid

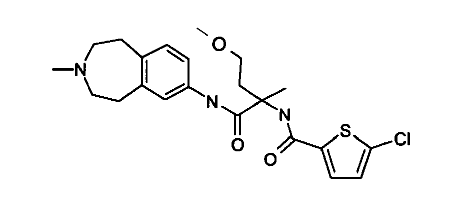

### (a) 2-Benzhydrilidenamino-4-methoxy-butansäureethylester

15.0 g (56 mmol) N-(Diphenylmethylen)glycinethylester werden in 150 ml THF auf -78°C gekühlt, mit 60 ml 1M NaHMDS-Lösung versetzt und 1 h gerührt. Dann werden 11 ml (117 mmol) 1-Brom-2-methoxyethan versetzt und langsam auf 5°C erwärmt. Nach Filtration wird durch mehrmalige Chromatographie aufgereinigt (Kieselgel, Petrolether:Essigester 9:1).
Ausbeute: 8.1 g

### (b) 2-Benzhydrilidenamino-4-methoxy-2-methylbutansäureethylester

Analog Beispiel 60 (a) wird 2-Benzhydrilidenamino-4-methoxy-butansäure mit Methyliodid zur Titelverbindung umgesetzt.
C₂₁H₂₅NO₃ (339.43)
Massenspektrum: (M+H)⁺ = 340

### (c) 5-Chlor-thiophen-2-carbonsäure-N-(1-ethoxycarbonyl-3-methoxy-1-methylpropyl)-amid

0.57 g 2-Benzhydrilidenamino-4-methoxy-2-methylbutansäureethylester in 2.5 ml THF werden mit 0.7 ml 4M HCl versetzt und 2 h bei Raumtemperatur gerührt. Anschließend wird mit Essigester und 1 M HCl versetzt. Die Wasserphase wird dreimal mit Essigester extrahiert und anschließend gefriergetrocknet. Das Rohprodukt wird mit 5-Chlorthiophencarbinsäurechlorid analog Beispiel 1 (e) zur Titelverbindung umgesetzt.
C₂₁H₂₅NO₃ (339.43)
Massenspektrum: (M+H)⁺ = 340

### (d) 5-Chlor-thiophen-2-carbonsäure-N-[3-methoxy-1-methyl-1-(3-methyl-2,3,4,5-tetrahydro-1H-benzo[d]azepin-7-ylcarbamoyl)-propyl]-amid

Zu einem Gemisch aus 130 mg (0.738 mmol) 3-Methyl-2,3,4,5-tetrahydro-1*H-*benzo[*d*]azepin-7-ylamin in Dichlormethan werden 0.60 ml einer 2M Lösung aus Trimethylaluminium in Toluol zugegeben und 15 min gerührt. Anschließend wird das Gemisch zu 233 mg (0.729 mmol) 5-Chlor-thiophen-2-carbonsäure-N-(1-ethoxycarbonyl-3-methoxy-1-methyl-propyl)-amid gegeben und 3 Tage gerührt.
Anschließend wird auf Eiswasser /2 N NaOH gegossen, mehrmals mit
Essigester extrahiert und mit Natriumsulfat getrocknet. Das Rohprodukt wird
chromatographisch gereinigt.
Rₜ-Wert: 2.53 min (HPLC-MS Methode 2)
C₂₂H₂₈ClN₃O₃S (450)
Massenspektrum: (M+H)⁺ = 450/452 (Chlorisotope)

### Beispiel 61

### (R)-5-Chlor-thiophen-2-carbonsäure-N-[2-methoxy-1-methyl-1-(3-methyl-2,3,4,5-tetrahydro-1H-benzo[d]azepin-7-ylcarbamoyl)-ethyl]-amid

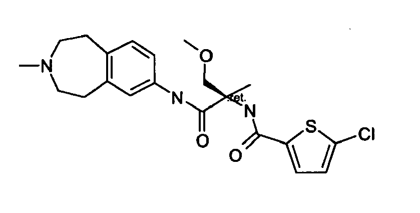

Ein Gemisch aus 1.5 g (6.8 mmol) N-tert.Butoxycarbonyl-α-methylserin in 100 ml Acetonitril wird unter heftigem Rühren innerhalb von 5 h im Stundentakt mit jeweils einem Fünftel von 7.8 g (34 mmol) Silberoxid und 4.3 ml (68 mmol) Jodmethan versetzt und es wird 4 Tage nachgerührt. Anschließend wird filtriert und das Rohprodukt wird mit 3-Methyl-2,3,4,5-tetrahydro-1*H*-benzo[*d*]azepin-7-ylamin und 5-Chlorthiophen-2-carbonsäurechlorid in Analogie zu den in Beispiel 1f, 55 und 58a beschriebenen Reaktionsbedingungen zur Titelverbindung umgesetzt.
R_{f}-Wert: 0.63 (Aluminiumoxid; Dichlormethan/Ethanol = 95:5)
C₂₁H₂₆ClN₃O₃S (435.97)
Massenspektrum: (M+H)⁺ = 436/438 (Chlorisotope)

In Analogie zu den, in den obigen Beispielen beschriebenen Synthesewegen können die folgenden Verbindungen aus Aminosäurederivaten, Benzazepinderivaten und Thiophencarbonsäurederivaten hergestellt werden:

| **Nr.** | **Strukturformel** | **Ausbeute Letzte Stufe** | **Massenpeak(s)** | **R_{f}-Wert bzw. Rt** |
|---|---|---|---|---|
| | **Name** | | | |
| **62** | 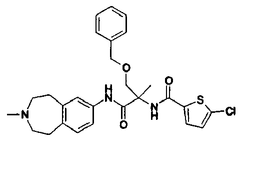 | 57 % | (M+H)⁺ = 512/514 (Chlorisotope) | Rt: 2.8 min HPLC-Methode 3a |
| | 5-Chlor-thiophen-2-carbonsäure-*N*-[2-benzyloxy-1-methyl-1-(3-methyl-2,3,4,5-tetrahydro-1*H*-benzo[*d*]azepin-7-ylcarbamoyl)-ethyl]-amid | | | |
| **63** | 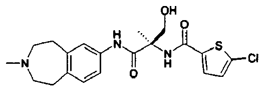 | 4% | (M+H)⁺ = 422/424 (Chlorisotope) | 2.25 min (HPLC; Methode 2) |
| | (R)-5-Chlor-thiophen-2-carbonsäure-*N*-[2-hydroxy-1-methyl-1-(3-methyl-2,3,4,5-tetrahydro-1*H*-benzo[*d*]azepin-7-ylcarbamoyl)-ethyl]-amid | | | |

### Beispiel 64

### 5-Brom-thiophen-2-carbonsäure-N-[3-hydroxy-1-methyl-1-(3-methyl-2,3,4,5-tetrahydro-1H-benzo[d]azepin-7-ylcarbamoyl)-propyl]-amid

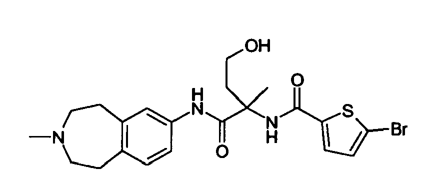

### (a) 2-[(5-Brom-thiophen-2-carbonyl)-amino]-propionsäuremethylester

5,18 g ( 25,30 mmol) 5-Bromthiophen-2-carbonsäure werden in 20 ml Thionylchlorid 1 h bei 60 °C gerührt und anschließend wird i.Vak eingeengt. 3,52 g ( 25,26 mmol) DL-OMe-Ala-HCL werden in 100 ml Dichlormethan mit 20 ml (142,25 mmol) TEA vorgelegt, dann wird bei 0 °C das Säurechlorid in 20 ml Dichlormethan zugetropft. Bei Raumtemperatur wird 16 h gerührt und i.Vak. eingeengt. Anschließend wird mit Natriumhydrogencarbonat-Lsg. versetzt und 3x mit Essigester extrahiert. Die organische Phase wird 1x mit 1 molarer Salzsäure und 1x mit ges.Natriumhydrogencarbonat-Lsg. gewaschen und mit Natriumsulfat getrocknet. Anschließend wird i.Vak. eingeengt und chromatographisch über Kieselgel (Laufmittel: Essigester/Petrolether 1:3 => 1:2) gereinigt.
Ausbeute: 6,0 g ( 82 %)
R_{f}-Wert: 0.17 (Kieselgel; Essigester/Petrolether = 30:70)
C₉H₁₀BrNO₃S (292.15)
Massenspektrum: (M+H)⁺ = 290/292 (Bromisotope)

### (b) 5-Brom-thiophen-2-carbonsäure-N-(3-methyl-2-oxo-tetrahydro-furan-3-yl)-amid (hergestellt analog J. Org. Chem., 1993, 58, 6966)

1,90 ml ( 22,62 mmol) Diisopropylamin und 5,00 ml ( 33,34 mmol) N,N,N,N-Tetramethyl-ethylendiamin (TMEDA) werden bei 0 °C in 110 ml THF vorgelegt und langsam mit 14,2 ml (22,62 mmol) n-Butyllithium 1.6 M in n-Hexan versetzt. Anschließend wird 20 min nachgerührt und auf - 78°C abgekühlt, dann werden 2,12 g (7,26 mmol) 2-[(5-Brom-thiophen-2-carbonyl)-amino]-propionsäuremethylester in 50 ml THF langsam zugetropft und 1h nachgerührt. In einem Zweihalskoblen werden 4,0 ml ( 80,91 mmol) Ethylenoxid bei -78 °C kondensiert und dieses dann zum Reaktionsgemisch gegeben. Bei Raumtemperatur wird 20 h gerührt. Anschließend wird Stickstoff durch das Reaktionsgefäß geleitet um überschüssiges Ethylenoxid auszutreiben, dann wird das Reaktionsgemisch mit wässriger Ammoniumchlorid-Lsg. versetzt und i.Vak. eingeengt. Der Rückstand wird mit 1 M Salzsäure und THF versetzt und mit Essigester extrahiert. Die organische Phase wird mit Natriumsulfat getrocknet und chromatographisch über RP-Material (Laufmittel: Wasser / Acetonitril 90:10 => 0:100) gereinigt.
Ausbeute: 0,20 g (9 %)
R_{f}-Wert: 0.52 (Kieselgel; Essigester/Petrolether = 30:10)
C₁₀H₁₀BrNO₃S (304.16)
Massenspektrum: (M+H)⁺ = 304/306 (Bromisotope)

### (c) 5-Brom-thiophen-2-carbonsäure-N-[3-hydroxy-1-methyl-1-(3-methyl-2,3,4,5-tetrahydro-1H-benzo[d]azepin-7-ylcarbamoyl)-propyl]-amid

0,16 g (0,66 mmol) 3-Methyl-2,3,4,5-tetrahydro-1H-benzo[d]azepin werden bei Raumtemperatur in 3 ml Dichlormethan suspendiert, mit 1,30 ml ( 2,60 mmol) 2M Trimethylaluminium Lsg. in Toluol tropfenweise versetzt und 30 min gerührt. 0,20 g (0,66 mmol) 5-Brom-thiophen-2-carbonsäure (3-methyl-2-oxo-tetrahydrofuran-3-yl)-amid in 6 ml THF zum Reaktionsgemisch getropft. Und 20 h bei Raumtemperatur gerührt. Anschließend wird die Reaktion auf 60 ml 2 M Natronlauge gegossen und 3x mit Essigester extrahiert. Die vereinigten organischen Phasen werden mit Natriumsulfat getrocknet und chromatographisch über RP-Material ( Microsorb C18 Varian Laufmittel: Wasser / Acetonitril 90:10 => 0:100) gereinigt.
Ausbeute: 0,14 g (38 %)
Rₜ-Wert: 2.22 min (HPLC-MS; Methode 3a)
C₂₁H₂₆BrN₃O₃S (480.43)
Massenspektrum: (M+H)⁺ = 480/482 (Bromisotope)

### Beispiel 65

### 5-Chlor-thiophen-2-carbonsäure-N-[1-methyl-3-dimethylaminocarbonyl-1-(3-methyl-2,3,4,5-tetrahydro-1H-benzo[d]azepin-7-ylcarbamoyl)-propyl]-amid

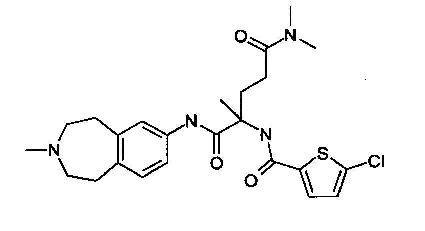

0,20 g (0,77 mmol) N- BOC-α-methyl-D,L-glutaminsäure werden in 5 ml THF unter Rühren bei Raumtemperatur mit 0,24 g (0,77 mmol) TBTU und 0,10 ml (0,77 mmol) TEA versetzt und 5 min gerührt. Anschließend werden 0,38 ml (0,77 mmol) Dimethylamin 2M in THF zugegeben und die Mischung 15 h bei Raumtemperatur gerührt. Das Reaktionsgemisch wird i.Vak eingeengt und analog den obigen Beispielen zur Titelverbindung umgesetzt.
R_{f}-Wert: 0.3 (Kieselgel; Dichlormethan/Ethanol/Ammoniak 80:20:2) C₂₄H₃₁ClN₄O₃S (491.05)
Massenspektrum: (M+H)⁺ = 491/493 (Chlorisotope)

In Analogie zu den, in den Beispielen beschriebenen Synthesewegen können die folgenden Verbindungen aus Aminosäurederivaten, Benzazepinderivaten und Thiophencarbonsäurederivaten hergestellt werden:

| | | | |
|---|---|---|---|
| **66** | 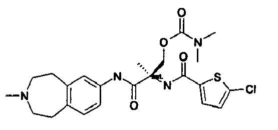 | (M+H)⁺ = 493/495 (Chlorisotope) | Rt: 2.5 min HPLC-Methode 2 |
| | (R)-5-Chlor-thiophen-2-carbonsäure-*N*-[2-dimethylaminocarbonyloxy-1-methyl-1-(3-methyl-2,3,4,5-tetrahydro-1*H*-benzo[*d*]azepin-7-ylcarbamoyl)-ethyl]-amid | | |
| **67** | 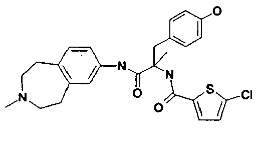 | (M+H)⁺ = 498/500 (Chlorisotope) | Rf: 0.48; Kieselgel; CH₂Cl₂/Ethan ol/NH₃ = 80:20:2 |
| | 5-Chlor-thiophen-2-carbonsäure-*N*-[2-(4-hydroxy-phenyl)-1-methyl-1-(3-methyl-2,3,4,5-tetrahydro-1*H*-benzo[*d*]azepin-7-ylcarbamoyl)-ethyl]-amid | | |
| **68** | 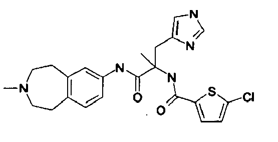 | (m+H)⁺ = 472/474 (Chlorisotope) | Rf: 0.5; Kieselgel; CH₂Cl₂/Ethan ol = 80:20 |
| | 5-Chlor-thiophen-2-carbonsäure-*N*-[2-(1H-imidazol-4-yl)-1-methyl-1-(3-methyl-2,3,4,5-tetrahydro-1*H*-benzo[*d*]azepin-7-ylcarbamoyl)-ethyl]-amid | | |

### Beispiel 69

### (R)-5-Chlor-thiophen-2-carbonsäure-N-[2-(3-methoxycarbonyl-propyloxy)-1-methyl-1-(3-methyl-2,3,4,5-tetrahydro-1H-benzo[d]azepin-7-ylcarbamoyl)-ethyl]-amid

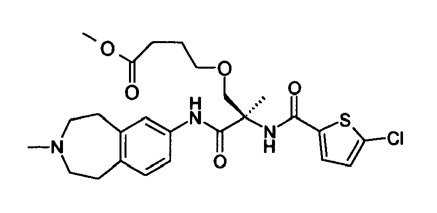

### (a) (R)-N-[2-(3-Methoxycarbonyl-prop-2-enyloxy)-1-methyl-1-(3-methyl-2,3,4,5-tetrahydro-1H-benzo[d]azepin-7-ylcarbamoyl)-ethyl]-carbaminsäuretert.butylester

Eine Mischung von 120 mg (0.226 mmol) (R)-*N*-[2-(Prop-2-enyloxy)-1-methyl-1-(3-methyl-2,3,4,5-tetrahydro-1*H*-benzo[*d*]azepin-7-ylcarbamoyl)ethyl]-carbaminsäuretert.butylester (hergestellt aus (R)-O-Allyl-α-methyl-N-butoxycarbonylserin und 3-Methyl-2,3,4,5-tetrahydro-1*H*-benzo[*d*]azepin-7-ylamin) in Methylenchlorid wird 30 min mit Argon gespült, mit 0.42 ml (4.66 mmol) Acrylsäuremethylester und Grubbs Katalysator 2. Generation versetzt und 4 h zum Sieden erhitzt. Anschließend wird eingeengt und chromatographisch gereinigt.
Rₜ-Wert: 2.53 min (HPLC-MS; Methode 2)

### (b) (R)-5-Chlor-thiophen-2-carbonsäure-N-[2-(3-methoxycarbonyl-propyloxy)-1-methyl-1-(3-methyl-2,3,4,5-tetrahydro-1H-benzo[d]azepin-7-ylcarbamoyl)-ethyl]-amid

(R)-*N*-[2-(3-Methoxycarbonyl-prop-2-enyloxy)-1-methyl-1-(3-methyl-2,3,4,5-tetrahydro-1*H*-benzo[*d*]azepin-7-ylcarbamoyl)-ethyl]-carbaminsäuretert.butylester wird durch eine 3-stufige Synthesesequenz analog Beispiel 1(c), 35 und 1(f) zur Titelverbindung umgesetzt.
Rₜ-Wert: 1.79 min (Methode 6)
C₂₅H₃₂ClN₃O₅S (522.06)
Massenspektrum: (M+H)⁺ = 522/524 (Chlorisotope)

### Beispiel 70

### (R)-5-Chlor-thiophen-2-carbonsäure-N-[2-(3-hydroxycarbonyl-propyloxy)-1-methyl-1-(3-methyl-2,3,4,5-tetrahydro-1H-benzo[d]azepin-7-ylcarbamoyl)-ethyl]-amid

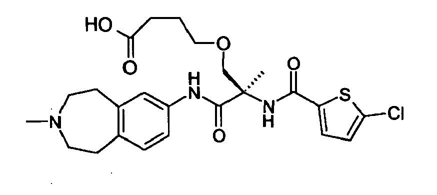

wird aus Beispiel 69 durch Verseifung mit Lithiumhydroxid analog Beispiel 8 (d) hergestellt.
Rₜ-Wert: 2.4 min (HPLC-MS; Methode 2)
C₂₄H₃₀ClN₃O₅S (508.03)
Massenspektrum: (M+H)⁺ = 508/510 (Chlorisotope)

### Beispiel 71

### 5-Chlor-thiophen-2-carbonsäure-N-[1-methyl-1-(3,5-dimethyl-2,3,4,5-tetrahydro-1H-benzo[d]azepin-7-ylcarbamoyl)-ethyl]-amid

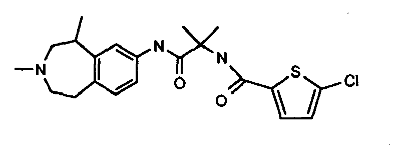

### (a) 3,5-Dimethyl-2,3,4,5-tetrahydro-1H-benzo[d]azepin-7-ylamin und 3,5-Dimethyl-2,3,4,5-tetrahydro-1H-benzo[d]azepin-8-ylamin

Ausgehend von 1-Methyl-2,3,4,5-tetrahydro-1*H-*benzo[*d*]azepin kann durch die Synthesesequenz Leuckart-Wallach-Reaktion analog Beispiel 1 (b), Nitrierung analog Beispiel 9(a) und Reduktion analog Beispiel 9(d) ein Gemisch der beiden Titelverbindungen hergestellt werden, das durch Chromatographie (Kieselgel, Methylenchlorid/(Ethanol: Ammoniak 95:5) 99/1-> 8/2) gereinigt werden kann:
3,5-Dimethyl-2,3,4,5-tetrahydro-1*H-*benzo[*d*]azepin-7-ylamin R_{f}-Wert: 0.70 (Kieselgel; Methylenchlorid/Methanol/Ammoniak 80/20/2) C₁₂H₁₈N₂ (190.29)
   Massenspektrum: (M+H)⁺ = 191
3,5-Dimethyl-2,3,4,5-tetrahydro-1*H-*benzo[*d*]azepin-8-ylamin R_{f}-Wert: 0.75 (Kieselgel; Methylenchlorid/Methanol/Ammoniak 80/20/2) C₁₂H₁₈N₂ (190.29)
   Massenspektrum: (M+H)⁺ = 191

### (b) 5-Chlor-thiophen-2-carbonsäure-N-[1-methyl-1-(3,5-dimethyl-2,3,4,5-tetrahydro-1H-benzo[d]azepin-7-ylcarbamoyl)-ethyl]-amid

wird durch Umsetzung von 3,5-Dimethyl-2,3,4,5-tetrahydro-1*H*-benzo[*d*]azepin-7-ylamin analog Beispiel 1 (f) hergestellt.
R_{f}-Wert: 0.6 (Kieselgel; Methylenchlorid/Methanol/Ammoniak 80/20/2) C₂₁H₂₆ClN₃O₂S (419.97)
Massenspektrum: (M+H)⁺ = 420/422 (Chlorisotope)

### Beispiel 72

### 5-Chlor-thiophen-2-carbonsäure-N-[1-methyl-1-(3,5-dimethyl-2,3,4,5-tetrahydro-1H-benzo[d]azepin-8-ylcarbamoyl)-ethyl]-amid

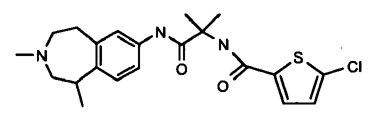

wird aus 3,5-Dimethyl-2,3,4,5-tetrahydro-1*H-*benzo[*d*]azepin-8-ylamin (s.Beispiel 71 a) analog Beispiel 1(f) hergestellt.
R_{f}-Wert: 0.8 (Kieselgel; Methylenchlorid/Methanol/Ammoniak 80/20/2) C₂₁H₂₆ClN₃O₂S (419.97)
Massenspektrum: (M+H)⁺ = 420/422 (Chlorisotope)

### Beispiel 73

### 5-Chlor-thiophen-2-carbonsäure-N-{1-methyl-1-[3-methyl-5-(4-aminophenyl)-2,3,4,5-tetrahydro-1H-benzo[d]azepin-7-ylcarbamoyl]-ethyl}-amid

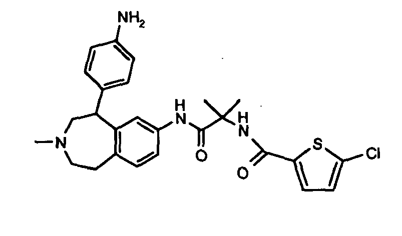

(a) 3-Methyl-5-phenyl-2,3,4,5-tetrahydro-1*H-*benzo[*d*]azepin 1.00 g (4.48 mmol) 1-Phenyl-2,3,4,5-tetrahydro-1*H-*benzo[*d*]azepin wird in 1.7 ml Ameisensäure aufgenommen und mit 1.22 ml 37% Formalinlösung versetzt, anschließend wird 2 h auf 70°C erwärmt. Das Reaktionsgemisch wird aufkonzentriert, mit 10 M NaOH basisch gestellt und mit Essigester extrahiert. Die organischen Phasen werden mit Natriumsulfat getrocknet, anschließend wird filtriert und eingedampft.
   R_{f}-Wert: 0.35 (Kieselgel; Methylenchlorid/Methanol/Ammoniak 95/5/0.5) C₁₇H₁₉N (237.34)
   Massenspektrum: (M+H)⁺ = 238
(b) 3-Methyl-5-(4-nitrophenyl)-7-nitro-2,3,4,5-tetrahydro-1*H-*benzo[*d*]azepin 3-Methyl-5-phenyl-2,3,4,5-tetrahydro-1*H-*benzo[*d*]azepin wird analog zu Beispiel 9(a) nitriert und chromatographisch gereinigt (Kieselgel:
   Petrolether/Essigester 1/1 -> 1/100)
   R_{f}-Wert: 0.2 (Kieselgel; Essigester)
   C₁₇H₁₇N₃O₄ (327.33)
   Massenspektrum: (M+H)⁺ = 328
(c) 5-Chlor-thiophen-2-carbonsäure-*N*-{1-methyl-1-[3-methyl-5-(4-aminophenyl)-2,3,4,5-tetrahydro-1*H-*benzo[*d*]azepin-7-ylcarbamoyl]-ethyl}-amid wird durch Hydrierung von 3-Methyl-5-(4-nitrophenyl)-7-nitro-2,3,4,5-tetrahydro-1*H-*benzo[*d*]azepin analog Beispiel 1 (c) und anschließende Umsetzung mit 2-[(5-Chlor-thiophen-2-yl)-carbonylamino]-2-methyl-propionsäure analog Beispiel 1 (f) hergestellt.
   R_{f}-Wert: 0.6 (Aluminiumoxid; Methylenchlorid/Ethanol 95/5)
   C₂₆H₂₉ClN₄O₂S (497.05)
   Massenspektrum: (M+H)⁺ = 497/499 (Chlorisotope)

In Analogie zu den, in den Beispielen beschriebenen Synthesewegen oder Literatursynthesewegen können die folgenden Verbindungen aus Aminosäurederivaten, Benzazepinderivaten und Thiophencarbonsäurederivaten hergestellt werden:

| | | | |
|---|---|---|---|
| **74** | 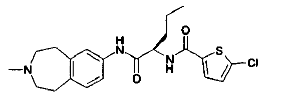 | (M+H)⁺ = 420/422 (Chlorisotope) | Rt: 2.63 min HPLC-Methode 2 |
| | (R)-5-Chlor-thiophen-2-carbonsäure-*N*-[1-(3-methyl-2,3,4,5-tetrahydro-1*H-*benzo[*d*]azepin-7-ylcarbamoyl)-butyl]-amid | | |
| **75** | 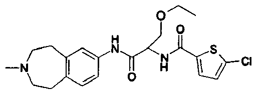 | (m+H)⁺ = 436/438 (Chlorisotope) | Rt: 2.56 min HPLC-Methode 2 |
| | 5-Chlor-thiophen-2-carbonsäure-*N*-[2-ethoxy-1-(3-methyl-2,3,4,5-tetrahydro-1*H-*benzo[*d*]azepin-7-ylcarbamoyl)-ethyl]-amid | | |
| **76** | 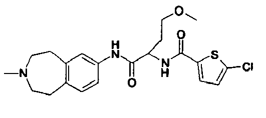 | (M+H)⁺ = 436/438 (Chlorisotope) | Rt: 0.92 min HPLC-Methode 5 |
| | 5-Chlor-thiophen-2-carbonsäure-*N*-[3-methoxy-1-(3-methyl-2,3,4,5-tetrahydro-1*H-*benzo[*d*]azepin-7-ylcarbamoyl)-propyl]-amid | | |
| **77** | 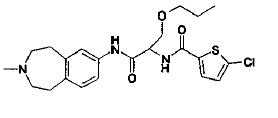 | (M+H)⁺ = 450/452 (Chlorisotope) | Rt: 2.63 min HPLC-Methode 2 |
| | 5-Chlor-thiophen-2-carbonsäure-*N*-[2-propyloxy-1-(3-methyl-2,3,4,5-tetrahydro-1*H-*benzo[*d*]azepin-7-ylcarbamoyl)-ethyl]-amid Rt: 4.62 min | | |
| **78** | 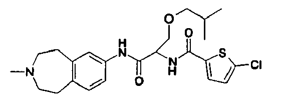 | (M+H)⁺ = 464/466 (Chlorisotope) | HPLC-Methode 3 |
| | 5-Chlor-thiophen-2-carbonsäure-*N*-[2-isobutyloxy-1-(3-methyl-2,3,4,5-tetrahydro-1*H-*benzo[*d*]azepin-7-ylcarbamoyl)-ethyl]-amid | | |
| **79** | 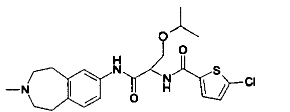 | (M+H)⁺ = 450/452 (Chlorisotope) | Rt: 4.32 min HPLC-Methode 3 |
| | 5-Chlor-thiophen-2-carbonsäure-*N*-[2-isopropyloxy-1-(3-methyl-2,3,4,5-tetrahydro-1*H-*benzo[*d*]azepin-7-ylcarbamoyl)-ethyl]-amid | | |
| **80** | 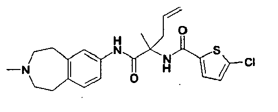 | (M+H)⁺ = 432/434 (Chlorisotope) | Rt: 4.30 min HPLC-Methode 3 |
| | 5-Chlor-thiophen-2-carbonsäure-*N*-[1-methyl-1-(3-methyl-2,3,4,5-tetrahydro-1*H-*benzo[*d*]azepin-7-ylcarbamoyl)-but-3-enyl]-amid | | |
| **81** | 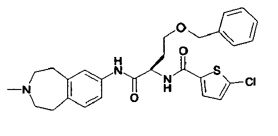 | (M+H)⁺ = 512/514 (Chlorisotope) | Rf: 0.25; Kieselgel; CH₂Cl₂/Etha nol/Ammonia k = 90:10:1 |
| | (R)-5-Chlor-thiophen-2-carbonsäure-*N*-[3-benzyloxy-1-(3-methyl-2,3,4,5-tetrahydro-1*H-*benzo[*d*]azepin-7-ylcarbamoyl)-propyl]-amid | | |
| **82** | 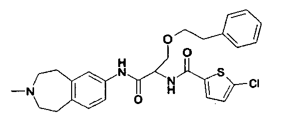 | (M+H)⁺ = 512/514 (Chlorisotope) | Rt: 4.82 min HPLC-Methode 3 |
| | (R)-5-Chlor-thiophen-2-carbonsäure-*N*-[3-phenethyloxy-1-(3-methyl-2,3,4,5-tetrahydro-1*H-*benzo[*d*]azepin-7-ylcarbamoyl)-ethyl]-amid | | |

### Beispiel 83

### 5-Chlor-thiophen-2-carbonsäure-N-[2-(2-methoxyethyloxy)-1-(3-methyl-2,3,4,5-tetrahydro-1H-benzo[d]azepin-7-ylcarbamoyl)-ethyl]-amid

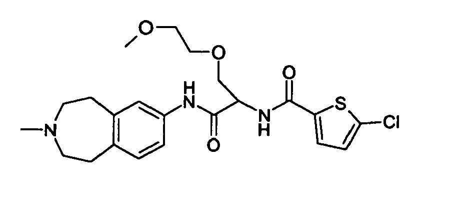

(a) N-(5-Chlorthiophen-2yl)carbonyl-aziridin-2-carbonsäuremethylester Eine Mischung aus 4.40 g (43.5 mmol) Aziridin-2-carbonsäuremethylester und 13.5 ml (97.3 mmol) Triethylamin in 30 ml Methylenchlorid wird unter Eiskühlung mit 8.80 g (48.6 mmol) 5-Chlorthiophencarbonsäurechlorid in 30 ml Methylenchlorid versetzt und anschließend nach Entfernung des Kühlbades 3 h bei Raumtemperatur gerührt. Anschließend wird mit Wasser verdünnt, mit Methylenchlorid extrahiert, mit Natriumsulfat getrocknet, aufkonzentriert und durch Chromatographie (Kieselgel, Petrolether/Essigester 80/15 -> 80/20) zur leicht verunreinigten Titelverbindung gereinigt.
   R_{f}-Wert: 0.42 (Kieselgel; Methylenchlorid/Methanol 80/20) C₉H₈ClNO₃S (245.68)
   Massenspektrum: (M+H)⁺ = 246/248 (Chlorisotope)
(b) 2-(5-Chlorthiophen-2yl)carbonylamino-3-(2-methoxyethyloxy)propionsäuremethylester
   Eine Mischung aus 0.30 g (ca. 1 mmol) N-(5-Chlorthiophen-2yl)carbonyl-aziridin-2-carbonsäuremethylester und 0.29 ml (3.7 mmol) 2-Methoxyethanol in 4 ml Methylenchlorid wird langsam mit 0.16 ml Bortrifluoridetherat versetzt und 3 h gerührt. Das Reaktionsgemisch wird konzentriert und chromatographisch (Kieselgel, Petrolether/Essigester 80/20 -> 40/60) gereingt.
   R_{f}-Wert: 0.05 (Kieselgel; Methylenchlorid/Methanol 80/20)
   C₁₂H₁₆ClNO₆S (321.78)
   Massenspektrum: (M+H)⁺ = 322/324 (Chlorisotope)
(c) 5-Chlor-thiophen-2-carbonsäure-*N*-[2-(2-methoxyethyloxy)-1-(3-methyl-2,3,4,5-tetrahydro-1*H-*benzo[*d*]azepin-7-ylcarbamoyl)-ethyl]-amid Die Tietelverbindung wird aus 2-(5-Chlorthiophen-2yl)carbonylamino-3-(2-methoxyethyloxy)propionsäuremethylester durch Lithiumhydroxidverseifung und anschließende Umsetzung mit 3-Methyl-2,3,4,5-tetrahydro-1*H-*benzo[*d*]azepin-7-ylamin mit EEDQ analog Beispiel 58(c) hergestellt.
   Rₜ-Wert: 3.98 min (Methode 3)
   C₂₂H₂₈ClN₃O₄S (465.99)
   Massenspektrum: (M+H)⁺ = 466/468 (Chlorisotope)

### Beispiel 84 und 85

### 1-[(5-Chlor-thiophen-2-yl)-carbonylamino]-3,4-dihydroxy-N-(3-methyl-2,3,4,5-tetrahydro-1H-benzo[d]azepin-7-yl)-cyclopentan-1-carbonsäureamid (84) und 1-[(5-Chlor-thiophen-2-yl)-carbonylamino]-3-formyloxy-4-hydroxy-N-(3-methyl-2,3,4,5-tetrahydro-1H-benzo[d]azepin-7-yl)-cyclopentan-1-carbonsäureamid (85)

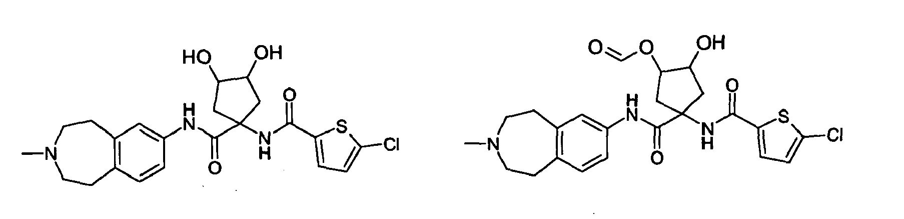

Eine Mischung aus 0.10 g (0.348 mmol) 1-[(5-Chlor-thiophen-2-yl)-carbonylamino]-3,4-epoxy-cyclopentan-1-carbonsäure, 0.113 g (0.352 mmol) TBTU und 0.116 ml (1.055 mmol) NMM in 1.5 ml DMF wird 30 min gerührt und dann mit 62 mg (0.352 mmol) 3-Methyl-2,3,4,5-tetrahydro-1*H-*benzo[*d*]azepin-7-ylamin versetzt und über Nacht gerührt. Es wird mit Wasser versetzt, mit Essigester extrahiert, aufkonzentriert und der Rückstand in DMF und Trifluoressigsäure aufgenommen.und chromatographisch mittels HPLC gereinigt.

### Beispiel 84

Rₜ-Wert: 3.53 min (Methode 3)
C₂₂H₂₆ClN₃O₄S (463.98)
Massenspektrum: (M+H)⁺ = 464/466 (Chlorisotope)

### Beispiel 85

Rₜ-Wert: 3.72 min (Methode 3)
C₂₃H₂₆ClN₃O₅S (491.99)
Massenspektrum: (M+H)⁺ = 492/494 (Chlorisotope)

### Beispiel 86

### 1-[(5-Chlor-thiophen-2-yl)-carbonylamino]-3,4-dimethoxy-N-(3-methyl-2,3,4,5-tetrahydro-1H-benzo[d]azepin-7-yl)-cyclopentan-1-carbonsäureamid

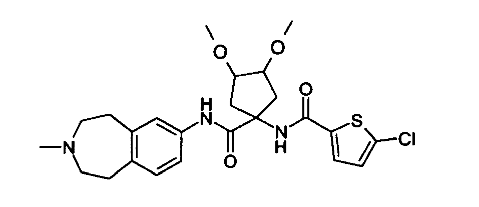

### (a) 1-[(5-Chlor-thiophen-2-yl)-carbonylamino]-3,4-epoxy-cyclopentan-1-carbonsäuremethylester

Eine Mischung aus 0.85 g (2.9 mmol) 1-[(5-Chlor-thiophen-2-yl)-carbonylamino]- cyclopent-3-en-1-carbonsäuremethylester und 20 ml Methylenchlorid wird bei 0°C mit 0.92 g 70%-iger meta-Chlorperbenzoesäure versetzt und 3 h bei Raumtemperatur gerührt. Die Mischung wird mit ges. Natriumhydrogencarbonatlösung gewaschen und konzentriert.
C₁₂H₁₂ClNO₄S (301.75)
Massenspektrum: (M+H)⁺ = 302/304 (Chlorisotope)

### (b) 1-[(5-Chlor-thiophen-2-yl)-carbonylamino]-3,4-dihydroxy-cyclopentan-1-carbonsäuremethylester

Eine Mischung aus 0.76 g (1.84 mmol) 1-[(5-Chlor-thiophen-2-yl)-carbonylamino]-3,4-epoxy-cyclopentan-1-carbonsäuremethylester, 3.0 ml Essigsäure und 0.38 g Kaliumhydrogensulfat werden 4h bei 40^C gerührt. Anschließend wird eingeengt, in DMF gelöst, mit Trifluoressigsäure acidifiziert und mittels präparaiver HPLC gereinigt.
C₁₂H₁₄ClNO₅S (319.76)
Massenspektrum: (M+H)⁺ = 320/322 (Chlorisotope)

### (c) 1-[(5-Chlor-thiophen-2-yl)-carbonylamino]-3,4-dimethoxy-N-(3-methyl-2,3,4,5-tetrahydro-1H-benzo[d]azepin-7-yl)-cyclopentan-1-carbonsäureamid

Die Titelverbindung wurde aus 1-[(5-Chlor-thiophen-2-yl)-carbonylamino]-3,4-dihydroxy-cyclopentan-1-carbonsäuremethylester durch Methylierung analog Beispiel 61 und anschließende Verseifung mit LiOH und Umsetzung mit 3-Methyl-2,3,4,5-tetrahydro-1*H-*benzo[*d*]azepin-7-ylamin hergestellt.
Rₜ-Wert: 4.09 min (HPLC-MS; Methode 3)
C₂₄H₃₀ClN₃O₄S (492.04)
Massenspektrum: (M+H)⁺ = 492/494 (Chlorisotope)

### Beispiel 87

### (R)-5-Brom-thiophen-2-carbonsäure-N-[2-(5-methyl-oxazol-2-yl)-1-(3-methyl-2,3,4,5-tetrahydro-1H-benzo[d]azepin-7-ylcarbamoyl)-ethyl]-amid

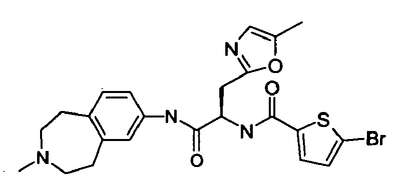

Eine Mischung aus 0.70 g (2.5 mmol) (R)-2-tert.Butoxycarbonylamino-3-propargylaminocarbonyl-propionsäuremethylester, 10 mg Goldtrichlorid und 9.0 ml Acetonitril wird 16 h bei 50°C gerührt. Anschließend wird filtriert, eingeengt und per HPLC gereinigt. Das Rohprodukt wird analog Beispiel 64(c) mit Methyl-2,3,4,5-tetrahydro-1*H-*benzo[*d*]azepin-7-ylamin und anschließender BOC-Abspaltung und Umsetzung mit 5-Bromthiophencarbonsäure analog Beispiel 1 (f) zur Titelverbindung umgesetzt.
Rₜ-Wert: 2.52 min (HPLC-MS; Methode 2)
C₂₃H₂₅BrN₄O₃S (517.45)
Massenspektrum: (M+H)⁺ = 517/519 (Bromisotope)

In Analogie zu den, in den Beispielen beschriebenen Synthesewegen oder Literatursynthesewegen können die folgenden Verbindungen aus Aminosäurederivaten, Benzazepinderivaten und Thiophencarbonsäurederivaten hergestellt werden:

| | | | |
|---|---|---|---|
| **88** | 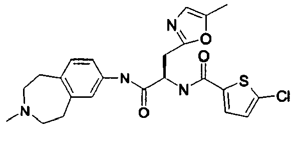 | (M+H)⁺ = 473/475 (Chlorisotope) | Rt: 2.48 min HPLC-Methode 2 |
| | (R)-5-Chlor-thiophen-2-carbonsäure-*N*-[2-(5-methyl-oxazol-2-yl)-1-(3-methyl-2,3,4,5-tetrahydro-1*H*-benzo[*d*]azepin-7-ylcarbamoyl)-ethyl]-amid | | |
| **89** | 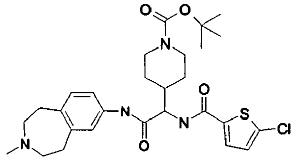 | (M+H)⁺ = 561/563 (Chlorisotope) | Rf: 0.37; Kieselgel; CH₂Cl₂/Ethan ol/Ammoniak = 90:10:1 |
| | 5-Chlor-thiophen-2-carbonsäure-*N*-[C-(1-tert.butoxycarbonylpiperidin-4-yl)-C-(3-methyl-2,3,4,5-tetrahydro-1*H*-benzo[*d*]azepin-7-ylcarbamoyl)-methyl]-amid | | |
| **90** | 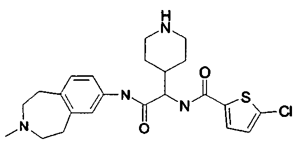 | (M+H)⁺ = 461/463 (Chlorisotope) | Rf: 0.42; RP8; MeOH/5%Na CI-Lsg. = 6/4 |
| | 5-Chlor-thiophen-2-carbonsäure-*N*-[C-(piperidin-4-yl)-C-(3-methyl-2,3,4,5-tetrahydro-1*H-*benzo[*d*]azepin-7-ylcarbamoyl)-methyl]-amid | | |
| **91** | 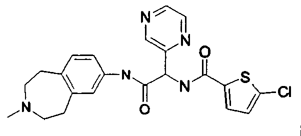 | (M+H)⁺ = 456/458 (Chlorisotope) | Rf: 0.35; RP8; MeOH/5%Na CI-Lsg. = 6/4 |
| | 5-Chlor-thiophen-2-carbonsäure-*N*-[C-(pyrazin-2-yl)-C-(3-methyl-2,3,4,5-tetrahydro-1*H-*benzo[*d*]azepin-7-ylcarbamoyl)-methyl]-amid | | |
| **92** | 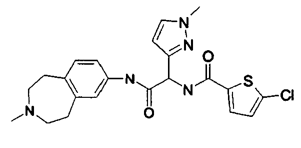 | (M+H)⁺ = 458/460 (Chlorisotope) | Rf: 0.6; Kieselgel; CH₂Cl₂/Ethan ol/Ammoniak = 80:20:2 |
| | 5-Chlor-thiophen-2-carbonsäure-*N*-[C-(1-methyl-pyrazol-3-yl)-C-(3-methyl-2,3,4,5-tetrahydro-1*H-*benzo[*d*]azepin-7-ylcarbamoyl)-methyl]-amid | | |
| **93** | 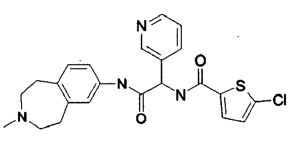 | (M+H)⁺ = 455/457 (Chlorisotope) | Rf: 0.54; Alox; CH₂Cl₂/Ethan ol = 95/5 |
| | 5-Chlor-thiophen-2-carbonsäure-*N*-[C-(pyridin-3-yl)-C-(3-methyl-2,3,4,5-tetrahydro-1*H-*benzo[*d*]azepin-7-ylcarbamoyl)-methyl]-amid | | |
| **94** | 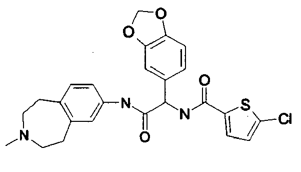 | (M+H)⁺ = 498/500 (Chlorisotope) | Rf: 0.75; Kieselgel; CH₂Cl₂/Ethan ol/Ammoniak = 80:20:2 |
| | 5-Chlor-thiophen-2-carbonsäure-*N*-[C-(3,4-methylendioxophenyl)-C-(3-methyl-2,3,4,5-tetrahydro-1*H-*benzo[*d*]azepin-7-ylcarbamoyl)-methyl]-amid | | |
| **95** | 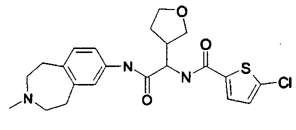 | (M+H)⁺ = 448/450 (Chlorisotope) | Rt: 2.72 min HPLC-Methode 4 |
| | 5-Chlor-thiophen-2-carbonsäure-*N*-[C-(tetrahydrofuran-3-yl)-C-(3-methyl-2,3,4,5-tetrahydro-1*H-*benzo[*d*]azepin-7-ylcarbamoyl)-methyl]-amid | | |
| **96** | 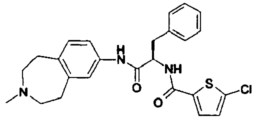 | (M+H)⁺ = 468/470 (Chlorisotope) | Rf: 0.79; Alox; CH₂Cl₂/Ethan ol = 95/5 |
| | (R)-5-Chlor-thiophen-2-carbonsäure-*N*-[2-phenyl-1-(3-methyl-2,3,4,5-tetrahydro-1*H*-benzo[*d*]azepin-7-ylcarbamoyl)-ethyl]-amid | | |
| **97** | 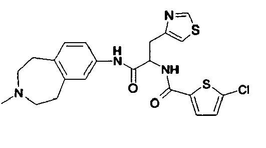 | (M+H)⁺ = 475/477 (Chlorisotope) | Rf: 0.56; Alox; CH₂Cl₂/Ethan ol = 95/5 |
| | 5-Chlor-thiophen-2-carbonsäure-*N*-[2-(thiazol-4-yl)-1-(3-methyl-2,3,4,5-tetrahydro-1*H*-benzo[*d*]azepin-7-ylcarbamoyl)-ethyl]-amid | | |
| **98** | 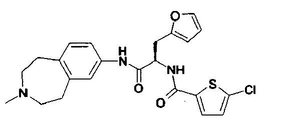 | (M+H)⁺ = 458/460 (Chlorisotope) | Rf: 0.77; Alox; CH₂Cl₂/Ethan ol = 95/5 |
| | (R)-5-Chlor-thiophen-2-carbonsäure-*N*-[2-(furan-2-yl)-1-(3-methyl-2,3,4,5-tetrahydro-1*H*-benzo[*d*]azepin-7-ylcarbamoyl)-ethyl]-amid Rf: 0.54; | | |
| **99** | 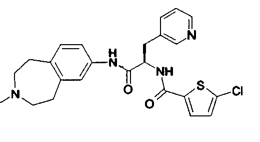 | (M+H)⁺ = 469/471 (Chlorisotope) | Kieselgel; CH₂Cl₂/Ethan ol/Ammoniak = 80:20:2 |
| | (R)-5-Chlor-thiophen-2-carbonsäure-*N-*[2-(pyridin-3-yl)-1-(3-methyl-2,3,4,5-tetrahydro-1*H*-benzo[*d*]azepin-7-ylcarbamoyl)-ethyl]-amid | | |
| **100** | 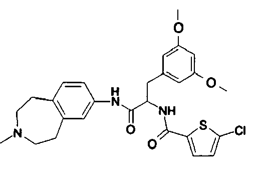 | (M+H)⁺ = 528/530 (Chlorisotope) | Rf: 0.27; Kieselgel; CH₂Cl₂/Ethan ol/Ammoniak = 90:10:1 |
| | 5-Chlor-thiophen-2-carbonsäure-*N*-[2-(3,5-dimethoxyphenyl)-1-(3-methyl-2,3,4,5-tetrahydro-1*H*-benzo[*d*]azepin-7-ylcarbamoyl)-ethyl]-amid | | |
| **101** | 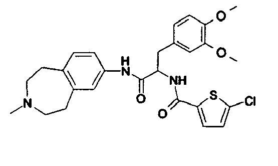 | (M+H)⁺ = 528/530 (Chlorisotope) | Rf: 0.7; Kieselgel; CH₂Cl₂/Ethan ol/Ammoniak = 80:20:2 |
| | 5-Chlor-thiophen-2-carbonsäure-*N*-[2-(3,4-dimethoxyphenyl)-1-(3-methyl-2,3,4,5-tetrahydro-1*H*-benzo[*d*]azepin-7-ylcarbamoyl)-ethyl]-amid | | |
| **102** | 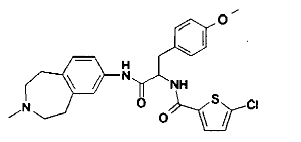 | (M+H)⁺ = 498/500 (Chlorisotope) | Rf: 0.7; Kieselgel; CH₂Cl₂/Ethan ol/Ammoniak = 80:20:2 |
| | 5-Chlor-thiophen-2-carbonsäure-*N*-[2-(4-methoxyphenyl)-1-(3-methyl-2,3,4,5-tetrahydro-1*H*-benzo[*d*]azepin-7-ylcarbamoyl)-ethyl]-amid | | |
| **103** | 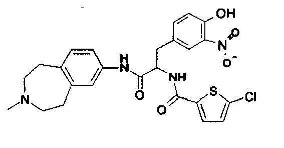 | (M+H)⁺ = 529/531 (Chlorisotope) | Rf: 0.18; Kieselgel; CH₂Cl₂/Ethan ol/Ammoniak = 80:20:2 |
| | 5-Chlor-thiophen-2-carbonsäure-*N*-[2-(4-hydroxy-3-nitro-phenyl)-1-(3-methyl-2,3,4,5-tetrahydro-1*H*-benzo[*d*]azepin-7-ylcarbamoyl)-ethyl]-amid | | |
| **104** | 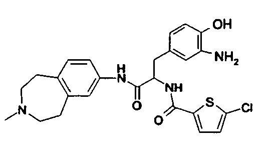 | (M+H)⁺ = 499/501 (Chlorisotope) | Rf: 0.30; Kieselgel; CH₂Cl₂/Ethan ol/Ammoniak = 80:20:2 |
| | 5-Chlor-thiophen-2-carbonsäure-*N*-[2-(4-hydroxy-3-amino-phenyl)-1-(3-methyl-2,3,4,5-tetrahydro-1*H*-benzo[*d*]azepin-7-ylcarbamoyl)-ethyl]-amid | | |
| **105** | 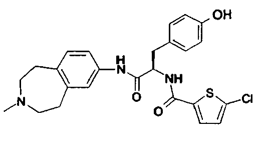 | (M+H)⁺ = 484/486 (Chlorisotope) | Rf: 0.5; Kieselgel; CH₂Cl₂/Ethan ol/Ammoniak = 80:20:2 |
| | (R)-5-Chlor-thiophen-2-carbonsäure-*N*-[2-(4-hydroxy-phenyl)-1-(3-methyl-2,3,4,5-tetrahydro-1*H*-benzo[*d*]azepin-7-ylcarbamoyl)-ethyl]-amid | | |
| **106** | 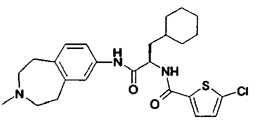 | (M+H)⁺ = 474/476 (Chlorisotope) | Rf: 0.18; Kieselgel; CH₂Cl₂/Ethan ol = 80:20 |
| | (R)-5-Chlor-thiophen-2-carbonsäure-*N*-[2-cyclohexyl-1-(3-methyl-2,3,4,5-tetrahydro-1*H*-benzo[*d*]azepin-7-ylcarbamoyl)-ethyl]-amid | | |
| **107** | 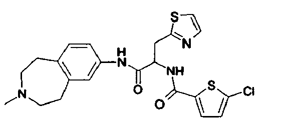 | (M+H)⁺ = 475/477 (Chlorisotope) | Rt: 2.7 min HPLC-Methode 4 |
| | 5-Chlor-thiophen-2-carbonsäure-*N*-[2-(thiazol-2-yl)-1-(3-methyl-2,3,4,5-tetrahydro-1*H*-benzo[*d*]azepin-7-ylcarbamoyl)-ethyl]-amid | | |
| **108** | 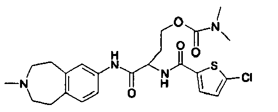 | (M+H)⁺ = 493/495 (Chlorisotope) | Rt: 4.14 min HPLC-Methode 3 |
| | 5-Chlor-thiophen-2-carbonsäure-*N*-[3-dimethylaminocarbonyloxy-1-(3-methyl-2,3,4,5-tetrahydro-1*H*-benzo[*d*]azepin-7-ylcarbamoyl)-propyl]-amid | | |
| **109** | 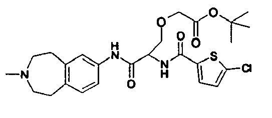 | (M+H)⁺ = 522/524 (Chlorisotope) | Rf: 0.74; Kieselgel; CH₂Cl₂/Ethan ol/Ammoniak = 80:20:2 |
| | 5-Chlor-thiophen-2-carbonsäure-*N*-[2-tert.butyloxycarbonylmethyloxy-1-(3-methyl-2,3,4,5-tetrahydro-1*H*-benzo[*d*]azepin-7-ylcarbamoyl)-ethyl]-amid | | |
| **110** | 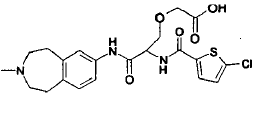 | (M+H)⁻ = 464/466 (Chlorisotope) | Rf: 0.45; RP8; MeOH/5%Na CI-Lsg. = 6/4 |
| | 5-Chlor-thiophen-2-carbonsäure-*N*-[2-hydroxycarbonylmethyloxy-1-(3-methyl-2,3,4,5-tetrahydro-1*H*-benzo[*d*]azepin-7-ylcarbamoyl)-ethyl]-amid | | |
| **111** | 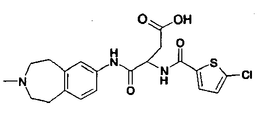 | (M+H)⁺ = 436/438 (Chlorisotope) | Rf: 0.65; RP8; MeOH/5%Na CI-Lsg. = 6/4 |
| | 5-Chlor-thiophen-2-carbonsäure-*N*-[2-hydroxycarbonyl-1-(3-methyl-2,3,4,5-tetrahydro-1*H*-benzo[*d*]azepin-7-ylcarbamoyl)-ethyl]-amid | | |
| **112** | 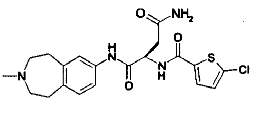 | (M+H)⁺ = 435/437 (Chlorisotope) | Rf: 0.25; Kieselgel; CH₂Cl₂/Ethan ol/Ammoniak = 80:20:2 |
| | (R)-5-Chlor-thiophen-2-carbonsäure-*N*-[2-aminocarbonyl-1-(3-methyl-2,3,4,5-tetrahydro-1*H*-benzo[d]azepin-7-ylcarbamoyl)-ethyl]-amid | | |
| **113** | 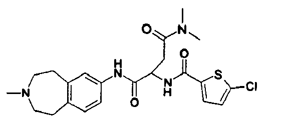 | (M+H)⁺ = 463/465 (Chlorisotope) | Rf: 0.6; Kieselgel; CH₂Cl₂/Ethan ol/Ammoniak = 80:20:2 |
| | 5-Chlor-thiophen-2-carbonsäure-*N*-[2-dimethylaminocarbonyl-1-(3-methyl-2,3,4,5-tetrahydro-1*H*-benzo[*d*]azepin-7-ylcarbamoyl)-ethyl]-amid | | |
| **114** | 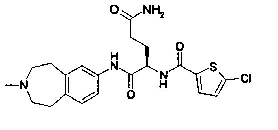 | (M+H)⁺ = 449/451 (Chlorisotope) | Rf: 0.2; Kieselgel; CH₂Cl₂/Ethan ol/Ammoniak = 80:20:2 |
| | (R)-5-Chlor-thiophen-2-carbonsäure-*N*-[3-aminocarbonyl-1-(3-methyl-2,3,4,5-tetrahydro-1*H*-benzo[*d*]azepin-7-ylcarbamoyl)-propyl]-amid | | |
| **115** | 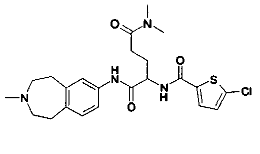 | (M+H)⁺ = 477/479 (Chlorisotope) | Rf: 0.3; Kieselgel; CH₂Cl₂/Ethan ol/Ammoniak = 80:20:2 |
| | 5-Chlor-thiophen-2-carbonsäure-*N*-[3-dimethylaminocarbonyl-1-(3-methyl-2,3,4,5-tetrahydro-1*H*-benzo[*d*]azepin-7-ylcarbamoyl)-propyl]-amid | | |
| **116** | 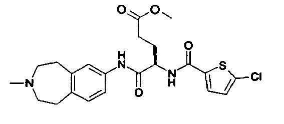 | (M+H)⁺ = 464/466 (Chlorisotope) | Rt: 0.94 min HPLC-Methode 5 |
| | (R)-5-Chlor-thiophen-2-carbonsäure-*N*-[3-methyloxycarbonyl-1-(3-methyl-2,3,4,5-tetrahydro-1*H*-benzo[*d*]azepin-7-ylcarbamoyl)-propyl]-amid | | |
| **117** | 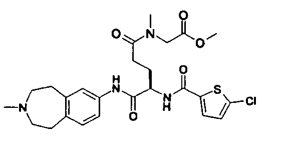 | (M+H)⁺ = 535/537 (Chlorisotope) | Rt: 0.90 min HPLC-Methode 5 |
| | (R)-5-Chlor-thiophen-2-carbonsäure-*N*-[3-(N-methyloxycarbonylmethyl-*N*-methyl-aminocarbonyl)-1-(3-methyl-2,3,4,5-tetrahydro-1*H*-benzo[*d*]azepin-7-ylcarbamoyl)-propyl]-amid | | |
| **118** | 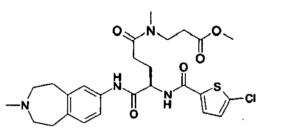 | (M+H)⁺ = 549/551 (Chlorisotope) | Rt: 0.89 min HPLC-Methode 5 |
| | (R)-5-Chlor-thiophen-2-carbonsäure-*N*-[3-(N-(2-methyloxycarbonyl-ethyl)-N-methyl-aminocarbonyl)-1-(3-methyl-2,3,4,5-tetrahydro-1*H-*benzo[*d*]azepin-7-ylcarbamoyl)-propyl]-amid | | |
| **119** | 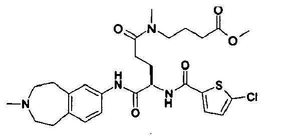 | (M+H)⁺ = 563/565 (Chlorisotope) | Rt: 0.89 min HPLC-Methode 5 |
| | (R)-5-Chlor-thiophen-2-carbonsäure-*N*-[3-(N-(3-methyloxycarbonyl-propyl)-N-methyl-aminocarbonyl)-1-(3-methyl-2,3,4,5-tetrahydro-1*H-*benzo[*d*]azepin-7-ylcarbamoyl)-propyl]-amid | | |
| **120** | 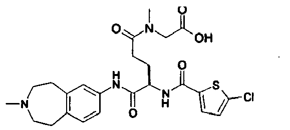 | (M+H)⁺ = 521/523 (Chlorisotope) | Rt: 0.84 min HPLC-Methode 5 |
| | (R)-5-Chlor-thiophen-2-carbonsäure-*N*-[3-(N-hydroxycarbonylmethyl-N-methyl-aminocarbonyl)-1-(3-methyl-2,3,4,5-tetrahydro-1*H-*benzo[*d*]azepin-7-ylcarbamoyl)-propyl]-amid | | |
| **121** | 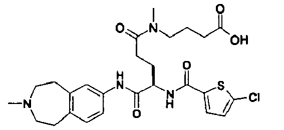 | (M+H)⁺ = 549/551 (Chlorisotope) | Rt: 0.85 min HPLC-Methode 5 |
| | (R)-5-Chlor-thiophen-2-carbonsäure-*N*-[3-(N-(3-hydroxycarbonyl-propyl)-N-methyl-aminocarbonyl)-1-(3-methyl-2,3,4,5-tetrahydro-1*H-*benzo[*d*]azepin-7-ylcarbamoyl)-propyl]-amid | | |
| **122** | 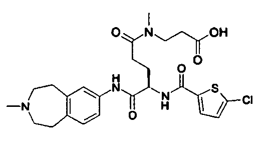 | (M+H)⁺ = 535/537 (Chlorisotope) | Rt: 0.84 min HPLC-Methode 5 |
| | (R)-5-Chlor-thiophen-2-carbonsäure-*N*-[3-(N-(2-hydroxycarbonyl-ethyl)-N-methyl-aminocarbonyl)-1-(3-methyl-2,3,4,5-tetrahydro-1*H-*benzo[*d*]azepin-7-ylcarbamoyl)-propyl]-amid | | |
| **123** | 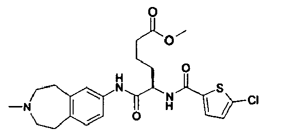 | (M+H)⁺ = 478/480 (Chlorisotope) | Rt: 2.55 min HPLC-Methode 2 |
| | (R)-5-Chlor-thiophen-2-carbonsäure-*N*-[4-methyloxycarbonyl-1-(3-methyl-2,3,4,5-tetrahydro-1*H*-benzo[*d*]azepin-7-ylcarbamoyl)-butyl]-amid | | |
| **124** | 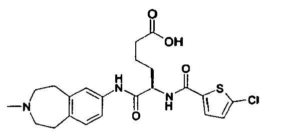 | (M+H)⁺ = 464/466 (Chlorisotope) | Rt: 3.87 min HPLC-Methode 3 |
| | (R)-5-Chlor-thiophen-2-carbonsäure-*N*-[4-hydroxycarbonyl-1-(3-methyl-2,3,4,5-tetrahydro-1*H-*benzo[*d*]azepin-7-ylcarbamoyl)-butyl]-amid | | |
| **125** | 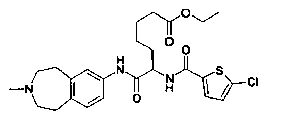 | (M+H)⁺ = 506/508 (Chlorisotope) | Rt: 2.71 min HPLC-Methode 2 |
| | (R)-5-Chlor-thiophen-2-carbonsäure-*N*-[5-ethyloxycarbonyl-1-(3-methyl-2,3,4,5-tetrahydro-1*H*-benzo[*d*]azepin-7-ylcarbamoyl)-pentyl]-amid | | |
| **126** | 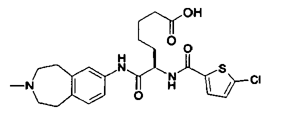 | (M+H)⁺ = 478/480 (Chlorisotope) | Rt: 3.96 min HPLC-Methode 3 |
| | (R)-5-Chlor-thiophen-2-carbonsäure-*N*-[5-hydroxycarbonyl-1-(3-methyl-2,3,4,5-tetrahydro-1*H*-benzo[*d*]azepin-7-ylcarbamoyl)-pentyl]-amid | | |
| **127** | 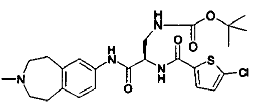 | (M+H)⁺ = 507/509 (Chlorisotope) | Rt: 4.43 min HPLC-Methode 3 |
| | (R)-5-Chlor-thiophen-2-carbonsäure-*N*-[2-tert.butyloxycarbonylamino-1-(3-methyl-2,3,4,5-tetrahydro-1*H-*benzo[*d*]azepin-7-ylcarbamoyl)-ethyl]-amid | | |
| **128** | 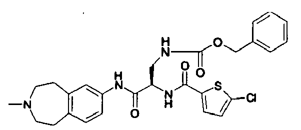 | (M+H)⁺ = 541/543 (Chlorisotope) | Rt: 1.03 min HPLC-Methode 5 |
| | (R)-5-Chlor-thiophen-2-carbonsäure-*N*-[2-benzyloxycarbonylamino-1-(3-methyl-2,3,4,5-tetrahydro-1H benzo[*d*]azepin-7-ylcarbamoyl)-ethyl]-amid | | |
| **129** | 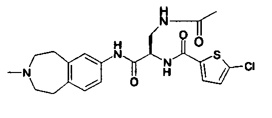 | (M+H)⁺ = 449/451 (Chlorisotope) | Rt: 0.84 min HPLC-Methode 5 |
| | (R)-5-Chlor-thiophen-2-carbonsäure-*N*-[2-acetylamino-1-(3-methyl-2,3,4,5-tetrahydro-1*H*-benzo[*d*]azepin-7-ylcarbamoyl)-ethyl]-amid | | |
| **130** | 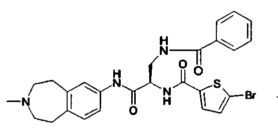 | (M+H)⁺ = 555/557 (Bromisotope) | Rt: 0.98 min HPLC-Methode 5 |
| | (R)-5-Brom-thiophen-2-carbonsäure-*N*-[2-benzoylamino-1-(3-methyl-2,3,4,5-tetrahydro-1*H*-benzo[*d*]azepin-7-ylcarbamoyl)-ethyl]-amid | | |
| **131** | 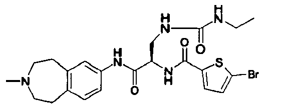 | (M+H)⁺ = 522/524 (Bromisotope) | Rt: 0.90 min HPLC-Methode 5 |
| | (R)-5-Brom-thiophen-2-carbonsäure-*N*-[2-ethylaminocarbonylamino-1-(3-methyl-2,3,4,5-tetrahydro-1*H-*benzo[*d*]azepin-7-ylcarbamoyl)-ethyl]-amid | | |
| **132** | 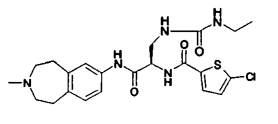 | (M+H)⁺ = 478/480 (Chlorisotope) | Rt: 0.88 min HPLC-Methode 5 |
| | (R)-5-Chlor-thiophen-2-carbonsäure-*N*-[2-ethylaminocarbonylamino-1-(3-methyl-2,3,4,5-tetrahydro-1*H* benzo[*d*]azepin-7-ylcarbamoyl)-ethyl]-amid | | |
| **133** | 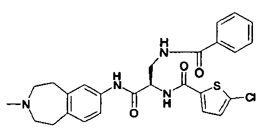 | (M+H)⁺ = 511/513 (Chlorisotope) | Rt: 0.97 min HPLC-Methode 5 |
| | (R)-5-Chlor-thiophen-2-carbonsäure-*N*-[2-benzoylamino-1-(3-methyl-2,3,4,5-tetrahydro-1*H*-benzo[*d*]azepin-7-ylcarbamoyl)-ethyl]-amid | | |
| **134** | 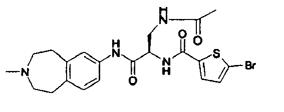 | (M+H)⁺ = 493/495 (Bromisotope) | Rt: 0.85 min HPLC-Methode 5 |
| | (R)-5-Brom-thiophen-2-carbonsäure-*N*-[2-acetylamino-1-(3-methyl-2,3,4,5-tetrahydro-1*H*-benzo[*d*]azepin-7-ylcarbamoyl)-ethyl]-amid | | |
| **135** | 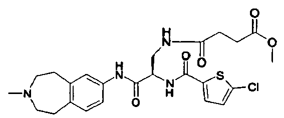 | (M+H)⁺ = 521/523 (Chlorisotope) | Rt: 3.99 min HPLC-Methode 3 |
| | (R)-5-Chlor-thiophen-2-carbonsäure-*N*-[2-(2-methoxycarbonyl-ethyl)carbonylamino-1-(3-methyl-2,3,4,5-tetrahydro-1*H*-benzo[*d*]azepin-7-ylcarbamoyl)-ethyl]-amid | | |
| **136** | 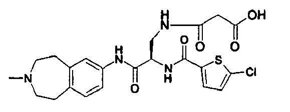 | (M+H)⁺ = 493/495 (Chlorisotope) | Rt: 3.61 min HPLC-Methode 3 |
| | (R)-5-Chlor-thiophen-2-carbonsäure-*N*-[2-hydroxycarbonylmethylcarbonylamino-1-(3-methyl-2,3,4,5-tetrahydro-1*H*-benzo[*d*]azepin-7-ylcarbamoyl)-ethyl]-amid | | |
| **137** | 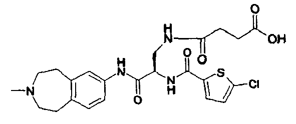 | (M+H)⁺ = 507/509 (Chlorisotope) | Rt: 3.64 min HPLC-Methode 3 |
| | (R)-5-Chlor-thiophen-2-carbonsäure-*N*-[2-(2-hydroxycarbonyl-ethyl)carbonylamino-1-(3-methyl-2,3,4,5-tetrahydro-1*H*-benzo[*d*]azepin-7-ylcarbamoyl)-ethyl]-amid | | |
| **138** | 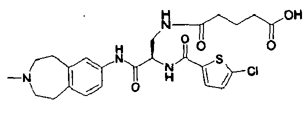 | (M+H)⁺ = 521/523 (Chlorisotope) | Rt: 3.66 min HPLC-Methode 3 |
| | (R)-5-Chlor-thiophen-2-carbonsäure-*N*-[2-(3-hydroxycarbonyl-propyl)carbonylamino-1-(3-methyl-2,3,4,5-tetrahydro-1*H*-benzo[*d*]azepin-7-ylcarbamoyl)-ethyl]-amid | | |
| **139** | 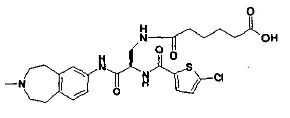 | (M+H)⁺ = 535/537 (Chlorisotope) | Rt: 3.71 min HPLC-Methode 3 |
| | (R)-5-Chlor-thiophen-2-carbonsäure-*N*-[2-(4-hydroxycarbonyl-butyl)carbonylamino-1-(3-methyl-2,3,4,5-tetrahydro-1*H-*benzo[*d*]azepin-7-ylcarbamoyl)-ethyl]-amid | | |
| **140** | 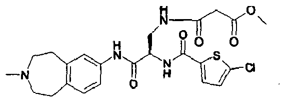 | (M+H)⁺ = 507/509 (Chlorisotope) | Rt: 3.83 min HPLC-Methode 3 |
| | (R)-5-Chlor-thiophen-2-carbonsäure-*N*-[2-methoxycarbonylmethylcarbonylamino-1-(3-methyl-2,3,4,5-tetrahydro-1*H*-benzo[*d*]azepin-7-ylcarbamoyl)-ethyl]-amid | | |
| **141** | 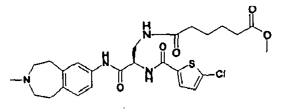 | (M+H)⁺ = 549/551 (Chlorisotope) | Rt: 4.04 min HPLC-Methode 3 |
| | (R)-5-Chlor-thiophen-2-carbonsäure-*N*-[2-(4-methoxycarbonyl-butyl)carbonylamino-1-(3-methyl-2,3,4,5-tetrahydro-1*H*-benzo[*d*]azepin-7-ylcarbamoyl)-ethyl]-amid | | |
| **142** | 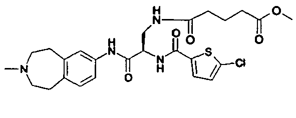 | (M+H)⁺ = 535/537 (Chlorisotope) | Rt: 3.95 min HPLC-Methode 3 |
| | (R)-5-Chlor-thiophen-2-carbonsäure-*N*-[2-(3-methoxycarbonyl-propyl)carbonylamino-1-(3-methyl-2,3,4,5-tetrahydro-1*H*-benzo[*d*]azepin-7-ylcarbamoyl)-ethyl]-amid | | |
| **143** | 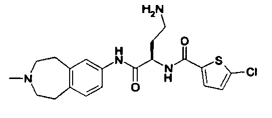 | (M+H)⁺ = 421/423 (Chlorisotope) | Rt: 0.77 min HPLC-Methode 5 |
| | (R)-5-Chlor-thiophen-2-carbonsäure-*N*-[3-amino-1-(3-methyl-2,3,4,5-tetrahydro-1*H*-benzo[*d*]azepin-7-ylcarbamoyl)-propyl]-amid | | |
| **144** | 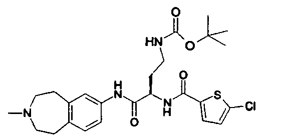 | (M+H)⁺ = 521/523 (Chlorisotope) | Rt: 1.02 min HPLC-Methode 5 |
| | (R)-5-Chlor-thiophen-2-carbonsäure-*N*-[3-tert.butoxycarbonylamino-1-(3-methyl-2,3,4,5-tetrahydro-1*H*-benzo[*d*]azepin-7-ylcarbamoyl)-propyl]-amid | | |
| **145** | 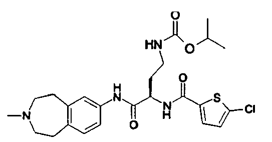 | (M+H)⁺ = 507/509 (Chlorisotope) | Rt: 0.97 min HPLC-Methode 5 |
| | (R)-5-Chlor-thiophen-2-carbonsäure-*N*-[3-isopropyloxycarbonylamino-1-(3-methyl-2,3,4,5-tetrahydro-1*H*-benzo[*d*]azepin-7-ylcarbamoyl)-propyl]-amid | | |
| **146** | 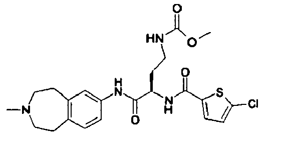 | (M+H)⁺ = 479/481 (Chlorisotope) | Rt: 0.90 min HPLC-Methode 5 |
| | (R)-5-Chlor-thiophen-2-carbonsäure-*N*-[3-methyloxycarbonylamino-1-(3-methyl-2,3,4,5-tetrahydro-1*H*-benzo[*d*]azepin-7-ylcarbamoyl)-propyl]-amid | | |
| **147** | 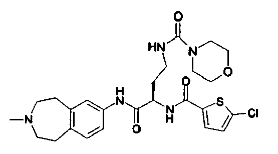 | (M+H)⁺ = 534/536 (Chlorisotope) | Rt: 0.87 min HPLC-Methode 5 |
| | (R)-5-Chlor-thiophen-2-carbonsäure-*N*-[3-(morpholin-4-yl)carbonylamino-1-(3-methyl-2,3,4,5-tetrahydro-1*H*-benzo[*d*]azepin-7-ylcarbamoyl)-propyl]-amid | | |
| **148** | 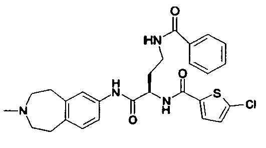 | (M+H)⁺= 525/527 (Chlorisotope) | Rt: 0.97 min HPLC-Methode 5 |
| | (R)-5-Chlor-thiophen-2-carbonsäure-*N*-[3-benzoylamino-1-(3-methyl-2,3,4,5-tetrahydro-1*H*-benzo[d]azepin-7-ylcarbamoyl)-propyl]-amid | | |
| **149** | 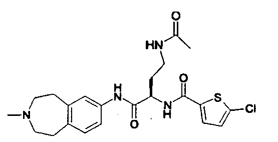 | (M+H)⁺ = 463/465 (Chlorisotope) | Rt: 0.85 min HPLC-Methode 5 |
| | (R)-5-Chlor-thiophen-2-carbonsäure-*N*-[3-acetylamino-1-(3-methyl-2,3,4,5-tetrahydro-1*H*-benzo[*d*]azepin-7-ylcarbamoyl)-propyl]-amid | | |
| **150** | 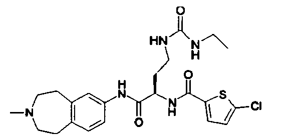 | (M+H)⁺ = 492/494 (Chlorisotope) | Rt: 0.88 min HPLC-Methode 5 |
| | (R)-5-Chlor-thiophen-2-carbonsäure-*N*-[3-ethylaminocarbonylamino-1-(3-methyl-2,3,4,5-tetrahydro-1*H*-benzo[*d*]azepin-7-ylcarbamoyl)-propyl]-amid | | |
| **151** | 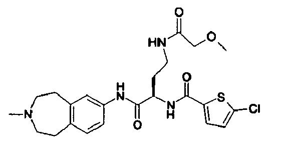 | (M+H)⁺ = 493/495 (Chlorisotope) | Rt: 0.88 min HPLC-Methode 5 |
| | (R)-5-Chlor-thiophen-2-carbonsäure-*N*-[3-methyloxymethylcarbonylamino-1-(3-methyl-2,3,4,5-tetrahydro-1*H*-benzo[*d*]azepin-7-ylcarbamoyl)-propyl]-amid | | |
| **152** | 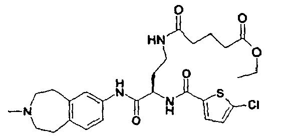 | (M+H)⁺ = 563/565 (Chlorisotope) | Rt: 0.94 min HPLC-Methode 5 |
| | (R)-5-Chlor-thiophen-2-carbonsäure-*N*-[3-(3-ethyloxycarbonyl-propyl)carbonylamino-1-(3-methyl-2,3,4,-tetrahydro-1*H*-benzo[*d*]azepin-7-ylcarbamoyl)-propyl]-amid | | |
| **153** | 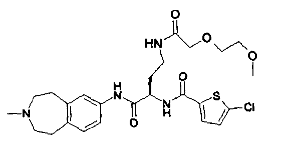 | (M+H)⁺ = 537/539 (Chlorisotope) | Rt: 0.89 min HPLC-Methode 5 |
| | (R)-5-Chlor-thiophen-2-carbonsäure-*N*-[3-(2-methyloxy-ethyl)methylcarbonylamino-1-(3-methyl-2,3,4,5-tetrahydro-1*H*-benzo[*d*]azepin-7-ylcarbamoyl)-propyl]-amid | | |
| **154** | 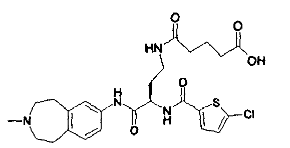 | (M+H)⁺ = 535/537 (Chlorisotope) | Rt: 0.85 min HPLC-Methode 5 |
| | (R)-5-Chlor-thiophen-2-carbonsäure-*N*-[3-(3-hydroxycarbonyl-propyl)carbonylamino-1-(3-methyl-2,3,4,5-tetrahydro-1*H*-benzo[*d*]azepin-7-ylcarbamoyl)-propyl]-amid Rt: 0.98 min | | |
| **155** | 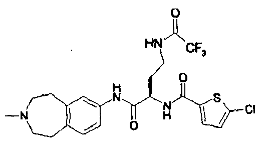 | (M+H)⁺ = 517/519 (Chlorisotope) | HPLC-Methode 5 |
| | (R)-5-Chlor-thiophen-2-carbonsäure-*N*-[3-trifluormethylcarbonylamino-1-(3-methyl-2,3,4,5-tetrahydro-1*H*-benzo[*d*]azepin-7-ylcarbamoyl)-propyl]-amid | | |
| **156** | 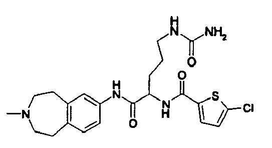 | (M+H)⁺ = 478/480 (Chlorisotope) | Rf: 0.2; Kieselgel; CH₂Cl₂/Ethan ol/Ammoniak = 80:20:2 |
| | 5-Chlor-thiophen-2-carbonsäure-*N*-[4-aminocarbonylamino-1-(3-methyl-2,3,4,5-tetrahydro-1*H*-benzo[*d*]azepin-7-ylcarbamoyl)-butyl]-amid | | |

### Beispiel 157

### 5-Chlor-thiophen-2-carbonsäure-N-[1-methyl-1-(3,3-dimethyl-2,3,4,5-tetrahydro-1H-benzo[d]azepinium-7-ylcarbamoyl]-ethyl}-amid iodid

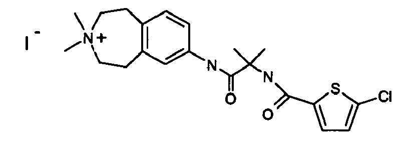

Eine Mischung aus 0.50 g (2.43 mmol) 3-Methyl-7-nitro-2,3,4,5-tetrahydro-1*H-*benzo[d]azepin, 0.44 ml (7.0 mmol) lodmethan und 2.0 ml Acetonitril werden 1 h bei Raumtemperatur und 1 h in der Siedehitze gerührt. Der ausgefallene Niederschlag wird abgesaugt und getrocknet. Anschließend wird analog Beispiel 1(c) hydriert und analog 1(f) zur Titelverbindung umgesetzt.
Rₜ-Wert: 2.7 min (HPLC-MS; Methode 4)
C₂₁H₂₇ClN₃O₂S I (547.88)
Massenspektrum: = 420/422 (Chlorisotope)

### Beispiel 158

### 1-[(5-Chlor-thiophen-2-yl)-carbonylamino]-N-(3-methyl-3-oxy-2,3,4,5-tetrahydro-1H-benzo[d]azepin-7-yl)-cyclopentan-1-carbonsäureamid

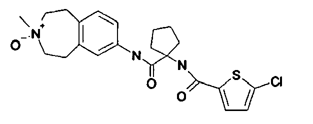

Eine Mischung aus 0.20 g (0.46 mmol) 1-[(5-Chlor-thiophen-2-yl)-carbonylamino]-*N*-(3-methyl-2,3,4,5-tetrahydro-1*H*-benzo[*d*]azepin-7-yl)-cyclopentan-1-carbonsäureamid, 0.12 ml 70%ige meta-Chlorperbenzoesäure und 10.0 ml Chloroform werden 2 h bei Raumtemperatur gerührt. Es wird mit Wasser und einigen Tropfen 2 N NaOH und Essigester verdünnt. Über Nacht wird die Mischung im Gefrierschrank aufbewahrt, nach dem Auftauen befindet sich zwischen den beiden Phase eine kristalline Schicht, die abfiltriert und getrocknet wird.
Rₜ-Wert: 3.14 min (HPLC-MS; Methode 4)
C₂₂H₂₆ClN₃O₃S (447.98)
Massenspektrum: (M+H)⁺ = 448/450 (Chlorisotope)

### Beispiel 159

### 5-Chlor-thiophen-2-carbonsäure-N-[1-(7-benzyl-6,7,8,9-tetrahydro-5H-pyrimido[4,5-d]azepin-2-ylcarbamoyl)-1-methyl-ethyl]-amid

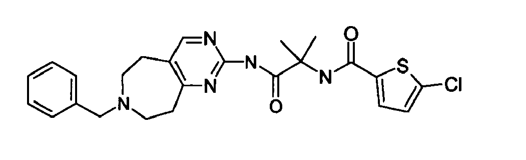

### (a) 1-Benzyl-5-chlor-azepan-4-carbaldehyd

310 ml (4 mol) DMF werden vorgelegt und bei 10-20 °C werden 273 ml (3 mol) Phosphoroxychlorid während 40 min zugetropft,anschließend wird 30 min nachgerührt und dann mit 400 ml Dichlormethan und 239,7 g (1 mol) 1-Benzyl-hexahydro-4*H*-azepinon versetzt und 4 h nachgerührt. Es wird auf 3 I Eiswasser gegossen und 30 min nachgerührt, anschließend wird mit Dichlormethan extrahiert. Die organische Phase wird getrocknet und i.Vak. eingeengt, anschließend wird das Rohprodukt mit Aceton verrieben.
Ausbeute: 177,8 g (62 %)
R_{f}-Wert: 0.69 (Kieselgel: Dichlormethan/Ethylacetat/Ethanol = 4:2:0,1) C₁₄H₁₆ClNO (249.74)

### (b) 7-Benzyl-6,7,8,9-tetrahydro-5H-pyrimido[4,5-d]azepin-2-ylamin

4,60 g (0,20 mol) Natrium werden in 250 ml Ethanol gelöst und bei Raumtemperatur mit 5,90 g (0,10 mmol) Guanidin Hydrochlorid und 24,97 g (0,10 mol) 1-Benzyl-5-chlor-azepan-4-carbaldehyd versetzt. Anschließend wird 5 h zum Rückfluß erhitzt, danach 15 h bei Raumtemperatur nachgerührt. Anschließend wird i.Vak. eingeengt, mit 500 ml Dichlormethan versetzt und 3 mal mit 400 ml Wasser gewaschen. Es wird chromatographisch über Kieselgel (Laufmittel: Dichlormethan/Methanol 95:5) gereinigt, i.Vak. eingeengt und mit Ethanol verrieben.
Ausbeute: 1,97 g (8 %)
C₁₅H₁₈N₄ (254.33)
Massenspektrum: (M+H)+ = 255

### (c) 5-Chlor-thiophen-2-carbonsäure-N-[1-(7-benzyl-6,7,8,9-tetrahydro-5H-pyrimido[4,5-d]azepin-2-ylcarbamoyl)-1-methyl-ethyl]-amid

0,63 g (2,75 mmol) 2-(5-Chlor-thiophen-2-yl)-4,4-dimethyl-4*H*-oxazol-5-on und 0,70 g (2,75 mmol) 7-benzyl-6,7,8,9-tetrahydro-5*H*-pyrimido[4,5-*d*]azepin-2-ylamin werden in 0,350 ml Eisessig, 3,15 ml Toluol und 3,5 ml DMF suspendiert und 20h bei 110 °C gerührt. Chromatographisch wird über Kieselgel (Laufmittel:Dichlormethan/Ethanol 100:0 => 93:7) getrennt. Anschließend wird mit Essigester versetzt und mit 5% iger Natriumhydrogencarbonat- Lösung und Wasser gewaschen, mit Natriumsulfat getrocknet und i.Vak. eingeengt.
Ausbeute: 0,20 g (15 %)
R_{f}-Wert: 0.5 (Kieselgel; Dichlormethan/Ethanol = 90:10)
C₂₄H₂₆ClN₅O₂S (484.01)
Massenspektrum: (M-H)- = 482/4 (Chlorisotope)

### Beispiel 160

### 5-Chlor-thiophen-2-carbonsäure-N-[1-methyl-1-(6,7,8,9-tetrahydro-5H-pyrido[2,3-d]azepin-2-ylcarbamoyl)-ethyl]-amid

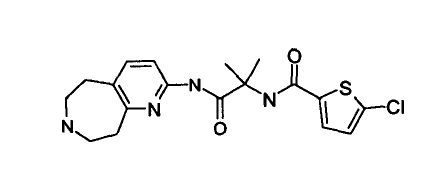

### (a) 6,8,9-Tetrahydro-5H-pyrido[2,3-d]azepin-2-ylamin

1,2 g (3,68 mmol) 7-Benzyl-6,7,8,9-tetrahydro-5*H*-pyrido[2,3-d]azepin-2-ylamin werden in 20 ml Methanol gelöst und mit 0,12 g Palladiumoxid versetzt. Man hydriert in einer Parr-Apparatur bei 50 °C bei 1 bar Wasserstoff-Druck. Anschließend wird der Katalysator abfiltriert und das Filtrat i.Vak. eingeengt. Es wird aus Methanol umkristallisiert.
Ausbeute: 0,50 g (57 %)
R_{f}-Wert: 0.21 (Kieselgel; Dichlormethan/Methanol/Ammoniak = 5:1:0,1) Schmelzpunkt: 290 °C
C₉H₁₃N₃ (163.22)

### (b) 2-Amino-5,6,8,9-tetrahydro-pyrido[2,3-d]azepin-7-carbonsäure tert.-butyl ester

0,38 g (1,61 mmol) 6,7,8,9-Tetrahydro-5*H*-pyrido[2,3-*d*]azepin-2-ylamin werden in 10 ml Dichlormethan suspendiert, anschließend werden 0,667 g (4,83 mmol) Kaliumcarbonat zugegeben und bei Raumtemperatur 10 min gerührt. Danach wird unter Eiskühlung 0,369 g (1,69 mmol) BOC-Anhydrid in 10 ml Dichlormethan langsam zugetropft, anschließend wird 17 h bei Raumtemperatur gerührt, mit 20 ml Dichlormethan verdünnt und mit Wasser gewaschen. Das Rohprodukt wird mit Natriumsulfat getrocknet, eingeengt und chromatographisch über Kieselgel
(Laufmittel: Dichlormethan/Ethanol 98:2=>90:10) gereinigt.
Ausbeute: 0,20 g (47 %)
R_{f}-Wert: 0.5 (Kieselgel; Dichlormethan/Ethanol = 90:10)
C₁₄H₂₁N₃O₂ (263.34)

### (c) 2-{2-[(5-Chlor-thiophen-2-yl)-carbamylamino]-2-methyl-propionylamino}-5,6,8,9-tetrahydro-pyrido[2,3-d]azepine-7-carbonsäure tert-butyl ester

Hergestellt analog Beispiel 1 (f) aus 2-Amino-5,6,8,9-tetrahydro-pyrido[2,3-d]azepin-7-carbonsäure tert.-butyl ester und 2-[(5-Chlor-thiophen-2-yl)-carbonylamino]-2-methyl-propionsäure mit HATU und NMM in DMF bei 70 °C und anschließender Reinigung über Kieselgel (Laufmittel: Dichlormethan/Ethanol 100:0=>97:3) gereinigt.
Ausbeute: 0,15 g (40 %)
R_{f}-Wert: 0.65 (Kieselgel; Dichlormethan/Ethanol = 90:10)
C₂₃H₂₉ClN₄O₄S (493.02)

### (d) 5-Chlor-thiophen-2-carbonsäure-N-[1-methyl-1-(6,7,8,9-tetrahydro-5H-pyrido[2,3-d]azepin-2-ylcarbamoyl)-ethyl]-amid

0,150 g (0,30 mmol) 2-{2-[(5-Chlor-thiophen-2-yl)-carbamylamino]-2-methyl-propionylamino}-5,6,8,9-tetrahydro-pyrido[2,3-d]azepine-7-carbonsäure tert-butyl ester werden bei Raumtemperatur in 2 ml Dichlormethan gelöst, mit 0,40 ml (5,23 mmol) Trifluoressigsäure versetzt und 2 h gerührt. Anschließend wird i.Vak. eingeengt und chromatographisch über RP-Material ( Zorbax StableBond C18, 3,5µm ; Laufmittel: Wasser/Acetonitril/Ameisensäure = 80:20:0,1 => 10:90:0,1 ) gereinigt.
Ausbeute: 0,080 g (52 %)
R_{f}-Wert: 0.25 (Kieselgel; Dichlormethan/Ethanol/Ammoniak = 90:10:1) C₁₈H₂₁ClN₄O₂S (392.91)
Massenspektrum: (M+H)+ = 393/5 (Chlorisotope)

### Beispiel 161

### 5-Chlor-thiophen-2-carbonsäure-N-[1-methyl-1-(7-methyl-6,7,8,9-tetrahydro-5H-pyrido[2,3-d]azepin-2-ylcarbamoyl)-ethyl]-amid

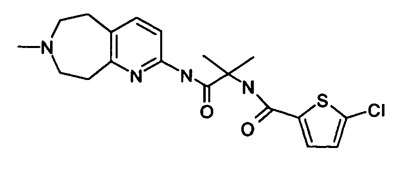

70 mg (0,14 mmol) 5-Chlor-thiophen-2-carbonsäure-*N*-[1-methyl-1-(6,7,8,9-tetrahydro-5*H*-pyrido[2,3-d]azepin-2-ylcarbamoyl)-ethyl]-amid werden in 1 ml (26,50 mmol) gelöst, dann werden 15,0 µl (0,20 mmol) 37% ige FormaldehydLösung in Wasser zugegeben und 3 h bei 90 °C gerührt. Anschließend wird i.Vak. eingeengt
Ausbeute: 40 mg (56 %)
R_{f}-Wert: 0.25 (Kieselgel; Dichlormethan/Ethanol/Ammoniak = 80:20:2) C₁₉H₂₃ClN₄O₂S (604.94)
Massenspektrum: (M+H)+ = 407/9 (Chlorisotope)

In Analogie zu den, in den Beispielen beschriebenen Synthesewegen oder Literatursynthesewegen können die folgenden Verbindungen aus Aminosäurederivaten, Benzazepinderivaten und Thiophencarbonsäurederivaten hergestellt werden:

| | | | |
|---|---|---|---|
| **162** | 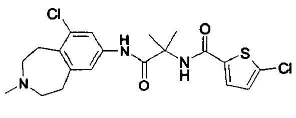 | (M+H)⁺ = 440/442/444 (Dichlorisotopenmuster) | Rf: 0.33; RP8; MeOH/5%Na Cl-Lsg. = 6/4 |
| | 5-Chlor-thiophen-2-carbonsäure-*N*-[1-methyl-1-(3-methyl-9-chlor-2,3,4,5-tetrahydro-1*H*-benzo[d]azepin-7-ylcarbamoyl]-ethyl}-amid | | |
| **163** | 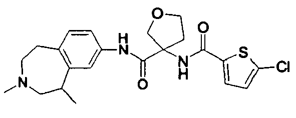 | (M+H)⁺ = 448/450 (Chlorisotope) | Rf: 0.75; Kieselgel; CH₂Cl₂/Ethan ol/Ammoniak = 80:20:2 |
| | 3-[(5-Chlor-thiophen-2-yl)-carbonylamino]-*N*-(3,5-dimethyl-2,3,4,5-tetrahydro-1*H*-benzo[*d*]azepin-7-yl)-tetrahydrofuran-3-carbonsäureamid | | |

### Beispiel 164

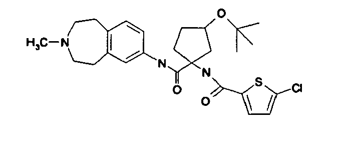

1-[(5-Chlor-thiophen-2-yl)-carbonylamino]-*N*-(3-methyl-2,3,4,5-tetrahydro-1*H-*benzo[*d*]azepin-7-yl)-cyclopentan-3-tert.butoxy-1-carbonsäureamid

### Beispiel 165

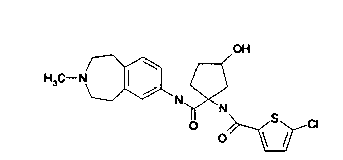

1-[(5-Chlor-thiophen-2-yl)-carbonylamino]-*N*-(3-methyl-2,3,4,5-tetrahydro-1*H-*benzo[*d*]azepin-7-yl)-cyclopentan-3-hydroxy-1-carbonsäureamid

### Beispiel 166

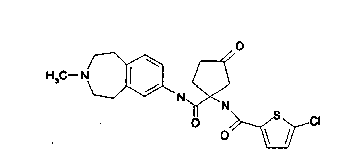

1-[(5-Chlor-thiophen-2-yl)-carbonylamino]-*N*-(3-methyl-2,3,4,5-tetrahydro-1*H-*benzo[*d*]azepin-7-yl)-cyclopentan-3-oxo-1-carbonsäureamid

### Beispiel 167

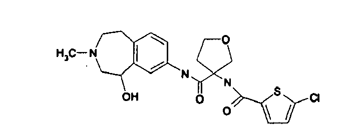

3-[(5-Chlor-thiophen-2-yl)-carbonylamino]-*N*-(3-methyl-5-hydroxy-2,3,4,5-tetrahydro-1*H*-benzo[*d*]azepin-7-yl)-tetrahydrofuran-3-carbonsäureamid

### Beispiel 168

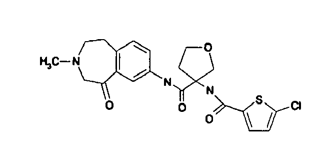

3-[(5-Chlor-thiophen-2-yl)-carbonylamino]-*N*-(3-methyl-5-oxo-2,3,4,5-tetrahydro-1*H*-benzo[*d*]azepin-7-yl)-tetrahydrofuran-3-carbonsäureamid

### Beispiel 169

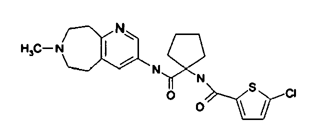

1-[(5-Chlor-thiophen-2-yl)-carbonylamino]-*N*-(7-methyl-6,7,8,9-tetrahydro-5*H-*pyrido[2,3-*d*]azepin-3-yl)-cyclopentan-1-carbonsäureamid

### Beispiel 170

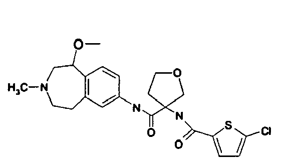

3-[(5-Chlor-thiophen-2-yl)-carbonylamino]-*N*-(3-methyl-1-methoxy-2,3,4,5-tetrahydro-1*H*-benzo[*d*]azepin-7-yl)-tetrahydrofuran-3-carbonsäureamid

### Beispiel 171

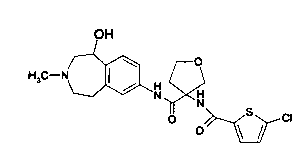

3-[(5-Chlor-thiophen-2-yl)-carbonylamino]-*N*-(3-methyl-1-hydroxy-2,3,4,5-tetrahydro-1*H*-benzo[*d*]azepin-7-yl)-tetrahydrofuran-3-carbonsäureamid

### Beispiel 172

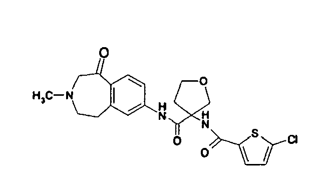

3-[(5-Chlor-thiophen-2-yl)-carbonylamino]-*N*-(3-methyl-1-oxo-2,3,4,5-tetrahydro-1*H*-benzo[*d*]azepin-7-yl)-tetrahydrofuran-3-carbonsäureamid

### Beispiel 173

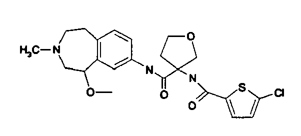

3-[(5-Chlor-thiophen-2-yl)-carbonylamino]-*N*-(3-methyl-5-methoxy-2,3,4,5-tetrahydro-1*H*-benzo[*d*]azepin-7-yl)-tetrahydrofuran-3-carbonsäureamid

### Beispiel 174

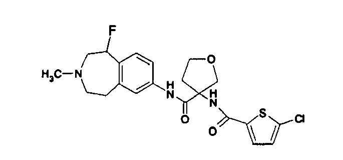

3-[(5-Chlor-thiophen-2-yl)-carbonylamino]-*N*-(3-methyl-1-fluor-2,3,4,5-tetrahydro-1*H*-benzo[*d*]azepin-7-yl)-tetrahydrofuran-3-carbonsäureamid

### Beispiel 175

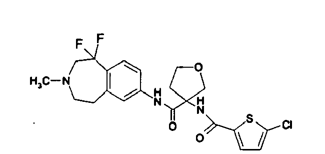

3-[(5-Chlor-thiophen-2-yl)-carbonylamino]-*N*-(3-methyl-1,1-difluor-2,3,4,5-tetrahydro-1*H*-benzo[*d*]azepin-7-yl)-tetrahydrofuran-3-carbonsäureamid

### Beispiel 176

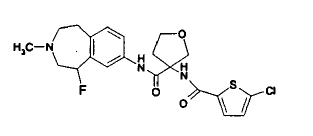

3-[(5-Chlor-thiophen-2-yl)-carbonylamino]-*N*-(3-methyl-5-fluor-2,3,4,5-tetrahydro-1*H*-benzo[*d*]azepin-7-yl)-tetrahydrofuran-3-carbonsäureamid

### Beispiel 177

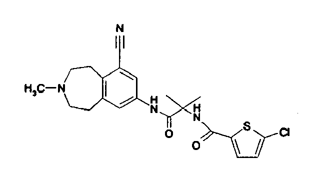

5-Chlor-thiophen-2-carbonsäure-*N*-[1-methyl-1-(3-methyl-9-cyano-2,3,4,5-tetrahydro-1*H*-benzo[*d*]azepin-7-ylcarbamoyl]-ethyl}-amid

### Beispiel 178

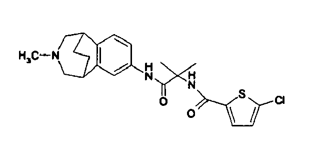

5-Chlor-thiophen-2-carbonsäure-*N*-[1-methyl-1-(10-methyl-10-azatricyclo[6.3.2.02,7]trideca-2(7),3,5-trien-4-ylcarbamoyl]-ethyl}-amid

### Beispiel 179

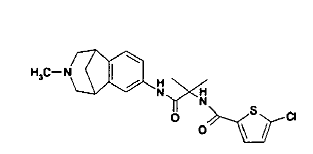

5-Chlor-thiophen-2-carbonsäure-*N*-[1-methyl-1-(10-methyl-10-Azatricyclo[6.3.1.02,7]dodeca-2(7),3,5-trien-4-ylcarbamoyl]-ethyl}-amid

### Beispiel 180

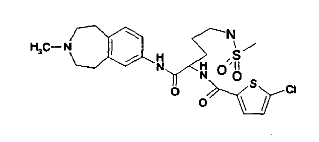

5-Chlor-thiophen-2-carbonsäure-*N*-[4-methylsulfonylamino-1-(3-methyl-2,3,4,5-tetrahydro-1*H*-benzo[*d*]azepin-7-ylcarbamoyl)-butyl]-amid

### Beispiel 181

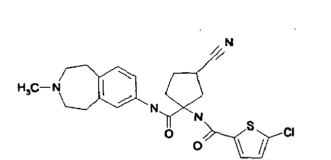

1-[(5-Chlor-thiophen-2-yl)-carbonylamino]-*N*-(3-methyl-2,3,4,5-tetrahydro-1*H-*benzo[*d*]azepin-7-yl)-3-cyano cyclopentan -1-carbonsäureamid

### Beispiel 182

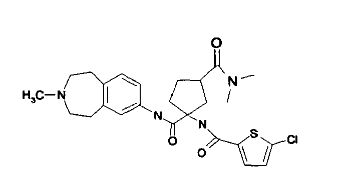

1-[(5-Chlor-thiophen-2-yl)-carbonylamino]-*N*-(3-methyl-2,3,4,5-tetrahydro-1*H-*benzo[*d*]azepin-7-yl)-3-dimethylaminocarbonyl cyclopentan -1-carbonsäureamid

### Beispiel 183

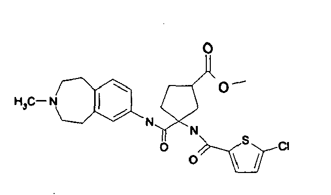

1-[(5-Chlor-thiophen-2-yl)-carbonylamino]-*N*-(3-methyl-2,3,4,5-tetrahydro-1*H-*benzo[*d*]azepin-7-yl)-3-methoxycarbonyl cyclopentan -1-carbonsäureamid

### Beispiel 184

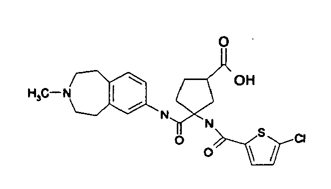

1-[(5-Chlor-thiophen-2-yl)-carbonylamino]-*N*-(3-methyl-2,3,4,5-tetrahydro-1*H-*benzo[*d*]azepin-7-yl)-3-hydroxycarbonyl cyclopentan -1-carbonsäureamid

### Beispiel 185

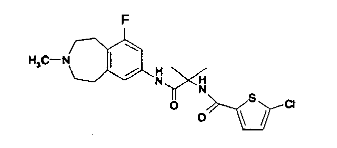

5-Chlor-thiophen-2-carbonsäure-*N*-[1-methyl-1-(3-methyl-9-cyano-2,3,4,5-tetrahydro-1*H*-benzo[*d*]azepin-7-ylcarbamoyl]-ethyl}-amid

### Beispiel 186

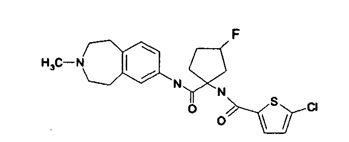

1-[(5-Chlor-thiophen-2-yl)-carbonylamino]-*N*-(3-methyl-2,3,4,5-tetrahydro-1*H-*benzo[*d*]azepin-7-yl)-3-cyano cyclopentan-1-carbonsäureamid

Die nachfolgenden Beispiele beschreiben die Herstellung pharmazeutischer Anwendungsformen, die als Wirkstoff eine beliebige Verbindung der allgemeinen Formel I enthalten.

### Beispiel A

### Trockenampulle mit 75 mg Wirkstoff pro 10 ml

### Zusammensetzung:

| | | |
|---|---|---|
| Wirkstoff | 75.0 mg | |
| Mannitol | 50.0 mg | |
| Wasser für Injektionszwecke | | ad 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel B

### Trockenampulle mit 35 mg Wirkstoff pro 2 ml

### Zusammensetzung:

| | | |
|---|---|---|
| Wirkstoff | 35.0 mg | |
| Mannitol | 100.0 mg | |
| Wasser für Injektionszwecke | | ad 2.0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel C

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel D

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

### Beispiel E

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel F

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel G

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Verbindungen der allgemeinen Formel (I) 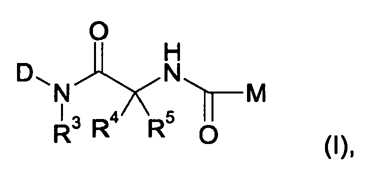 in denen
D ein substituiertes bicyclisches Ringsystem der Formel (II) darstellt, 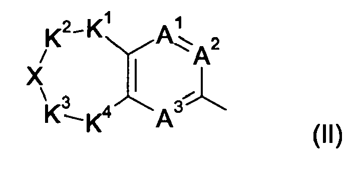 in dem
K¹ und K⁴ jeweils unabhängig voneinander eine -CH₂-, -CHR^{7a}-, -CR^{7b}R^{7c}- oder eine -C(O)-Gruppe bedeuten, und wobei R^{7a}/R ⁷b /R^{7c} jeweils unabhängig voneinander ein Fluoratom, eine Hydroxy-, C₁- ₅-Alkyloxy-, Tetrahydrofuranyl-, Oxetanyl-, Amino-, C₁₋₅-Alkylamino-, Di-(C₁₋₅-alkyl)-amino, C₃₋₅-Cycloalkylenimino- oder C₁₋₅-Alkylcarbonylaminogruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋ ₅-alkyl-, C₁₋₅-Alkylamino-C₁₋₅-alkyl-, Di-(C₁₋₅-alkyl)-amino-C₁₋₅-alkyl- , C₄₋₇-Cycloalkylenimino-C₁₋₅-alkyl-, Carboxy-C₀₋₅-alkyl-, C₁₋₅- Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl- C₀₋₅-alkyl-, C₄₋₇-Cycloalkyleniminocarbonyl-C₀₋₅-alkyl-gruppe, eine Phenylgruppe oder eine 5- oder 6-gliedrige Heteroarylgruppe, die durch 1-2 Substituenten ausgewählt aus einer Nitro-, Amino-, Hydroxy- Methoxy-, Cyano-, C₁₋₅-alkyl-gruppe oder einem Fluor-, Chlor- oder Bromatom substituiert sein kann, bedeutet, wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2- Imidazolidinon-, 2-Oxazolidinon-, Tetrahydro-2(1H)-pyrimidinon-, [1,3]Oxazinan-2-on-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem N-Atom durch eine -CO-Gruppe ersetzt sein kann, und/oder dessen Iminogruppen jeweils durch eine C₁₋₃-Alkyl- oder C₁₋ ₃-Alkylcarbonyl-gruppe substituiert sein können, und/oder bei dem das Schwefelatom zu einer Sulfoxid oder Sulfongruppe oxidiert sein kann,
K² und K³ jeweils unabhängig voneinander eine -CH₂-, -CHR^{8a}-, -CR^{8b}R^{8c}- oder eine -C(O)- Gruppe bedeuten, und wobei
R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁₋ ₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di- (C₁₋₅-alkyl)-aminocarbonyl-C₀₋₅-alkyl-, C₄₋₇- Cycloalkyleniminocarbonyl- C₀₋₅-alkyl-gruppe bedeutet, oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)- pyrimidinon-, [1,3]Oxazinan-2-on-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine -CH₂-Gruppe neben einem Stickstoffatom durch eine -CO-Gruppe ersetzt sein kann, und/oder dessen Iminogruppen jeweils durch eine C₁₋₃-Alkyl- oder C₁₋ ₃-Alkylcarbonyl-gruppe substituiert sein können, und/oder bei dem das Schwefelatom zu einer Sulfoxid oder Sulfongruppe oxidiert sein kann, mit der Massgabe, dass ein durch R^{8b} oder R^{8c} eingebrachtes Heteroatom nicht durch nur ein Kohlenstoffatom von X in Formel (I) entfernt sein darf, und insgesamt in Formel (II) maximal vier Reste ausgewählt aus R^{7a}, R^{7b}, R^{7c} R^{8a}, R^{8b} und R^{8c} vorhanden sein dürfen, und
X ein Sauerstoff- oder Schwefelatom, eine Sulfen-, Sulfon-, oder eine NR¹-Gruppe bedeutet, in der
R¹ ein Wasserstoffatom oder eine Hydoxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆- Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl- sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅- Alkyloxycarbonyl-, C₄₋₇-Cycloalkyleniminocarbonyl-gruppe bedeutet, wobei die in den voranstehend genannten Gruppen befindlichen Methylen- und Methylgruppen zusätzlich durch eine C₁₋₃-Alkyl-, Carboxy-, C₁₋₅-Alkoxycarbonyl- gruppe substituiert sein können oder durch eine Hydroxy-, C₁₋₅-Alkyloxy-, Amino-, C₁₋₅-Alkylamino-, C₁₋ ₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind, und in dem
A¹ entweder N oder CR¹⁰ bedeutet,
A² entweder N oder CR¹¹ bedeutet,
A³ entweder N oder CR¹² bedeutet, wobei R¹⁰, R¹¹ und R¹² jeweils unabhängig voneinander ein Wasserstoff-, Fluor-, Chlor-, Brom- oder lodatom, oder eine C₁₋ ₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋ ₇-Cycloalkylenimino-gruppe bedeuten, oder
D eine der vier Gruppen (II-1), (II-2), (II-3) oder (II-4) darstellt 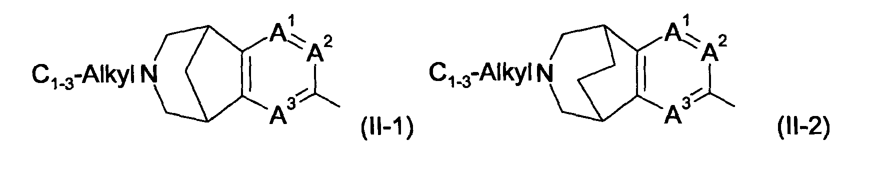 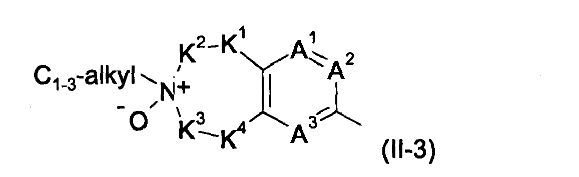 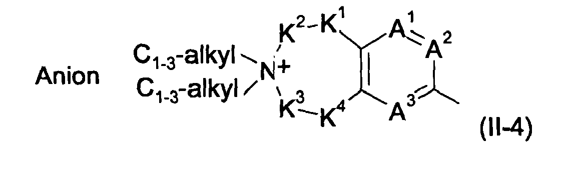 in der die Reste A1, A2, A3, K1, K2, K3, K4 wie oben definiert sind, und Anion in (II-4) ein Fluorid, Chlorid, Bromid, Iodid, Sulfat, Hydrogensulfat, Phosphat, Hydrogenphosphat, Benzoat, Salicylat, Succinat, Citrat oder Tartrat bedeutet,
R³ ein Wasserstoffatom oder eine C₁₋₃-Alkylgruppe bedeutet, und
R⁴ und R⁵ jeweils unabhängig voneinander ein Wasserstoffatom, eine geradkettige oder verzweigte C₁₋₆-Alkyl-, C₂₋₆-Alkenyl- oder C₂₋₆-Alkinylgruppe, wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder wobei ein oder zwei Wasserstoffatome der geradkettigen oder verzweigten C₁₋₆-Alkyl-, C₂₋₆-Alkenyl- oder C₂₋₆-Alkinylgruppe in ihren Methylen- und/oder Methylfragmenten gegebenenfalls jeweils unabhängig voneinander durch eine C₃₋₇- Cycloalkylgruppe, eine Nitril-, Hydroxy-oder C₁₋₅-Alkyloxygruppe, wobei die Wasserstoffatome der C₁₋₅-Alkyloxygruppe gegebenenfalls ganz oder teilweise durch Fluoratome ersetzt sein können, eine Allyloxy-, Propargyloxy-, Phenylmethyloxy-, Phenethyloxy-, C₁₋₅-Alkylcarbonyloxy-, C₁₋₅-Alkyloxycarbonyloxy-, Carboxy- C₁₋₅-alkyloxy-, C₁₋₅-Alkyloxycarbonyl-C₁₋₅-alkyloxy-, C₁₋₅-Alkyloxy- C₂₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₁₋₅-Alkyl-aminocarbonyloxy-, Di-(C₁₋₅-alkyl)- aminocarbonyloxy-, C₄₋₇-Cycloalkyleniminocarbonyl-, Aminosulfonyl-, C₁₋₅-Alkylaminosulfonyl-, Di-(C₁₋₅-alkyl)- aminosulfonyl-, C₄₋₇-Cycloalkyleniminosulfonyl-, Di-(C₁₋₅-alkyl)- phosphoryl-, Amino-, C₁₋₅-Alkylamino-, Di-(C₁₋₅-alkyl)-amino-, C₁₋₅-Alkylcarbonylamino-, Trifluoracetylamino-, C₁₋₅-Alkyloxy- C₁₋₅-alkylcarbonylamino-, Phenylcarbonylamino-, C₁₋₅-Alkylaminocarbonylamino-, Di-(C₁₋₅-alkyl)- aminocarbonylamino-, C₁₋₅-Alkyloxy-carbonylamino-, Phenylmethyloxy-carbonylamino-, C₁₋₅-Alkyloxy-C₂₋₅-alkyloxy-C₁₋₂- alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonyl- aminogruppe, 4-Morpholinocarbonylamino-, oder eine Morpholinyl- , Thiomorpholinyl-, Pyrrolidinyl-, Piperidinyl-, Piperazinyl-, Tetrahydrofuranyl-, Tetrahydropyranylgruppe ersetzt sein können, wobei die vorgenannten Carbo- und Heterocyclen im Ring jeweils durch 1 bis 4 C₁₋₃-Alkyl- oder C₁₋₃- Alkylcarbonylgruppen oder jeweils durch 1 oder 2 Oxogruppen substituiert sein können, und/oder wobei die vorgenannten Phenyl und Heteroarylreste durch 1 oder 2 Substituenten ausgewahlt aus Fluor-, Chlor-, Brom-, Methyl-, Methoxy-, Amino- oder Trifluormethyl ersetzt sein können oder zwei benachbarte Kohlenstoffatome eines Phenylrings durch eine -CH₂-O- CH₂-Gruppe substituiert sein können, und/oder wobei die vorgenannten Alkylgruppen durch eine Cyano- C₁₋₅-Alkyloxycarbonyl- oder Carboxy-gruppe substituiert sein können, wobei die vorgenannten Carbonsäure- oder Sulfonsäure- amide gegebenenfalls am Stickstoff zusätzlich durch eine C₁₋₅-Alkylgruppe substituiert sein können, und/oder wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, eine Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl-, C₄₋₇-Cycloalkyleniminocarbonylgruppe, eine Phenyl-, mono- oder bicyclische Heteroaryl-, Phenyl-C₁₋₅-alkyl- oder mono- oder bicyclische Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom- und lodatome, und C₁₋₅-Alkyl-, Trifluormethyl-, Benzyl-, Amino-, Nitro-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann, oder zwei benachbarte Kohlenstoffatome eines Phenylrings durch eine -CH₂-O-CH₂-Gruppe substituiert sein können, bedeuten, oder eine C₃₋₇Cycloalkyl-, Morpholinyl-, Thiomorpholinyl-, Pyrrolidinyl-, Piperidinyl-, Piperazinyl-, Tetrahydrofuranyl-, Tetrahydropyranylgruppe bedeuten, die gegebenenfalls durch ein bis zwei unabhängig voneinander ausgewählte Reste aus C₁₋₃-Alkyl-, Acetyl-, C₁₋₅Alkyloxycarbonyl-, und Hydroxycarbonyl- substituiert sein kann, oder
R⁴ und R⁵ zusammen mit dem Kohlenstoffatom, an dem sie gebunden sind, eine C₃₋₇-Cycloalkyl- oder C₅₋₇-Cycloalkenylgruppe bilden, wobei eine der Methylengruppen einer C₄₋₇-Cycloalkylgruppe durch ein Sauerstoff- oder Schwefelatom oder eine -NH-, -N(C₁₋₅- Alkyl), -N(C₁₋₄-Alkylcarbonyl)-, -N(C₁₋₄-Alkyloxycarbonyl)- oder eine Carbonyl-, Sulfinyl- oder Sulfonylgruppe ersetzt sein kann, und/oder wobei zwei direkt benachbarte Methylengruppen einer C₄₋₇-Cycloalkylgruppe zusammen durch eine -C(O)NH-, - C(O)N(C₁₋₅-Alkyl)-, -S(O)₂NH-, oder -S(O)₂N(C₁₋₅Alkyl)-Gruppe ersetzt sein können, und/oder wobei 1 bis 3 Kohlenstoffatome einer C₃₋₇-Cycloalkylgruppe gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, Formyloxy-, C₁₋₅-Alkylcarbonyloxy-, C₁₋₅-Alkylsulfanyl-, C₁₋₅-Alkylsulfonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di- (C₁₋₅-alkyl)-amino-, C₁₋₅-Alkylcarbonylamino-, C₁₋₅-Alkyl- sulfonylamino-, N-(C₁₋₅-Alkylsulfonyl)-C₁₋₅-alkylamino-, C₃₋₆-Cycloalkylcarbonylamino-, Nitril-, Carboxy-C₁₋₅-alkyl, C₁₋₅- Alkyloxycarbonyl-C₁₋₅-alkyl, Carboxy-, C₁₋₅-Alkyloxycarbonyl-, Aminocarbonyl-, C₁₋₅-Alkylaminocarbonyl-, Di-(C₁₋₅-alkyl)- aminocarbonyl- oder C₄₋₇-Cycloalkyleniminocarbonylgruppe substituiert sein können, und/oder wobei 1 bis 2 Kohlenstoffatome einer C₅₋₇-Cycloalkenylgruppe gegebenenfalls unabhängig voneinander durch jeweils eine C₁₋₅- Alkyl-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₃₋₆-Cycloalkyleniminocarbonyl-, Aminosulfonyl-, C₁₋₅-Alkylaminosulfonyl-, Di-(C₁₋₅-alkyl)-aminosulfonyl-, C₃₋₆-Cycloalkyleniminosulfonylgruppen oder 1-2 Fluoratome substituiert sein können, und/oder 1 bis 2 Kohlenstoffatome einer C₄₋₇-Cycloalkenylgruppe, die nicht durch eine Doppelbindung an ein anderes Kohlenstoffatom gebunden sind, gegebenenfalls unabhängig voneinander durch eine Hydroxy-, C₁₋₅-Alkyloxy-, C₁₋₅-Alkylcarbonyloxy-, C₁₋₅-Alkylsulfanyl-, C₁₋₅-Alkylsulfonyl-, Amino-, C₁₋₅-Alkylamino-, Di-(C₁₋₅-alkyl)-amino-, C₁₋₅-Alkylcarbonylamino-, C₁₋₅-Alkyl- sulfonylamino-, N-(C₁₋₅-Alkylsulfonyl)-C₁₋₅-alkylamino- oder C₃₋₆-Cycloalkylcarbonylaminogruppen substituiert sein können, mit der Maßgabe, dass eine solche, zusammen aus R⁴ und R⁵ gebildete C₃₋₇-Cycloalkyl- oder C₅₋₇-Cycloalkenylgruppe, in der zwei Heteroatome im Cyclus aus der Gruppe Sauerstoff und Stickstoff durch genau eine gegebenenfalls substituierte -CH₂-Gruppe voneinander getrennt sind, und/oder in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder in der ein an die cyclische Gruppe gebundener Substituent, der sich **dadurch** auszeichnet, daß ein Heteroatom aus der Gruppe Sauerstoff, Stickstoff, Schwefel und Fluor direkt an die cyclische Gruppe gebunden ist, von einem anderen Heteroatom aus der Gruppe Sauerstoff, Stickstoff und Schwefel, mit Ausnahme der Sulfongruppe, durch genau eine, gegebenenfalls substituierte, Methylengruppe getrennt ist,
und/oder in der zwei Atome im Ring eine -O-O- oder -S-O-Bindung bilden, ausgeschlossen ist,
M einen Thiophenring gemäß Formel (III) darstellt, 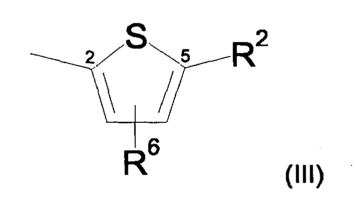 der über die 2-Position an die Carbonylgruppe in Formel (I) gebunden ist und der in 5-Position durch R² und gegebenenfalls zusätzlich durch R⁶ substituiert ist, wobei
R² ein Wasserstoff- , Fluor-, Chlor-, Brom- oder lodatom oder eine Methoxy-, C₁₋₂-Alkyl-, Formyl-, NH₂CO- oder Ethinyl-Gruppe bedeutet,
R⁶ ein Wasserstoff-, Fluor-, Chlor-, Brom- oder lodatom oder eine C₁₋₂- Alkyl- oder Amino-Gruppe bedeutet, wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei die 6-gliedrige Heteroarylgruppe ein, zwei oder drei Stickstoffatome, und die 5-gliedrige Heteroarylgruppe eine gegebenenfalls durch eine C₁₋₃-Alkylgruppe substituierte Iminogruppe, ein Sauerstoff- oder Schwefelatom, oder eine gegebenenfalls durch eine C₁₋₃-Alkylgruppe substituierte Iminogruppe oder ein Sauerstoff- oder Schwefelatom und zusätzlich ein oder zwei Stickstoffatome, oder eine gegebenenfalls durch eine C₁₋₃-Alkylgruppe substituierte Iminogruppe und drei Stickstoffatome, enthält, und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)- amino- oder C₃₋₆-Cycloalkyleniminogruppe substituierter Phenylring ankondensiert sein kann, und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und lod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt wurde, ganz oder teilweise durch Fluoratome ersetzt sein können,
deren Tautomere, deren Enantiomere, deren Diastereomere, deren Gemische und deren Salze.

2. Verbindungen der allgemeinen Formel (I) nach Anspruch 1, in denen
D ein substituiertes bicyclisches Ringsystem der Formel (II) darstellt, 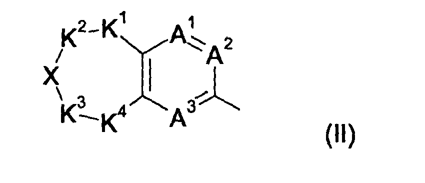 in dem
K¹ und K⁴ jeweils unabhängig voneinander eine -CH₂-, -CHR^{7a}-, -CR^{7b}R^{7c}- oder eine -C(O)-Gruppe bedeuten, wobei
R^{7a}/R^{7b}/R^{7c} jeweils unabhängig voneinander ein Fluoratom, eine Hydroxy-, C₁₋ ₅-Alkyloxygruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅- alkyl-gruppe bedeutet, oder eine Phenylgruppe, die durch 1-2 Substituenten ausgewählt aus einer Nitro-, Amino-, Hydroxy- Methoxy-, Cyano-, C₁₋₅-alkyl-gruppe oder einem Fluor-, Chlor- oder Bromatom substituiert sein kann, oder eine 5- oder 6- gliedriger Heteroarylgruppe, bedeutet
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Tetrahydrofuran- oder Tetrahydropyran-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und
K² und K³ jeweils unabhängig voneinander eine -CH₂-, -CHR^{8a}-, -CR^{8b}R^{8c}- oder eine -C(O)- Gruppe bedeuten, und wobei
R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁₋ ₅-Alkyloxy-C₁₋₅-alkyl-gruppe bedeutet, oder zwei Reste R^{8b}/R^{8c} zusammen mit dem Ringkohlenstoffatom einen 3-, 4-, 5-, 6- oder 7-gliedrigen Carbocyclus oder einen Cyclopenten-, Cyclohexen-, Oxetan-, Tetrahydrofuran-, Tetrahydropyran-ring bilden können, wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, mit der Massgabe, dass ein durch R^{8b} oder R^{8c} eingebrachtes Heteroatom nicht durch nur ein Kohlenstoffatom von X in Formel (I) entfernt sein darf, und insgesamt in Formel (II) maximal vier Reste ausgewählt aus R^{7a}, R^{7b}, R^{7c} R^{8a}, R^{8b} und R^{8c} vorhanden sein dürfen, und
X ein Sauerstoff- oder Schwefelatom, eine Sulfen-, Sulfon- oder eine NR¹-Gruppe bedeutet, in der
R¹ ein Wasserstoffatom oder eine C₁₋₅-Alkyl-, C₂₋₅-Alkenyl-CH₂-, C₂₋₅-Alkinyl-CH₂, C₃₋₆- Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkyl- sulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅- Alkyloxycarbonyl-, C₄₋₇-Cycloalkyleniminocarbonyl-gruppe bedeutet, wobei die in den voranstehend genannten Gruppen befindlichen Methylen- und Methylgruppen zusätzlich durch eine C₁₋₃Alkyl-, Carboxy-, C₁₋₅-Alkoxycarbonyl- gruppe substituiert sein können oder durch eine Hydroxy-, C₁₋₅-Alkyloxy-, Amino-, C₁₋₅-Alkylamino-, C₁₋ ₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind, und in dem A¹, A², und A³ jeweils wie in der 1. Ausführungsform beschrieben definiert sind, oder
D eine der vier Gruppen (II-1a), (II-2a), (II-3) oder (II-4) darstellt 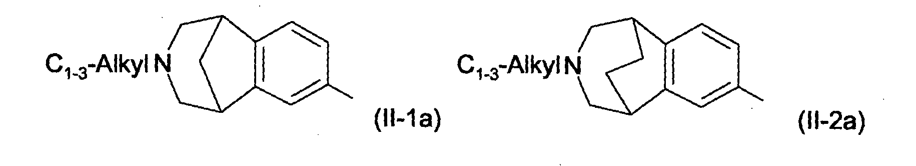 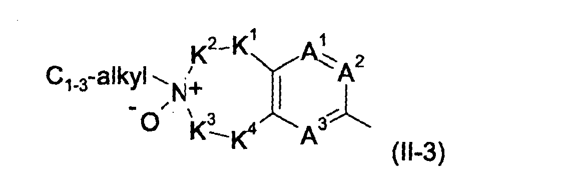 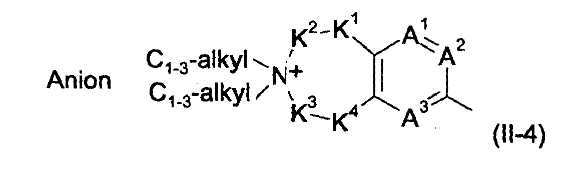
in der die Reste A1, A2, A3, K1, K2, K3, K4 wie oben definiert sind, und Anion in (II-4) ein Fluorid, Chlorid, Bromid, Iodid, Sulfat, Hydrogensulfat, Phosphat, Hydrogenphosphat, Benzoat, Salicylat, Succinat, Citrat oder Tartrat bedeutet, und
R³ ein Wasserstoffatom bedeutet, und
R⁴, R⁵ und M jeweils wie in der 1. Ausführungsform beschrieben definiert sind,
deren Tautomere, deren Enantiomere, deren Diastereomere, deren Gemische und deren Salze.

3. Verbindungen der allgemeinen Formel (I) nach Anspruch 1 oder 2, in denen
D ein substituiertes bicyclisches Ringsystem der Formel (II) darstellt, 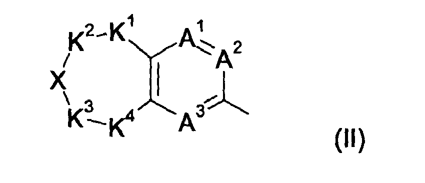 in dem K1, K2, K3 und K4 wie in Anspruch 1 oder 2 definiert sind, und wobei
X eine NR¹-Gruppe bedeutet, in der
R¹ ein Wasserstoffatom oder eine C₁₋₅-Alkyl-, C₂₋₅-Alkenyl-CH₂-, C₂₋₅-Alkinyl-CH₂, C₃₋₆- Cycloalkyl-, C₄₋₆-Cycloalkenylgruppe bedeutet, wobei die in den voranstehend genannten Gruppen befindlichen Methylen- und Methylgruppen zusätzlich durch eine C₁₋₃-Alkyl-, Carboxy-, C₁₋₅- Alkoxycarbonyl-gruppe substituiert sein können oder durch eine Hydroxy-, C₁₋₅-Alkyloxy-, Amino-, C₁₋₅- Alkylamino-, C₁₋₅-Dialkylamino- oder C₄₋₇- Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an das Stickstoff atom gebunden sind, und in dem
A¹ entweder N oder CR¹⁰ bedeutet,
A² entweder N oder CR¹¹ bedeutet,
A³ entweder N oder CR¹² bedeutet, wobei R¹⁰, R¹¹ und R¹² jeweils unabhängig voneinander ein Wasserstoff- , Fluor-, Chlor-, Bromatom oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-gruppe bedeuten, oder
D eine der vier Gruppen (II-1a), (II-2a), (II-3) oder (11-4) darstellt 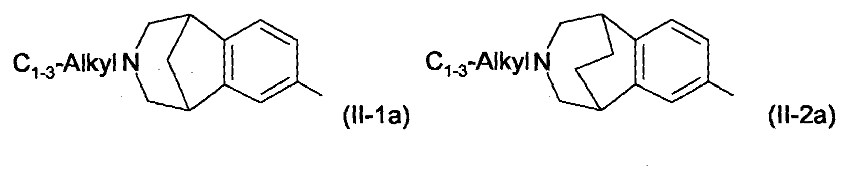 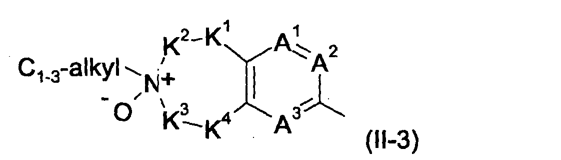 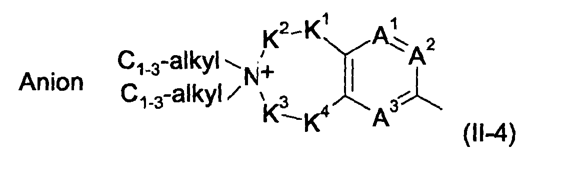 in der die Reste A1, A2, A3, K1, K2, K3, K4 wie oben definiert sind, und Anion in (II-4) aus der Gruppe Fluorid, Chlorid, Bromid, Iodid, Sulfat, Phosphat, Benzoat, Salicylat, Succinat, Citrat und Tartrat ausgewählt werden kann,und
R³, R⁴, R⁵ und M jeweils wie in Anspruch 1 oder 2 definiert sind, wobei R⁶ ein Wasserstoffatom bedeutet,
deren Tautomere, deren Enantiomere, deren Diastereomere, deren Gemische und deren Salze.

4. Verbindungen der allgemeinen Formel (I) nach einem der Ansprüche 1 bis 3, in denen
D, R³ und M jeweils wie in einem der Ansprüche 1 bis 3 definiert sind, und
R⁴ eine geradkettige oder verzweigte C₃₋₆-Alkenyl- oder C₃₋₆-Alkinylgruppe, eine geradkettige oder verzweigte C₁₋₆-Alkylgruppe, wobei die Wasserstoffatome der geradkettigen oder verzweigten C₁₋₆-Alkylgruppe gegebenenfalls ganz oder teilweise durch Fluoratome ersetzt sein können, und bei der gegebenenfalls ein bis zwei Wasserstoffatome unabhängig voneinander durch eine C₃₋₇-Cycloalkyl-, Hydroxy-, C₁₋₅-Alkyloxy-, Phenylmethyloxy-, Phenethyloxy-, Carboxy- C₁₋₅-alkyloxy-, C₁₋₅-Alkyloxycarbonyl-C₁₋₅-alkyloxy-, C₁₋₅-Alkyloxy- C₂₋₅-alkyloxy-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)- aminocarbonyl-, C₁₋₅-Alkyl-aminocarbonyloxy-, Di-(C₁₋₅-alkyl)- aminocarbonyloxy-, C₄₋₇-Cycloalkyleniminocarbonyl-, Amino-, C₁₋₅- Alkylamino- oder Di-(C₁₋₅-alkyl)-aminogruppe C₁₋₅-Alkylcarbonylamino-, Trifluoracetylamino-, C₁₋₅-Alkyloxy- C₁₋₅-alkylcarbonylamino-, Phenylcarbonylamino-, C₁₋₅-Alkylaminocarbonylamino-, Di-(C₁₋₅-alkyl)- aminocarbonylamino-, C₁₋₅-Alkyloxy-carbonylamino-, Phenylmethyloxy-carbonylamino-, C₁₋₅-Alkyloxy-C₂₋₅-alkyloxy-C₁₋₂- alkylcarbonylamino-, C₁₋₅-Alkylsulfonylamino-, C₃₋₆-Cycloalkylcarbonylaminogruppe, 4-Morpholinocarbonylamino- gruppe ersetzt sein können, wobei die vorgenannten Carbo- und Heterocyclen im Ring jeweils durch 1 bis 4 C₁₋₃-Alkyl- oder C₁₋₃- Alkylcarbonylgruppen oder jeweils durch 1 oder 2 Oxogruppen substituiert sein können, und/oder wobei die vorgenannten Phenyl und Heteroarylreste durch 1 bis 2 Substituenten ausgewahlt aus Fluor-, Chlor-, Brom-, Methyl-, Methoxy-, oder Trifluormethyl ersetzt sein können, oder zwei benachbarte Kohlenstoffatome eines Phenylrings durch eine -CH₂-O-CH₂-Gruppe substituiert sein können, und/oder wobei die vorgenannten Alkylgruppen durch eine Cyano- C₁₋₅-Alkyloxycarbonyl- oder Carboxy-gruppe substituiert sein können, wobei die vorgenannten Carbonsäure- oder Sulfonsäure- amide gegebenenfalls am Stickstoff zusätzlich durch eine C₁₋₅-Alkylgruppe substituiert sein können, eine Phenyl-, Phenyl-C₁₋₂-alkyl-, Hetetoaryl-C₁₋₂-alkyl- oder C-verknüpfte Heteroarylgruppe, wobei die Heteroarylgruppe ausgewählt ist aus der Gruppe bestehend aus Imidazolyl-, Furanyl-, Thiophenyl-, Thiazolyl-, Pyrazolyl-, Tetrazolyl-, Benzimidazolyl-, Indolyl-, Pyrimidinyl-, Pyrazinyl- Oxazolyl-, 1,2,4-Triazolyl- und Pyridinyl-, und die im Phenyl- oder Heteroarylteil gegebenenfalls ein- bis zweifach durch gleiche oder unterschiedliche Substituenten ausgewählt aus Chlor- oder Fluoratomen oder C₁₋₃-Alkyl-, Benzyl-, Hydroxy-, Amino-, CF₃-, CH₃O- oder CHF₂O- gruppen, substituiert sein kann, bedeutet,
R⁵ ein Wasserstoffatom, eine geradkettige oder verzweigte C₁₋₄-Alkylgruppe, wobei die Wasserstoffatome der geradkettigen oder verzweigten C₁₋₄-Alkylgruppen gegebenenfalls ganz oder teilweise durch Fluoratome ersetzt sein können, oder eine Propargyl- oder C₁₋₃Alkyloxy- C₁₋₃-alkylgruppe, bedeutet, oder
R⁴ und R⁵ zusammen mit dem Kohlenstoffatom, an dem sie gebunden sind, eine C₅₋₆-Cycloalkenyl- oder C₃₋₇-Cycloalkylgruppe bilden, wobei eine der Methylengruppen einer C₄₋₇-Cycloalkylgruppe durch ein Sauerstoff- oder Schwefelatom oder eine -NH-, -N(C₁₋₅- Alkyl), -N(C₁₋₄-Alkylcarbonyl)-, Carbonyl-, Sulfinyl- oder eine Sulfonyl-Gruppe ersetzt sein kann, oder zwei direkt benachbarte Methylengruppen einer C₄₋₇-Cycloalkylgruppe zusammen durch eine -C(O)NH-, -C(O)N(C₁₋₅-Alkyl)-, -S(O)₂NH-, oder -S(O)₂N(C₁₋ ₅-Alkyl)-Gruppe ersetzt sein können, und/oder wobei 1 bis 2 Kohlenstoffatome einer C₃₋₇-Cycloalkylgruppe gegebenenfalls unabhängig voneinander durch jeweils ein oder zwei Fluoratome oder eine oder zwei C₁₋₅- Alkyl-gruppen oder eine Hydroxy-, C₁₋₅-Alkyloxy-, Formyloxy-, Amino-, C₁₋₅-Alkylamino-, Di-(C₁₋₅-alkyl)-amino-, C₁₋₅-Alkylcarbonylamino-, C₃₋₆-Cycloalkylcarbonylamino-, Nitril-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können, mit der Maßgabe, dass eine solche, zusammen aus R⁴ und R⁵ gebildete C₃₋₇-Cycloalkylgruppe, in der zwei Heteroatome im Cyclus aus der Gruppe Sauerstoff und Stickstoff durch genau eine gegebenenfalls substituierte -CH₂-Gruppe voneinander getrennt sind, und/oder in der eine oder beide Methylengruppen des Cyclus, die direkt mit dem Kohlenstoffatom verbunden sind, an dem die Reste R⁴ und R⁵ angebunden sind, durch ein Heteroatom aus der Gruppe Sauerstoff, Stickstoff und Schwefel ersetzt sind, und/oder in der ein an die cyclische Gruppe gebundener Substituent, der sich **dadurch** auszeichnet, daß ein Heteroatom aus der Gruppe Sauerstoff, Stickstoff, Schwefel und Fluoratom direkt an die cyclische Gruppe gebunden ist, von einem anderen Heteroatom aus der Gruppe Sauerstoff, Stickstoff und Schwefel durch genau eine, gegebenenfalls substituierte, Methylengruppe getrennt ist, und/oder in der zwei Atome im Ring eine -O-O- oder -S-O-Bindung bilden,
ausgeschlossen ist,
deren Tautomere, deren Enantiomere, deren Diastereomere, deren Gemische und deren Salze.

5. Verbindungen der allgemeinen Formel (I) nach einem der Ansprüche 1 bis 4, in denen
D ein substituiertes bicyclisches Ringsystem der Formel (II) darstellt, 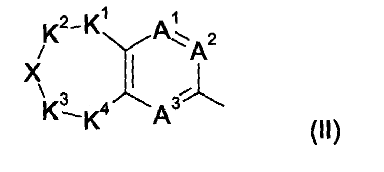 in dem
K¹ und K⁴ jeweils unabhängig voneinander eine -CH₂-, -CHR^{7a}- oder eine - CR^{7b}R^{7c}- Gruppe bedeuten, wobei
R^{7a}/R^{7b}/R^{7c} jeweils unabhängig voneinander eine C₁₋₂-Alkylgruppe oder eine Phenylgruppe, die durch 1 oder 2 Substituenten ausgewählt aus einer Nitro-, Amino-, Hydroxy- Methoxy-, Cyano-, C₁₋₅-alkyl-gruppe oder einem Fluor-, Chlor- oder Bromatom substituiert sein kann, bedeutet,
K² und K³ jeweils eine -CH₂-Gruppe bedeutet
X eine NR¹-Gruppe bedeutet, in der
R¹ ein Wasserstoffatom oder eine C₁₋₅-Alkyl-, C₂₋₄-Alkenyl-CH₂-, C₂₋₄-Alkinyl-CH₂- oder C₃₋₆-Cycloalkylgruppe bedeutet, wobei die in den voranstehend genannten C₂₋₅-Alkyl-Gruppen befindlichen Methylen- und Methylgruppen durch ein bis drei Fluoratome substituiert sein können, sofern die Methylen- oder Methylgruppen nicht direkt an das Stickstoffatom gebunden sind, und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff- , Fluor- oder Chloratom, oder eine C₁₋₃-Alkyl-, CF₃-, Hydroxy oder CH₃O-gruppe, bedeuten, oder
D eine der Gruppen (II-3) oder (II-4) darstellt 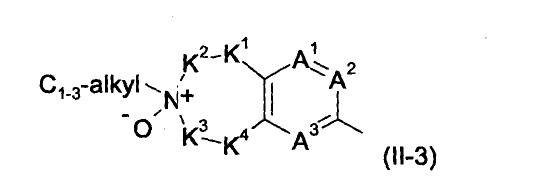 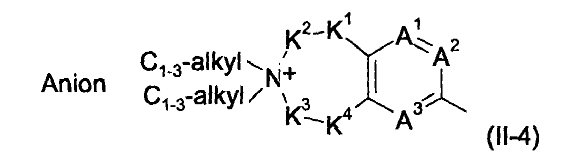 in der die Reste A1, A2, A3, K1, K2, K3, K4 wie oben definiert sind, und das Anion in (II-4) aus der Gruppe Fluorid, Chlorid, Bromid, Iodid, Sulfat, Phosphat, Benzoat, Salicylat, Succinat, Citrat oder Tartrat ausgewählt werden kann,und
R³ ein Wasserstoffatom bedeutet,
R⁴ eine geradkettige oder verzweigte C₃₋₆-Alkenyl- oder C₃₋₆-Alkinylgruppe, eine geradkettige oder verzweigte C₁₋₄-Alkylgruppe, wobei die Wasserstoffatome der geradkettigen oder verzweigten C₁₋₄-Alkylgruppe gegebenenfalls teilweise durch bis zu vier Fluoratome ersetzt sein können, und bei der gegebenenfalls ein bis zwei Wasserstoffatome unabhängig voneinander durch eine C₃₋₇-Cycloalkyl-, Hydroxy-, C₁₋₅-Alkyloxy-, Phenylmethyloxy-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino- oder Di-(C₁₋₅-alkyl)-amino-, C₁₋₅-Alkylcarbonylamino-, Carboxy-C₁₋₅-alkylcarbonylamino- oder eine C₁₋₅-Alkyloxycarbonyl-C₁₋₅-alkylcarbonylamino-gruppe ersetzt sein können, wobei die vorgenannten Phenylreste durch 1 oder 2 Substituenten ausgewahlt aus Fluor-, Chlor-, Brom-, Methyl-, Methoxy-, oder Trifluormethyl- ersetzt sein können, oder wobei die vorgenannten Carbonsäureamide gegebenenfalls am Stickstoff zusätzlich durch eine C₁₋₅- Alkylgruppe substituiert sein können, eine Phenyl-, Phenyl-C₁₋₂-alkyl-, Heteroaryl-C₁₋₂-alkyl- oder C-verknüpfte Heteroarylgruppe, wobei die Heteroarylgruppe ausgewählt ist aus der Gruppe bestehend aus Imidazolyl-, Furanyl-, Thiophenyl-, Thiazolyl-, Pyrazolyl-, Tetrazolyl-, Benzimidazolyl-, Indolyl-, Pyrimidinyl-, Pyrazinyl-, Oxazolyl-, und Pyridinyl-, und die im Phenyl- oder Heteroarylteil gegebenenfalls ein- bis zweifach durch gleiche oder unterschiedliche Substituenten ausgewählt aus Chlor- oder Fluoratomen oder C₁₋₃-Alkyl-, CF₃-, HO-, CH₃O- oder CHF₂O-gruppen, substituiert sein kann, bedeutet,
R⁵ ein Wasserstoffatom, eine geradkettige oder verzweigte C₁₋₄-Alkylgruppe, eine Propargyl- oder C₁₋₃Alkyloxy- C₁₋₃-alkylgruppe, bedeutet, oder
R⁴ und R⁵ zusammen mit dem Kohlenstoffatom, an dem sie gebunden sind, eine C₅₋₆-Cycloalkenyl- oder C₃₋₇-Cycloalkylgruppe bilden, wobei eine der Methylengruppen einer C₄₋₇-Cycloalkylgruppe durch ein Sauerstoff- oder Schwefelatom oder eine Sulfonyl- Gruppe ersetzt sein kann, oder wobei 1 bis 2 Kohlenstoffatome einer C₃₋₇-Cycloalkylgruppe gegebenenfalls unabhängig voneinander durch jeweils ein oder zwei Fluoratome, oder eine oder zwei C₁₋₅-Alkyl-gruppen, oder eine Hydroxy-, C₁₋₅-Alkyloxy-, Formyloxy-, Nitril-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe, substituiert sein können, mit der Maßgabe, dass eine solche, zusammen aus R⁴ und R⁵ gebildete C₃₋₇-Cycloalkylgruppe, in der eine der Methylengruppen des Cyclus, die direkt mit dem Kohlenstoffatom verbunden ist, an dem die Reste R⁴ und R⁵ angebunden sind, durch ein Sauerstoff- oder Schwefelatom ersetzt ist, ausgeschlossen ist, und
M einen Thiophenring gemäß Formel (III) darstellt, 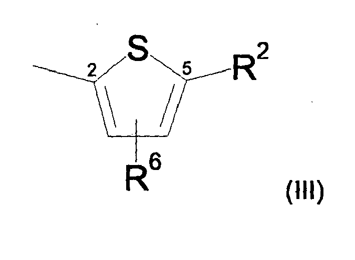 der über die 2-Position an die Carbonylgruppe in Formel (I) gebunden ist und der in 5-Position durch R² substituiert ist, wobei
R² ein Chlor- oder Bromatom oder eine Ethinyl-Gruppe bedeutet, und
R⁶ ein Wasserstoffatom bedeutet,
wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
deren Tautomere, deren Enantiomere, deren Diastereomere, deren Gemische und deren Salze.

6. Verbindungen der allgemeinen Formel (I) nach einem der Ansprüche 1 bis 5, in denen der Rest
D ein substituiertes bicyclisches Ringsystem der Formel (II) darstellt, 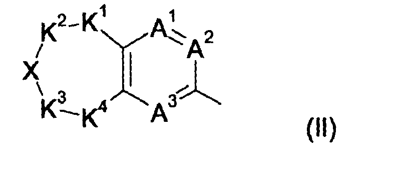 in dem
K¹ und K⁴ jeweils unabhängig voneinander eine -CH₂-, -CHR^{7a}- oder eine - CR^{7b}R^{7c}- Gruppe bedeuten, wobei
R^{7a}/R^{7b}/R^{7c} jeweils unabhängig voneinander eine C₁₋₂-Alkylgruppe bedeutet,
K² und K³ jeweils eine -CH₂-Gruppe bedeutet,
X eine NR¹-Gruppe bedeutet, in der
R¹ ein Wasserstoffatom oder eine C₁₋₅-Alkyl- oder C₃₋₆-Cycloalkylgruppe bedeutet, wobei die in den voranstehend genannten Gruppen befindlichen Methylen- und Methylgruppen ein bis drei Wasserstoffatome durch Fluoratome ersetzt sein können, sofern die Methylen- oder Methylgruppen nicht direkt an das Stickstoffatom gebunden sind,
und in dem
A¹ CR¹⁰ bedeutet,
A² CR¹¹ bedeutet,
A³ CR¹² bedeutet, wobei R¹⁰, R¹¹ und R¹² jeweils unabhängig voneinander ein Wasserstoff- , Fluor- oder Chloratom, oder eine C₁₋₃-Alkyl-, CF₃-, HO-, CH₃O -gruppe, bedeuten, oder
D die Gruppe (II-4) darstellt 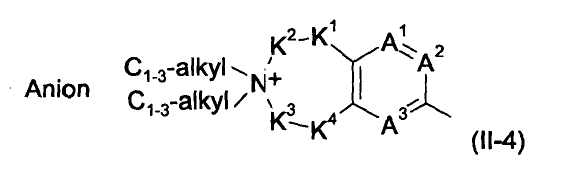 in der die Reste A1, A2, A3, K1, K2, K3, K4 wie oben definiert sind, und das Anion in (II-4) aus der Gruppe Fluorid, Chlorid, Bromid, lodid, Sulfat, Phosphat, Benzoat, Salicylat, Succinat, Citrat oder Tartrat ausgewählt werden kann,
deren Tautomere, deren Enantiomere, deren Diastereomere, deren Gemische und deren Salze.

7. Verbindungen der allgemeinen Formel (I) nach einem der Ansprüche 1 bis 6, in denen weder
R⁴ noch R⁵ ein Wasserstoffatom bedeuten dürfen,
deren Tautomere, deren Enantiomere, deren Diastereomere, deren Gemische und deren Salze.

8. Verbindungen der allgemeinen Formel (I) nach einem der Ansprüche 1 bis 6, in denen
R⁴ und R⁵ zusammen mit dem Kohlenstoffatom, an dem sie gebunden sind, eine C₅₋₆-Cycloalkenyl- oder C₃₋₇-Cycloalkylgruppe bilden, wobei eine der Methylengruppen einer C₄₋₇-Cycloalkylgruppe durch ein Sauerstoff- oder Schwefelatom ersetzt sein kann, mit der Maßgabe, dass eine solche, zusammen aus R⁴ und R⁵ gebildete C₃₋₇-Cycloalkylgruppe, in der eine der Methylengruppen des Cyclus, die direkt mit dem Kohlenstoffatom verbunden ist, an dem die Reste R⁴ und R⁵ angebunden sind, durch ein Sauerstoff- oder Schwefelatom ersetzt ist, ausgeschlossen ist,
deren Tautomere, deren Enantiomere, deren Diastereomere, deren Gemische und deren Salze.

9. Folgende Verbindungen der allgemeinen Formel (I) nach Anspruch 1:
3-[(5-Brom-thiophen-2-yl)-carbonylamino]-*N*-(3-methyl-2,3,4,5-tetrahydro-1*H-*benzo[*d*]azepin-7-yl)-tetrahydrofuran-3-carbonsäureamid,
3-[(5-Chlor-thiophen-2-yl)-carbonylamino]-*N*-(3-methyl-2,3,4,5-tetrahydro-1*H-*benzo[*d*]azepin-7-yl)-tetrahydrofuran-3-carbonsäureamid,
5-Chlor-thiophen-2-carbonsäure-*N*-[1-(3-ethyl-2,3,4,5-tetrahydro-1*H-*benzo[*d*]azepin-7-ylcarbamoyl)-1-methyl-ethyl]-amid,
5-Ethinyl-N-[1-methyl-1-(3-methyl-2,3,4,5-tetrahydro-1*H*-benzo[*d*]azepin-7-ylcarbamoyl)-ethyl]-thiophen-2-carbonsäureamid,
5-Chlor-thiophen-2-carbonsäure-*N*-[1-methyl-1-(3-methyl-2,3,4,5-tetrahydro-1*H-*benzo[d]azepin-7-ylcarbamoyl]-ethyl}-amid,
5-Brom-thiophen-2-carbonsäure-*N*-[1-methyl-1-(3-methyl-2,3,4,5-tetrahydro-1*H-*benzo[d]azepin-7-ylcarbamoyl]-ethyl}-amid,
5-Chlor-thiophen-2-carbonsäure-*N*-[2-methoxy-1-(3-methyl-2,3,4,5-tetrahydro-1*H*-benzo[*d*]azepin-7-ylcarbamoyl)-ethyl]-amid,
1-[(5-Brom-thiophen-2-yl)-carbonylamino]-*N*-(3-methyl-2,3,4,5-tetrahydro-1*H-*benzo[*d*]azepin-7-yl)-cyclopentan-1-carbonsäureamid,
1-[(5-Chlor-thiophen-2-yl)-carbonylamino]-*N*-(3-methyl-2,3,4,5-tetrahydro-1*H-*benzo[*d*]azepin-7-yl)-cyclopentan-1-carbonsäureamid,
3-[(5-Chlor-thiophen-2-yl)-carbonylamino]-*N*-(3-methyl-2,3,4,5-tetrahydro-1*H-*benzo[*d*]azepin-7-yl)-tetrahydrothiophen-3-carbonsäureamid,
1-[(5-Chlor-thiophen-2-yl)carbonylamino]-N-(3-methyl-2,3,4,5-tetrahydro-1H-benzo[d]azepin-7-yl)-cyclobutan-1-carbonsäureamid,
1-[(5-Brom-thiophen-2-yl)carbonylamino]-N-(3-methyl-2,3,4,5-tetrahydro-1H-benzo[d]azepin-7-yl)-cyclopent-3-en-1-carbonsäureamid,
1-[(5-Chlor-thiophen-2-yl)carbonylamino]-N-(3-methyl-2,3,4,5-tetrahydro-1H-benzo[d]azepin-7-yl)-cyclohexan-1-carbonsäureamid,
(R)-5-Chlor-thiophen-2-carbonsäure-*N*-[2-benzyloxy-1-(3-methyl-2,3,4,5-tetrahydro-1*H*-benzo[*d*]azepin-7-ylcarbamoyl)-ethyl]-amid,
5-Chlor-thiophen-2-carbonsäure-*N*-[2-benzyloxy-1-methyl-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[2-hydroxy-1-methyl-1-(3-methyl-2,3,4,5-tetrahydro-1*H*-benzo[*d*]azepin-7-ylcarbamoyl)-ethyl]-amid,
5-Brom-thiophen-2-carbonsäure-N-[3-hydroxy-1-methyl-1-(3-methyl-2,3,4,5-tetrahydro-1H-benzo[d]azepin-7-ylcarbamoyl)-propyl]-amid,
6-Chlor-thiophen-2-carbonsäure-N-[1-methyl-3-dimethylaminocarbonyl-1-(3-methyl-2,3,4,5-tetrahydro-1*H*-benzo[*d*]azepin-7-ylcarbamoyl)-propyl]-amid,
5-Chlor-thiophen-2-carbonsäure-*N*-[2-(4-hydroxy-phenyl)-1-methyl-1-(3-methyl-2,3,4,5-tetrahydro-1*H*-benzo[*d*]azepin-7-ylcarbamoyl)-ethyl]-amid, 5-Chlor-thiophen-2-carbonsäure-*N*-[1-methyl-1-(3,5-dimethyl-2,3,4,5-tetrahydro-1*H*-benzo[*d*]azepin-7-ylcarbamoyl)-ethyl]-amid,
B-Chlor-thiophen-2-carbonsäure-*N*-{1-methyl-1-[3-methyl-5-(4-aminophenyl)-2,3,4,5-tetrahydro-1*H*-benzo[*d*]azepin-7-ylcarbamoyl]-ethyl}-amid,
5-Chlor-thiophen-2-carbonsäure-*N*-[2-ethoxy-1-(3-methyl-2,3,4,5-tetrahydro-1*H-*benzo[*d*]azepin-7-ylcarbamoyl)-ethyl]-amid,
5-Chlor-thiophen-2-carbonsäure-*N*-[3-methoxy-1-(3-methyl-2,3,4,5-tetrahydro-1*H*-benzo[*d*]azepin-7-ylcarbamoyl)-propyl]-amid,
5-Chlor-thiophen-2-carbonsäure-*N*-[2-isopropyloxy-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[3-benzyloxy-1-(3-methyl-2,3,4,5-tetrahydro-1*H*-benzo[*d*]azepin-7-ylcarbamoyl)-propyl]-amid,
1-[(5-Chlor-thiophen-2-yl)-carbonylamino]-3,4-dimethoxy-*N*-(3-methyl-2,3,4,5-tetrahydro-1*H*-benzo[*d*]azepin-7-yl)-cyclopentan-1-carbonsäureamid,
5-Chlor-thiophen-2-carbonsäure-*N*-[C-(1-methyl-pyrazol-3-yl)-C-(3-methyl-2,3,4,5-tetrahydro-1*H*-benzo[*d]*azepin-7-ylcarbamoyl)-methyl]-amid,
(R)-5-Chlor-thiophen-2-carbonsäure-*N*-[2-phenyl-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[2-(furan-2-yl)-1-(3-methyl-2,3,4,5-tetrahydro-1*H*-benzo[*d*]azepin-7-ylcarbamoyl)-ethyl]-amid,
5-Chlor-thiophen-2-carbonsäure-*N*-[2-(4-methoxyphenyl)-1-(3-methyl-2,3,4,5-tetrahydro-1*H*-benzo[*d*]azepin-7-ylcarbamoyl)-ethyl]-amid,
5-Chlor-thiophen-2-carbonsäure-*N*-[2-(4-hydroxy-3-nitro-phenyl)-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[2-(4-hydroxy-phenyl)-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[2-cyclohexyl-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[3-aminocarbonyl-1-(3-methyl-2,3,4,5-tetrahydro-1*H*-benzo[*d*]azepin-7-ylcarbamoyl)-propyl]-amid,
(R)-5-Chlor-thiophen-2-carbonsäure-*N*-[2-acetylamino-1-(3-methyl-2,3,4,5-tetrahydro-1*H*-benzo[*d*]azepin-7-ylcarbamoyl)-ethyl]-amid,
(R)-5-Brom-thiophen-2-carbonsäure-*N*-[2-benzoylamino-1-(3-methyl-2,3,4,5-tetrahydro-1*H*-benzo[*d*]azepin-7-ylcarbamoyl)-ethyl]-amid,
(R)-5-Chlor-thiaphen-2-carbonsäure-*N*-[2-(2-hydroxycarbonyl-ethyl)carbonylamino-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[2-(2-hydroxycarbonyl-ethyl)carbonylamino-1-(3-methyl-2,3,4,5-tetrahydro-1*H*-benzo[*d*]azepin-7-ylcarbamoyl)-ethyl]-amid,
(R)-5-Chlor-thiophen-2-carbonsäure-*N*-[2-(4-methoxycarbonyl-butyl)carbonylamino-1-(3-methyl-2,3,4,5-tetrahydro-1*H*-benzo[*d*]azepin-7-ylcarbamoyl)-ethyl]-amid,
5-Chlor-thiophen-2-carbonsäure-*N*-[1-methyl-1-(3,3-dimethyl-2,3,4,5-tetrahydro-1*H*-benzo[*d*]azepinium-7-ylcarbamoyl]-ethyl}-amid iodid,
3-[(5-Chlor-thiophen-2-yl)-carbonylamino]-*N*-(3,5-dimethyl-2,3,4,5-tetrahydro-1*H*-benzo[*d*]azepin-7-yl)-tetrahydrofuran-3-carbonsäureamid,
sowie deren Tautomere, deren Enantiomere, deren Diastereomere, deren Gemische und deren Salze.

10. Physiologisch verträgliche Salze der Verbindungen gemäß einem der Ansprüche 1 bis 9.

11. Arzneimittel, enthaltend eine Verbindung nach mindestens einem der Ansprüche 1 bis 9 oder ein physiologisch verträgliches Salz gemäß Anspruch 10, neben gegebenenfalls einem oder mehreren inerten Trägerstoffen und/oder Verdünnungsmitteln.

12. Verwendung einer Verbindung nach mindestens einem der Ansprüche 1 bis 9 oder eines physiologisch verträglichen Salzes gemäß Anspruch 10 zur Herstellung eines Arzneimittels mit einem inhibitorischen Effekt auf Faktor Xa und/oder einem inhibitorischen Effekt auf verwandte Serinproteasen.

13. Verfahren zur Herstellung eines Arzneimittels gemäß Anspruch 11, **dadurch gekennzeichnet, dass** auf nichtchemischem Wege eine Verbindung nach mindestens einem der Ansprüche 1 bis 9 oder ein physiologisch verträgliches Salz gemäß Anspruch 10 in einen oder mehrere inerte Trägerstoffe und/oder Verdünnungsmittel eingearbeitet wird.

## Claims

1. Compounds of general formula (I) 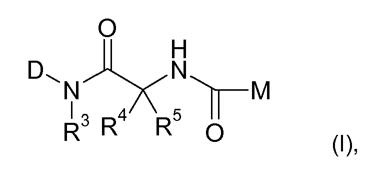 wherein
D denotes a substituted bicyclic ring system of formula (II), 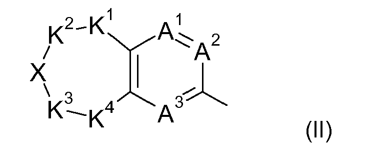 wherein
K¹ and K⁴ each independently of one another denote a -CH₂-, -CHR^{7a}-, -CR^{7b}R^{7c}- or a -C(O)- group, and
R^{7a}/R^{7b}/R^{7c} each independently of one another denote a fluorine atom, a hydroxy, C₁₋₅-alkyloxy, tetrahydrofuranyl, oxetanyl, amino, C₁₋₅-alkylamino, di- (C₁₋₅-alkyl)-amino, C₃₋₅-cycloalkyleneimino or C₁₋₅-alkylcarbonylamino group, a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl, C₁₋₅-alkylamino-C₁₋₅-alkyl, di-(C₁₋₅-alkyl)-amino-C₁₋₅-alkyl, C₄₋₇- cycloalkyleneimino-C₁₋₅-alkyl, carboxy-C₀₋₅-alkyl, C₁₋₅-alkyloxycarbonyl- C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl, C₄₋₇-cycloalkyleneiminocarbonyl- C₀₋₅-alkyl- group, a phenyl group or a 5- or 6-membered heteroaryl group which may be substituted by 1-2 substituents selected from among a nitro, amino, hydroxy, methoxy, cyano, C₁₋₅-alkyl group or a fluorine, chlorine or bromine atom, wherein the two groups R^{7b}/R^{7c} cannot both simultaneously be bound to the cyclic carbon atom via a heteroatom, except if -C(R^{7b}R^{7c})- corresponds to a -CF₂- group, or two groups R^{7b}/R^{7c} together with the cyclic carbon atom may form a 3, 4, 5-, 6- or 7-membered saturated carbocyclic group or a cyclopentene, cyclohexene, oxetane, azetidine, thietane, tetrahydrofuran, pyrrolidine, tetrahydrothiophene, tetrahydropyran, piperidine, pentamethylene sulphide, hexamethyleneimine, 1.3-dioxolane, 1,4-dioxane, hexahydropyridazine, piperazine, thiomorpholine, morpholine, 2- imidazolidinone, 2-oxazolidinone, tetrahydro-2(1H)-pyrimidinone, [1,3]oxazinan-2-one ring, wherein the methylene groups thereof may be substituted by 1-2 C₁₋₃-alkyl or CF₃ groups, and/or the methylene groups thereof, if they are not bound to a heteroatom, may be substituted by 1-2 fluorine atoms, and/or wherein a -CH₂ group adjacent to an N atom may be replaced by a -CO group, and/or the imino groups of which may be substituted in each case by a C₁₋₃-alkyl or C₁₋₃-alkylcarbonyl group, and/or wherein the sulphur atom may be oxidised to form a sulphoxide or sulphone group,
K² and K³ each independently of one another denote a -CH₂-, -CHR^{8a}-, -CR^{8b}R^{8c}- or a -C(O)- group, and
R^{8a}/R^{8b}/R^{8c} each independently of one another denote a C₁₋₅-alkyl group which may be substituted by 1-3 fluorine atoms, a hydroxy-C₁₋₅-alkyl, C₁₋₅-alkyloxy- C₁₋₅-alkyl, amino-C₁₋₅-alkyl, C₁₋₅-alkylamino-C₁₋₅-alkyl, di-(C₁₋₅-alkyl)- amino-C₁₋₅-alkyl, C₄₋₇-cycloalkyleneimino-C₁₋₅-alkyl, carboxy-C₀₋₅-alkyl, C₁₋₅-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅- alkyl, C₄₋₇-cycloalkyleneiminocarbonyl-C₀₋₅-alkyl group, or two groups R^{3b}/R^{3c} together with the cyclic carbon atom may form a 3, 4, 5-, 6- or 7-membered saturated carbocyclic group or a cyclopentene, cyclohexene, oxetane, azetidine, thietane, tetrahydrofuran, pyrrolidine, tetrahydrothiophene, tetrahydropyran, piperidine, pentamethylene sulphide, hexamethyleneimine, hexahydropyridazine, tetrahydro-2(1H)- pyrimidinone, [1,3]oxazinan-2-one ring, wherein the methylene groups thereof may be substituted by 1-2 C₁₋₃-alkyl or CF₃ groups, and/or the methylene groups thereof, if they are not bound to a heteroatom, may be substituted by 1-2 fluorine atoms, and/or wherein a -CH₂ group next to a nitrogen atom may be replaced by a -CO group, and/or the imino groups of which may be substituted in each case by a C₁₋₃-alkyl or C₁₋₃-alkylcarbonyl group, and/or wherein the sulphur atom may be oxidised to form a sulphoxide or sulphone group, with the proviso that a heteroatom introduced by R^{8b} or R^{8c} may not be separated from X in formula (I) by only one carbon atom, and in all there should be no more than four groups selected from among R^{7a}, R^{7b}, R^{7c}, R^{8a}, R^{8b} and R^{8c} in formula (II), and
X denotes an oxygen or sulphur atom, a sulphene, sulphone or an NR¹ group, wherein
R¹ denotes a hydrogen atom or a hydroxy, C₁₋₃-alkyloxy, amino, C₁₋₃-alkylamino, di-(C₁₋₃-alkyl)-amino, a C₁₋₅-alkyl, C₂₋₅-alkenyl-CH₂, C₂₋₅-alkynyl-CH₂, C₃₋₆-cycloalkyl, C₄₋₆-cycloalkenyl, oxetan-3-yl, tetrahydrofuran-3-yl, benzyl, C₁₋₅-alkyl-carbonyl, trifluoromethylcarbonyl, C₃₋₆-cycloalkyl-carbonyl, C₁₋₅-alkyl- sulphonyl, C₃₋₆-cycloalkyl-sulphonyl, aminocarbonyl, C₁₋₅-alkylaminocarbonyl, di-(C₁₋₅-alkyl)-aminocarbonyl, C₁₋₅- alkyloxycarbonyl, C₄₋₇-cycloalkyleneiminocarbonyl group, wherein the methylene and methyl groups present in the above-mentioned groups may additionally be substituted by a C₁₋₃-alkyl, carboxy, C₁₋₅-alkoxycarbonyl group or by a hydroxy, C₁₋₅-alkyloxy, amino, C₁₋₅-alkylamino, C₁₋₅- dialkylamino or C₄₋₇-cycloalkyleneimino group, provided that the methylene or methyl groups are not directly bound to a heteroatom selected from among O, N or S, and/or one to three hydrogen atoms may be replaced by fluorine atoms, provided that the methylene or methyl groups are not directly bound to a heteroatom selected from among O, N or S,
and wherein
A¹ denotes either N or CR¹⁰,
A² denotes either N or CR¹¹,
A³ denotes either N or CR¹², wherein R¹⁰, R¹¹ and R¹² each independently of one another represent a hydrogen, fluorine, chlorine, bromine or iodine atom, or a C₁₋₅-alkyl, CF₃, C₂₋₅ -alkenyl, C₂₋₅-alkynyl, a cyano, carboxy, C₁₋₅-alkyloxycarbonyl, hydroxy, C₁₋₃-alkyloxy, CF₃O, CHF₂O, CH₂FO, amino, C₁₋₅-alkylamino, di-(C₁₋₅-alkyl)-amino or C₄₋₇-cycloalkyleneimino group, or
D denotes one of the four groups (II-1), (II-2), (II-3) or (II-4) 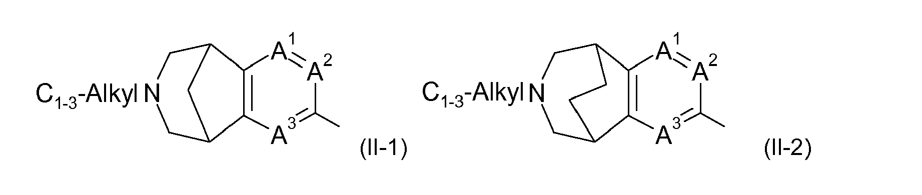 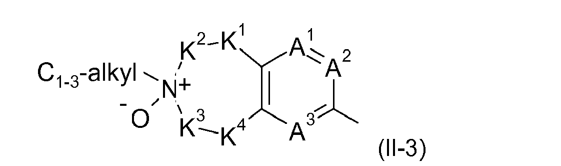 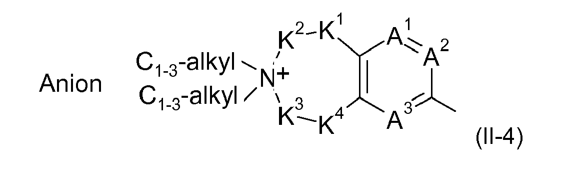 wherein the groups A1, A2, A3, K1, K2, K3, K4 are as hereinbefore defined, and Anion in (II-4) denotes a fluoride, chloride, bromide, iodide, sulphate, hydrogen sulphate, phosphate, hydrogen phosphate, benzoate, salicylate, succinate, citrate or tartrate,
R³ denotes a hydrogen atom or a C₁₋₃-alkyl group, and
R⁴ and R⁵ each independently of one another represent a hydrogen atom, a straight-chain or branched C₁₋₆-alkyl, C₂₋₆-alkenyl or C₂₋₆-alkynyl group, wherein the hydrogen atoms of the methylene and/or methyl fragments of the straight-chain or branched C₁₋₆-alkyl, C₂₋₆-alkenyl or C₂₋₆-alkynyl group may optionally be wholly or partly replaced by fluorine atoms, and/or one or two hydrogen atoms of the straight-chain or branched C₁₋₆-alkyl, C₂₋₆-alkenyl or C₂₋₆-alkynyl group in their methylene and/or methyl fragments may optionally each be replaced independently of one another by a C₃₋₇-cycloalkyl group, a nitrile, hydroxy or C₁₋₅-alkyloxy group, wherein the hydrogen atoms of the C₁₋₅-alkyloxy group may optionally be wholly or partly replaced by fluorine atoms, an allyloxy, propargyloxy, phenylmethyloxy, phenethyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, C₁₋₅-alkyloxy-C₂₋₅-alkyloxy, mercapto, C₁₋₅-alkylsulphanyl, C₁₋₅-alkylsulphinyl, C₁₋₅-alkylsulphonyl, carboxy, C₁₋₅-alkyloxycarbonyl, aminocarbonyl, C₁₋₅-alkylaminocarbonyl, di- (C₁₋₅-alkyl)-aminocarbonyl, C₁₋₅-alkyl-aminocarbonyloxy, di-(C₁₋₅-alkyl)- aminocarbonyloxy, C₄₋₇-cycloalkyleneiminocarbonyl, aminosulphonyl, C₁₋₅-alkylaminosulphonyl, di-(C₁₋₅-alkyl)-aminosulphonyl, C₄₋₇- cycloalkyleneiminosulphonyl, di-(C₁₋₅-alkyl)-phosphoryl, amino, C₁₋₅-alkylamino, di-(C₁₋₅-alkyl)-amino, C₁₋₅-alkylcarbonylamino, trifluoracetylamino, C₁₋₅-alkyloxy-C₁₋₅-alkylcarbonylamino, phenylcarbonylamino, C₁₋₅-alkylaminocarbonylamino, di-(C₁₋₅-alkyl)- aminocarbonylamino, C₁₋₅-alkyloxy-carbonylamino, phenylmethyloxy- carbonylamino, C₁₋₅-alkyloxy-C₂₋₅-alkyloxy-C₁₋₂-alkylcarbonylamino, C₁₋₅-alkylsulphonylamino, N-(C₁₋₅-alkylsulphonyl)-C₁₋₅-alkylamino, C₃₋₆- cycloalkylcarbonylamino group, 4-morpholinocarbonylamino, or a morpholinyl, thiomorpholinyl, pyrrolidinyl, piperidinyl, piperazinyl, tetrahydrofuranyl, tetrahydropyranyl group, wherein the above-mentioned carbo- and heterocycles in the ring may each be substituted by 1 to 4 C₁₋₃-alkyl or C₁₋₃-alkylcarbonyl groups or may each be substituted by 1 or 2 oxo groups, and/or the above-mentioned phenyl and heteroaryl groups may be replaced by 1 or 2 substituents selected from among fluorine, chlorine, bromine, methyl, methoxy, amino or trifluoromethyl or two adjacent carbon atoms of a phenyl ring may be substituted by a -CH₂-O-CH₂ group, and/or the above-mentioned alkyl groups may be substituted by a cyano-C₁₋₅-alkyloxycarbonyl or carboxy group, wherein the above-mentioned carboxylic acid or sulphonic acid amide may optionally be additionally substituted at the nitrogen by a C₁₋₅-alkyl group, and/or the hydrogen atoms of the sp²-hybridised carbon atoms of the straight-chain or branched C₂₋₆-alkenyl group may optionally be wholly or partly replaced by fluorine atoms, a carboxy, aminocarbonyl, C₁₋₅-alkylaminocarbonyl, C₃₋₆-cycloalkylamino- carbonyl, di-(C₁₋₅-alkyl)-aminocarbonyl, C₁₋₅-alkyloxycarbonyl, C₄₋₇- cycloalkyleneiminocarbonyl group, a phenyl, mono- or bicyclic heteroaryl, phenyl-C₁₋₅-alkyl or mono- or bicyclic heteroaryl-C₁₋₅-alkyl group, which may optionally be mono- to trisubstituted in the phenyl or heteroaryl moiety by identical or different substituents selected from among fluorine, chlorine, bromine and iodine atoms, and C₁₋₅-alkyl, trifluoromethyl, benzyl, amino, nitro, C₁₋₅-alkyl-amino, di-(C₁₋₅-alkyl)- amino, hydroxy, C₁₋₅-alkyloxy, mono-, di- or trifluoromethoxy, carboxy- and C₁₋₅-alkyloxycarbonyl group, or two adjacent carbon atoms of a phenyl ring may be substituted by a -CH₂-O-CH₂- group, or a C₃₋₇cycloalkyl, morpholinyl, thiomorpholinyl, pyrrolidinyl, piperidinyl, piperazinyl, tetrahydrofuranyl, tetrahydropyranyl group, which may optionally be substituted by one to two groups selected independently of one another from among C₁₋₃-alkyl, acetyl, C₁₋₅- alkyloxycarbonyl, and hydroxycarbonyl, or
R⁴ and R⁵ together with the carbon atom to which they are bound, form a C₃₋₇-cycloalkyl or C₅₋₇-cycloalkenyl group, wherein one of the methylene groups of a C₄₋₇-cycloalkyl group may be replaced by an oxygen or sulphur atom or an -NH-, -N(C₁₋₅-alkyl) -, -N(C₁₋₄-alkylcarbonyl)-, -N(C₁₋₄-alkyloxycarbonyl)- or a carbonyl, sulphinyl or sulphonyl group, and/or two directly adjacent methylene groups of a C₄₋₇-cycloalkyl group may together be replaced by a -C(O)NH-, -C(O)N(C₁₋₅-alkyl)-, -S(O)₂NH-, or -S(O)₂N(C₁₋₅-alkyl)- group, and/or 1 to 3 carbon atoms of a C₃₋₇-cycloalkyl group may each optionally be substituted independently of one another by one or two fluorine atoms or one or two C₁₋₅-alkyl groups or a hydroxy, C₁₋₅-alkyloxy, formyloxy, C₁₋₅-alkylcarbonyloxy, C₁₋₅-alkylsulphanyl, C₁₋₅-alkylsulphonyl, aminosulphonyl, C₁₋₅-alkylaminosulphonyl, di-(C₁₋₅-alkyl)- aminosulphonyl, C₄₋₇-cycloalkyleneiminosulphonyl, amino, C₁₋₅-alkylamino, di-(C₁₋₅-alkyl)-amino, C₁₋₅-alkylcarbonylamino, C₁₋₅-alkyl- sulphonylamino, *N*-(C₁₋₅-alkylsulphonyl)-C₁₋₅-alkylamino, C₃₋₆- cycloalkylcarbonylamino, Nitril, carboxy-C₁₋₅-alkyl, C₁₋₅-alkyloxycarbonyl- C₁₋₅-alkyl, carboxy, C₁₋₅-alkyloxycarbonyl, aminocarbonyl, C₁₋₅-alkylaminocarbonyl, di-(C₁₋₅-alkyl)-aminocarbonyl or C₄₋₇- cycloalkyleneiminocarbonyl group, and/or 1 to 2 carbon atoms of a C₅₋₇-cycloalkenyl group may optionally be substituted independently of one another by in each case a C₁₋₅-alkyl, nitrile, carboxy-C₁₋₅-alkyl, C₁₋₅-alkyloxycarbonyl-C₁₋₅-alkyl, carboxy, C₁₋₅-alkyloxycarbonyl, aminocarbonyl, C₁₋₅-alkylaminocarbonyl, di- (C₁₋₅-alkyl)-aminocarbonyl, C₃₋₆-cycloalkyleneiminocarbonyl, aminosulphonyl, C₁₋₅-alkylaminosulphonyl, di-(C₁₋₅-alkyl)- aminosulphonyl, C₃₋₆-cycloalkyleneiminosulphonyl groups or 1-2 fluorine atoms, and/or 1 to 2 carbon atoms of a C₄₋₇-cycloalkenyl group which are not bound to another carbon atom by a double bond, may optionally be substituted independently of one another by a hydroxy, C₁₋₅-alkyloxy, C₁₋₅-alkylcarbonyloxy, C₁₋₅-alkylsulphanyl, C₁₋₅-alkylsulphonyl, amino, C₁₋₅-alkylamino, di-(C₁₋₅-alkyl)-amino, C₁₋₅-alkylcarbonylamino, C₁₋₅-alkyl- sulphonylamino, *N*-(C₁₋₅-alkylsulphonyl)-C₁₋₅-alkylamino or C₃₋₆- cycloalkylcarbonylamino groups, with the proviso that a C₃₋₇-cycloalkyl or C₅₋₇-cycloalkenyl group of this kind formed from R⁴ and R⁵ together, wherein two heteroatoms in the cyclic group selected from among oxygen and nitrogen are separated from one another by precisely one optionally substituted -CH₂- group, and/or wherein one or both methylene groups of the cyclic group which are joined directly to the carbon atom to which the groups R⁴ and R⁵ are linked, are replaced by a heteroatom selected from among oxygen, nitrogen and sulphur, and/or wherein a substituent bound to the cyclic group, which is **characterised in that** a heteroatom selected from among oxygen, nitrogen, sulphur and fluorine is bound directly to the cyclic group, is separated from one other heteroatom selected from among oxygen, nitrogen and sulphur, with the exception of the sulphone group, by precisely one, optionally substituted, methylene group, and/or wherein two atoms in the ring form an -O-O- or -S-O- bond, is excluded,
M denotes a thiophene ring according to formula (III), 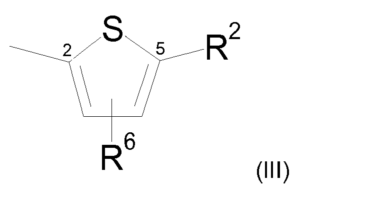 which is bound to the carbonyl group in formula (I) via the 2-position and which is substituted in the 5-position by R² and optionally additionally by R⁶, wherein
R² denotes a hydrogen, fluorine, chlorine, bromine or iodine atom or a methoxy, C₁₋₂-alkyl, formyl, NH₂CO or ethynyl group,
R⁶ denotes a hydrogen, fluorine, chlorine, bromine or iodine atom or a C₁₋₂- alkyl or amino group,
wherein, unless otherwise stated, the term "heteroaryl group" mentioned hereinbefore in the definitions denotes a monocyclic 5- or 6-membered heteroaryl group, wherein
the 6-membered heteroaryl group contains one, two or three nitrogen atoms, and
the 5-membered heteroaryl group contains an imino group optionally substituted by a C₁₋₃-alkyl group, or an oxygen or sulphur atom, or
an imino group optionally substituted by a C₁₋₃-alkyl group or an oxygen or sulphur atom and additionally one or two nitrogen atoms, or
an imino group optionally substituted by a C₁₋₃-alkyl group and three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group phenyl ring may be fused to the above-mentioned monocyclic heteroaryl groups via two adjacent carbon atoms,
and the bond is effected via a nitrogen atom or a carbon atom of the heterocyclic moiety or a fused-on phenyl ring,
and wherein, unless otherwise stated, by the term "halogen atom" mentioned hereinbefore in the definitions is meant an atom selected from among fluorine, chlorine, bromine and iodine,
and wherein the alkyl, alkenyl, alkynyl and alkyloxy groups contained in the previously mentioned definitions which have more than two carbon atoms may, unless otherwise stated, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless otherwise stated, may be wholly or partly replaced by fluorine atoms, the tautomers, enantiomers, diastereomers, mixtures and salts thereof.

2. Compounds of general formula (I) according to claim 1, wherein
D denotes a substituted bicyclic ring system of formula (II), 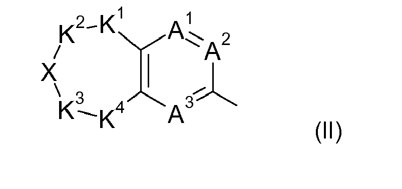 wherein
K¹ and K⁴ each independently of one another denote a -CH₂-, -CHR^{7a}-, -CR^{7b}R^{7c}- or a -C(O)- group, wherein
R^{7a}/R^{7b}/R^{7c} each independently of one another denote a fluorine atom, a hydroxy, C₁₋₅-alkyloxy group, a C₁₋₅-alkyl group which may be substituted by 1-3 fluorine atoms, a hydroxy-C₁₋₅-alkyl, C₁₋₅-alkyloxy-C₁₋₅-alkyl group, or a phenyl group which may be substituted by 1-2 substituents selected from among a nitro, amino, hydroxy- methoxy, cyano, C₁₋₅-alkyl group or a fluorine, chlorine or bromine atom, or a 5- or 6-membered heteroaryl group, wherein the two groups R^{7b}/R^{7c} cannot both simultaneously be bound to the cyclic carbon atom via a heteroatom, except if -C(R^{7b}R^{7c})- corresponds to a -CF₂- group, or two groups R^{7b}/R^{7c} may form, together with the cyclic carbon atom, a 3-, 4-, 5-, 6- or 7-membered saturated carbocyclic group or a cyclopentene, cyclohexene, oxetane, tetrahydrofuran or tetrahydropyran ring, wherein the methylene groups thereof may be substituted by 1-2 C₁₋₃-alkyl or CF₃ groups, and/or the methylene groups thereof, if they are not bound to a heteroatom, may be substituted by 1-2 fluorine atoms, and
K² and K³ each independently of one another represent a -CH₂-, -CHR^{8a}-, -CR^{8b}R^{8c}- or a -C(O)- group, and
R^{8a}/R^{8b}/R^{8c} each independently of one another denote a C₁₋₅-alkyl group which may be substituted by 1-3 fluorine atoms, a hydroxy-C₁₋₅-alkyl, C₁₋₅-alkyloxy- C₁₋₅-alkyl group, or two groups R^{3b}/R^{3c} may form, together with the cyclic carbon atom, a 3-, 4-, 5-, 6- or 7-membered carbocyclic group or a cyclopentene, cyclohexene, oxetane, tetrahydrofuran, tetrahydropyran ring, wherein the methylene groups thereof may be substituted by 1-2 C₁₋₃-alkyl or CF₃ groups, and/or the methylene groups thereof, if they are not bound to a heteroatom, may be substituted by 1-2 fluorine atoms, with the proviso that a heteroatom introduced by R^{8b} or R^{8c} must not be separated from X in formula (I) by only one carbon atom, and in all in formula (II) there should be no more than four groups selected from among R^{7a}, R^{7b}, R^{7c}, R^{8a}, R^{8b} and R^{8c}, and
X denotes an oxygen or sulphur atom, a sulphene, sulphone or an NR¹ group, wherein
R¹ denotes a hydrogen atom or a C₁₋₅-alkyl, C₂₋₅-alkenyl-CH₂, C₂₋₅-alkynyl-CH₂, C₃₋₆-cycloalkyl, C₄₋₆- cycloalkenyl, oxetan-3-yl, tetrahydrofuran-3-yl, benzyl, C₁₋₅-alkyl- carbonyl, trifluoromethylcarbonyl, C₃₋₆-cycloalkyl-carbonyl, C₁₋₅- alkyl-sulphonyl, C₃₋₆-cycloalkyl-sulphonyl, aminocarbonyl, C₁₋₅-alkylaminocarbonyl, di-(C₁₋₅-alkyl)-aminocarbonyl, C₁₋₅- alkyloxycarbonyl, C₄₋₇-cycloalkyleneiminocarbonyl group, wherein the methylene and methyl groups present in the above-mentioned groups may additionally be substituted by a C₁₋₃-alkyl, carboxy, C₁₋₅-alkoxycarbonyl group or by a hydroxy, C₁₋₅-alkyloxy, amino, C₁₋₅-alkylamino, C₁₋₅- dialkylamino or C₄₋₇-cycloalkyleneimino group, provided that the methylene or methyl groups are not directly bound to a heteroatom selected from among O, N or S, and/or one to three hydrogen atoms may be replaced by fluorine atoms, provided that the methylene or methyl groups are not directly bound to a heteroatom selected from among O, N or S, and wherein A¹, A², and A³ are each defined as described in the 1 st embodiment, or
D denotes one of the four groups (II-1a), (II-2a), (II-3) or (II-4) 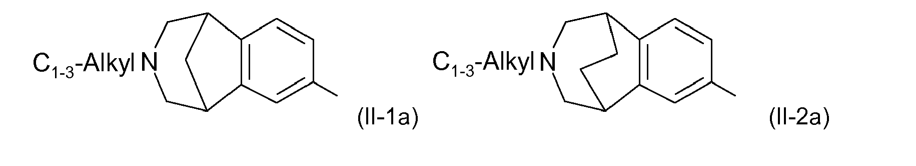 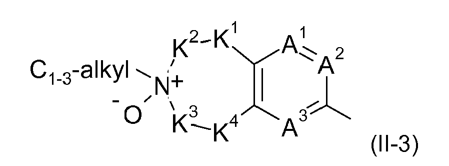 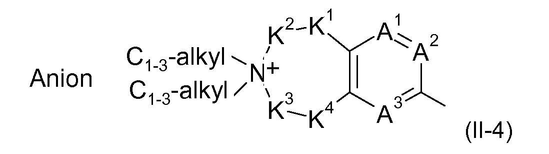 wherein the groups A1, A2, A3, K1, K2, K3, K4 are as hereinbefore defined, and Anion in (II-4) denotes a fluoride, chloride, bromide, iodide, sulphate, hydrogen sulphate, phosphate, hydrogen phosphate, benzoate, salicylate, succinate, citrate or tartrate,
and
R³ denotes a hydrogen atom, and
R⁴, R⁵ and M are each defined as described in the 1 st embodiment,
the tautomers, enantiomers, diastereomers, mixtures and salts thereof.

3. Compounds of general formula (I) according to claim 1 or 2, wherein
D denotes a substituted bicyclic ring system of formula (II), 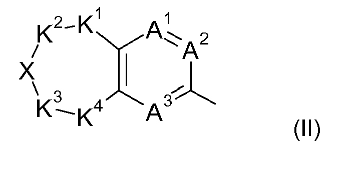 wherein K1, K2, K3 and K4 are defined as in claim 1 or 2, and
X denotes an NR¹ group, wherein
R¹ denotes a hydrogen atom or a C₁₋₅-alkyl, C₂₋₅-alkenyl-CH₂, C₂₋₅-alkynyl-CH₂, C₃₋₆-cycloalkyl, C₄₋₆-cycloalkenyl group, wherein the methylene and methyl groups present in the above-mentioned groups may additionally be substituted by a C₁₋₃-alkyl, carboxy, C₁₋₅-alkoxycarbonyl group or by a hydroxy, C₁₋₅-alkyloxy, amino, C₁₋₅-alkylamino, C₁₋₅- dialkylamino or C₄₋₇-cycloalkyleneimino group, provided that the methylene or methyl groups are not directly bound to a heteroatom selected from among O, N or S, and/or one to three hydrogen atoms may be replaced by fluorine atoms, so long as the methylene or methyl groups are not directly bound to the nitrogen atom, and wherein
A¹ denotes either N or CR¹⁰,
A² denotes either N or CR¹¹,
A³ denotes either N or CR¹², wherein R¹⁰, R¹¹ and R¹² each independently of one another represent a hydrogen, fluorine, chlorine, bromine atom or a C₁₋₅-alkyl, CF₃, a cyano, carboxy, C₁₋₅-alkyloxycarbonyl, hydroxy, C₁₋₃-alkyloxy, CF₃O, CHF₂O, CH₂FO group, or
D denotes one of the four groups (II-1a), (II-2a), (II-3) or (II-4) 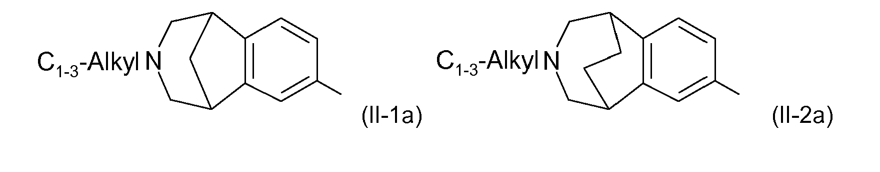 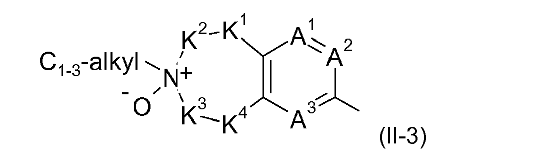 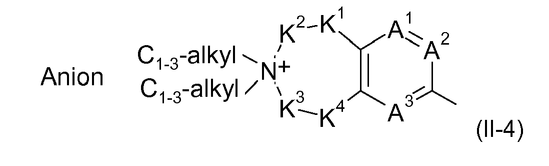 wherein the groups A1, A2, A3, K1, K2, K3, K4 are as hereinbefore defined, and Anion in (II-4) may be selected from among fluoride, chloride, bromide, iodide, sulphate, phosphate, benzoate, salicylate, succinate, citrate and tartrate, and
R³, R⁴, R⁵ and M are each defined as in claim 1 or 2, wherein R⁶ denotes a hydrogen
atom,
the tautomers, enantiomers, diastereomers, mixtures and salts thereof.

4. Compounds of general formula (I) according to one of claims 1 to 3, wherein
D, R³ and M are each defined as in one of claims 1 to 3, and
R⁴ denotes a straight-chain or branched C₃₋₆-alkenyl or C₃₋₆-alkynyl group, a straight-chain or branched C₁₋₆-alkyl group, wherein the hydrogen atoms of the straight-chain or branched C₁₋₆-alkyl group may optionally be wholly or partly replaced by fluorine atoms, and wherein optionally one to two hydrogen atoms may be replaced independently of one another by a C₃₋₇-cycloalkyl, hydroxy, C₁₋₅-alkyloxy, phenylmethyloxy, phenethyloxy, carboxy-C₁₋₅-alkyloxy, C₁₋₅-alkyloxycarbonyl-C₁₋₅-alkyloxy, C₁₋₅-alkyloxy-C₂₋₅-alkyloxy, C₁₋₅-alkylsulphanyl, C₁₋₅-alkylsulphinyl, C₁₋₅-alkylsulphonyl, carboxy, C₁₋₅-alkyloxycarbonyl, aminocarbonyl, C₁₋₅-alkylaminocarbonyl, di- (C₁₋₅-alkyl)-aminocarbonyl, C₁₋₅-alkyl-aminocarbonyloxy, di-(C₁₋₅-alkyl)- aminocarbonyloxy, C₄₋₇-cycloalkyleneiminocarbonyl, amino, C₁₋₅- alkylamino or di-(C₁₋₅-alkyl)-amino group C₁₋₅-alkylcarbonylamino, trifluoracetylamino, C₁₋₅-alkyloxy-C₁₋₅-alkylcarbonylamino, phenylcarbonylamino, C₁₋₅-alkylaminocarbonylamino, di-(C₁₋₅-alkyl)- aminocarbonylamino, C₁₋₅-alkyloxy-carbonylamino, phenylmethyloxy- carbonylamino, C₁₋₅-alkyloxy-C₂₋₅-alkyloxy-C₁₋₂-alkylcarbonylamino, C₁₋₅-alkylsulphonylamino, C₃₋₆-cycloalkylcarbonylamino group, 4- morpholinocarbonylamino- group, wherein the above-mentioned carbo- and heterocycles in the ring may each be substituted by 1 to 4 C₁₋₃-alkyl or C₁₋₃-alkylcarbonyl groups or may each be substituted by 1 or 2 oxo groups, and/or the above-mentioned phenyl and heteroaryl groups may be replaced by 1 to 2 substituents selected from among fluorine, chlorine, bromine, methyl, methoxy, or trifluoromethyl, or two adjacent carbon atoms of a phenyl ring may be substituted by a -CH₂-O-CH₂- group, and/or the above-mentioned alkyl groups may be substituted by a cyano-C₁₋₅-alkyloxycarbonyl or carboxy group, wherein the above-mentioned carboxylic acid or sulphonic acid amide may optionally additionally be substituted at the nitrogen by a C₁₋₅-alkyl group, a phenyl, phenyl-C₁₋₂-alkyl, heteroaryl-C₁₋₂-alkyl or C-linked heteroaryl group, wherein the heteroaryl group is selected from among imidazolyl, furanyl, thiophenyl, thiazolyl, pyrazolyl, tetrazolyl, benzimidazolyl, indolyl, pyrimidinyl, pyrazinyl- oxazolyl, 1,2,4-triazolyl and pyridinyl, and which may optionally be mono- to disubstituted in the phenyl or heteroaryl moiety by identical or different substituents selected from among chlorine or fluorine atoms or C₁₋₃-alkyl, benzyl, hydroxy, amino, CF₃, CH₃O or CHF₂O groups,
R⁵ denotes a hydrogen atom, a straight-chain or branched C₁₋₄-alkyl group, wherein the hydrogen atoms of the straight-chain or branched C₁₋₄-alkyl groups may optionally be wholly or partly replaced by fluorine atoms, or a propargyl or C₁₋₃alkyloxy- C₁₋₃-alkyl group, or
R⁴ and R⁵ together with the carbon atom to which they are bound, form a C₅₋₆-cycloalkenyl or C₃₋₇-cycloalkyl group, wherein one of the methylene groups of a C₄₋₇-cycloalkyl group may be replaced by an oxygen or sulphur atom or an -NH-, -N(C₁₋₅-alkyl)-, -N(C₁₋₄-alkylcarbonyl)-, carbonyl, sulphinyl or a sulphonyl group, or two immediately adjacent methylene groups of a C₄₋₇-cycloalkyl group may together be replaced by a -C(O)NH-, -C(O)N(C₁₋₅-alkyl)-, -S(O)₂NH- or -S(O)₂N(C₁₋₅-alkyl)- group, and/or 1 to 2 carbon atoms of a C₃₋₇-cycloalkyl group may optionally be substituted independently of one another by in each case one or two fluorine atoms or one or two C₁₋₅-alkyl groups or a hydroxy, C₁₋₅-alkyloxy, formyloxy, amino, C₁₋₅-alkylamino, di-(C₁₋₅-alkyl)-amino, C₁₋₅-alkylcarbonylamino, C₃₋₆-cycloalkylcarbonylamino, nitrile, carboxy,
C₁₋₅-alkyloxycarbonyl, aminocarbonyl, C₁₋₅-alkylaminocarbonyl, di- (C₁₋₅-alkyl)-aminocarbonyl or C₄₋₇-cycloalkyleneiminocarbonyl group,
with the proviso that a C₃₋₇-cycloalkyl group of this kind, formed from R⁴ and R⁵ together,
wherein two heteroatoms in the cyclic group selected from among oxygen and nitrogen are separated from one another by precisely one optionally substituted -CH₂- group, and/or
wherein one or both methylene groups of the cyclic group which are joined directly to the carbon atom to which the groups R⁴ and R⁵ are bound, are replaced by a heteroatom selected from among oxygen, nitrogen and sulphur, and/or
wherein a substituent bound to the cyclic group, which is **characterised in that** a heteroatom selected from among oxygen, nitrogen, sulphur and fluorine atom is bound directly to the cyclic group, is separated from another heteroatom selected from among oxygen, nitrogen and sulphur by precisely one optionally substituted methylene group, and/or
wherein two atoms in the ring form a -O-O- or -S-O- bond,
is excluded,
the tautomers, enantiomers, diastereomers, mixtures and salts thereof.

5. Compounds of general formula (I) according to one of claims 1 to 4, wherein
D denotes a substituted bicyclic ring system of formula (II), 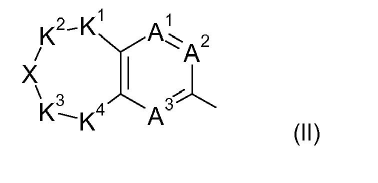 wherein
K¹ and K⁴ each independently of one another represent a -CH₂-, -CHR^{7a}- or a -CR^{7b}R^{7c}- group, wherein
R^{7a}/R^{7b}/R^{7c} each independently of one another denote a C₁₋₂-alkyl group or a phenyl group which may be substituted by 1 or 2 substituents selected from among a nitro, amino, hydroxyl, methoxy, cyano, C₁₋₅-alkyl group or a fluorine, chlorine or bromine atom,
K² and K³ each denote a -CH₂- group
X denotes an NR¹ group, wherein
R¹ denotes a hydrogen atom or a C₁₋₅-alkyl, C₂₋₄-alkenyl-CH₂, C₂₋₄-alkynyl-CH₂ or C₃₋₆-cycloalkyl group, wherein the methylene and methyl groups present in the previously mentioned C₂₋₅-alkyl groups may be substituted by one to three fluorine atoms, as long as the methylene or methyl groups are not directly bound to the nitrogen atom, and wherein
A¹ denotes either N or CR¹⁰,
A² denotes either N or CR¹¹,
A³ denotes either N or CR¹², wherein R¹⁰, R¹¹ and R¹² each independently of one another represent a hydrogen, fluorine or chlorine atom, or a C₁₋₃-alkyl, CF₃, hydroxy or CH₃O group, or
D denotes one of the groups (II-3) or (II-4) 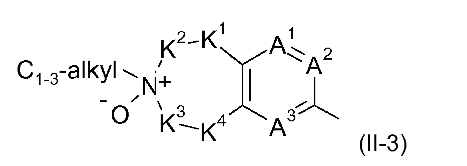 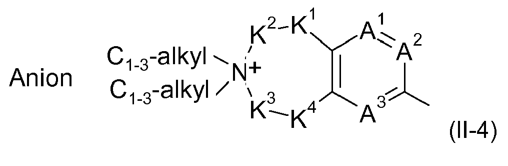 wherein the groups A1, A2, A3, K1, K2, K3, K4 are as hereinbefore defined, and the anion in (II-4) may be selected from among fluoride, chloride, bromide, iodide, sulphate, phosphate, benzoate, salicylate, succinate, citrate or tartrate, and
R³ denotes a hydrogen atom,
R⁴ denotes a straight-chain or branched C₃₋₆-alkenyl or C₃₋₆-alkynyl group, a straight-chain or branched C₁₋₄-alkyl group, wherein the hydrogen atoms of the straight-chain or branched C₁₋₄-alkyl group may optionally be partially replaced by up to four fluorine atoms, and wherein optionally one to two hydrogen atoms may be replaced independently of one another by a C₃₋₇-cycloalkyl, hydroxy, C₁₋₅-alkyloxy, phenylmethyloxy, C₁₋₅-alkylsulphanyl, C₁₋₅-alkylsulphinyl, C₁₋₅-alkylsulphonyl, carboxy, C₁₋₅-alkyloxycarbonyl, aminocarbonyl, C₁₋₅-alkylaminocarbonyl, di-(C₁₋₅-alkyl)-aminocarbonyl, C₄₋₇- cycloalkyleneiminocarbonyl, amino, C₁₋₅-alkylamino or di-(C₁₋₅-alkyl)- amino, C₁₋₅-alkylcarbonylamino, carboxy-C₁₋₅-alkylcarbonylamino or a C₁₋₅-alkyloxycarbonyl-C₁₋₅-alkylcarbonylamino group, wherein the above-mentioned phenyl groups may be replaced by 1 or 2 substituents selected from fluorine, chlorine, bromine, methyl, methoxy, or trifluoromethyl, or wherein the above- mentioned carboxylic acid amide may optionally be additionally substituted at the nitrogen by a C₁₋₅-alkyl group, a phenyl, phenyl-C₁₋₂-alkyl, heteroaryl-C₁₋₂-alkyl or C-linked heteroaryl group, wherein the heteroaryl group is selected from among imidazolyl, furanyl, thiophenyl, thiazolyl, pyrazolyl, tetrazolyl, benzimidazolyl, indolyl, pyrimidinyl, pyrazinyl, oxazolyl, and pyridinyl, and which may optionally be mono- to disubstituted in the phenyl or heteroaryl moiety by identical or different substituents selected from among chlorine or fluorine atoms or C₁₋₃-alkyl, CF₃, HO, CH₃O or CHF₂O groups,
R⁵ denotes a hydrogen atom, a straight-chain or branched C₁₋₄-alkyl group, a propargyl or C₁₋₃-alkyloxy-C₁₋₃-alkyl group, or
R⁴ and R⁵ together with the carbon atom to which they are bound form a C₅₋₆-cycloalkenyl or C₃₋₇-cycloalkyl group, wherein one of the methylene groups of a C₄-₇-cycloalkyl group may be replaced by an oxygen or sulphur atom or a sulphonyl group, or 1 to 2 carbon atoms of a C₃-₇-cycloalkyl group may optionally be substituted independently of one another by in each case one or two fluorine atoms, or one or two C₁₋₅-alkyl groups, or a hydroxy, C₁₋₅-alkyloxy, formyloxy, nitrile, carboxy, C₁₋₅-alkyloxycarbonyl, aminocarbonyl, C₁₋₅-alkylaminocarbonyl, di-(C₁₋₅-alkyl)-aminocarbonyl or C₄₋₇-cycloalkyleneiminocarbonyl group, with the proviso that a C₃₋₇-cycloalkyl group of this kind formed from R⁴ and R⁵ together, wherein one of the methylene groups of the cyclic group which is linked directly to the carbon atom to which the groups R⁴ and R⁵ are bound, is replaced by an oxygen or sulphur atom, is excluded, and
M denotes a thiophene ring according to formula (III), 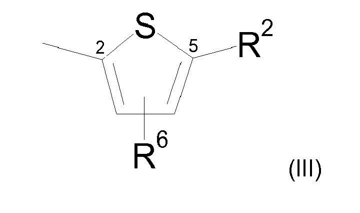 which is bound to the carbonyl group in formula (I) via the 2-position and is substituted by R² in the 5-position, where
R² denotes a chlorine or bromine atom or an ethynyl group, and
R⁶ denotes a hydrogen atom,
wherein the alkyl, alkenyl, alkynyl and alkyloxy groups contained in the previously mentioned definitions which have more than two carbon atoms may, unless otherwise stated, be straight-chain or branched and the alkyl groups in the previously mentioned dialkylated groups, for example the dialkylamino groups, may be identical or different, the tautomers, enantiomers, diastereomers, mixtures and salts thereof.

6. Compounds of general formula (I) according to one of claims 1 to 5, wherein the group
D denotes a substituted bicyclic ring system of formula (II), 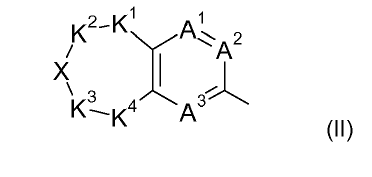 wherein
K¹ and K⁴ each independently of one another represent a -CH₂-, -CHR^{7a}- or a -CR^{7b}R^{7c}- group, where
R^{7a}/R^{7b}/R^{7c} each independently of one another denote a C₁₋₂-alkyl group,
K² and K³ each denote a -CH₂- group,
X denotes an NR¹ group, wherein
R¹ denotes a hydrogen atom or a C₁₋₅-alkyl or C₃₋₆-cycloalkyl group, wherein in the methylene and methyl groups present in the above-mentioned groups one to three hydrogen atoms may be replaced by fluorine atoms, provided that the methylene or methyl groups are not directly bound to the nitrogen atom, and wherein
A¹ denotes CR¹⁰,
A² denotes CR¹¹,
A³ denotes CR¹², where R¹⁰, R¹¹ and R¹² each independently of one another represent a hydrogen, fluorine or chlorine atom, or a C₁₋₃-alkyl, CF₃, HO, CH₃O group, or
D denotes the group (II-4) 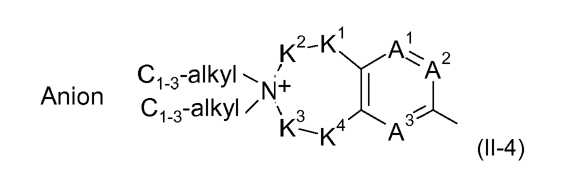 wherein the groups A1, A2, A3, K1, K2, K3, K4 are as hereinbefore defined, and the anion in (II-4) may be selected from among fluoride, chloride, bromide, iodide, sulphate, phosphate, benzoate, salicylate, succinate, citrate or tartrate,
the tautomers, enantiomers, diastereomers, mixtures and salts thereof.

7. Compounds of general formula (I) according to one of claims 1 to 6, wherein neither
R⁴ nor R⁵ may represent a hydrogen atom,
the tautomers, enantiomers, diastereomers, mixtures and salts thereof.

8. Compounds of general formula (I) according to one of claims 1 to 6, wherein
R⁴ and R⁵ together with the carbon atom to which they are bound, form a C₅₋₆-cycloalkenyl or C₃₋₇-cycloalkyl group, wherein one of the methylene groups of a C₄-₇-cycloalkyl group may be replaced by an oxygen or sulphur atom, with the proviso that a C₃-₇-cycloalkyl group of this kind formed from R⁴ and R⁵ together,
wherein one of the methylene groups of the cyclic group, which is linked directly to the carbon atom, to which the groups R⁴ and R⁵ are bound, is replaced by an oxygen or sulphur atom,
is excluded, the tautomers, enantiomers, diastereomers, mixtures and salts thereof.

9. The following compounds of general formula (I) according to claim 1:
3-[(5-bromo-thiophen-2-yl)-carbonylamino]-*N*-(3-methyl-2,3,4,5-tetrahydro-1*H-*benzo[*d*]azepin-7-yl)-tetrahydrofuran-3-carboxylic acid amide,
3-[(5-chloro-thiophen-2-yl)-carbonylamino]-*N-*(3-methyl-2,3,4,5-tetrahydro-1*H-*benzo[*d*]azepin-7-yl)-tetrahydrofuran-3-carboxylic acid amide,
5-chloro-thiophene-2-carboxylic acid-*N-*[1-(3-ethyl-2,3,4,5-tetrahydro-1*H-*benzo[*d*]azepin-7-ylcarbamoyl)-1-methyl-ethyl]-amide,
5-ethynyl-*N*-[1-methyl-1-(3-methyl-2,3,4,5-tetrahydro-1*H*-benzo[*d*]azepin-7-ylcarbamoyl)-ethyl]-thiophene-2-carboxylic acid amide,
5-chloro-thiophene-2-carboxylic acid-*N-*[1-methyl-1-(3-methyl-2,3,4,5-tetrahydro-1*H-*benzo[d]azepin-7-ylcarbamoyl]-ethyl}-amide,
5-bromo-thiophene-2-carboxylic acid-*N-*[1-methyl-1-(3-methyl-2,3,4,5-tetrahydro-1*H-*benzo[d]azepin-7-ylcarbamoyl]-ethyl}-amide,
5-chloro-thiophene-2-carboxylic acid-*N-*[2-methoxy-1-(3-methyl-2,3,4,5-tetrahydro-1*H-*benzo[*d*]azepin-7-ylcarbamoyl)-ethyl]-amide,
1-[(5-bromo-thiophen-2-yl)-carbonylamino]-*N-*(3-methyl-2,3,4,5-tetrahydro-1*H-*benzo[*d*]azepin-7-yl)-cyclopentane-1-carboxylic acid amide,
1-[(5-chloro-thiophen-2-yl)-carbonylamino]-*N-*(3-methyl-2,3,4,5-tetrahydro-1*H-*benzo[*d*]azepin-7-yl)-cyclopentane-1-carboxylic acid amide,
3-[(5-chloro-thiophen-2-yl)-carbonylamino]-*N-*(3-methyl-2,3,4,5-tetrahydro-1*H-*benzo[*d*]azepin-7-yl)-tetrahydrothiophene-3-carboxylic acid amide,
1-[(5-chloro-thiophen-2-yl)carbonylamino]-*N-*(3-methyl-2,3,4,5-tetrahydro-1H-benzo[d]azepin-7-yl)-cyclobutane-1-carboxylic acid amide,
1-[(5-bromo-thiophen-2-yl)carbonylamino]-*N-*(3-methyl-2,3,4,5-tetrahydro-1H-benzo[d]azepin-7-yl)-cyclopent-3-ene-1-carboxylic acid amide,
1-[(5-chloro-thiophen-2-yl)carbonylamino]-*N-*(3-methyl-2,3,4,5-tetrahydro-1H-benzo[d]azepin-7-yl)-cyclohexane-1-carboxylic acid amide,
(R)-5-chloro-thiophene-2-carboxylic acid-*N-*[2-benzyloxy-1-(3-methyl-2,3,4,5-tetrahydro-1*H-*benzo[*d*]azepin-7-ylcarbamoyl)-ethyl]-amide,
5-chloro-thiophene-2-carboxylic acid-*N-*[2-benzyloxy-1-methyl-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[2-hydroxy-1-methyl-1-(3-methyl-2,3,4,5-tetrahydro-1*H-*benzo[*d*]azepin-7-ylcarbamoyl)-ethyl]-amide,
5-bromo-thiophene-2-carboxylic acid-N-[3-hydroxy-1-methyl-1-(3-methyl-2,3,4,5-tetrahydro-1H-benzo[d]azepin-7-ylcarbamoyl)-propyl]-amide,
5-chloro-thiophene-2-carboxylic acid-N-[1-methyl-3-dimethylaminocarbonyl-1-(3-methyl-2,3,4,5-tetrahydro-1*H-*benzo[*d*]azepin-7-ylcarbamoyl)-propyl]-amide,
5-chloro-thiophene-2-carboxylic acid-*N-* [2-(4-hydroxy-phenyl)-1-methyl-1-(3-methyl-2,3,4,5-tetrahydro-1*H-*benzo[*d*]azepin-7-ylcarbamoyl)-ethyl]-amide,
5-chloro-thiophene-2-carboxylic acid-*N-*[1-methyl-1-(3,5-dimethyl-2,3,4,5-tetrahydro-1*H-*benzo[*d*]azepin-7-ylcarbamoyl)-ethyl]-amide,
5-chloro-thiophene-2-carboxylic acid-*N-*{1-methyl-1-[3-methyl-5-(4-aminophenyl)-2,3,4,5-tetrahydro-1*H-*benzo[*d*]azepin-7-ylcarbamoyl]-ethyl}-amide,
5-chloro-thiophene-2-carboxylic acid-*N-*[2-ethoxy-1-(3-methyl-2,3,4,5-tetrahydro-1*H-*benzo[*d*]azepin-7-ylcarbamoyl)-ethyl]-amide,
5-chloro-thiophene-2-carboxylic acid-*N-*[3-methoxy-1-(3-methyl-2,3,4,5-tetrahydro-1*H-*benzo[*d*]azepin-7-ylcarbamoyl)-propyl]-amide,
5-chloro-thiophene-2-carboxylic acid-*N-*[2-isopropyloxy-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[3-benzyloxy-1-(3-methyl-2,3,4,5-tetrahydro-1*H-*benzo[*d*]azepin-7-ylcarbamoyl)-propyl]-amide,
1-[(5-chloro-thiophen-2-yl)-carbonylamino]-3,4-dimethoxy-*N-*(3-methyl-2,3,4,5-tetrahydro-1*H-*benzo[*d*]azepin-7-yl)-cyclopentane-1-carboxylic acid amide,
5-chloro-thiophene-2-carboxylic acid-*N-*[C-(1-methyl-pyrazol-3-yl)-C-(3-methyl-2,3,4,5-tetrahydro-1*H-*benzo[*d*]azepin-7-ylcarbamoyl)-methyl]-amide,
(R)-5-chloro-thiophene-2-carboxylic acid-*N-*[2-phenyl-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-* [2-(furan-2-yl)-1-(3-methyl-2,3,4,5-tetrahydro-1*H-*benzo[*d*]azepin-7-ylcarbamoyl)-ethyl]-amide,
5-chloro-thiophene-2-carboxylic acid-*N-* [2-(4-methoxyphenyl)-1-(3-methyl-2,3,4,5-tetrahydro-1*H-*benzo[*d*]azepin-7-ylcarbamoyl)-ethyl]-amide,
5-chloro-thiophene-2-carboxylic acid-*N-* [2-(4-hydroxy-3-nitro-phenyl)-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-* [2-(4-hydroxy-phenyl)-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[2-cyclohexyl-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[3-aminocarbonyl-1-(3-methyl-2,3,4,5-tetrahydro-1*H-*benzo[*d*]azepin-7-ylcarbamoyl)-propyl]-amide,
(R)-5-chloro-thiophene-2-carboxylic acid-*N-*[2-acetylamino-1-(3-methyl-2,3,4,5-tetrahydro-1*H-*benzo[*d*]azepin-7-ylcarbamoyl)-ethyl]-amide,
(R)-5-bromo-thiophene-2-carboxylic acid-*N-*[2-benzoylamino-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[2-(2-hydroxycarbonyl-ethyl)carbonylamino-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[2-(2-hydroxycarbonyl-ethyl)carbonylamino-1-(3-methyl-2,3,4,5-tetrahydro-1*H-*benzo[*d*]azepin-7-ylcarbamoyl)-ethyl]-amide,
(R)-5-chloro-thiophene-2-carboxylic acid-*N-*[2-(4-methoxycarbonyl-butyl)carbonylamino-1-(3-methyl-2,3,4,5-tetrahydro-1*H-*benzo[*d*]azepin-7-ylcarbamoyl)-ethyl]-amide,
5-chloro-thiophene-2-carboxylic acid-*N-*[1-methyl-1-(3,3-dimethyl-2,3,4,5-tetrahydro-1*H-*benzo[d]azepinium-7-ylcarbamoyl]-ethyl}-amide iodide,
3-[(5-chloro-thiophen-2-yl)-carbonylamino]-*N-*(3,5-dimethyl-2,3,4,5-tetrahydro-1*H-*benzo[*d*]azepin-7-yl)-tetrahydrofuran-3-carboxylic acid amide
and their tautomers, enantiomers, diastereomers, the mixtures thereof and the salts thereof.

10. Physiologically acceptable salts of the compounds according to one of claims 1 to 9.

11. Pharmaceutical compositions containing a compound according to at least one of claims 1 to 9 or a physiologically acceptable salt according to claim 10 optionally together with one or more inert carriers and/or diluents.

12. Use of a compound according to at least one of claims 1 to 9 or a physiologically acceptable salt according to claim 10 for preparing a pharmaceutical composition with an inhibitory effect on factor Xa and/or an inhibitory effect on related serine proteases.

13. Process for preparing a pharmaceutical composition according to claim 11, **characterised in that** by a non-chemical method a compound according to at least one of claims 1 to 9 or a physiologically acceptable salt according to claim 10 is incorporated in one or more inert carriers and/or dilue

## Revendications

1. Composés de formule générale (I) 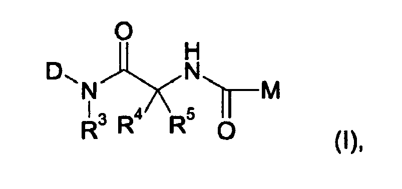
D dans lesquels
représente un système cyclique bicyclique substitué de formule (II) 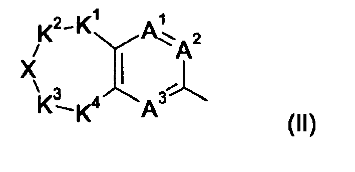 dans lequel
K¹ et K⁴ représentent chacun indépendamment l'un de l'autre un groupe -CH₂-, -CHR^{7a}-, -CR^{7b}R^{7c}- ou un groupe -C(O)-, et où R^{7a}/R^{7b}/R^{7c}
représentent chacun indépendamment les uns des autres un atome de fluor, un groupe hydroxy, C₁₋₅-alkyloxy, tétrahydrofuranyle, oxétanyle, amino, C₁₋₅-alkylamino, di-(C₁₋₅-alkyl)-amino, C₃₋₅-cycloalkylèneimino ou C₁₋₅-alkylcarbonylamino,
un groupe C₁₋₅-alkyle, qui peut être substitué par 1 - 3 atomes de fluor, un groupe hydroxy-C₁₋₅-alkyle, C₁₋₅-alkyloxy-C₁₋₅-alkyle, amino-C₁₋₅-alkyle, C₁-₅-alkylamino-C₁₋₅-alkyle, di-(C₁₋₅-alkyl)-amino-C₁₋₅-alkyle, C₄₋₇-cyclo-alkylèneimino-C₁₋₅-alkyle, carboxy-C₀₋₅-alkyle, C₁₋₅-alkyloxycarbonyl-C₀₋₅-alkyle, aminocarbonyl-C₀₋₅-alkyle, C₁₋₅-alkylaminocarbonyl-C₀₋₅-alkyle, di-(C₁₋₅-alkyl)-aminocarbonyl-C₀₋₅-alkyle, C₄₋₇-cycloalkylèneiminocarbonyl-C₀₋₅-alkyle,
un groupe phényle ou un groupe hétéroaryle à 5 ou 6 chaînons, qui peut être substitué par 1 - 2 substituants choisis parmi un groupe nitro, amino, hydroxy, méthoxy, cyano, C₁₋₅-alkyle ou un atome de fluor, de chlore ou de brome,
où les deux groupements R^{7b}/R^{7c} ne peuvent pas être liés en même temps par un hétéroatome à l'atome de carbone du cycle, sauf si -C(R^{7b}R^{7c})- correspond à un groupe -CF₂-,
ou
deux groupements R^{7b}/R^{7c} peuvent former avec l'atome de carbone du cycle un carbocycle saturé à 3, 4, 5, 6 ou 7 chaînons ou un cycle cyclopentène, cyclohexène, oxétane, azétidine, thiétane, tétrahydrofurane, pyrrolidine, tétrahydrothiophène, tétrahydropyrane, pipéridine, pentaméthylènesulfure, hexaméthylèneimine, 1,3-dioxolane, 1,4-dioxane, hexahydropyridazine, pipérazine, thiomorpholine, morpholine, 2-imidazolidinone, 2-oxazolidinone, tétrahydro-2(1H)-pyrimidinone, [1,3]oxazinan-2-one,
où ses groupes méthylène peuvent être substitués par 1 - 2 groupes C₁-₃-alkyle ou CF₃, et/ou
ses groupes méthylène, dans la mesure où ils ne sont pas liés à un hétéroatome, peuvent être substitués par 1 - 2 atomes de fluor, et/ou dans lequel un groupe -CH₂- à côté d'un atome N peut être remplacé par un groupe -CO-, et/ou
ses groupes imino peuvent être substitués dans chaque cas par un groupe C₁₋₃-alkyle ou C₁₋₃-alkylcarbonyle, et/ou dans lequel l'atome de soufre peut être oxydé en un groupe sulfoxyde ou sulfone,
K² et K³ représentent chacun indépendamment l'un de l'autre un groupe -CH₂-, -CHR^{8a}-, -CR^{8b}R^{8c}- ou un groupe -C(O)-, et où R^{8a}/R^{8b}/R^{8c}
représentent chacun indépendamment les uns des autres un groupe C₁₋₅-alkyle, qui peut être substitué par 1 - 3 atomes de fluor, un groupe hydroxy-C₁₋₅-alkyle, C₁₋₅-alkyloxy-C₁₋₅-alkyle, amino-C₁₋₅-alkyle, C₁₋₅-alkylamino-C₁₋₅-alkyle, di-(C₁₋₅-alkyl)-amino-C₁₋₅-alkyle, C₄₋₇-cycloalkylèneimino-C₁₋₅-alkyle, carboxy-C₀₋₅-alkyle, C₁₋₅-alkyloxycarbonyl-C₀-₅-alkyle, aminocarbonyl-C₀-₅-alkyle, C₁₋₅-alkylaminocarbonyl-C₀₋₅-alkyle, di-(C₁₋₅-alkyl)-aminocarbonyl-C₀₋₅-alkyle, C₄₋₇-cycloalkylèneiminocarbonyl-C₀₋₅-alkyle,
ou deux groupements R^{8b}/R^{8c} peuvent former avec l'atome de carbone du cycle un carbocycle saturé à 3, 4, 5, 6 ou 7 chaînons ou un cycle cyclopentène, cyclohexène, oxétane, azétidine, thiétane, tétrahydrofurane, pyrrolidine, tétrahydrothiophène, tétrahydropyrane, pipéridine, pentaméthylènesulfure, hexaméthylèneimine, hexahydropyridazine, tétrahydro-2(1H)-pyrimidinone, [1,3]oxazinan-2-one,
où ses groupes méthylène peuvent être substitués par 1 - 2 groupe C₁₋₃-alkyle ou CF₃, et/ou
ses groupes méthylène, dans la mesure où ils ne sont pas liés à un hétéroatome, peuvent être substitués par 1 - 2 atomes de fluor, et/ou dans lequel un groupe -CH₂- à côté d'un atome d'azote peut être remplacé par un groupe -CO-, et/ou
ses groupes imino peuvent être substitués dans chaque cas par un groupe C₁₋₃-alkyle ou C₁₋₃-alkylcarbonyle, et/ou dans lequel l'atome de soufre peut être oxydé en un groupe sulfoxyde ou sulfone,
avec la condition qu'un hétéroatome introduit par R^{8b} ou R^{8c} ne doit pas être séparé de X dans la formule (I) par seulement un atome de carbone, et
au total dans la formule (II) au maximum quatre groupements choisis parmi R^{7a}, R^{7b}, R^{7c}, R^{8a}, R^{8b} et R^{8c} peuvent être présents, et X représente un atome d'oxygène ou de soufre, un groupe sulfène, sulfone ou un groupe NR¹, dans lequel
R¹ représente un atome d'hydrogène ou un groupe hydroxy, C₁₋₃-alkyloxy, amino, C₁₋₃-alkylamino, di-(C₁₋₃-alkyl)-amino, un groupe C₁₋₅-alkyle, C₂₋₅-alcényl-CH₂, C₂₋₅-alcynyl-CH₂, C₃₋₆-cycloalkyle, C₄-₆-cycloalcényle, oxétan-3-yle, tétrahydrofuran-3-yle, benzyle, C₁₋₅-alkyl-carbonyle, trifluorométhylcarbonyle, C₃₋₆-cycloalkyl-carbonyle, C₁₋₅-alkyl-sulfonyle, C₃₋₆-cycloalkyl-sulfonyle, aminocarbonyle, C₁₋₅-alkylaminocarbonyle, di-(C₁₋₅-alkyl)-aminocarbonyle, C₁₋₅-alkyloxycarbonyle, C₄₋₇-cycloalkylène-iminocarbonyle,
où les groupes méthylène et méthyle qui se trouvent dans les groupes cités précédemment peuvent être substitués en outre par un groupe C₁₋₃-alkyle, carboxy, C₁₋₅-alcoxycarbonyle ou par un groupe hydroxy, C₁₋₅-alkyloxy, amino, C₁₋₅-alkylamino, C₁₋₅-dialkylamino ou C₄₋₇-cycloalkylèneimino, dans la mesure où les groupes méthylène ou méthyle ne sont pas liés directement à un hétéroarome du groupe O, N et S, et/ou un à trois atomes d'hydrogène peuvent être remplacés par des atomes de fluor, dans la mesure où les groupes méthylène ou méthyle ne sont pas liés directement à un hétéroatome du groupe O, N et S,
et dans lequel
A¹ représente N ou CR¹⁰,
A² représente N ou CR¹¹,
A³ représente N ou CR¹²,
où R¹⁰, R¹¹ et R¹² représentent dans chaque cas indépendamment les uns des autres
un atome d'hydrogène, de fluor, de chlore, de brome ou d'iode, ou un groupe C₁₋₅-alkyle, CF₃, C₂₋₅-alcényle, C₂₋₅-alcynyle, un groupe cyano, carboxy, C₁₋₅-alkyloxycarbonyle, hydroxy, C₁₋₃-alkyloxy, CF₃O-, CHF₂O-, CH₂FO-, amino, C₁-₅-alkylamino, di-(C₁₋₅-alkyl)-amino ou C₄-₇-cyclo-alkylèneimino, ou
D représente l'un des quatre groupes (II-1), (II-2), (II-3) ou (II-4) 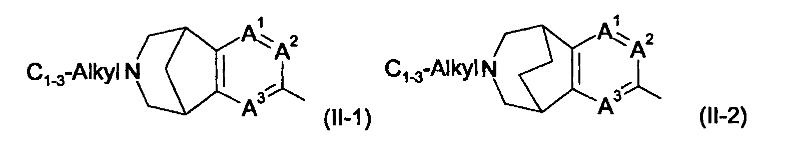 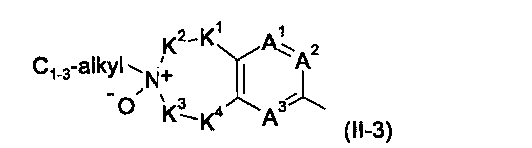 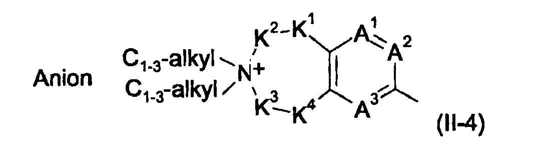 dans lequel les groupements A1, A2, A3, K1, K2, K3, K4 sont définis comme ci-dessus, et
l'anion dans (II-4) représente un fluorure, chlorure, bromure, iodure, sulfate, hydrogénosulfate, phosphate, hydrogénophosphate, benzoate, salicylate, succinate, citrate ou tartrate,
R³ représente un atome d'hydrogène ou un groupe C₁₋₃-alkyle, et
R⁴ et R⁵ représentent dans chaque cas indépendamment l'un de l'autre
un atome d'hydrogène, un groupe C₁₋₆-alkyle, C₂₋₆-alcényle ou C₂₋₆-alcynyle linéaire ou ramifié,
où les atomes d'hydrogène des fragments méthylène et/ou méthyle du groupe C₁₋₆-alkyle, C₂₋₆-alcényle ou C₂₋₆-alcynyle linéaire ou ramifié peuvent éventuellement être remplacés totalement ou partiellement par des atomes de fluor, et/ou
où un ou deux atomes d'hydrogène du groupe C₁₋₆-alkyle, C₂₋₆-alcényle ou C₂₋₆-alcynyle linéaire ou ramifié peuvent éventuellement être remplacés indépendamment l'un de l'autre dans leurs fragments méthylène et/ou méthyle par un groupe C₃₋₇-cycloalkyle, un groupe nitrile, hydroxy ou C₁₋₅-alkyloxy,
où les atomes d'hydrogène du groupe C₁₋₅-alkyloxy peuvent éventuellement être remplacés totalement ou partiellement par des atomes de fluor,
un groupe allyloxy, propargyloxy, phénylméthyloxy, phénéthyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, C₁₋₅-alkyloxy-C₂₋₅-alkyloxy, mercapto, C₁₋₅-alkylsulfanyle, C₁₋₅-alkylsulfinyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₁₋₅-alkyl-aminocarbonyloxy, di-(C₁₋₅-alkyl)-aminocarbonyloxy, C₄₋₇-cycloalkylèneiminocarbonyle, aminosulfonyle, C₁₋₅-alkylaminosulfonyle, di-(C₁₋₅-alkyl)-aminosulfonyle, C₄₋₇-cycloalkylène-iminosulfonyle, di-(C₁₋₅-alkyl)-phosphoryle, amino, C₁₋₅-alkylamino, di-(C₁₋₅-alkyl)-amino, C₁₋₅-alkylcarbonylamino, trifluoroacétylamino, C₁₋₅-alkyloxy-C₁₋₅-alkylcarbonylamino, phénylcarbonylamino, C₁₋₅-alkylamino-carbonylamino, di-(C₁₋₅-alkyl)-aminocarbonylamino, C₁₋₅-alkyloxy-carbonylamino, phénylméthyloxy-carbonylamino, C₁₋₅-alkyloxy-C₂₋₅-alkyloxy-C₁₋₂-alkylcarbonylamino, C₁₋₅-alkylsulfonylamino, *N-*(C₁₋₅-alkylsulfonyl)-C₁₋₅-alkylamino, C₃₋₆-cycloalkylcarbonylamino, 4-morpholinocarbonylamino ou un groupe morpholinyle, thiomorpholinyle, pyrrolidinyle, pipéridinyle, pipérazinyle, tétrahydrofuranyle, tétrahydropyranyle,
où les carbo- et hétérocycles précités peuvent être substitués dans le cycle dans chaque cas par 1 à 4 groupes C₁₋₃-alkyle ou C₁₋₃-alkylcarbonyle ou dans chaque cas par 1 ou 2 groupes oxo, et/ou où les groupements phényle et hétéroaryle précités peuvent être remplacés par 1 ou 2 substituants choisis parmi le fluor, le chlore, le brome, méthyle, méthoxy, amino ou trifluorométhyle ou bien deux atomes de carbone voisins d'un cycle phényle peuvent être substitués par un groupe -CH₂-O-CH₂-, et/ou
où les groupes alkyle précités peuvent être substitués par un groupe cyano-C₁₋₅-alkyloxycarbonyle ou carboxy,
où les amides d'acides carboxyliques ou d'acides sulfoniques précités peuvent éventuellement être substitués en outre sur l'azote par un groupe C₁₋₅-alkyle,
et/ou où les atomes d'hydrogène des atomes de carbone hybridés sp² du groupe C₂₋₆-alcényle linéaire ou ramifié peuvent éventuellement être remplacés totalement ou partiellement par des atomes de fluor,
un groupe carboxy, aminocarbonyle, C₁₋₅-alkylamino carbonyle, C₃₋₆-cycloalkylaminocarbonyle, di-(C₁₋₅-alkyl)-aminocarbonyle, C₁₋₅-alkyloxy-carbonyle, C₄-₇-cycloalkylèneiminocarbonyle,
un groupe phényle, hétéroaryle mono- ou bicyclique, phényl-C₁₋₅-alkyle ou hétéroaryl-C₁₋₅-alkyle mono- ou bicyclique,
qui peut éventuellement être substitué une à trois fois dans la partie phényle ou hétéroaryle par des substituants identiques ou différents choisis dans le groupe consistant en les atomes de fluor, de chlore, de brome et d'iode, et les groupes C₁₋₅-alkyle, trifluorométhyle, benzyle, amino, nitro, C₁₋₅-alkylamino, di-(C₁₋₅-alkyl)-amino, hydroxy, C₁₋₅-alkyloxy, mono-, di- ou trifluorométhoxy, carboxy et C₁₋₅-alkyloxycarbonyle,
ou deux atomes de carbone voisins d'un cycle phényle peuvent être substitués par un groupe -CH₂-O-CH₂-, ou
un groupe C₃₋₇-cycloalkyle, morpholinyle, thiomorpholinyle, pyrrolidinyle, pipéridinyle, pipérazinyle, tétrahydrofuranyle, tétrahydropyranyle,
qui peut éventuellement être substitué par un à deux groupements choisis indépendamment l'un de l'autre parmi C₁₋₃-alkyle, acétyle, C₁₋₅-alkyloxycarbonyle et hydroxycarbonyle, ou
R⁴ et R⁵ forment avec l'atome de carbone auquel ils sont liés un groupe C₃₋₇-cycloalkyle ou C₅₋₇-cycloalcényle,
où l'un des groupes méthylène d'un groupe C₄-₇-cycloalkyle peut être remplacé par un atome d'oxygène ou de soufre ou un groupe -NH-, -N(C₁₋₅-alkyle), -N(C₁₋₄-alkylcarbonyle), -N(C₁₋₄-alkyloxycarbonyle) ou un groupe carbonyle, sulfinyle ou sulfonyle, et/ou
où deux groupes méthylène directement voisins d'un groupe C₄₋₇-cycloalkyle peuvent être remplacés ensemble par un groupe -C(O)NH-, -C(O)N(C₁₋₅-alkyle)-, -S(O)₂NH- ou -S(O)₂N(C₁₋₅-alkyle), et/ou où 1 à 3 atomes de carbone d'un groupe C₃₋₇-cycloalkyle peuvent éventuellement être substitués indépendamment les uns des autres par dans chaque cas un ou deux atomes de fluor ou un ou deux groupes C₁₋₅-alkyle ou un groupe hydroxy, C₁₋₅-alkyloxy, formyloxy, C₁₋₅-alkylcarbonyloxy, C₁₋₅-alkylsulfanyle, C₁₋₅-alkylsulfonyle, aminosulfonyle, C₁₋₅-alkyl-aminosulfonyle, di-(C₁-₅-alkyl)-aminosulfonyle, C₄₋₇-cycloalkylène-iminosulfonyle, amino, C₁₋₅-alkylamino, di-(C₁₋₅-alkyl)-amino, C₁₋₅-alkylcarbonylamino, C₁₋₅-alkylsulfonylamino, *N-*(C₁₋₅-alkylsulfonyl)-C₁₋₅-alkylamino, C₃₋₆-cycloalkylcarbonylamino, nitrile, carboxy- C₁₋₅-alkyle, C₁₋₅-alkyloxycarbonyl-C₁₋₅₋alkyle, carboxy, C₁₋₅-alkyloxycarbonyle, aminocarbonyle, C₁₋₅-alkylaminocarbonyle, di-(C₁₋₅-alkyl)-aminocarbonyle ou C₄₋₇-cycloalkylèneiminocarbonyle, et/ou
où 1 à 2 atomes de carbone d'un groupe C₅₋₇-cycloalcényle peuvent éventuellement être substitués indépendamment l'un de l'autre par dans chaque cas un groupe C₁₋₅-alkyle, nitrile, carboxy-C₁₋₅-alkyle, C₁₋₅-alkyloxycarbonyl-C₁₋₅-alkyle, carboxy, C₁₋₅-alkyloxycarbonyle, aminocarbonyle, C₁₋₅-alkylaminocarbonyle, di-(C₁₋₅-alkyl)-aminocarbonyle, C₃₋₆-cyclcoalkylèneiminocarbonyle, aminosulfonyle, C₁₋₅-alkylamino-sulfonyle, di-(C₁₋₅-alkyl)-aminosulfonyle, C₃₋₆-cycloalkylèneiminosulfonyle ou 1 - 2 atomes de fluor, et/ou
où 1 ou 2 atomes de carbone d'un groupe C₄-₇-cycloalcényle, qui ne sont pas liés par une double liaison à un autre atome de carbone, peuvent éventuellement être substitués indépendamment l'un de l'autre par un groupe hydroxy, C₁₋₅-alkyloxy, C₁₋₅-alkylcarbonyloxy, C₁₋₅-alkylsulfanyle, C₁₋₅-alkylsulfonyle, amino, C₁₋₅-alkylamino, di-(C₁₋₅-alkyl)-amino, C₁₋₅-alkylcarbonylamino, C₁₋₅-alkylsulfonylamino , *N-*(C₁₋₅-alkylsulfonyl)-C₁₋₅-alkylamino ou C₃₋₆-cycloalkylcarbonylamino,
avec la condition qu'un tel groupe C₃₋₇-cycloalkyle ou C₅₋₇-cycloalcényle formé à partir de R⁴ et R⁵,
dans lequel deux hétéroatomes du groupe oxygène et azote dans le cycle sont séparés l'un de l'autre par exactement un groupe -CH₂-éventuellement substitué, et/ou
dans lequel un groupe méthylène ou les deux groupes méthylène du cycle, qui sont liés directement à l'atome de carbone auquel les groupements R⁴ et R⁵ sont liés, sont remplacés par un hétéroatome du groupe oxygène, azote et soufre, et/ou dans lequel un substituant lié au groupe cyclique, qui se **caractérise en ce qu'**un hétéroatome du groupe oxygène, azote, soufre et fluor est lié directement au groupe cyclique, est séparé par un autre hétéroatome du groupe oxygène, azote et soufre, à l'exception du groupe sulfone, par exactement un groupe méthylène éventuellement substitué, et/ou dans lequel deux atomes dans le cycle forment une liaison -O-O- ou -S-O-,
est exclu,
M représente un cycle thiophène selon la formule (III) 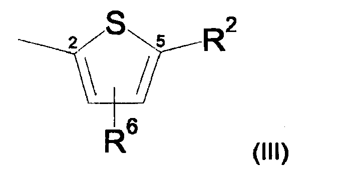 qui est lié par la position 2 au groupe carbonyle dans la formule (I) et qui est substitué à la position 5 par R² et éventuellement en outre par R⁶, où
R² représente un atome d'hydrogène, de fluor, de chlore, de brome ou d'iode ou un groupe méthoxy, C₁₋₂-alkyle, formyle, NH₂CO- ou éthynyle,
R⁶ représente un atome d'hydrogène, de fluor, de chlore, de brome ou d'iode ou un groupe C₁₋₂-alkyle ou amino,
où, sauf indication contraire, par l'expression « groupe hétéroaryle » citée précédemment dans les définitions, on doit comprendre un groupe hétéroaryle à 5 ou 6 chaînons monocyclique, où
le groupe hétéroaryle à 6 chaînons contient un, deux ou trois atomes d'azote, et le groupe hétéroaryle à 5 chaînons contient un groupe imino éventuellement substitué par un groupe C₁₋₃-alkyle, un atome d'oxygène ou de soufre, ou
un groupe imino éventuellement substitué par un groupe C₁₋₃-alkyle ou un atome d'oxygène ou de soufre et en outre un ou deux atomes d'azote, ou
un groupe imino éventuellement substitué par un groupe C₁₋₃-alkyle et trois atomes d'azote,
et en outre un cycle phényle éventuellement substitué par un atome de fluor, de chlore ou de brome, un groupe C₁₋₃-alkyle, hydroxy, C₁₋₃-alkyloxy, amino, C₁₋₃-alkylamino, di-(C₁₋₃-alkyl)-amino ou C₃₋₆-cycloalkylèneimino peut être condensé aux groupes hétéroaryle monocycliques cités précédemment par le biais de deux atomes de carbone voisins,
et la liaison se fait par le biais d'un atome d'azote ou par le biais d'un atome de carbone de la partie hétérocyclique ou d'un cycle phényle condensé,
et où, sauf indication contraire, par l'expression « atome d'halogène » citée précédemment dans les définitions on doit comprendre un atome du groupe fluor, chlore, brome et iode,
et où les groupes alkyle, alcényle, alcynyle et alkyloxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
et où les atomes d'hydrogène des groupes méthyle ou éthyle contenus dans les définitions citées précédemment, sauf indication contraire, peuvent être remplacés totalement ou partiellement par des atomes de fluor,
leurs tautomères, leurs énantiomères, leurs diastéréoisomères, leurs mélanges et leurs sels.

2. Composés de formule générale (I) selon la revendication 1, dans lesquels
D représente un système cyclique bicyclique substitué de formule (II) 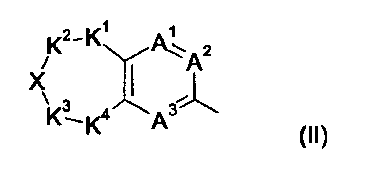 dans lequel
K¹ et K⁴ représentent chacun indépendamment l'un de l'autre un groupe -CH₂-, -CHR^{7a}-, -CR^{7b}- ou un groupe -C(O)-, où R^{7a}/R^{7b}/R^{7c}
représentent chacun indépendamment les uns des autres un atome de fluor, un groupe hydroxy, C₁₋₅-alkyloxy, un groupe C₁₋₅-alkyle, qui peut être substitué par 1 - 3 atomes de fluor, un groupe hydroxy-C₁₋₅-alkyle, C₁₋₅-alkyloxy-C₁₋₅-alkyle, ou un groupe phényle, qui peut être substitué par 1 - 2 substituants choisis parmi un groupe nitro, amino, hydroxy, méthoxy, cyano, C₁₋₅-alkyle ou un atome de fluor, de chlore ou de brome, ou un groupe hétéroaryle à 5 ou 6 chaînons,
où les deux groupements R⁷/R^{7c} ne peuvent pas être liés en même temps par un hétéroatome à l'atome de carbone du cycle, sauf si - C(R^{7b}R^{7c})- correspond à un groupe -CF₂-,
ou
deux groupements R^{7b}/R^{7c} peuvent former avec l'atome de carbone du cycle un carbocycle saturé à 3, 4, 5, 6 ou 7 chaînons ou un cycle cyclopentène, cyclohexène, oxétane, tétrahydrofurane ou tétrahydropyrane,
où ses groupes méthylène peuvent être substitués par 1 - 2 groupes C₁₋₃-alkyle ou CF₃, et/ou
ses groupes méthylène, dans la mesure où ils ne sont pas liés à un hétéroatome, peuvent être substitués par 1 - 2 atomes de fluor, et
K² et K³ représentent chacun indépendamment l'un de l'autre un groupe -CH₂-, ₋CHR^{8a}-, -CR^{8b}R^{8c}- ou un groupe -C(O)-, et où
R^{8a}/R^{8b}/R^{8c}
représentent chacun indépendamment les uns des autres un groupe C₁₋₅-alkyle, qui peut être substitué par 1 - 3 atomes de fluor, un groupe hydroxy-C₁₋₅-alkyle, C₁₋₅-alkyloxy-C₁₋₅-alkyle,
ou deux groupements R^{8b}/R^{8c} peuvent former avec l'atome de carbone du cycle un carbocycle à 3, 4, 5, 6 ou 7 chaînons ou un cycle cyclopentène, cyclohexène, oxétane, tétrahydrofurane, tétrahydropyrane,
où ses groupes méthylène peuvent être substitués par 1 - 2 groupe C₁₋₃-alkyle ou CF₃, et/ou
ses groupes méthylène, dans la mesure où ils ne sont pas liés à un hétéroatome, peuvent être substitués par 1 - 2 atomes de fluor,
avec la condition qu'un hétéroatome introduit par R^{8b} ou R^{8c} ne doit pas être distant de X dans la formule (I) de seulement un atome de carbone, et
au total dans la formule (II) au maximum quatre groupements choisis parmi R^{7a}, R^{7b}, R^{7c}, R^{8a}, R^{8b} et R^{8c} peuvent être présents, et
X représente un atome d'oxygène ou de soufre, un groupe sulfène, sulfone ou un groupe NR¹, dans lequel
R¹ représente un atome d'hydrogène ou un groupe C₁₋₅-alkyle, C₂₋₅-alcényl-CH₂, C₂₋₅-alcynyl-CH₂, C₃₋₆-cycloalkyle, C₄₋₆-cycloalcényle, oxétan-3-yle, tétrahydrofuran-3-yle, benzyle, C₁₋₅-alkyl-carbonyle, trifluorométhylcarbonyle, C₃₋₆-cycloalkyl-carbonyle, C₁₋₅-alkyl-sulfonyle, C₃₋₆-cycloalkyl-sulfonyle, aminocarbonyle, C₁₋₅-alkylaminocarbonyle, di-(C₁₋₅-alkyl)-aminocarbonyle, C₁₋₅-alkyloxycarbonyle, C₄₋₇-cycloalkylène-iminocarbonyle,
où les groupes méthylène et méthyle qui se trouvent dans les groupes cités précédemment peuvent être substitués en outre par un groupe C₁-₃-alkyle, carboxy, C₁₋₅-alcoxycarbonyle ou par un groupe hydroxy, C₁-₅-alkyloxy, amino, C₁₋₅-alkylaniino, C₁₋₅-dialkylaniino ou C₄₋₇-cycloalkylèneimino, dans la mesure où les groupes méthylène ou méthyle ne sont pas liés directement à un hétéroarome du groupe O, N et S, et/ou
un à trois atomes d'hydrogène peuvent être remplacés par des atomes de fluor, dans la mesure où les groupes méthylène ou méthyle ne sont pas liés directement à un hétéroatome du groupe O, N et S,
et dans lequel
A¹, A² et A³ sont définis dans chaque cas comme décrit dans la 1^{ère} forme de réalisation, ou
D représente l'un des quatre groupes (II-1a), (II-2a), (II-3) ou (II-4) 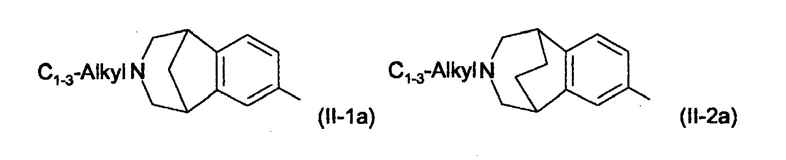 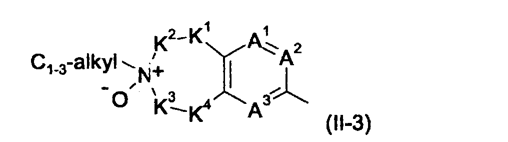 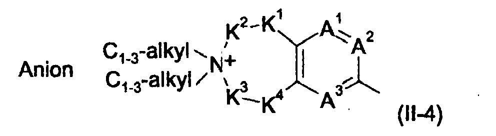 dans lequel les groupements A1, A2, A3, K1, K2, K3, K4 sont définis comme ci-dessus, et
l'anion dans (II-4) représente un fluorure, chlorure, bromure, iodure, sulfate, hydrogénosulfate, phosphate, hydrogénophosphate, benzoate, salicylate, succinate, citrate ou tartrate,
et
R³ représente un atome d'hydrogène, et
R⁴, R⁵ et M sont définis dans chaque cas comme décrit dans la 1^{ère} forme de réalisation,
leurs tautomères, leurs énantiomères, leurs diastéréoisomères, leurs mélanges et leurs sels.

3. Composés de formule générale (I) selon la revendication 1 ou 2, dans lesquels
D représente un système cyclique bicyclique substitué de formule (II) 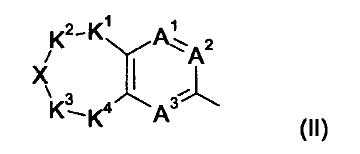 dans lequel
K1, K2, K3 et K4 sont définis comme dans la revendication 1 ou 2, et où
X représente un groupe NR¹, dans lequel
R¹ représente un atome d'hydrogène ou un groupe C₁₋₅-alkyle, C₂₋₅-alcényl-CH₂, C₂₋₅-alcynyl-CH₂, C₃₋₆-cycloalkyle, C₄₋₆-cycloalcényle,
où les groupes méthylène et méthyle qui se trouvent dans les groupes cités précédemment peuvent être substitués en outre par un groupe C₁₋₃-alkyle, carboxy, C₁₋₅-alcoxycarbonyle ou par un groupe hydroxy, C₁₋₅-alkyloxy, amino, C₁₋₅-alkylamino, C₁₋₅-dialkylamino ou C₄₋₇-cycloalkylèneimino, dans la mesure où les groupes méthylène ou méthyle ne sont pas liés directement à un hétéroarome du groupe O, N et S, et/ou
un à trois atomes d'hydrogène peuvent être remplacés par des atomes de fluor, dans la mesure où les groupes méthylène ou méthyle ne sont pas liés directement à l'atome d'azote,
et dans lequel
A¹ représente N ou CR¹⁰,
A² représente N ou CR¹¹,
A³ représente N ou CR¹²,
où R¹⁰, R¹¹ et R¹² représentent dans chaque cas indépendamment les uns des autres
un atome d'hydrogène, de fluor, de chlore, de brome ou un groupe C₁₋₅-alkyle, CF₃, un groupe cyano, carboxy, C₁₋₅-alkyloxycarbonyle, hydroxy, C₁-₃-alkyloxy, CF₃O-, CHF₂O-, CH₂FO-, ou
D représente l'un des quatre groupes (II-1a), (II-2a), (II-3) ou (II-4) 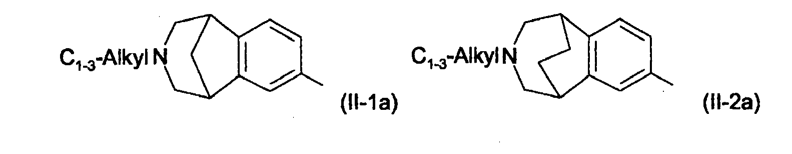 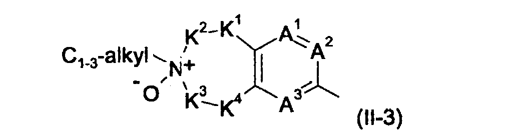 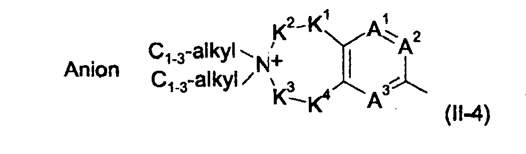 dans lequel les groupements A1, A2, A3, K1, K2, K3, K4 sont définis comme ci-dessus, et
l'anion dans (II-4) peut être choisi dans le groupe fluorure, chlorure, bromure, iodure, sulfate, phosphate, benzoate, salicylate, succinate, citrate et tartrate, et
R³, R⁴, R⁵et M sont définis dans chaque cas comme dans la revendication 1 ou 2, où R⁶ représente un atome d'hydrogène,
leurs tautomères, leurs énantiomères, leurs diastéréoisomères, leurs mélanges et leurs sels.

4. Composés de formule générale (I) selon l'une des revendications 1 à 3, dans lesquels
D, R³ et M sont définis dans chaque cas comme dans l'une des revendications 1 à 3, et
R⁴ représente un groupe C₃₋₆-alcényle ou C₃₋₆-alcynyle linéaire ou ramifié, un groupe C₁₋₆-alkyle linéaire ou ramifié,
où les atomes d'hydrogène du groupe C₁₋₆-alkyle linéaire ou ramifié peuvent éventuellement être remplacés totalement ou partiellement par des atomes de fluor, et dans lequel un à deux atomes d'hydrogène peuvent éventuellement être remplacés indépendamment l'un de l'autre par un groupe C₃₋₇-cycloalkyle, hydroxy, C₁₋₅-alkyloxy, phénylméthyloxy, phénéthyloxy, carboxy-C₁₋₅-alkyloxy, C₁₋₅-alkyloxycarbonyl-C₁₋₅-alkyloxy, C₁₋₅-alkyloxy-C₂₋₅-alkyloxy, C₁₋₅-alkylsulfanyle, C₁₋₅-alkylsulfinyle, C₁₋₅-alkylsulfonyle, carboxy, C₁₋₅-alkyloxycarbonyle, aminocarbonyle, C₁₋₅-alkylaminocarbonyle, di-(C₁₋₅alkyl)-aminocarbonyle, C₁₋₅-alkyl-amino-carbonyloxy, di-(C₁₋₅-alkyl)-aminocarbonyloxy, C₄₋₇-cycloalkylène-iminocarbonyle, amino, C₁₋₅-alkylamino ou di-(C₁₋₅-alkyl)-amino, C₁₋₅-alkylcarbonylamino, trifluoroacétylamino, C₁₋₅-alkyloxy-C₁₋₅-alkylcarbonylamino, phénylcarbonylamino, C₁₋₅-alkylaminocarbonylamino, di-(C₁₋₅-alkyl)-aminocarbonylamino, C₁₋₅-alkyloxy-carbonylamino, phénylméthyloxy-carbonylamino, C₁₋₅-alkyloxy-C₂₋₅-alkyloxy-C₁₋₂-alkylcarbonylamino, C₁₋₅-alkylsulfonylamino, C₃₋₆-cycloalkylcarbonyl-amino, 4-morpholinocarbonylamino,
où les carbo- et hétérocycles précités peuvent être substitués dans le cycle dans chaque cas par 1 à 4 groupes C₁₋₃-alkyle ou C₁₋₃-alkylcarbonyle ou dans chaque cas par 1 ou 2 groupes oxo, et/ou où les groupements phényle et hétéroaryle précités peuvent être remplacés par 1 à 2 substituants choisis parmi le fluor, le chlore, le brome, méthyle, méthoxy ou trifluorométhyle, ou bien deux atomes de carbone voisins d'un cycle phényle peuvent être substitués par un groupe -CH₂-O-CH₂-, et/ou
où les groupes alkyle précités peuvent être substitués par un groupe cyano-C₁₋₅-alkyloxycarbonyle ou carboxy,
où les amides d'acides carboxyliques ou d'acides sulfoniques précités peuvent éventuellement être substitués en outre sur l'azote par un groupe C₁₋₅-alkyle,
un groupe phényle, phényl-C₁₋₂-alkyle, hétéroaryl-C₁₋₂-alkyle ou hétéroaryle lié par C, où le groupe hétéroaryle est choisi dans le groupe consistant en imidazolyle, furanyle, thiophényle, thiazolyle, pyrazolyle, tétrazolyle, benzimidazolyle, indolyle, pyrimidinyle, pyrazinyle, oxazolyle, 1,2,4-triazolyle et pyridinyle, et qui peut éventuellement être substitué une à deux fois dans la partie phényle ou hétéroaryle par des substituants identiques ou différents choisis parmi les atomes de chlore ou de fluor ou les groupes C₁₋₃-alkyle, benzyle, hydroxy, amino, CF₃, CH₃O ou CHF₂O,
R⁵ représente un atome d'hydrogène, un groupe C₁₋₄-alkyle linéaire ou ramifié, où les atomes d'hydrogène des groupes C₁₋₄-alkyle linéaires ou ramifiés peuvent éventuellement être remplacés totalement ou partiellement par des atomes de fluor, ou un groupe propargyle ou C₁₋₃-alkyloxy-C₁₋₃-alkyle, ou
R⁴ et R⁵ orment avec l'atome de carbone auquel ils sont liés un groupe C₅₋₆₋ cycloalcényle ou C₃₋₇-cycloalkyle,
où l'un des groupes méthylène d'un groupe C₄₋₇-cycloalkyle peut être remplacé par un atome d'oxygène ou de soufre ou un groupe -NH-, -N(C₁₋₅-alkyle), -N(C₁₋₄-alkylcarbonyle), carbonyle, sulfinyle ou un groupe sulfonyle, ou deux groupes méthylène d'un groupe C₄₋₇-cycloalkyle directement voisins peuvent être remplacés ensemble par un groupe -C(O)NH-, -C(O)N(C₁-₅-alkyle)-, -S(O)₂NH- ou -S(O)₂N(C₁₋₅-alkyle), et/ou
où 1 à 2 atomes de carbone d'un groupe C₃₋₇-cycloalkyle peuvent éventuellement être substitués indépendamment l'un de l'autre par dans chaque cas un ou deux atomes de fluor ou un ou deux groupes C₁₋₅-alkyle ou un groupe hydroxy, C₁₋₅-alkyloxy, formyloxy, amino, C₁₋₅-alkylaniino, di-(C₁₋₅-alkyl)-amino, C₁₋₅-alkylcarbonylamino, C₃₋₆-cycloalkylcarbonylamino, nitrile, carboxy, C₁₋₅-alkyloxycarbonyle, aminocarbonyle, C₁₋₅-alkylaminocarbonyle, di-(C₁₋₅-alkyl)-aminocarbonyle ou C₄-₇-cycloalkylène-iminocarbonyle,
avec la condition qu'un tel groupe C₃₋₇-cycloalkyle formé à partir de R⁴ et R⁵
dans lequel deux hétéroatomes dans le cycle du groupe oxygène et azote sont séparés l'un de l'autre par exactement un groupe -CH₂-éventuellement substitué, et/ou
dans lequel un groupe méthylène ou les deux groupes méthylène du cycle, qui sont liés directement à l'atome de carbone auquel les groupements R⁴ et R⁵ sont liés, sont remplacés par un hétéroatome du groupe oxygène, azote et soufre, et/ou
dans lequel un substituant lié au groupe cyclique, qui se **caractérise en ce qu'**un hétéroatome du groupe atome d'oxygène, d'azote, de soufre et de fluor est lié directement au groupe cyclique, est séparé par un autre hétéroatome du groupe oxygène, azote et soufre par exactement un groupe méthylène éventuellement substitué, et/ou dans lequel deux atomes dans le cycle forment une liaison -O-O- ou -S-O-, est exclu, leurs tautomères, leurs énantiomères, leurs diastéréoisomères, leurs mélanges et leurs sels.

5. Composés de formule générale (I) selon l'une des revendications 1 à 4, dans lesquels
D représente un système cyclique bicyclique substitué de formule (II) 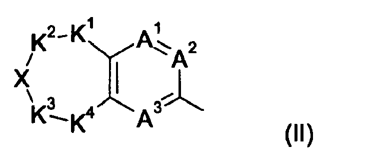 dans lequel
K¹ et K⁴ représentent chacun indépendamment l'un de l'autre un groupe -CH₂-, -CHR^{7a}- ou -CR^{7b}R^{7c}-, où
R^{7a}/R^{7b}/R^{7c}
représentent dans chaque cas indépendamment les uns des autres un groupe C₁₋₂-alkyle ou un groupe phényle, qui peut être substitué par 1 ou 2 substituants choisis parmi un groupe nitro, amino, hydroxy, méthoxy, cyano, C₁₋₅-alkyle ou un atome de fluor, de chlore ou de brome,
K² et K³ représentent chacun un groupe -CH₂-,
X représente un groupe NR¹, dans lequel R¹ représente un atome d'hydrogène ou
un groupe C₁₋₅-alkyle, C₂₋₄-alcényl-CH₂-, C₂₋₄-alcynyl-CH₂- ou C₃₋₆-cycloalkyle,
où les groupes méthylène et méthyle qui se trouvent dans les groupes C₂₋₅-alkyle cités précédemment peuvent être substitués par un à trois atomes de fluor, dans la mesure où les groupes méthylène ou méthyle ne sont pas liés directement à l'atome d'azote,
et dans lequel
A¹ représente N ou CR¹⁰,
A² représente N ou CR¹¹,
A³ représente N ou CR¹²,
où R¹⁰, R¹¹ et R¹² représentent dans chaque cas indépendamment les uns des autres
un atome d'hydrogène, de fluor ou de chlore, ou un groupe C₁₋₃-alkyle, CF₃, hydroxy ou CH₃O-,
ou
D représente l'un des groupes (II-3) ou (II-4) 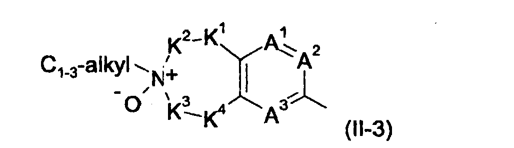 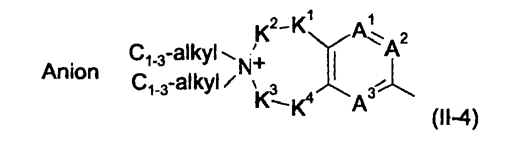 dans lequel les groupements A1, A2, A3, K1, K2, K3, K4 sont définis comme ci-dessus, et l'anion dans (II-4) peut être choisi dans le groupe fluorure, chlorure, bromure, iodure, sulfate, phosphate, benzoate, salicylate, succinate, citrate ou tartrate, et
R³ représente un atome d'hydrogène,
R⁴ représente un groupe C₃₋₆-alcényle ou C₃₋₆-alcynyle linéaire ou ramifié,
un groupe C₁₋₄-alkyle linéaire ou ramifié,
où les atomes d'hydrogène du groupe C₁₋₄-alkyle linéaire ou ramifié peuvent éventuellement être remplacés partiellement par jusqu'à quatre atomes de fluor,
et dans lequel éventuellement un à deux atomes d'hydrogène peuvent être remplacés indépendamment l'un de l'autre par un groupe C₃₋₇-cycloalkyle, hydroxy, C₁₋₅-alkyloxy, phénylméthyloxy, C₁₋₅-alkylsulfanyle, C₁₋₅-alkylsulfinyle, C₁₋₅-alkylsulfonyle, carboxy, C₁₋₅-alkyloxycarbonyle, aminocarbonyle, C₁₋₅-alkylaminocarbonyle, di-(C₁₋₅-alkyl)-aminocarbonyle, C₄₋₇-cycloalkylèneiminocarbonyle, amino, C₁₋₅-alkylamino ou di-(C₁₋₅-alkyl)-amino, C₁₋₅-alkylcarbonylamino, carboxy-C₁₋₅-alkylcarbonylamino ou C₁₋₅-alkyloxycarbonyl-C₁₋₅-alkylcarbonylamino,
où les groupements phényle précités peuvent être remplacés par 1 ou 2 substituants choisis parmi le fluor, le chlore, le brome, méthyle, méthoxy ou trifluorométhyle, ou bien où les amides d'acides carboxyliques précités peuvent éventuellement être substitués en outre sur l'azote par un groupe C₁₋₅-alkyle,
un groupe phényle, phényl-C₁₋₂-alkyle, hétéroaryl-C₁₋₂-alkyle ou hétéroaryle lié par C, où le groupe hétéroaryle est choisi dans le groupe consistant en imidazolyle, furanyle, thiophényle, thiazolyle, pyrazolyle, tétrazolyle, benzimidazolyle, indolyle, pyrimidinyle, pyrazinyle, oxazolyle et pyridinyle, et qui peut éventuellement être substitué une à deux fois dans la partie phényle ou hétéroaryle par des substituants identiques ou différents choisis parmi les atomes de chlore ou de fluor ou les groupes C₁₋₃-alkyle, CF₃, OH, CH₃O ou CHF₂O,
R⁵ représente un atome d'hydrogène, un groupe C₁₋₄-alkyle linéaire ou ramifié, un groupe propargyle ou C₁₋₃-alkyloxy-C₁₋₃-alkyle, ou
R⁴ et R⁵ forment avec l'atome de carbone auquel ils sont liés un groupe C₅₋₆-cycloalcényle ou C₃₋₇-cycloalkyle,
où l'un des groupes méthylène d'un groupe C₄₋₇-cycloalkyle peut être remplacé par un atome d'oxygène ou de soufre ou un groupe sulfonyle, ou où 1 à 2 atomes de carbone d'un groupe C₃₋₇-cycloalkyle peuvent éventuellement être substitués indépendamment l'un de l'autre par dans chaque cas un ou deux atomes de fluor ou un ou deux groupes C₁₋₅-alkyle ou un groupe hydroxy, C₁₋₅-alkyloxy, formyloxy, nitrile, carboxy, C₁₋₅-alkyloxycarbonyle, aminocarbonyle, C₁₋₅-alkylaminocarbonyle, di-(C₁₋₅-alkyl)-aminocarbonyle ou C₄₋₇-cycloalkylèneiminocarbonyle,
avec la condition qu'un tel groupe C₃₋₇-cycloalkyle formé à partir de R⁴ et R⁵ ,
dans lequel l'un des groupes méthylène du cycle, qui est lié directement à l'atome de carbone auquel les groupements R⁴ et R⁵ sont liés, est remplacé par un atome d'oxygène ou de soufre,
est exclu, et
M représente un cycle thiophène selon la formule (III) 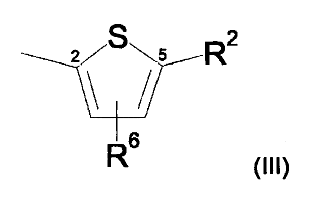 qui est lié par la position 2 au groupe carbonyle dans la formule (I) et qui est substitué à la position 5 par R², où
R² représente un atome de chlore ou de brome ou un groupe éthynyle, et
R⁶ représente un atome d'hydrogène,
où les groupes alkyle, alcényle, alcynyle et alkyloxy contenus dans les définitions citées précédemment, qui comportent plus de deux atomes de carbone, sauf indication contraire, peuvent être linéaires ou ramifiés et les groupes alkyle dans les groupements dialkylés cités précédemment, par exemple les groupes dialkylamino, peuvent être identiques ou différents,
leurs tautomères, leurs énantiomères, leurs diastéréoisomères, leurs mélanges et leurs sels.

6. Composés de formule générale (I) selon l'une des revendications 1 à 5, dans lesquels le groupement
D représente un système cyclique bicyclique substitué de formule (II) 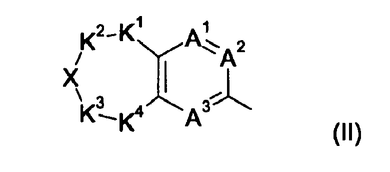 dans lequel
K¹ et K⁴ représentent chacun indépendamment l'un de l'autre un groupe -CH₂-, -CHR^{7a}- ou -CR^{7b}R^{7c}-, où
R^{7a}/R^{7b}/R^{7c}
représentent dans chaque cas indépendamment les uns des autres un groupe C₁₋₂-alkyle,
K² et K³ représentent chacun un groupe -CH₂-,
X représente un groupe NR¹, dans lequel R¹ représente un atome d'hydrogène ou
un groupe C₁₋₅-alkyle ou C₃₋₆-cycloalkyle,
où les groupes méthylène et méthyle qui se trouvent dans les groupes cités précédemment un à trois atomes d'hydrogène peuvent être remplacés par des atomes de fluor, dans la mesure où les groupes méthylène ou méthyle ne sont pas liés directement à l'atome d'azote, et dans lequel
A¹ représente CR¹⁰,
A² représente CR¹¹,
A³ représente CR¹²,
où R¹⁰, R¹¹ et R¹² représentent dans chaque cas indépendamment les uns des autres
un atome d'hydrogène, de fluor ou de chlore, ou un groupe C₁₋₃-alkyle, CF₃, HO-, CH₃O-,
ou
D représente le groupe (II-4) 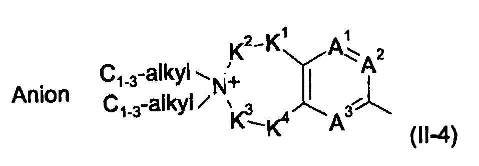 dans lequel les groupements A1, A2, A3, K1, K2, K3, K4 sont définis comme ci-dessus, et l'anion dans (II-4) peut être choisi dans le groupe fluorure, chlorure, bromure, iodure, sulfate, phosphate, benzoate, salicylate, succinate, citrate ou tartrate,
leurs tautomères, leurs énantiomères, leurs diastéréoisomères, leurs mélanges et leurs sels.

7. Composés de formule générale (I) selon l'une des revendications 1 à 6, dans lesquels
ni R⁴ ni R⁵ ne peuvent représenter un atome d'hydrogène,
leurs tautomères, leurs énantiomères, leurs diastéréoisomères, leurs mélanges et leurs sels.

8. Composés de formule générale (I) selon l'une des revendications 1 à 6, dans lesquels
R⁴ et R⁵ forment avec l'atome de carbone auquel ils sont liés un groupe C₅₋₆-cycloalcényle ou C₃₋₇-cycloalkyle, où l'un des groupes méthylène d'un groupe C₄₋₇-cycloalkyle peut être remplacé par un atome d'oxygène ou de soufre,
avec la condition qu'un tel groupe C₃₋₇-cycloalkyle formé à partir de R⁴ et R⁵,
dans lequel l'un des groupes méthylène du cycle, qui est lié directement à l'atome de carbone auquel les groupements R⁴ et R⁵ sont liés, est remplacé par un atome d'oxygène ou de soufre,
est exclu,
leurs tautomères, leurs énantiomères, leurs diastéréoisomères, leurs mélanges et leurs sels.

9. Composés de formule générale (I) selon la revendication 1 suivants :
3-[(5-bromo-thiophén-2-yl)-carbonylamino]-*N-*(3-méthyl-2,3,4,5-tétrahydro-1*H-*benzo[*d*]azépin-7-yl)-tétrahydrofurane-3-carboxamide,
3-[(5-chloro-thiophén-2-yl)-carbonylamino]-*N-*(3-méthyl-2,3,4,5-tétrahydro-1*H-*benzo[*d*]azépin-7-yl)-tétrahydrofurane-3-carboxamide,
*N*-[1-(3-éthyl-2,3,4,5-tétrahydro-1*H-*benzo[*d*]azépin-7-ylcarbamoyl)-1-méthyl-éthyl]-amide d'acide 5-chloro-thiophène-2-carboxylique,
5-éthynyl-N-[1-méthyl-1-(3-méthyl-2,3,4,5-tétrahydro-1*H-*benzo[*d*]azépin-7-ylcarbamoyl)-éthyl]-thiophène-2-carboxamide,
*N*-[1-méthyl-1-(3-méthyl-2,3,4,5-tétrahydro-1*H-*benzo[*d*]azépin-7-ylcarbamoyl]-éthyl}-amide d'acide 5-chloro-thiophène-2-carboxylique,
*N-*[1-méthyl-1-(3-méthyl-2,3,4,5-tétrahydro-1*H-*benzo[*d*]azépin-7-ylcarbamoyl]-éthyl}-amide d'acide 5-bromo-thiophène-2-carboxylique,
*N-*[2-méthoxy-1-(3-méthyl-2,3,4,5-tétrahydro-1*H-*benzo[*d*]azépin-7-ylcarbamoyl)-éthyl]-amide d'acide 5-chloro-thiophène-2-carboxylique,
1-[(5-bromo-thiophén-2-yl)-carbonylamino]-*N-*(3-méthyl-2,3,4,5-tétrahydro-1*H-*benzo[*d*]azépin-7-yl)-cyclopentane-1-carboxamide,
1-[(5-chloro-thiophén-2-yl)-carbonylamino]-*N-*(3-méthyl-2,3,4,5-tétrahydro-1*H-*benzo[*d*]azépin-7-yl)-cyclopentane-1-carboxamide,
3-[(5-chloro-thiophén-2-yl)-carbonylamino]-*N-*(3-méthyl-2,3,4,5-tétrahydro-1*H-*benzo[*d*]azépin-7-yl)-tétrahydrothiophène-3-carboxamide,
1-[(5-chloro-thiophén-2-yl)-carbonylamino]-*N-*(3-méthyl-2,3,4,5-tétrahydro-1*H-*benzo[*d*]azépin-7-yl)-cyclobutane-1-carboxamide,
1-[(5-bromo-thiophén-2-yl)-carbonylamino]-*N-*(3-méthyl-2,3,4,5-tétrahydrro-1*H-*benzo[*d*]azépin-7-yl)-cyclopent-3-ène-1-carboxamide,
1-[(5-chloro-thiophén-2-yl)-carbonylamino]-*N-*(3-méthyl-2,3,4,5-tétrahydro-1*H-*benzo[*d*]azépin-7-yl)-cyclohexane-1-carboxamide,
(R)-*N-*[2-benzyloxy-1-(3-méthyl-2,3,4,5-tétrahydro-1*H-*benzo[*d*]azépin-7-yl-carbamoyl)-éthyl]-amide d'acide 5-chloro-thiophène-2-carboxylique,
*N-*[2-benzyloxy-1-méthyl-1-(3-méthyl-2,3,4,5-tétrahydro-1*H-*benzo[*d*]azépin-7-ylcarbamoyl)-éthyl]-amide d'acide 5-chloro-thiophène-2-carboxylique,
(R)-*N-*[2-hydroxy-1-méthyl-1-(3-méthyl-2,3,4,5-tétrahydro-1*H-*benzo[*d*]azépin-7-ylcarbamoyl)-éthyl]-amide d'acide 5-chloro-thiophène-2-carboxylique,
*N-*[3-hydroxy-1-méthyl-1-(3-méthyl-2,3,4,5-tétrahydro-1*H-*benzo[*d*]azépin-7-ylcarbamoyl)-propyl]-amide d'acide 5-bromo-thiophène-2-carboxylique,
*N-*[1-méthyl-3-diméthylaminocarbonyl-1-(3-méthyl-2,3,4,5-tétrahydro-1*H-*benzo[*d*]azépin-7-ylcarbamoyl)-propyl]-amide d'acide 5-chloro-thiophène-2-carboxylique,
*N-*[2-(4-hydroxy-phényl)-1-méthyl-1-(3-méthyl-2,3,4,5-tétrahydro-1*H-*benzo-[*d*]azépin-7-ylcarbamoyl)-éthyl]-amide d'acide 5-chloro-thiophène-2-carboxylique,
*N-*[1-méthyl-1-(3,5-diméthyl-2,3,4,5-tétrahydro-1*H-*benzo[*d*]azépin-7-yl-carbamoyl)-éthyl]-amide d'acide 5-chloro-thiophène-2-carboxylique,
*N-*[1-méthyl-1-(3-méthyl-5-(4-aminophényl)-2,3,4,5-tétrahydro-1*H-*benzo[*d*]-azépin-7-ylcarbamoyl)-éthyl]-amide d'acide 5-chloro-thiophène-2-carboxylique,
*N-*[2-éthoxy-1-(3-méthyl-2,3,4,5-tétrahydro-1*H-*benzo[*d*]azépin-7-ylcarbamoyl)-éthyl]-amide d'acide 5-chloro-thiophène-2-carboxylique,
*N-*[3-méthoxy-1-(3-méthyl-2,3,4,5-tétrahydro-1*H-*benzo[*d*]azépin-7-ylcarbamoyl)-propyl]-amide d'acide 5-chloro-thiophène-2-carboxylique,
*N-*[2-isopropyloxy-1-(3-méthyl-2,3,4,5-tétrahydro-1*H-*benzo[*d*]azépin-7-yl-carbamoyl)-éthyl]-amide d'acide 5-chloro-thiophène-2-carboxylique,
(R)-*N-*[3-benzyloxy-1-(3-méthyl-2,3,4,5-tétrahydro-1*H-*benzo[*d*]azépin-7-yl-carbamoyl)-propyl]-amide d'acide 5-chloro-thiophène-2-carboxylique,
1-[(5-chloro-thiophén-2-yl)-carbonylamino]-3,4-diméthoxy-*N-*(3-méthyl-2,3,4,5-tétrahydro-1*H-*benzo[*d*]azépin-7-yl)-cyclopentane-1-carboxamide,
*N-*[C-(1-méthyl-pyrazol-3-yl)-C-(3-méthyl-2,3,4,5-tétrahydro-1*H-*benzo[*d*]azépin-7-ylcarbamoyl)-méthyl]-amide d'acide 5-chloro-thiophène-2-carboxylique,
(R)-*N-*[2-phényl-1-(3-méthyl-2,3,4,5-tétrahydro-1*H-*benzo[*d*]azépin-7-yl-carbamoyl)-éthyl]-amide d'acide 5-chloro-thiophène-2-carboxylique,
(R)-*N-*[2-(furan-2-yl)-1-(3-méthyl-2,3,4,5-tétrahydro-1*H-*benzo[*d*]azépin-7-ylcarbamoyl)-éthyl]-amide d'acide 5-chloro-thiophène-2-carboxylique,
*N-*[2-(4-méthoxyphényl)-1-(3-méthyl-2,3,4,5-tétrahydro-1*H-*benzo[*d*]azépin-7-ylcarbamoyl)-éthyl]-amide d'acide 5-chloro-thiophène-2-carboxylique,
*N-*[2-(4-hydroxy-3-nitro-phényl)-1-(3-méthyl-2,3,4,5-tétrahydro-1*H-*benzo[*d*]-azépin-7-ylcarbamoyl)-éthyl]-amide d'acide 5-chloro-thiophène-2-carboxylique,
(R)-*N-*[2-(4-hydroxy-phényl)-1-(3-méthyl-2,3,4,5-tétrahydro-1*H-*benzo[*d*]azépin-7-ylcarbamoyl)-éthyl]-amide d'acide 5-chloro-thiophène-2-carboxylique, (R)-*N-*[2-cyclohexyl-1-(3-méthyl-2,3,4,5-tétrahydro-1*H-*benzo[*d*]azépin-7-yl-carbamoyl)-éthyl]-amide d'acide 5-chloro-thiophène-2-carboxylique,
(R)-*N-*[3-aminocarbonyl-l-(3-méthyl-2,3,4,5-tétrahydro-1*H-*benzo[*d*]azépin-7-ylcarbamoyl)-propyl]-amide d'acide 5-chloro-thiophène-2-carboxylique,
(R)-*N-*[2-acétylamino-1-(3-méthyl-2,3,4,5-tétrahydro-1*H-*benzo[*d*]azépin-7-yl-carbamoyl)-éthyl]-amide d'acide 5-chloro-thiophène-2-carboxylique,
(R)-*N-*[2-benzoylamino-1-(3-méthyl-2,3,4,5-tétrahydro-1*H-*benzo[*d*]azépin-7-yl-carbamoyl)-éthyl]-amide d'acide 5-bromo-thiophène-2-carboxylique,
(R)-*N-*[2-(2-hydroxycarbonyléthyl)carbonylamino-1-(3-méthyl-2,3,4,5-tétrahydro-1*H-*benzo[*d*]azépin-7-ylcarbamoyl)-éthyl]-amide d'acide 5-chloro-thiophène-2-carboxylique,
(R)-*N-*[2-(2-hydroxycarbonyléthyl)carbonylamino-1-(3-méthyl-2,3,4,5-tétrahydro-1*H-*benzo[*d*]azépin-7-ylcarbamoyl)-éthyl]-amide d'acide 5-chloro-thiophène-2-carboxylique,
(R)-*N-*[2-(4-méthoxycarbonylbutyl)carbonylamino-1-(3-méthyl-2,3,4,5-tétrahydro-1*H-*benzo[*d*]azépin-7-ylcarbamoyl)-éthyl]-amide d'acide 5-chloro-thiophène-2-carboxylique,
iodure de *N-*[1-méthyl-1-(3,3-diméthyl-2,3,4,5-tétrahydro-1*H-*benzo[*d*]azépinium-7-ylcarbamoyl)-éthyl]-amide d'acide 5-chloro-thiophène-2-carboxylique,
3-[(5-chloro-thiophén-2-yl)-carbonylamino]-*N-*(3,5-diméthyl-2,3,4,5-tétrahydro-1*H-*benzo[*d*]azépin-7-yl)-tétrahydrofurane-1-carboxamide,
ainsi que leurs tautomères, leurs énantiomères, leurs diastéréoisomères, leurs mélanges et leurs sels.

10. Sels physiologiquement acceptables des composés selon l'une des revendications 1 à 9.

11. Médicament contenant un composé selon au moins l'une des revendications 1 à 9 ou un sel physiologiquement acceptable selon la revendication 10 outre éventuellement un ou plusieurs vecteurs et/ou diluants inertes.

12. Utilisation d'un composé selon au moins l'une des revendications 1 à 9 ou d'un sel physiologiquement acceptable selon la revendication 10 pour la production d'un médicament ayant un effet inhibiteur sur le facteur Xa et/ou un effet inhibiteur sur des sérine protéases apparentées.

13. Procédé pour la production d'un médicament selon la revendication 11 **caractérisé en ce que**, par voie non chimique, un composé selon au moins l'une des revendications 1 à 9 ou un sel physiologiquement acceptable selon la revendication 10 est incorporé dans un ou plusieurs vecteurs et/ou diluants inertes.
